# EUROPEAN PATENT SPECIFICATION

(11) **EP 3 036 331 B1**
(45) Date of publication and mention of the grant of the patent: **16.06.2021**
(21) Application number: 14753252.7
(22) Date of filing: 20.08.2014
(51) Int. Cl.: C12N 15/82, C12N 9/88, A01H 5/00

(54) **ALS INHIBITOR HERBICIDE TOLERANT MUTANT PLANTS**
HERBIZIDTOLERANTE MUTANTE PFLANZEN ALS ALS-HEMMER
PLANTES MUTANTES TOLÉRANTES AUX HERBICIDES INHIBITEURS D'ALS

(30) Priority: 21.08.2013 EP 13181128; 10.12.2013 EP 13196378
(43) Date of publication of application: 29.06.2016
(73) Proprietor: BASF Agricultural Solutions Seed US LLC, Florham Park, NJ 07932 (US)
(72) Inventor: RUITER, Rene, 9070 Heusden (Destelbergen) (BE); HAIN, Rüdiger, 60594 Frankfurt (DE); JOHANN, Gerhard, 51399 Burscheid (DE); LABER, Bernd, 65510 Idstein (DE)
(74) Representative: BASF IP Association
(86) International application number: PCT/EP2014/067716
(87) International publication number: WO 2015/024957

(56) References cited:
- WO-A1-2010/147636
- WO-A1-2012/150333
- WO-A2-2008/124495
- WO-A2-2011/114232

### SEQUENCE LISTING

<110> Bayer CropScience NV Bayer CropScience AG Ruiter, Rene Hain, Ruediger Johann, Gerhard Laber, Bernd
<120> ALS INHIBITOR HERBICIDE TOLERANT MUTANT PLANTS
<130> BCS13-2013
<160> 38
<170> PatentIn version 3.5
<210> 1
   <211> 1965
   <212> DNA
   <213> Brassica napus
<220>
   <221> CDS
   <222> (1)..(1965)
<400> 1
   atg gcg gcg gca aca tcg tct tct ccg atc tcc tta acc gct aaa cct      48
   Met Ala Ala Ala Thr Ser Ser Ser Pro Ile Ser Leu Thr Ala Lys Pro
   1               5                   10                  15
   tct tcc aaa tcc cct cta ccc att tcc aga ttc tcc ctt ccc ttc tcc      96
   Ser Ser Lys Ser Pro Leu Pro Ile Ser Arg Phe Ser Leu Pro Phe Ser
               20                  25                  30
   tta acc cca cag aaa gac tcc tcc cgt ctc cac cgt cct ctc gcc atc     144
   Leu Thr Pro Gln Lys Asp Ser Ser Arg Leu His Arg Pro Leu Ala Ile
           35                  40                  45
   tcc gcc gtt ctc aac tca ccc gtc aat gtc gca cct cct tcc cct gaa     192
   Ser Ala Val Leu Asn Ser Pro Val Asn Val Ala Pro Pro Ser Pro Glu
       50                  55                  60
   aaa acc gac aag aac aag act ttc gtc tcc cgc tac gct ccc gac gag     240
   Lys Thr Asp Lys Asn Lys Thr Phe Val Ser Arg Tyr Ala Pro Asp Glu
   65                  70                  75                  80
   ccc cgc aag ggt gct gat atc ctc gtc gaa gcc ctc gag cgt caa ggc     288
   Pro Arg Lys Gly Ala Asp Ile Leu Val Glu Ala Leu Glu Arg Gln Gly
                   85                  90                  95
   gtc gaa acc gtc ttt gct tat ccc gga ggt gct tcc atg gag atc cac     336
   Val Glu Thr Val Phe Ala Tyr Pro Gly Gly Ala Ser Met Glu Ile His
               100                 105                 110
   caa gcc ttg act cgc tcc tcc acc atc cgt aac gtc ctt ccc cgt cac     384
   Gln Ala Leu Thr Arg Ser Ser Thr Ile Arg Asn Val Leu Pro Arg His
           115                 120                 125
   gaa caa gga gga gtc ttc gcc gcc gag ggt tac gct cgt tcc tcc ggc     432
   Glu Gln Gly Gly Val Phe Ala Ala Glu Gly Tyr Ala Arg Ser Ser Gly
       130                 135                 140
   aaa ccg gga atc tgc ata gcc act tcg ggt ccc gga gct acc aac ctc        480
   Lys Pro Gly Ile Cys Ile Ala Thr Ser Gly Pro Gly Ala Thr Asn Leu
   145                 150                 155                 160
   gtc agc ggg tta gca gac gcg atg ctt gac agt gtt cct ctt gtc gcc        528
   Val Ser Gly Leu Ala Asp Ala Met Leu Asp Ser Val Pro Leu Val Ala
                   165                 170                 175
   att aca gga cag gtc cct cgc cgg atg atc ggt act gac gcc ttc caa        576
   Ile Thr Gly Gln Val Pro Arg Arg Met Ile Gly Thr Asp Ala Phe Gln
               180                 185                 190
   gag aca cca atc gtt gag gta acg agg tct att acg aaa cat aac tat        624
   Glu Thr Pro Ile Val Glu Val Thr Arg Ser Ile Thr Lys His Asn Tyr
           195                 200                 205
   ttg gtg atg gat gtt gat gac ata cct agg atc gtt caa gaa gct ttc        672
   Leu Val Met Asp Val Asp Asp Ile Pro Arg Ile Val Gln Glu Ala Phe
       210                 215                 220
   ttt cta gct act tcc ggt aga ccc gga ccg gtt ttg gtt gat gtt cct        720
   Phe Leu Ala Thr Ser Gly Arg Pro Gly Pro Val Leu Val Asp Val Pro
   225                 230                 235                 240
   aag gat att cag cag cag ctt gcg att cct aac tgg gat caa cct atg        768
   Lys Asp Ile Gln Gln Gln Leu Ala Ile Pro Asn Trp Asp Gln Pro Met
                   245                 250                 255
   cgc tta cct ggc tac atg tct agg ttg cct cag cct ccg gaa gtt tct        816
   Arg Leu Pro Gly Tyr Met Ser Arg Leu Pro Gln Pro Pro Glu Val Ser
               260                 265                 270
   cag tta ggt cag atc gtt agg ttg atc tcg gag tct aag agg cct gtt        864
   Gln Leu Gly Gln Ile Val Arg Leu Ile Ser Glu Ser Lys Arg Pro Val
           275                 280                 285
   ttg tac gtt ggt ggt gga agc ttg aac tcg agt gaa gaa ctg ggg aga        912
   Leu Tyr Val Gly Gly Gly Ser Leu Asn Ser Ser Glu Glu Leu Gly Arg
       290                 295                 300
   ttt gtc gag ctt act ggg atc ccc gtt gcg agt act ttg atg ggg ctt        960
   Phe Val Glu Leu Thr Gly Ile Pro Val Ala Ser Thr Leu Met Gly Leu
   305                 310                 315                 320
   ggc tct tat cct tgt aac gat gag ttg tcc ctg cag atg ctt ggc atg       1008
   Gly Ser Tyr Pro Cys Asn Asp Glu Leu Ser Leu Gln Met Leu Gly Met
                   325                 330                 335
   cac ggg act gtg tat gct aac tac gct gtg gag cat agt gat ttg ttg       1056
   His Gly Thr Val Tyr Ala Asn Tyr Ala Val Glu His Ser Asp Leu Leu
               340                 345                 350
   ctg gcg ttt ggt gtt agg ttt gat gac cgt gtc acg gga aag ctc gag       1104
   Leu Ala Phe Gly Val Arg Phe Asp Asp Arg Val Thr Gly Lys Leu Glu
           355                 360                 365
   gct ttc gct agc agg gct aaa att gtg cac ata gac att gat tct gct       1152
   Ala Phe Ala Ser Arg Ala Lys Ile Val His Ile Asp Ile Asp Ser Ala
       370                 375                 380
   gag att ggg aag aat aag aca cct cac gtg tct gtg tgt ggt gat gta       1200
   Glu Ile Gly Lys Asn Lys Thr Pro His Val Ser Val Cys Gly Asp Val
   385                 390                 395                 400
   aag ctg gct ttg caa ggg atg aac aag gtt ctt gag aac cgg gcg gag      1248
   Lys Leu Ala Leu Gln Gly Met Asn Lys Val Leu Glu Asn Arg Ala Glu
                   405                 410                 415
   gag ctc aag ctt gat ttc ggt gtt tgg agg agt gag ttg agc gag cag      1296
   Glu Leu Lys Leu Asp Phe Gly Val Trp Arg Ser Glu Leu Ser Glu Gln
               420                 425                 430
   aaa cag aag ttc cct ttg agc ttc aaa acg ttt gga gaa gcc att cct      1344
   Lys Gln Lys Phe Pro Leu Ser Phe Lys Thr Phe Gly Glu Ala Ile Pro
           435                 440                 445
   ccg cag tac gcg att cag atc ctc gac gag cta acc gaa ggg aag gca      1392
   Pro Gln Tyr Ala Ile Gln Ile Leu Asp Glu Leu Thr Glu Gly Lys Ala
       450                 455                 460
   att atc agt act ggt gtt gga cag cat cag atg tgg gcg gcg cag ttt      1440
   Ile Ile Ser Thr Gly Val Gly Gln His Gln Met Trp Ala Ala Gln Phe
   465                 470                 475                 480
   tac aag tac agg aag ccg aga cag tgg ctg tcg tca tca ggc ctc gga      1488
   Tyr Lys Tyr Arg Lys Pro Arg Gln Trp Leu Ser Ser Ser Gly Leu Gly
                   485                 490                 495
   gct atg ggt ttt gga ctt cct gct gcg att gga gcg tct gtg gcg aac      1536
   Ala Met Gly Phe Gly Leu Pro Ala Ala Ile Gly Ala Ser Val Ala Asn
               500                 505                 510
   cct gat gcg att gtt gtg gat att gac ggt gat gga agc ttc ata atg      1584
   Pro Asp Ala Ile Val Val Asp Ile Asp Gly Asp Gly Ser Phe Ile Met
           515                 520                 525
   aac gtt caa gag ctg gcc aca atc cgt gta gag aat ctt cct gtg aag      1632
   Asn Val Gln Glu Leu Ala Thr Ile Arg Val Glu Asn Leu Pro Val Lys
       530                 535                 540
   ata ctc ttg tta aac aac cag cat ctt ggg atg gtc atg caa tgg gaa      1680
   Ile Leu Leu Leu Asn Asn Gln His Leu Gly Met Val Met Gln Trp Glu
   545                 550                 555                 560
   gat cgg ttc tac aaa gct aac aga gct cac act tat ctc ggg gac ccg      1728
   Asp Arg Phe Tyr Lys Ala Asn Arg Ala His Thr Tyr Leu Gly Asp Pro
                   565                 570                 575
   gca agg gag aac gag atc ttc cct aac atg ctg cag ttt gca gga gct      1776
   Ala Arg Glu Asn Glu Ile Phe Pro Asn Met Leu Gln Phe Ala Gly Ala
               580                 585                 590
   tgc ggg att cca gct gcg aga gtg acg aag aaa gaa gaa ctc cga gaa      1824
   Cys Gly Ile Pro Ala Ala Arg Val Thr Lys Lys Glu Glu Leu Arg Glu
           595                 600                 605
   gct att cag aca atg ctg gat aca cca gga cca tac ctg ttg gat gtg      1872
   Ala Ile Gln Thr Met Leu Asp Thr Pro Gly Pro Tyr Leu Leu Asp Val
       610                 615                 620
   ata tgt ccg cac caa gaa cat gtg tta ccg atg atc cca agt ggt ggc      1920
   Ile Cys Pro His Gln Glu His Val Leu Pro Met Ile Pro Ser Gly Gly
   625                 630                 635                 640
   act ttc aaa gat gta ata aca gaa ggg gat ggt cgc act aag tac          1965
   Thr Phe Lys Asp Val Ile Thr Glu Gly Asp Gly Arg Thr Lys Tyr
   645 650 655
<210> 2
   <211> 655
   <212> PRT
   <213> Brassica napus
<400> 2
   Met Ala Ala Ala Thr Ser Ser Ser Pro Ile Ser Leu Thr Ala Lys Pro
   1 5 10 15
   Ser Ser Lys Ser Pro Leu Pro Ile Ser Arg Phe Ser Leu Pro Phe Ser
   20 25 30
   Leu Thr Pro Gln Lys Asp Ser Ser Arg Leu His Arg Pro Leu Ala Ile
   35 40 45
   Ser Ala Val Leu Asn Ser Pro Val Asn Val Ala Pro Pro Ser Pro Glu
   50 55 60
   Lys Thr Asp Lys Asn Lys Thr Phe Val Ser Arg Tyr Ala Pro Asp Glu
   65 70 75 80
   Pro Arg Lys Gly Ala Asp Ile Leu Val Glu Ala Leu Glu Arg Gln Gly
   85 90 95
   Val Glu Thr Val Phe Ala Tyr Pro Gly Gly Ala Ser Met Glu Ile His
   100 105 110
   Gln Ala Leu Thr Arg Ser Ser Thr Ile Arg Asn Val Leu Pro Arg His
   115 120 125
   Glu Gln Gly Gly Val Phe Ala Ala Glu Gly Tyr Ala Arg Ser Ser Gly
   130 135 140
   Lys Pro Gly Ile Cys Ile Ala Thr Ser Gly Pro Gly Ala Thr Asn Leu
   145 150 155 160
   Val Ser Gly Leu Ala Asp Ala Met Leu Asp Ser Val Pro Leu Val Ala
   165 170 175
   Ile Thr Gly Gln Val Pro Arg Arg Met Ile Gly Thr Asp Ala Phe Gln
   180 185 190
   Glu Thr Pro Ile Val Glu Val Thr Arg Ser Ile Thr Lys His Asn Tyr
   195 200 205
   Leu Val Met Asp Val Asp Asp Ile Pro Arg Ile Val Gln Glu Ala Phe
       210                 215                 220
   Phe Leu Ala Thr Ser Gly Arg Pro Gly Pro Val Leu Val Asp Val Pro
   225                 230                 235                 240
   Lys Asp Ile Gln Gln Gln Leu Ala Ile Pro Asn Trp Asp Gln Pro Met
                   245                 250                 255
   Arg Leu Pro Gly Tyr Met Ser Arg Leu Pro Gln Pro Pro Glu Val Ser
               260                 265                 270
   Gln Leu Gly Gln Ile Val Arg Leu Ile Ser Glu Ser Lys Arg Pro Val
           275                 280                 285
   Leu Tyr Val Gly Gly Gly Ser Leu Asn Ser Ser Glu Glu Leu Gly Arg
       290                 295                 300
   Phe Val Glu Leu Thr Gly Ile Pro Val Ala Ser Thr Leu Met Gly Leu
   305                 310                 315                 320
   Gly Ser Tyr Pro Cys Asn Asp Glu Leu Ser Leu Gln Met Leu Gly Met
                   325                 330                 335
   His Gly Thr Val Tyr Ala Asn Tyr Ala Val Glu His Ser Asp Leu Leu
               340                 345                 350
   Leu Ala Phe Gly Val Arg Phe Asp Asp Arg Val Thr Gly Lys Leu Glu
           355                 360                 365
   Ala Phe Ala Ser Arg Ala Lys Ile Val His Ile Asp Ile Asp Ser Ala
       370                 375                 380
   Glu Ile Gly Lys Asn Lys Thr Pro His Val Ser Val Cys Gly Asp Val
   385                 390                 395                 400
   Lys Leu Ala Leu Gln Gly Met Asn Lys Val Leu Glu Asn Arg Ala Glu
                   405                 410                 415
   Glu Leu Lys Leu Asp Phe Gly Val Trp Arg Ser Glu Leu Ser Glu Gln
               420                 425                 430
   Lys Gln Lys Phe Pro Leu Ser Phe Lys Thr Phe Gly Glu Ala Ile Pro
           435                 440                 445
   Pro Gln Tyr Ala Ile Gln Ile Leu Asp Glu Leu Thr Glu Gly Lys Ala
       450                 455                 460
   Ile Ile Ser Thr Gly Val Gly Gln His Gln Met Trp Ala Ala Gln Phe
   465                 470                 475                 480
   Tyr Lys Tyr Arg Lys Pro Arg Gln Trp Leu Ser Ser Ser Gly Leu Gly
                   485                 490                 495
   Ala Met Gly Phe Gly Leu Pro Ala Ala Ile Gly Ala Ser Val Ala Asn
               500                 505                 510
   Pro Asp Ala Ile Val Val Asp Ile Asp Gly Asp Gly Ser Phe Ile Met
           515                 520                 525
   Asn Val Gln Glu Leu Ala Thr Ile Arg Val Glu Asn Leu Pro Val Lys
       530                 535                 540
   Ile Leu Leu Leu Asn Asn Gln His Leu Gly Met Val Met Gln Trp Glu
   545                 550                 555                 560
   Asp Arg Phe Tyr Lys Ala Asn Arg Ala His Thr Tyr Leu Gly Asp Pro
                   565                 570                 575
   Ala Arg Glu Asn Glu Ile Phe Pro Asn Met Leu Gln Phe Ala Gly Ala
               580                 585                 590
   Cys Gly Ile Pro Ala Ala Arg Val Thr Lys Lys Glu Glu Leu Arg Glu
           595                 600                 605
   Ala Ile Gln Thr Met Leu Asp Thr Pro Gly Pro Tyr Leu Leu Asp Val
       610                 615                 620
   Ile Cys Pro His Gln Glu His Val Leu Pro Met Ile Pro Ser Gly Gly
   625                 630                 635                 640
   Thr Phe Lys Asp Val Ile Thr Glu Gly Asp Gly Arg Thr Lys Tyr
                   645                 650                 655
<210> 3
   <211> 1956
   <212> DNA
   <213> Brassica napus
<220>
   <221> CDS
   <222> (1)..(1956)
<400> 3
   atg gcg gcg gca aca tcg tct tct ccg atc tcc tta acc gct aaa cct        48
   Met Ala Ala Ala Thr Ser Ser Ser Pro Ile Ser Leu Thr Ala Lys Pro
   1               5                   10                  15
   tct tcc aaa tcc cct cta ccc att tcc aga ttc tcc ctt ccc ttc tcc      96
   Ser Ser Lys Ser Pro Leu Pro Ile Ser Arg Phe Ser Leu Pro Phe Ser
               20                  25                  30
   tta acc cca cag aaa ccc tcc tcc cgt ctc cac cgt cct ctc gcc atc     144
   Leu Thr Pro Gln Lys Pro Ser Ser Arg Leu His Arg Pro Leu Ala Ile
           35                  40                  45
   tcc gcc gtt ctc aac tca ccc gtc aat gtc gca cct gaa aaa acc gac     192
   Ser Ala Val Leu Asn Ser Pro Val Asn Val Ala Pro Glu Lys Thr Asp
       50                  55                  60
   aag atc aag act ttc atc tcc cgc tac gct ccc gac gag ccc cgc aag     240
   Lys Ile Lys Thr Phe Ile Ser Arg Tyr Ala Pro Asp Glu Pro Arg Lys
   65                  70                  75                  80
   ggt gct gat atc ctc gtg gaa gcc ctc gag cgt caa ggc gtc gaa acc     288
   Gly Ala Asp Ile Leu Val Glu Ala Leu Glu Arg Gln Gly Val Glu Thr
                   85                  90                  95
   gtc ttc gct tat ccc gga ggt gcc tcc atg gag atc cac caa gcc ttg     336
   Val Phe Ala Tyr Pro Gly Gly Ala Ser Met Glu Ile His Gln Ala Leu
               100                 105                 110
   act cgc tcc tcc acc atc cgt aac gtc ctc ccc cgt cac gaa caa gga     384
   Thr Arg Ser Ser Thr Ile Arg Asn Val Leu Pro Arg His Glu Gln Gly
           115                 120                 125
   gga gtc ttc gcc gcc gag ggt tac gct cgt tcc tcc ggc aaa ccg gga     432
   Gly Val Phe Ala Ala Glu Gly Tyr Ala Arg Ser Ser Gly Lys Pro Gly
       130                 135                 140
   atc tgc ata gcc act tcg ggt ccc gga gct acc aac ctc gtc agc ggg     480
   Ile Cys Ile Ala Thr Ser Gly Pro Gly Ala Thr Asn Leu Val Ser Gly
   145                 150                 155                 160
   tta gcc gac gcg atg ctt gac agt gtt cct ctc gtc gcc atc aca gga     528
   Leu Ala Asp Ala Met Leu Asp Ser Val Pro Leu Val Ala Ile Thr Gly
                   165                 170                 175
   cag gtc cct cgc cgg atg atc ggt act gac gcc ttc caa gag acg cca     576
   Gln Val Pro Arg Arg Met Ile Gly Thr Asp Ala Phe Gln Glu Thr Pro
               180                 185                 190
   atc gtt gag gta acg agg tct att acg aaa cat aac tat ctg gtg atg     624
   Ile Val Glu Val Thr Arg Ser Ile Thr Lys His Asn Tyr Leu Val Met
           195                 200                 205
   gat gtt gat gac ata cct agg atc gtt caa gaa gca ttc ttt cta gct     672
   Asp Val Asp Asp Ile Pro Arg Ile Val Gln Glu Ala Phe Phe Leu Ala
       210                 215                 220
   act tcc ggt aga ccc gga ccg gtt ttg gtt gat gtt cct aag gat att     720
   Thr Ser Gly Arg Pro Gly Pro Val Leu Val Asp Val Pro Lys Asp Ile
   225                 230                 235                 240
   cag cag cag ctt gcg att cct aac tgg gat caa cct atg cgc ttg cct     768
   Gln Gln Gln Leu Ala Ile Pro Asn Trp Asp Gln Pro Met Arg Leu Pro
                   245                 250                 255
   ggc tac atg tct agg ctg cct cag cca ccg gaa gtt tct cag tta ggc     816
   Gly Tyr Met Ser Arg Leu Pro Gln Pro Pro Glu Val Ser Gln Leu Gly
               260                 265                 270
   cag atc gtt agg ttg atc tcg gag tct aag agg cct gtt ttg tac gtt         864
   Gln Ile Val Arg Leu Ile Ser Glu Ser Lys Arg Pro Val Leu Tyr Val
           275                 280                 285
   ggt ggt gga agc ttg aac tcg agt gaa gaa ctg ggg aga ttt gtc gag         912
   Gly Gly Gly Ser Leu Asn Ser Ser Glu Glu Leu Gly Arg Phe Val Glu
       290                 295                 300
   ctt act ggg atc cct gtt gcg agt acg ttg atg ggg ctt ggc tct tat         960
   Leu Thr Gly Ile Pro Val Ala Ser Thr Leu Met Gly Leu Gly Ser Tyr
   305                 310                 315                 320
   cct tgt aac gat gag ttg tcc ctg cag atg ctt ggc atg cac ggg act        1008
   Pro Cys Asn Asp Glu Leu Ser Leu Gln Met Leu Gly Met His Gly Thr
                   325                 330                 335
   gtg tat gct aac tac gct gtg gag cat agt gat ttg ttg ctg gcg ttt        1056
   Val Tyr Ala Asn Tyr Ala Val Glu His Ser Asp Leu Leu Leu Ala Phe
               340                 345                 350
   ggt gtt agg ttt gat gac cgt gtc acg gga aag ctc gag gcg ttt gcg        1104
   Gly Val Arg Phe Asp Asp Arg Val Thr Gly Lys Leu Glu Ala Phe Ala
           355                 360                 365
   agc agg gct aag att gtg cac ata gac att gat tct gct gag att ggg        1152
   Ser Arg Ala Lys Ile Val His Ile Asp Ile Asp Ser Ala Glu Ile Gly
       370                 375                 380
   aag aat aag aca cct cac gtg tct gtg tgt ggt gat gta aag ctg gct        1200
   Lys Asn Lys Thr Pro His Val Ser Val Cys Gly Asp Val Lys Leu Ala
   385                 390                 395                 400
   ttg caa ggg atg aac aag gtt ctt gag aac cgg gcg gag gag ctc aag        1248
   Leu Gln Gly Met Asn Lys Val Leu Glu Asn Arg Ala Glu Glu Leu Lys
                   405                 410                 415
   ctt gat ttc ggt gtt tgg agg agt gag ttg agc gag cag aaa cag aag        1296
   Leu Asp Phe Gly Val Trp Arg Ser Glu Leu Ser Glu Gln Lys Gln Lys
               420                 425                 430
   ttc ccg ttg agc ttc aaa acg ttt gga gaa gcc att cct ccg cag tac        1344
   Phe Pro Leu Ser Phe Lys Thr Phe Gly Glu Ala Ile Pro Pro Gln Tyr
           435                 440                 445
   gcg att cag gtc cta gac gag cta acc caa ggg aag gca att atc agt        1392
   Ala Ile Gln Val Leu Asp Glu Leu Thr Gln Gly Lys Ala Ile Ile Ser
       450                 455                 460
   act ggt gtt gga cag cat cag atg tgg gcg gcg cag ttt tac aag tac        1440
   Thr Gly Val Gly Gln His Gln Met Trp Ala Ala Gln Phe Tyr Lys Tyr
   465                 470                 475                 480
   agg aag ccg agg cag tgg ctg tcg tcc tca gga ctc gga gct atg ggt        1488
   Arg Lys Pro Arg Gln Trp Leu Ser Ser Ser Gly Leu Gly Ala Met Gly
                   485                 490                 495
   ttc gga ctt cct gct gcg att gga gcg tct gtg gcg aac cct gat gcg        1536
   Phe Gly Leu Pro Ala Ala Ile Gly Ala Ser Val Ala Asn Pro Asp Ala
               500                 505                 510
   att gtt gtg gac att gac ggt gat gga agc ttc ata atg aac gtt caa        1584
   Ile Val Val Asp Ile Asp Gly Asp Gly Ser Phe Ile Met Asn Val Gln
           515                 520                 525
   gag ctg gcc aca atc cgt gta gag aat ctt cct gtg aag ata ctc ttg       1632
   Glu Leu Ala Thr Ile Arg Val Glu Asn Leu Pro Val Lys Ile Leu Leu
       530                 535                 540
   tta aac aac cag cat ctt ggg atg gtc atg caa tgg gaa gat cgg ttc       1680
   Leu Asn Asn Gln His Leu Gly Met Val Met Gln Trp Glu Asp Arg Phe
   545                 550                 555                 560
   tac aaa gct aac aga gct cac act tat ctc ggg gac ccg gca agg gag       1728
   Tyr Lys Ala Asn Arg Ala His Thr Tyr Leu Gly Asp Pro Ala Arg Glu
                   565                 570                 575
   aac gag atc ttc cct aac atg ctg cag ttt gca gga gct tgc ggg att       1776
   Asn Glu Ile Phe Pro Asn Met Leu Gln Phe Ala Gly Ala Cys Gly Ile
               580                 585                 590
   cca gct gcg aga gtg acg aag aaa gaa gaa ctc cga gaa gct att cag       1824
   Pro Ala Ala Arg Val Thr Lys Lys Glu Glu Leu Arg Glu Ala Ile Gln
           595                 600                 605
   aca atg ctg gat aca cct gga ccg tac ctg ttg gat gtc atc tgt ccg       1872
   Thr Met Leu Asp Thr Pro Gly Pro Tyr Leu Leu Asp Val Ile Cys Pro
       610                 615                 620
   cac caa gaa cat gtg tta ccg atg atc cca agt ggt ggc act ttc aaa       1920
   His Gln Glu His Val Leu Pro Met Ile Pro Ser Gly Gly Thr Phe Lys
   625                 630                 635                 640
   gat gta ata acc gaa ggg gat ggt cgc act aag tac                       1956
   Asp Val Ile Thr Glu Gly Asp Gly Arg Thr Lys Tyr
                   645                 650
<210> 4
   <211> 652
   <212> PRT
   <213> Brassica napus
<400> 4
   Met Ala Ala Ala Thr Ser Ser Ser Pro Ile Ser Leu Thr Ala Lys Pro
   1               5                   10                  15
   Ser Ser Lys Ser Pro Leu Pro Ile Ser Arg Phe Ser Leu Pro Phe Ser
               20                  25                  30
   Leu Thr Pro Gln Lys Pro Ser Ser Arg Leu His Arg Pro Leu Ala Ile
           35                  40                  45
   Ser Ala Val Leu Asn Ser Pro Val Asn Val Ala Pro Glu Lys Thr Asp
       50                  55                  60
   Lys Ile Lys Thr Phe Ile Ser Arg Tyr Ala Pro Asp Glu Pro Arg Lys
   65                  70                  75                  80
   Gly Ala Asp Ile Leu Val Glu Ala Leu Glu Arg Gln Gly Val Glu Thr
                   85                  90                  95
   Val Phe Ala Tyr Pro Gly Gly Ala Ser Met Glu Ile His Gln Ala Leu
               100                 105                 110
   Thr Arg Ser Ser Thr Ile Arg Asn Val Leu Pro Arg His Glu Gln Gly
           115                 120                 125
   Gly Val Phe Ala Ala Glu Gly Tyr Ala Arg Ser Ser Gly Lys Pro Gly
       130                 135                 140
   Ile Cys Ile Ala Thr Ser Gly Pro Gly Ala Thr Asn Leu Val Ser Gly
   145                 150                 155                 160
   Leu Ala Asp Ala Met Leu Asp Ser Val Pro Leu Val Ala Ile Thr Gly
                   165                 170                 175
   Gln Val Pro Arg Arg Met Ile Gly Thr Asp Ala Phe Gln Glu Thr Pro
               180                 185                 190
   Ile Val Glu Val Thr Arg Ser Ile Thr Lys His Asn Tyr Leu Val Met
           195                 200                 205
   Asp Val Asp Asp Ile Pro Arg Ile Val Gln Glu Ala Phe Phe Leu Ala
       210                 215                 220
   Thr Ser Gly Arg Pro Gly Pro Val Leu Val Asp Val Pro Lys Asp Ile
   225                 230                 235                 240
   Gln Gln Gln Leu Ala Ile Pro Asn Trp Asp Gln Pro Met Arg Leu Pro
                   245                 250                 255
   Gly Tyr Met Ser Arg Leu Pro Gln Pro Pro Glu Val Ser Gln Leu Gly
               260                 265                 270
   Gln Ile Val Arg Leu Ile Ser Glu Ser Lys Arg Pro Val Leu Tyr Val
           275                 280                 285
   Gly Gly Gly Ser Leu Asn Ser Ser Glu Glu Leu Gly Arg Phe Val Glu
       290                 295                 300
   Leu Thr Gly Ile Pro Val Ala Ser Thr Leu Met Gly Leu Gly Ser Tyr
   305                 310                 315                 320
   Pro Cys Asn Asp Glu Leu Ser Leu Gln Met Leu Gly Met His Gly Thr
                   325                 330                 335
   Val Tyr Ala Asn Tyr Ala Val Glu His Ser Asp Leu Leu Leu Ala Phe
               340                 345                 350
   Gly Val Arg Phe Asp Asp Arg Val Thr Gly Lys Leu Glu Ala Phe Ala
           355                 360                 365
   Ser Arg Ala Lys Ile Val His Ile Asp Ile Asp Ser Ala Glu Ile Gly
       370                 375                 380
   Lys Asn Lys Thr Pro His Val Ser Val Cys Gly Asp Val Lys Leu Ala
   385                 390                 395                 400
   Leu Gln Gly Met Asn Lys Val Leu Glu Asn Arg Ala Glu Glu Leu Lys
                   405                 410                 415
   Leu Asp Phe Gly Val Trp Arg Ser Glu Leu Ser Glu Gln Lys Gln Lys
               420                 425                 430
   Phe Pro Leu Ser Phe Lys Thr Phe Gly Glu Ala Ile Pro Pro Gln Tyr
           435                 440                 445
   Ala Ile Gln Val Leu Asp Glu Leu Thr Gln Gly Lys Ala Ile Ile Ser
       450                 455                 460
   Thr Gly Val Gly Gln His Gln Met Trp Ala Ala Gln Phe Tyr Lys Tyr
   465                 470                 475                 480
   Arg Lys Pro Arg Gln Trp Leu Ser Ser Ser Gly Leu Gly Ala Met Gly
                   485                 490                 495
   Phe Gly Leu Pro Ala Ala Ile Gly Ala Ser Val Ala Asn Pro Asp Ala
               500                 505                 510
   Ile Val Val Asp Ile Asp Gly Asp Gly Ser Phe Ile Met Asn Val Gln
           515                 520                 525
   Glu Leu Ala Thr Ile Arg Val Glu Asn Leu Pro Val Lys Ile Leu Leu
       530                 535                 540
   Leu Asn Asn Gln His Leu Gly Met Val Met Gln Trp Glu Asp Arg Phe
   545                 550                 555                 560
   Tyr Lys Ala Asn Arg Ala His Thr Tyr Leu Gly Asp Pro Ala Arg Glu
                   565                 570                 575
   Asn Glu Ile Phe Pro Asn Met Leu Gln Phe Ala Gly Ala Cys Gly Ile
               580                 585                 590
   Pro Ala Ala Arg Val Thr Lys Lys Glu Glu Leu Arg Glu Ala Ile Gln
           595                 600                 605
   Thr Met Leu Asp Thr Pro Gly Pro Tyr Leu Leu Asp Val Ile Cys Pro
       610                 615                 620
   His Gln Glu His Val Leu Pro Met Ile Pro Ser Gly Gly Thr Phe Lys
   625                 630                 635                 640
   Asp Val Ile Thr Glu Gly Asp Gly Arg Thr Lys Tyr
                   645                 650
<210> 5
   <211> 1956
   <212> DNA
   <213> Brassica juncea
<220>
   <221> CDS
   <222> (1)..(1956)
<400> 5
   atg gcg gcg gca aca tcg tct tct ccg atc tcc tta acc gct aaa cct        48
   Met Ala Ala Ala Thr Ser Ser Ser Pro Ile Ser Leu Thr Ala Lys Pro
   1               5                   10                  15
   tct tcc aaa tcc cct cta ccc att tcc aga ttc tcc ctt ccc ttc tcc        96
   Ser Ser Lys Ser Pro Leu Pro Ile Ser Arg Phe Ser Leu Pro Phe Ser
               20                  25                  30
   tta acc cca cag aaa ccc tcc tcc cgt ctc cac cgt cct ctc gcc atc       144
   Leu Thr Pro Gln Lys Pro Ser Ser Arg Leu His Arg Pro Leu Ala Ile
           35                  40                  45
   tcc gcc gtt ctc aac tca ccc gtc aat gtc gca cct gaa aaa acc gac       192
   Ser Ala Val Leu Asn Ser Pro Val Asn Val Ala Pro Glu Lys Thr Asp
       50                  55                  60
   aag atc aag act ttc atc tcc cgc tac gct ccc gac gag ccc cgc aag       240
   Lys Ile Lys Thr Phe Ile Ser Arg Tyr Ala Pro Asp Glu Pro Arg Lys
   65                  70                  75                  80
   ggt gct gat atc ctc gtg gaa gcc ctc gag cgt caa ggc gtc gaa acc       288
   Gly Ala Asp Ile Leu Val Glu Ala Leu Glu Arg Gln Gly Val Glu Thr
                   85                  90                  95
   gtc ttc gct tat ccc gga ggt gcc tcc atg gag atc cac caa gcc ttg       336
   Val Phe Ala Tyr Pro Gly Gly Ala Ser Met Glu Ile His Gln Ala Leu
               100                 105                 110
   act cgc tcc tcc acc atc cgt aac gtc ctc ccc cgt cac gaa caa gga       384
   Thr Arg Ser Ser Thr Ile Arg Asn Val Leu Pro Arg His Glu Gln Gly
           115                 120                 125
   gga gtc ttc gcc gcc gag ggt tac gct cgt tcc tcc ggc aaa ccg gga       432
   Gly Val Phe Ala Ala Glu Gly Tyr Ala Arg Ser Ser Gly Lys Pro Gly
       130                 135                 140
   atc tgc att gcc act tcg ggt ccc gga gct acc aac ctc gtc agc ggg         480
   Ile Cys Ile Ala Thr Ser Gly Pro Gly Ala Thr Asn Leu Val Ser Gly
   145                 150                 155                 160
   tta gcc gac gcg atg ctt gac agt gtt cct ctc gtc gcc att aca gga         528
   Leu Ala Asp Ala Met Leu Asp Ser Val Pro Leu Val Ala Ile Thr Gly
                   165                 170                 175
   cag gtc cct cgc cgg atg atc ggt act gac gcc ttc caa gag acg cca         576
   Gln Val Pro Arg Arg Met Ile Gly Thr Asp Ala Phe Gln Glu Thr Pro
               180                 185                 190
   atc gtt gag gta acg agg tct att acg aaa cat aac tat ctg gtg atg         624
   Ile Val Glu Val Thr Arg Ser Ile Thr Lys His Asn Tyr Leu Val Met
           195                 200                 205
   gat gtt gat gac ata cct agg atc gtt caa gaa gct ttc ttt cta gct         672
   Asp Val Asp Asp Ile Pro Arg Ile Val Gln Glu Ala Phe Phe Leu Ala
       210                 215                 220
   act tcc ggt aga ccc gga ccg gtt ttg gtt gac gtt cct aag gat att         720
   Thr Ser Gly Arg Pro Gly Pro Val Leu Val Asp Val Pro Lys Asp Ile
   225                 230                 235                 240
   cag cag cag ctt gcg att cct aac tgg gat caa cct atg cgc ttg cct         768
   Gln Gln Gln Leu Ala Ile Pro Asn Trp Asp Gln Pro Met Arg Leu Pro
                   245                 250                 255
   ggc tac atg tct agg ctg cct cag cca ccg gaa gtt tct cag tta ggt         816
   Gly Tyr Met Ser Arg Leu Pro Gln Pro Pro Glu Val Ser Gln Leu Gly
               260                 265                 270
   cag atc gtt agg ttg atc tcg gag tct aag agg cct gtt ttg tac gtt         864
   Gln Ile Val Arg Leu Ile Ser Glu Ser Lys Arg Pro Val Leu Tyr Val
           275                 280                 285
   ggt ggt gga agc ttg aac tcg agt gaa gaa ctg ggg aga ttt gtc gag         912
   Gly Gly Gly Ser Leu Asn Ser Ser Glu Glu Leu Gly Arg Phe Val Glu
       290                 295                 300
   ctt act ggg atc cct gtt gcg agt acg ttg atg ggg ctt ggc tct tat         960
   Leu Thr Gly Ile Pro Val Ala Ser Thr Leu Met Gly Leu Gly Ser Tyr
   305                 310                 315                 320
   cct tgt aac gat gag ttg tcc ctg cag atg ctt ggc atg cac ggg act        1008
   Pro Cys Asn Asp Glu Leu Ser Leu Gln Met Leu Gly Met His Gly Thr
                   325                 330                 335
   gtg tat gct aac tac gct gtg gag cat agt gat ttg ttg ctg gcg ttt        1056
   Val Tyr Ala Asn Tyr Ala Val Glu His Ser Asp Leu Leu Leu Ala Phe
               340                 345                 350
   ggt gtt agg ttt gat gac cgt gtc acg gga aag ctc gag gcg ttt gcg        1104
   Gly Val Arg Phe Asp Asp Arg Val Thr Gly Lys Leu Glu Ala Phe Ala
           355                 360                 365
   agc agg gct aag att gtg cac ata gac att gat tct gct gag att ggg        1152
   Ser Arg Ala Lys Ile Val His Ile Asp Ile Asp Ser Ala Glu Ile Gly
       370                 375                 380
   aag aat aag aca cct cac gtg tct gtg tgt ggt gat gta aag ctg gct        1200
   Lys Asn Lys Thr Pro His Val Ser Val Cys Gly Asp Val Lys Leu Ala
   385                 390                 395                 400
   ttg caa ggg atg aac aag gtt ctt gag aac cgg gcg gag gag ctc aag        1248
   Leu Gln Gly Met Asn Lys Val Leu Glu Asn Arg Ala Glu Glu Leu Lys
                   405                 410                 415
   ctt gat ttc ggt gtt tgg agg agt gag ttg agc gag cag aaa cag aag        1296
   Leu Asp Phe Gly Val Trp Arg Ser Glu Leu Ser Glu Gln Lys Gln Lys
               420                 425                 430
   ttc ccg ttg agc ttc aaa acg ttt gga gaa gcc att cct ccg cag tac        1344
   Phe Pro Leu Ser Phe Lys Thr Phe Gly Glu Ala Ile Pro Pro Gln Tyr
           435                 440                 445
   gcg att cag gtc cta gac gag cta acc caa ggg aag gca att atc agt        1392
   Ala Ile Gln Val Leu Asp Glu Leu Thr Gln Gly Lys Ala Ile Ile Ser
       450                 455                 460
   act ggt gtt gga cag cat cag atg tgg gcg gcg cag ttt tac aag tac        1440
   Thr Gly Val Gly Gln His Gln Met Trp Ala Ala Gln Phe Tyr Lys Tyr
   465                 470                 475                 480
   agg aag ccg agg cag tgg ctg tcg tcc tca gga ctc gga gct atg ggt        1488
   Arg Lys Pro Arg Gln Trp Leu Ser Ser Ser Gly Leu Gly Ala Met Gly
                   485                 490                 495
   ttc gga ctt cct gct gcg att gga gcg tct gtg gcg aac cct gat gcg        1536
   Phe Gly Leu Pro Ala Ala Ile Gly Ala Ser Val Ala Asn Pro Asp Ala
               500                 505                 510
   att gtt gtg gac att gac ggt gat gga agc ttc ata atg aac gtt caa        1584
   Ile Val Val Asp Ile Asp Gly Asp Gly Ser Phe Ile Met Asn Val Gln
           515                 520                 525
   gag ctg gcc aca atc cgt gta gag aat ctt cct gtg aag ata ctc ttg        1632
   Glu Leu Ala Thr Ile Arg Val Glu Asn Leu Pro Val Lys Ile Leu Leu
       530                 535                 540
   tta aac aac cag cat ctt ggg atg gtc atg caa tgg gaa gat cgg ttc        1680
   Leu Asn Asn Gln His Leu Gly Met Val Met Gln Trp Glu Asp Arg Phe
   545                 550                 555                 560
   tac aaa gct aac aga gct cac act tat ctc ggg gac ccg gca agg gag        1728
   Tyr Lys Ala Asn Arg Ala His Thr Tyr Leu Gly Asp Pro Ala Arg Glu
                   565                 570                 575
   aac gag atc ttc cct aac atg ctg cag ttt gca gga gct tgc ggg att        1776
   Asn Glu Ile Phe Pro Asn Met Leu Gln Phe Ala Gly Ala Cys Gly Ile
               580                 585                 590
   cca gct gcg aga gtg acg aag aaa gaa gaa ctc cga gaa gct att cag        1824
   Pro Ala Ala Arg Val Thr Lys Lys Glu Glu Leu Arg Glu Ala Ile Gln
           595                 600                 605
   aca atg ctg gat aca cct gga ccg tac ctg ttg gat gtc atc tgt ccg        1872
   Thr Met Leu Asp Thr Pro Gly Pro Tyr Leu Leu Asp Val Ile Cys Pro
       610                 615                 620
   cac caa gaa cat gtg tta ccg atg atc cca agt ggt ggc act ttc aaa        1920
   His Gln Glu His Val Leu Pro Met Ile Pro Ser Gly Gly Thr Phe Lys
   625                 630                 635                 640
   gat gta ata acc gaa ggg gat ggt cgc act aag tac                    1956
   Asp Val Ile Thr Glu Gly Asp Gly Arg Thr Lys Tyr
                   645                 650
<210> 6
   <211> 652
   <212> PRT
   <213> Brassica juncea
<400> 6
   Met Ala Ala Ala Thr Ser Ser Ser Pro Ile Ser Leu Thr Ala Lys Pro
   1               5                   10                  15
   Ser Ser Lys Ser Pro Leu Pro Ile Ser Arg Phe Ser Leu Pro Phe Ser
               20                  25                  30
   Leu Thr Pro Gln Lys Pro Ser Ser Arg Leu His Arg Pro Leu Ala Ile
           35                  40                  45
   Ser Ala Val Leu Asn Ser Pro Val Asn Val Ala Pro Glu Lys Thr Asp
       50                  55                  60
   Lys Ile Lys Thr Phe Ile Ser Arg Tyr Ala Pro Asp Glu Pro Arg Lys
   65                  70                  75                  80
   Gly Ala Asp Ile Leu Val Glu Ala Leu Glu Arg Gln Gly Val Glu Thr
                   85                  90                  95
   Val Phe Ala Tyr Pro Gly Gly Ala Ser Met Glu Ile His Gln Ala Leu
               100                 105                 110
   Thr Arg Ser Ser Thr Ile Arg Asn Val Leu Pro Arg His Glu Gln Gly
           115                 120                 125
   Gly Val Phe Ala Ala Glu Gly Tyr Ala Arg Ser Ser Gly Lys Pro Gly
       130                 135                 140
   Ile Cys Ile Ala Thr Ser Gly Pro Gly Ala Thr Asn Leu Val Ser Gly
   145                 150                 155                 160
   Leu Ala Asp Ala Met Leu Asp Ser Val Pro Leu Val Ala Ile Thr Gly
                   165                 170                 175
   Gln Val Pro Arg Arg Met Ile Gly Thr Asp Ala Phe Gln Glu Thr Pro
               180                 185                 190
   Ile Val Glu Val Thr Arg Ser Ile Thr Lys His Asn Tyr Leu Val Met
           195                 200                 205
   Asp Val Asp Asp Ile Pro Arg Ile Val Gln Glu Ala Phe Phe Leu Ala
   210 215 220
   Thr Ser Gly Arg Pro Gly Pro Val Leu Val Asp Val Pro Lys Asp Ile
   225 230 235 240
   Gln Gln Gln Leu Ala Ile Pro Asn Trp Asp Gln Pro Met Arg Leu Pro
   245 250 255
   Gly Tyr Met Ser Arg Leu Pro Gln Pro Pro Glu Val Ser Gln Leu Gly
   260 265 270
   Gln Ile Val Arg Leu Ile Ser Glu Ser Lys Arg Pro Val Leu Tyr Val
   275 280 285
   Gly Gly Gly Ser Leu Asn Ser Ser Glu Glu Leu Gly Arg Phe Val Glu
   290 295 300
   Leu Thr Gly Ile Pro Val Ala Ser Thr Leu Met Gly Leu Gly Ser Tyr
   305 310 315 320
   Pro Cys Asn Asp Glu Leu Ser Leu Gln Met Leu Gly Met His Gly Thr
   325 330 335
   Val Tyr Ala Asn Tyr Ala Val Glu His Ser Asp Leu Leu Leu Ala Phe
   340 345 350
   Gly Val Arg Phe Asp Asp Arg Val Thr Gly Lys Leu Glu Ala Phe Ala
   355 360 365
   Ser Arg Ala Lys Ile Val His Ile Asp Ile Asp Ser Ala Glu Ile Gly
   370 375 380
   Lys Asn Lys Thr Pro His Val Ser Val Cys Gly Asp Val Lys Leu Ala
   385 390 395 400
   Leu Gln Gly Met Asn Lys Val Leu Glu Asn Arg Ala Glu Glu Leu Lys
   405 410 415
   Leu Asp Phe Gly Val Trp Arg Ser Glu Leu Ser Glu Gln Lys Gln Lys
   420 425 430
   Phe Pro Leu Ser Phe Lys Thr Phe Gly Glu Ala Ile Pro Pro Gln Tyr
   435 440 445
   Ala Ile Gln Val Leu Asp Glu Leu Thr Gln Gly Lys Ala Ile Ile Ser
   450 455 460
   Thr Gly Val Gly Gln His Gln Met Trp Ala Ala Gln Phe Tyr Lys Tyr
   465                 470                 475                 480
   Arg Lys Pro Arg Gln Trp Leu Ser Ser Ser Gly Leu Gly Ala Met Gly
                   485                 490                 495
   Phe Gly Leu Pro Ala Ala Ile Gly Ala Ser Val Ala Asn Pro Asp Ala
               500                 505                 510
   Ile Val Val Asp Ile Asp Gly Asp Gly Ser Phe Ile Met Asn Val Gln
           515                 520                 525
   Glu Leu Ala Thr Ile Arg Val Glu Asn Leu Pro Val Lys Ile Leu Leu
       530                 535                 540
   Leu Asn Asn Gln His Leu Gly Met Val Met Gln Trp Glu Asp Arg Phe
   545                 550                 555                 560
   Tyr Lys Ala Asn Arg Ala His Thr Tyr Leu Gly Asp Pro Ala Arg Glu
                   565                 570                 575
   Asn Glu Ile Phe Pro Asn Met Leu Gln Phe Ala Gly Ala Cys Gly Ile
               580                 585                 590
   Pro Ala Ala Arg Val Thr Lys Lys Glu Glu Leu Arg Glu Ala Ile Gln
           595                 600                 605
   Thr Met Leu Asp Thr Pro Gly Pro Tyr Leu Leu Asp Val Ile Cys Pro
       610                 615                 620
   His Gln Glu His Val Leu Pro Met Ile Pro Ser Gly Gly Thr Phe Lys
   625                 630                 635                 640
   Asp Val Ile Thr Glu Gly Asp Gly Arg Thr Lys Tyr
                   645                 650
<210> 7
   <211> 1965
   <212> DNA
   <213> Brassica juncea
<220>
   <221> CDS
   <222> (1)..(1965)
<400> 7
   atg gcg gcg gca aca tcg tct tct cca atc tcc ttc acc gct aaa cct       48
   Met Ala Ala Ala Thr Ser Ser Ser Pro Ile Ser Phe Thr Ala Lys Pro
   1               5                   10                  15
   tct tcc aaa tcc ctt tta ccc att tcc aga ttc tcc ctt ccc ttc tcc          96
   Ser Ser Lys Ser Leu Leu Pro Ile Ser Arg Phe Ser Leu Pro Phe Ser
               20                  25                  30
   tta atc ccg cag aaa ccc tcc tcc ctt cgc cac agt cct ctc tcc atc         144
   Leu Ile Pro Gln Lys Pro Ser Ser Leu Arg His Ser Pro Leu Ser Ile
           35                  40                  45
   tca gcc gtt ctc aac aca ccc gtc aat gtc gca cct cct tcc cct gaa         192
   Ser Ala Val Leu Asn Thr Pro Val Asn Val Ala Pro Pro Ser Pro Glu
       50                  55                  60
   aaa att gaa aag aac aag act ttc atc tcc cgc tac gct ccc gac gag         240
   Lys Ile Glu Lys Asn Lys Thr Phe Ile Ser Arg Tyr Ala Pro Asp Glu
   65                  70                  75                  80
   ccc cgc aag ggc gcc gat atc ctc gtc gaa gcc ctc gag cgt caa ggc         288
   Pro Arg Lys Gly Ala Asp Ile Leu Val Glu Ala Leu Glu Arg Gln Gly
                   85                  90                  95
   gtc gaa acc gtc ttc gct tac ccg gga ggt gct tcc atg gag atc cac         336
   Val Glu Thr Val Phe Ala Tyr Pro Gly Gly Ala Ser Met Glu Ile His
               100                 105                 110
   caa gcc tta act cga tcc tct acc atc cgt aac gtc ctc ccc cgt cac         384
   Gln Ala Leu Thr Arg Ser Ser Thr Ile Arg Asn Val Leu Pro Arg His
           115                 120                 125
   gaa caa gga gga gtc ttt gcc gcc gag ggt tac gct cgt tcc tct ggt         432
   Glu Gln Gly Gly Val Phe Ala Ala Glu Gly Tyr Ala Arg Ser Ser Gly
       130                 135                 140
   aaa ccg gga atc tgc ata gcc acg tca ggt ccc gga gcc acc aac ctc         480
   Lys Pro Gly Ile Cys Ile Ala Thr Ser Gly Pro Gly Ala Thr Asn Leu
   145                 150                 155                 160
   gtt agc ggt tta gcc gac gcg atg ctc gac agt gtc cct ctc gtc gct         528
   Val Ser Gly Leu Ala Asp Ala Met Leu Asp Ser Val Pro Leu Val Ala
                   165                 170                 175
   att aca gga cag gtc cct cgt cgg atg att ggt act gac gcg ttc cag         576
   Ile Thr Gly Gln Val Pro Arg Arg Met Ile Gly Thr Asp Ala Phe Gln
               180                 185                 190
   gag acg cca atc gtt gag gta acg agg tct att acg aaa cat aac tat         624
   Glu Thr Pro Ile Val Glu Val Thr Arg Ser Ile Thr Lys His Asn Tyr
           195                 200                 205
   ctg gtc atg gat gtt gat gac ata cct agg atc gtg caa gag gct ttc         672
   Leu Val Met Asp Val Asp Asp Ile Pro Arg Ile Val Gln Glu Ala Phe
       210                 215                 220
   ttt cta gct act tcc ggt aga ccc gga ccg gtt tta gtt gat gtt cct         720
   Phe Leu Ala Thr Ser Gly Arg Pro Gly Pro Val Leu Val Asp Val Pro
   225                 230                 235                 240
   aag gat att cag cag cag ctt gcg att cct aac tgg gat cag cct atg         768
   Lys Asp Ile Gln Gln Gln Leu Ala Ile Pro Asn Trp Asp Gln Pro Met
                   245                 250                 255
   cgc tta cct ggt tac atg tct agg ctg cct cag cct ccg gaa gtt tct         816
   Arg Leu Pro Gly Tyr Met Ser Arg Leu Pro Gln Pro Pro Glu Val Ser
               260                 265                 270
   cag tta ggg cag atc gtt agg ttg atc tct gaa tct aag agg cct gtt      864
   Gln Leu Gly Gln Ile Val Arg Leu Ile Ser Glu Ser Lys Arg Pro Val
           275                 280                 285
   ttg tat gtt ggt ggt gga agc ttg aac tcg agt gat gaa ctg ggg agg      912
   Leu Tyr Val Gly Gly Gly Ser Leu Asn Ser Ser Asp Glu Leu Gly Arg
       290                 295                 300
   ttt gtg gag ctt act ggg atc cct gtc gcg agt act ttg atg ggg ctt      960
   Phe Val Glu Leu Thr Gly Ile Pro Val Ala Ser Thr Leu Met Gly Leu
   305                 310                 315                 320
   ggt tct tat cct tgt aac gat gag ttg tct ctg cag atg ctt ggt atg     1008
   Gly Ser Tyr Pro Cys Asn Asp Glu Leu Ser Leu Gln Met Leu Gly Met
                   325                 330                 335
   cac ggg act gtg tac gct aat tac gct gtg gag cat agt gat ttg ttg     1056
   His Gly Thr Val Tyr Ala Asn Tyr Ala Val Glu His Ser Asp Leu Leu
               340                 345                 350
   ctg gcg ttt ggt gtt agg ttt gat gac cgt gtc act gga aag ctc gag     1104
   Leu Ala Phe Gly Val Arg Phe Asp Asp Arg Val Thr Gly Lys Leu Glu
           355                 360                 365
   gct ttt gcg agc agg gct aag att gtg cac att gac att gat tct gct     1152
   Ala Phe Ala Ser Arg Ala Lys Ile Val His Ile Asp Ile Asp Ser Ala
       370                 375                 380
   gag att ggg aag aac aag acg cct cat gtg tct gtg tgt ggt gat gtt     1200
   Glu Ile Gly Lys Asn Lys Thr Pro His Val Ser Val Cys Gly Asp Val
   385                 390                 395                 400
   aag ctg gct ttg caa ggg atg aac aag gtt ctt gag aac cga gca gag     1248
   Lys Leu Ala Leu Gln Gly Met Asn Lys Val Leu Glu Asn Arg Ala Glu
                   405                 410                 415
   gag ctc aag ctt gac ttc gga gtt tgg agg agt gaa ttg agc gag cag     1296
   Glu Leu Lys Leu Asp Phe Gly Val Trp Arg Ser Glu Leu Ser Glu Gln
               420                 425                 430
   aaa caa aag ttc ccg ttg agt ttt aaa acg ttt gga gaa gct att cct     1344
   Lys Gln Lys Phe Pro Leu Ser Phe Lys Thr Phe Gly Glu Ala Ile Pro
           435                 440                 445
   cca cag tac gcg att cag gtc ctc gac gag cta acc gat ggg aag gca     1392
   Pro Gln Tyr Ala Ile Gln Val Leu Asp Glu Leu Thr Asp Gly Lys Ala
       450                 455                 460
   atc atc agt act ggt gtt ggg caa cat cag atg tgg gcg gcg cag ttt     1440
   Ile Ile Ser Thr Gly Val Gly Gln His Gln Met Trp Ala Ala Gln Phe
   465                 470                 475                 480
   tac aag tac agg aag ccg agg cag tgg ttg tca tca tca ggc ctt gga     1488
   Tyr Lys Tyr Arg Lys Pro Arg Gln Trp Leu Ser Ser Ser Gly Leu Gly
                   485                 490                 495
   gct atg ggt ttt gga ctt cct gct gcc att gga gcg tct gtg gcg aac     1536
   Ala Met Gly Phe Gly Leu Pro Ala Ala Ile Gly Ala Ser Val Ala Asn
               500                 505                 510
   cct gat gcg att gtt gtg gac att gac ggt gac gga agc ttc atc atg        1584
   Pro Asp Ala Ile Val Val Asp Ile Asp Gly Asp Gly Ser Phe Ile Met
           515                 520                 525
   aat gtt caa gag ctg gcc aca atc cgt gta gag aat ctt cct gtg aag        1632
   Asn Val Gln Glu Leu Ala Thr Ile Arg Val Glu Asn Leu Pro Val Lys
       530                 535                 540
   gta ctc ttg tta aac aac cag cat ctt ggc atg gtt atg caa tgg gaa        1680
   Val Leu Leu Leu Asn Asn Gln His Leu Gly Met Val Met Gln Trp Glu
   545                 550                 555                 560
   gat cgg ttc tac aaa gct aac aga gct cac act tat ctc ggg gat ccg        1728
   Asp Arg Phe Tyr Lys Ala Asn Arg Ala His Thr Tyr Leu Gly Asp Pro
                   565                 570                 575
   gca aag gag aac gag atc ttc cca aac atg ctg cag ttt gca gga gcc        1776
   Ala Lys Glu Asn Glu Ile Phe Pro Asn Met Leu Gln Phe Ala Gly Ala
               580                 585                 590
   tgt ggg att cca gct gcg agg gtg acg aag aaa gaa gaa ctc cga gat        1824
   Cys Gly Ile Pro Ala Ala Arg Val Thr Lys Lys Glu Glu Leu Arg Asp
           595                 600                 605
   gct att cag aca atg ctg gat aca cca gga cca tac ctg ttg gat gtg        1872
   Ala Ile Gln Thr Met Leu Asp Thr Pro Gly Pro Tyr Leu Leu Asp Val
       610                 615                 620
   atc tgt ccg cac caa gag cat gtg tta ccg atg atc cca agt ggt ggt        1920
   Ile Cys Pro His Gln Glu His Val Leu Pro Met Ile Pro Ser Gly Gly
   625                 630                 635                 640
   act ttc aaa gat gtc ata aca gaa ggg gat ggt cgc act aag tac            1965
   Thr Phe Lys Asp Val Ile Thr Glu Gly Asp Gly Arg Thr Lys Tyr
                   645                 650                 655
<210> 8
   <211> 655
   <212> PRT
   <213> Brassica juncea
<400> 8
   Met Ala Ala Ala Thr Ser Ser Ser Pro Ile Ser Phe Thr Ala Lys Pro
   1               5                   10                  15
   Ser Ser Lys Ser Leu Leu Pro Ile Ser Arg Phe Ser Leu Pro Phe Ser
               20                  25                  30
   Leu Ile Pro Gln Lys Pro Ser Ser Leu Arg His Ser Pro Leu Ser Ile
           35                  40                  45
   Ser Ala Val Leu Asn Thr Pro Val Asn Val Ala Pro Pro Ser Pro Glu
       50                  55                  60
   Lys Ile Glu Lys Asn Lys Thr Phe Ile Ser Arg Tyr Ala Pro Asp Glu
   65                  70                  75                  80
   Pro Arg Lys Gly Ala Asp Ile Leu Val Glu Ala Leu Glu Arg Gln Gly
   85 90 95
   Val Glu Thr Val Phe Ala Tyr Pro Gly Gly Ala Ser Met Glu Ile His
   100 105 110
   Gln Ala Leu Thr Arg Ser Ser Thr Ile Arg Asn Val Leu Pro Arg His
   115 120 125
   Glu Gln Gly Gly Val Phe Ala Ala Glu Gly Tyr Ala Arg Ser Ser Gly
   130 135 140
   Lys Pro Gly Ile Cys Ile Ala Thr Ser Gly Pro Gly Ala Thr Asn Leu
   145 150 155 160
   Val Ser Gly Leu Ala Asp Ala Met Leu Asp Ser Val Pro Leu Val Ala
   165 170 175
   Ile Thr Gly Gln Val Pro Arg Arg Met Ile Gly Thr Asp Ala Phe Gln
   180 185 190
   Glu Thr Pro Ile Val Glu Val Thr Arg Ser Ile Thr Lys His Asn Tyr
   195 200 205
   Leu Val Met Asp Val Asp Asp Ile Pro Arg Ile Val Gln Glu Ala Phe
   210 215 220
   Phe Leu Ala Thr Ser Gly Arg Pro Gly Pro Val Leu Val Asp Val Pro
   225 230 235 240
   Lys Asp Ile Gln Gln Gln Leu Ala Ile Pro Asn Trp Asp Gln Pro Met
   245 250 255
   Arg Leu Pro Gly Tyr Met Ser Arg Leu Pro Gln Pro Pro Glu Val Ser
   260 265 270
   Gln Leu Gly Gln Ile Val Arg Leu Ile Ser Glu Ser Lys Arg Pro Val
   275 280 285
   Leu Tyr Val Gly Gly Gly Ser Leu Asn Ser Ser Asp Glu Leu Gly Arg
   290 295 300
   Phe Val Glu Leu Thr Gly Ile Pro Val Ala Ser Thr Leu Met Gly Leu
   305 310 315 320
   Gly Ser Tyr Pro Cys Asn Asp Glu Leu Ser Leu Gln Met Leu Gly Met
   325 330 335
   His Gly Thr Val Tyr Ala Asn Tyr Ala Val Glu His Ser Asp Leu Leu
               340                 345                 350
   Leu Ala Phe Gly Val Arg Phe Asp Asp Arg Val Thr Gly Lys Leu Glu
           355                 360                 365
   Ala Phe Ala Ser Arg Ala Lys Ile Val His Ile Asp Ile Asp Ser Ala
       370                 375                 380
   Glu Ile Gly Lys Asn Lys Thr Pro His Val Ser Val Cys Gly Asp Val
   385                 390                 395                 400
   Lys Leu Ala Leu Gln Gly Met Asn Lys Val Leu Glu Asn Arg Ala Glu
                   405                 410                 415
   Glu Leu Lys Leu Asp Phe Gly Val Trp Arg Ser Glu Leu Ser Glu Gln
               420                 425                 430
   Lys Gln Lys Phe Pro Leu Ser Phe Lys Thr Phe Gly Glu Ala Ile Pro
           435                 440                 445
   Pro Gln Tyr Ala Ile Gln Val Leu Asp Glu Leu Thr Asp Gly Lys Ala
       450                 455                 460
   Ile Ile Ser Thr Gly Val Gly Gln His Gln Met Trp Ala Ala Gln Phe
   465                 470                 475                 480
   Tyr Lys Tyr Arg Lys Pro Arg Gln Trp Leu Ser Ser Ser Gly Leu Gly
                   485                 490                 495
   Ala Met Gly Phe Gly Leu Pro Ala Ala Ile Gly Ala Ser Val Ala Asn
               500                 505                 510
   Pro Asp Ala Ile Val Val Asp Ile Asp Gly Asp Gly Ser Phe Ile Met
           515                 520                 525
   Asn Val Gln Glu Leu Ala Thr Ile Arg Val Glu Asn Leu Pro Val Lys
       530                 535                 540
   Val Leu Leu Leu Asn Asn Gln His Leu Gly Met Val Met Gln Trp Glu
   545                 550                 555                 560
   Asp Arg Phe Tyr Lys Ala Asn Arg Ala His Thr Tyr Leu Gly Asp Pro
                   565                 570                 575
   Ala Lys Glu Asn Glu Ile Phe Pro Asn Met Leu Gln Phe Ala Gly Ala
   580 585 590
   Cys Gly Ile Pro Ala Ala Arg Val Thr Lys Lys Glu Glu Leu Arg Asp
   595 600 605
   Ala Ile Gln Thr Met Leu Asp Thr Pro Gly Pro Tyr Leu Leu Asp Val
   610 615 620
   Ile Cys Pro His Gln Glu His Val Leu Pro Met Ile Pro Ser Gly Gly
   625 630 635 640
   Thr Phe Lys Asp Val Ile Thr Glu Gly Asp Gly Arg Thr Lys Tyr
   645 650 655
<210> 9
   <211> 2010
   <212> DNA
   <213> Arabidopsis thaliana
<220>
   <221> CDS
   <222> (1)..(2010)
<400> 9
   atg gcg gcg gca aca aca aca aca aca aca tct tct tcg atc tcc ttc        48
   Met Ala Ala Ala Thr Thr Thr Thr Thr Thr Ser Ser Ser Ile Ser Phe
   1               5                   10                  15
   tcc acc aaa cca tct cct tcc tcc tcc aaa tca cca tta cca atc tcc        96
   Ser Thr Lys Pro Ser Pro Ser Ser Ser Lys Ser Pro Leu Pro Ile Ser
               20                  25                  30
   aga ttc tcc ctc cca ttc tcc cta aac ccc aac aaa tca tcc tcc tcc       144
   Arg Phe Ser Leu Pro Phe Ser Leu Asn Pro Asn Lys Ser Ser Ser Ser
           35                  40                  45
   tcc cgc cgc cgc ggt atc aaa tcc agc tct ccc tcc tcc atc tcc gcc       192
   Ser Arg Arg Arg Gly Ile Lys Ser Ser Ser Pro Ser Ser Ile Ser Ala
       50                  55                  60
   gtg ctc aac aca acc acc aat gtg aca acc act ccc tct cca acc aaa       240
   Val Leu Asn Thr Thr Thr Asn Val Thr Thr Thr Pro Ser Pro Thr Lys
   65                  70                  75                  80
   cct acc aaa ccc gaa aca ttc atc tcc cga ttc gct cca gat caa ccc       288
   Pro Thr Lys Pro Glu Thr Phe Ile Ser Arg Phe Ala Pro Asp Gln Pro
                   85                  90                  95
   cgc aaa ggc gct gat atc ctc gtc gaa gct tta gaa cgt caa ggc gta       336
   Arg Lys Gly Ala Asp Ile Leu Val Glu Ala Leu Glu Arg Gln Gly Val
               100                 105                 110
   gaa acc gta ttc gct tac cct gga ggt gca tca atg gag att cac caa       384
   Glu Thr Val Phe Ala Tyr Pro Gly Gly Ala Ser Met Glu Ile His Gln
           115                 120                 125
   gcc tta acc cgc tct tcc tca atc cgt aac gtc ctt cct cgt cac gaa       432
   Ala Leu Thr Arg Ser Ser Ser Ile Arg Asn Val Leu Pro Arg His Glu
       130                 135                 140
   caa gga ggt gta ttc gca gca gaa gga tac gct cga tcc tca ggt aaa      480
   Gln Gly Gly Val Phe Ala Ala Glu Gly Tyr Ala Arg Ser Ser Gly Lys
   145                 150                 155                 160
   cca ggt atc tgt ata gcc act tca ggt ccc gga gct aca aat ctc gtt      528
   Pro Gly Ile Cys Ile Ala Thr Ser Gly Pro Gly Ala Thr Asn Leu Val
                   165                 170                 175
   agc gga tta gcc gat gcg ttg tta gat agt gtt cct ctt gta gca atc      576
   Ser Gly Leu Ala Asp Ala Leu Leu Asp Ser Val Pro Leu Val Ala Ile
               180                 185                 190
   aca gga caa gtc cct cgt cgt atg att ggt aca gat gcg ttt caa gag      624
   Thr Gly Gln Val Pro Arg Arg Met Ile Gly Thr Asp Ala Phe Gln Glu
           195                 200                 205
   act ccg att gtt gag gta acg cgt tcg att acg aag cat aac tat ctt      672
   Thr Pro Ile Val Glu Val Thr Arg Ser Ile Thr Lys His Asn Tyr Leu
       210                 215                 220
   gtg atg gat gtt gaa gat atc cct agg att att gag gaa gct ttc ttt      720
   Val Met Asp Val Glu Asp Ile Pro Arg Ile Ile Glu Glu Ala Phe Phe
   225                 230                 235                 240
   tta gct act tct ggt aga cct gga cct gtt ttg gtt gat gtt cct aaa      768
   Leu Ala Thr Ser Gly Arg Pro Gly Pro Val Leu Val Asp Val Pro Lys
                   245                 250                 255
   gat att caa caa cag ctt gcg att cct aat tgg gaa cag gct atg aga      816
   Asp Ile Gln Gln Gln Leu Ala Ile Pro Asn Trp Glu Gln Ala Met Arg
               260                 265                 270
   tta cct ggt tat atg tct agg atg cct aaa cct ccg gaa gat tct cat      864
   Leu Pro Gly Tyr Met Ser Arg Met Pro Lys Pro Pro Glu Asp Ser His
           275                 280                 285
   ttg gag cag att gtt agg ttg att tct gag tct aag aag cct gtg ttg      912
   Leu Glu Gln Ile Val Arg Leu Ile Ser Glu Ser Lys Lys Pro Val Leu
       290                 295                 300
   tat gtt ggt ggt ggt tgt ttg aat tct agc gat gaa ttg ggt agg ttt      960
   Tyr Val Gly Gly Gly Cys Leu Asn Ser Ser Asp Glu Leu Gly Arg Phe
   305                 310                 315                 320
   gtt gag ctt acg ggg atc cct gtt gcg agt acg ttg atg ggg ctg gga     1008
   Val Glu Leu Thr Gly Ile Pro Val Ala Ser Thr Leu Met Gly Leu Gly
                   325                 330                 335
   tct tat cct tgt gat gat gag ttg tcg tta cat atg ctt gga atg cat     1056
   Ser Tyr Pro Cys Asp Asp Glu Leu Ser Leu His Met Leu Gly Met His
               340                 345                 350
   ggg act gtg tat gca aat tac gct gtg gag cat agt gat ttg ttg ttg     1104
   Gly Thr Val Tyr Ala Asn Tyr Ala Val Glu His Ser Asp Leu Leu Leu
           355                 360                 365
   gcg ttt ggg gta agg ttt gat gat cgt gtc acg ggt aag ctt gag gct     1152
   Ala Phe Gly Val Arg Phe Asp Asp Arg Val Thr Gly Lys Leu Glu Ala
       370                 375                 380
   ttt gct agt agg gct aag att gtt cat att gat att gac tcg gct gag        1200
   Phe Ala Ser Arg Ala Lys Ile Val His Ile Asp Ile Asp Ser Ala Glu
   385                 390                 395                 400
   att ggg aag aat aag act cct cat gtg tct gtg tgt ggt gat gtt aag        1248
   Ile Gly Lys Asn Lys Thr Pro His Val Ser Val Cys Gly Asp Val Lys
                   405                 410                 415
   ctg gct ttg caa ggg atg aat aag gtt ctt gag aac cga gcg gag gag        1296
   Leu Ala Leu Gln Gly Met Asn Lys Val Leu Glu Asn Arg Ala Glu Glu
               420                 425                 430
   ctt aag ctt gat ttt gga gtt tgg agg aat gag ttg aac gta cag aaa        1344
   Leu Lys Leu Asp Phe Gly Val Trp Arg Asn Glu Leu Asn Val Gln Lys
           435                 440                 445
   cag aag ttt ccg ttg agc ttt aag acg ttt ggg gaa gct att cct cca        1392
   Gln Lys Phe Pro Leu Ser Phe Lys Thr Phe Gly Glu Ala Ile Pro Pro
       450                 455                 460
   cag tat gcg att aag gtc ctt gat gag ttg act gat gga aaa gcc ata        1440
   Gln Tyr Ala Ile Lys Val Leu Asp Glu Leu Thr Asp Gly Lys Ala Ile
   465                 470                 475                 480
   ata agt act ggt gtc ggg caa cat caa atg tgg gcg gcg cag ttc tac        1488
   Ile Ser Thr Gly Val Gly Gln His Gln Met Trp Ala Ala Gln Phe Tyr
                   485                 490                 495
   aat tac aag aaa cca agg cag tgg cta tca tca gga ggc ctt gga gct        1536
   Asn Tyr Lys Lys Pro Arg Gln Trp Leu Ser Ser Gly Gly Leu Gly Ala
               500                 505                 510
   atg gga ttt gga ctt cct gct gcg att gga gcg tct gtt gct aac cct        1584
   Met Gly Phe Gly Leu Pro Ala Ala Ile Gly Ala Ser Val Ala Asn Pro
           515                 520                 525
   gat gcg ata gtt gtg gat att gac gga gat gga agc ttt ata atg aat        1632
   Asp Ala Ile Val Val Asp Ile Asp Gly Asp Gly Ser Phe Ile Met Asn
       530                 535                 540
   gtg caa gag cta gcc act att cgt gta gag aat ctt cca gtg aag gta        1680
   Val Gln Glu Leu Ala Thr Ile Arg Val Glu Asn Leu Pro Val Lys Val
   545                 550                 555                 560
   ctt tta tta aac aac cag cat ctt ggc atg gtt atg caa tgg caa gat        1728
   Leu Leu Leu Asn Asn Gln His Leu Gly Met Val Met Gln Trp Gln Asp
                   565                 570                 575
   cgg ttc tac aaa gct aac cga gct cac aca ttt ctc ggg gat ccg gct        1776
   Arg Phe Tyr Lys Ala Asn Arg Ala His Thr Phe Leu Gly Asp Pro Ala
               580                 585                 590
   cag gag gac gag ata ttc ccg aac atg ttg ctg ttt gca gca gct tgc        1824
   Gln Glu Asp Glu Ile Phe Pro Asn Met Leu Leu Phe Ala Ala Ala Cys
           595                 600                 605
   ggg att cca gcg gcg agg gtg aca aag aaa gca gat ctc cga gaa gct        1872
   Gly Ile Pro Ala Ala Arg Val Thr Lys Lys Ala Asp Leu Arg Glu Ala
       610                 615                 620
   att cag aca atg ctg gat aca cca gga cct tac ctg ttg gat gtg att        1920
   Ile Gln Thr Met Leu Asp Thr Pro Gly Pro Tyr Leu Leu Asp Val Ile
   625                 630                 635                 640
   tgt ccg cac caa gaa cat gtg ttg ccg atg atc ccg agt ggt ggc act       1968
   Cys Pro His Gln Glu His Val Leu Pro Met Ile Pro Ser Gly Gly Thr
                   645                 650                 655
   ttc aac gat gtc ata acg gaa gga gat ggc cgg att aaa tac               2010
   Phe Asn Asp Val Ile Thr Glu Gly Asp Gly Arg Ile Lys Tyr
               660                 665                 670
<210> 10
   <211> 670
   <212> PRT
   <213> Arabidopsis thaliana
<400> 10
   Met Ala Ala Ala Thr Thr Thr Thr Thr Thr Ser Ser Ser Ile Ser Phe
   1               5                   10                  15
   Ser Thr Lys Pro Ser Pro Ser Ser Ser Lys Ser Pro Leu Pro Ile Ser
               20                  25                  30
   Arg Phe Ser Leu Pro Phe Ser Leu Asn Pro Asn Lys Ser Ser Ser Ser
           35                  40                  45
   Ser Arg Arg Arg Gly Ile Lys Ser Ser Ser Pro Ser Ser Ile Ser Ala
       50                  55                  60
   Val Leu Asn Thr Thr Thr Asn Val Thr Thr Thr Pro Ser Pro Thr Lys
   65                  70                  75                  80
   Pro Thr Lys Pro Glu Thr Phe Ile Ser Arg Phe Ala Pro Asp Gln Pro
                   85                  90                  95
   Arg Lys Gly Ala Asp Ile Leu Val Glu Ala Leu Glu Arg Gln Gly Val
               100                 105                 110
   Glu Thr Val Phe Ala Tyr Pro Gly Gly Ala Ser Met Glu Ile His Gln
           115                 120                 125
   Ala Leu Thr Arg Ser Ser Ser Ile Arg Asn Val Leu Pro Arg His Glu
       130                 135                 140
   Gln Gly Gly Val Phe Ala Ala Glu Gly Tyr Ala Arg Ser Ser Gly Lys
   145                 150                 155                 160
   Pro Gly Ile Cys Ile Ala Thr Ser Gly Pro Gly Ala Thr Asn Leu Val
                   165                 170                 175
   Ser Gly Leu Ala Asp Ala Leu Leu Asp Ser Val Pro Leu Val Ala Ile
               180                 185                 190
   Thr Gly Gln Val Pro Arg Arg Met Ile Gly Thr Asp Ala Phe Gln Glu
           195                 200                 205
   Thr Pro Ile Val Glu Val Thr Arg Ser Ile Thr Lys His Asn Tyr Leu
       210                 215                 220
   Val Met Asp Val Glu Asp Ile Pro Arg Ile Ile Glu Glu Ala Phe Phe
   225                 230                 235                 240
   Leu Ala Thr Ser Gly Arg Pro Gly Pro Val Leu Val Asp Val Pro Lys
                   245                 250                 255
   Asp Ile Gln Gln Gln Leu Ala Ile Pro Asn Trp Glu Gln Ala Met Arg
               260                 265                 270
   Leu Pro Gly Tyr Met Ser Arg Met Pro Lys Pro Pro Glu Asp Ser His
           275                 280                 285
   Leu Glu Gln Ile Val Arg Leu Ile Ser Glu Ser Lys Lys Pro Val Leu
       290                 295                 300
   Tyr Val Gly Gly Gly Cys Leu Asn Ser Ser Asp Glu Leu Gly Arg Phe
   305                 310                 315                 320
   Val Glu Leu Thr Gly Ile Pro Val Ala Ser Thr Leu Met Gly Leu Gly
                   325                 330                 335
   Ser Tyr Pro Cys Asp Asp Glu Leu Ser Leu His Met Leu Gly Met His
               340                 345                 350
   Gly Thr Val Tyr Ala Asn Tyr Ala Val Glu His Ser Asp Leu Leu Leu
           355                 360                 365
   Ala Phe Gly Val Arg Phe Asp Asp Arg Val Thr Gly Lys Leu Glu Ala
       370                 375                 380
   Phe Ala Ser Arg Ala Lys Ile Val His Ile Asp Ile Asp Ser Ala Glu
   385                 390                 395                 400
   Ile Gly Lys Asn Lys Thr Pro His Val Ser Val Cys Gly Asp Val Lys
                   405                 410                 415
   Leu Ala Leu Gln Gly Met Asn Lys Val Leu Glu Asn Arg Ala Glu Glu
               420                 425                 430
   Leu Lys Leu Asp Phe Gly Val Trp Arg Asn Glu Leu Asn Val Gln Lys
   435 440 445
   Gln Lys Phe Pro Leu Ser Phe Lys Thr Phe Gly Glu Ala Ile Pro Pro
   450 455 460
   Gln Tyr Ala Ile Lys Val Leu Asp Glu Leu Thr Asp Gly Lys Ala Ile
   465 470 475 480
   Ile Ser Thr Gly Val Gly Gln His Gln Met Trp Ala Ala Gln Phe Tyr
   485 490 495
   Asn Tyr Lys Lys Pro Arg Gln Trp Leu Ser Ser Gly Gly Leu Gly Ala
   500 505 510
   Met Gly Phe Gly Leu Pro Ala Ala Ile Gly Ala Ser Val Ala Asn Pro
   515 520 525
   Asp Ala Ile Val Val Asp Ile Asp Gly Asp Gly Ser Phe Ile Met Asn
   530 535 540
   Val Gln Glu Leu Ala Thr Ile Arg Val Glu Asn Leu Pro Val Lys Val
   545 550 555 560
   Leu Leu Leu Asn Asn Gln His Leu Gly Met Val Met Gln Trp Gln Asp
   565 570 575
   Arg Phe Tyr Lys Ala Asn Arg Ala His Thr Phe Leu Gly Asp Pro Ala
   580 585 590
   Gln Glu Asp Glu Ile Phe Pro Asn Met Leu Leu Phe Ala Ala Ala Cys
   595 600 605
   Gly Ile Pro Ala Ala Arg Val Thr Lys Lys Ala Asp Leu Arg Glu Ala
   610 615 620
   Ile Gln Thr Met Leu Asp Thr Pro Gly Pro Tyr Leu Leu Asp Val Ile
   625 630 635 640
   Cys Pro His Gln Glu His Val Leu Pro Met Ile Pro Ser Gly Gly Thr
   645 650 655
   Phe Asn Asp Val Ile Thr Glu Gly Asp Gly Arg Ile Lys Tyr
   660 665 670
<210> 11
   <211> 1980
   <212> DNA
   <213> Gossypium hirsutum
<220>
   <221> CDS
   <222> (1)..(1980)
<400> 11
   atg gcg gct gcc act tcg aac tcc gct ttg cct aag ctt tct acg tta       48
   Met Ala Ala Ala Thr Ser Asn Ser Ala Leu Pro Lys Leu Ser Thr Leu
   1               5                   10                  15
   act tcg tcc ttc aaa tct tcc ata ccc att tcc aaa tcc agc ctt ccc       96
   Thr Ser Ser Phe Lys Ser Ser Ile Pro Ile Ser Lys Ser Ser Leu Pro
               20                  25                  30
   ttc tcc aca acc cct caa aag ccc acc cct tac cgc tcc ttc gac gtt      144
   Phe Ser Thr Thr Pro Gln Lys Pro Thr Pro Tyr Arg Ser Phe Asp Val
           35                  40                  45
   tcc tgc tct ctt tct cat gca agc tcc aac ccc aga tcc gct gcc gca      192
   Ser Cys Ser Leu Ser His Ala Ser Ser Asn Pro Arg Ser Ala Ala Ala
       50                  55                  60
   tcc gtt acc caa aaa act gct cct ccc cat tat ttc att tcc agg tat      240
   Ser Val Thr Gln Lys Thr Ala Pro Pro His Tyr Phe Ile Ser Arg Tyr
   65                  70                  75                  80
   gcg gat gat gag ccc cgg aaa ggc gct gat atc ctg gtg gaa gcc ctg      288
   Ala Asp Asp Glu Pro Arg Lys Gly Ala Asp Ile Leu Val Glu Ala Leu
                   85                  90                  95
   gaa cgt gaa ggg gtc aag gat gtg ttt gcc tac cca ggt gga gct tca      336
   Glu Arg Glu Gly Val Lys Asp Val Phe Ala Tyr Pro Gly Gly Ala Ser
               100                 105                 110
   atg gag atc cac cag gct tta acc cgc tca aaa atc atc cga aat gtc      384
   Met Glu Ile His Gln Ala Leu Thr Arg Ser Lys Ile Ile Arg Asn Val
           115                 120                 125
   ctt ccg cga cac gag caa ggt ggg gtc ttt gcc gcc gag ggt tac gcg      432
   Leu Pro Arg His Glu Gln Gly Gly Val Phe Ala Ala Glu Gly Tyr Ala
       130                 135                 140
   cgc tcc tct ggc att tcc ggc gtt tgc att gcg acg tct ggc cct ggg      480
   Arg Ser Ser Gly Ile Ser Gly Val Cys Ile Ala Thr Ser Gly Pro Gly
   145                 150                 155                 160
   gca acc aac ttg gtg agt ggt ctc gct gat gca atg ctc gat agt atc      528
   Ala Thr Asn Leu Val Ser Gly Leu Ala Asp Ala Met Leu Asp Ser Ile
                   165                 170                 175
   cct ctc gtg gcg atc act ggt caa gtc cct cgt cgg atg atc ggt acc      576
   Pro Leu Val Ala Ile Thr Gly Gln Val Pro Arg Arg Met Ile Gly Thr
               180                 185                 190
   gat gct ttc cag gaa act cca att gtt gag gta aca agg tct att acg      624
   Asp Ala Phe Gln Glu Thr Pro Ile Val Glu Val Thr Arg Ser Ile Thr
           195                 200                 205
   aag cat aat tat ctt gtt ctt gat gtg gat gat att cct agg att gtt      672
   Lys His Asn Tyr Leu Val Leu Asp Val Asp Asp Ile Pro Arg Ile Val
       210                 215                 220
   agt gag gct ttc ttt tta gct tca tcg ggc agg ccg gga cct gtt ctg        720
   Ser Glu Ala Phe Phe Leu Ala Ser Ser Gly Arg Pro Gly Pro Val Leu
   225                 230                 235                 240
   att gat gtt cct aag gat ata caa cag caa ctt gct gtt cct aaa tgg        768
   Ile Asp Val Pro Lys Asp Ile Gln Gln Gln Leu Ala Val Pro Lys Trp
                   245                 250                 255
   aac cat tct ctt aga ttg cca ggg tat ttg tct agg ttg cct aag gct        816
   Asn His Ser Leu Arg Leu Pro Gly Tyr Leu Ser Arg Leu Pro Lys Ala
               260                 265                 270
   ccc gct gag gct cat ctc gaa cag atc gtg aga ttg gtt tct gag tct        864
   Pro Ala Glu Ala His Leu Glu Gln Ile Val Arg Leu Val Ser Glu Ser
           275                 280                 285
   aag aag cct gtt tta tat gtt ggt ggt ggg tgt ttg aac tct agt gag        912
   Lys Lys Pro Val Leu Tyr Val Gly Gly Gly Cys Leu Asn Ser Ser Glu
       290                 295                 300
   gag ttg aag agg ttt gtt gag ctt aca ggg ata ccc gtt gca agt act        960
   Glu Leu Lys Arg Phe Val Glu Leu Thr Gly Ile Pro Val Ala Ser Thr
   305                 310                 315                 320
   ttg atg ggt ctt gga gcc ttt ccg att tcg gat gag ttg tcg tta caa       1008
   Leu Met Gly Leu Gly Ala Phe Pro Ile Ser Asp Glu Leu Ser Leu Gln
                   325                 330                 335
   atg ctt ggg atg cac gga act gtg tat gcc aat tat gct gtt gat aag       1056
   Met Leu Gly Met His Gly Thr Val Tyr Ala Asn Tyr Ala Val Asp Lys
               340                 345                 350
   agt gat ttg ttg ctt gct ttt gga gtg aga ttt gat gat agg gtg aca       1104
   Ser Asp Leu Leu Leu Ala Phe Gly Val Arg Phe Asp Asp Arg Val Thr
           355                 360                 365
   gga aaa ctt gag gct ttt gcc agc cgg gca aag att gtg cat atc gat       1152
   Gly Lys Leu Glu Ala Phe Ala Ser Arg Ala Lys Ile Val His Ile Asp
       370                 375                 380
   atc gac tct gct gag att ggg aag aac aag cag cct cat atg tca gtg       1200
   Ile Asp Ser Ala Glu Ile Gly Lys Asn Lys Gln Pro His Met Ser Val
   385                 390                 395                 400
   tgt tcc gat gtg aaa ttg gca ttg cag ggg ata aat aag ata ttg gag       1248
   Cys Ser Asp Val Lys Leu Ala Leu Gln Gly Ile Asn Lys Ile Leu Glu
                   405                 410                 415
   acc acg gga gct aag ctg aat ctt gat tat tcg gaa tgg agg cag gag       1296
   Thr Thr Gly Ala Lys Leu Asn Leu Asp Tyr Ser Glu Trp Arg Gln Glu
               420                 425                 430
   tta aac gag cag aag ctg aaa ttc cct ttg agt tac aag acc ttt ggt       1344
   Leu Asn Glu Gln Lys Leu Lys Phe Pro Leu Ser Tyr Lys Thr Phe Gly
           435                 440                 445
   gaa gct att cca cct caa tat gca att cag gtt ctt gat gaa tta act       1392
   Glu Ala Ile Pro Pro Gln Tyr Ala Ile Gln Val Leu Asp Glu Leu Thr
       450                 455                 460
   ggc ggg aat gca att ata agt acc ggt gtt ggc cag cat caa atg tgg       1440
   Gly Gly Asn Ala Ile Ile Ser Thr Gly Val Gly Gln His Gln Met Trp
   465                 470                 475                 480
   gct gct caa ttt tac aag tat aag aag cct cgt caa tgg tta acg tct        1488
   Ala Ala Gln Phe Tyr Lys Tyr Lys Lys Pro Arg Gln Trp Leu Thr Ser
                   485                 490                 495
   ggg gga ttg ggt gct atg gga ttt gga ttg cct gct gct att gga gct        1536
   Gly Gly Leu Gly Ala Met Gly Phe Gly Leu Pro Ala Ala Ile Gly Ala
               500                 505                 510
   gct gtt gca aac ccg gag gca gtt gtt gta gac atc gac ggt gat gga        1584
   Ala Val Ala Asn Pro Glu Ala Val Val Val Asp Ile Asp Gly Asp Gly
           515                 520                 525
   agt ttt atc atg aat gtg caa gag ttg gcg act atg cgt gtg gaa aat        1632
   Ser Phe Ile Met Asn Val Gln Glu Leu Ala Thr Met Arg Val Glu Asn
       530                 535                 540
   ctt ccg gtt aag ata tta ttg ttg aat aat cag cat ttg ggt atg gtt        1680
   Leu Pro Val Lys Ile Leu Leu Leu Asn Asn Gln His Leu Gly Met Val
   545                 550                 555                 560
   gtt caa tgg gag gac cgg ttt tac aag gca aac agg gct cat aca tac        1728
   Val Gln Trp Glu Asp Arg Phe Tyr Lys Ala Asn Arg Ala His Thr Tyr
                   565                 570                 575
   ttg gga gac cca tcc aac gag tcg gaa ata ttc cca aat atg ttg aaa        1776
   Leu Gly Asp Pro Ser Asn Glu Ser Glu Ile Phe Pro Asn Met Leu Lys
               580                 585                 590
   ttt gct gaa gca tgc ggg ata cca gct gcc cgg gtg acg aag aaa gaa        1824
   Phe Ala Glu Ala Cys Gly Ile Pro Ala Ala Arg Val Thr Lys Lys Glu
           595                 600                 605
   gat ctc aaa gca gca att cag aaa atg ttg gac act cct gga cct tac        1872
   Asp Leu Lys Ala Ala Ile Gln Lys Met Leu Asp Thr Pro Gly Pro Tyr
       610                 615                 620
   ttg ttg gat gtg att gtc cca cat caa gaa cat gtc ctg cct atg atc        1920
   Leu Leu Asp Val Ile Val Pro His Gln Glu His Val Leu Pro Met Ile
   625                 630                 635                 640
   ccc agt gga ggc gct ttc aaa gat gtg atc aca gag ggt gat gga aga        1968
   Pro Ser Gly Gly Ala Phe Lys Asp Val Ile Thr Glu Gly Asp Gly Arg
                   645                 650                 655
   aca caa tat tga                                                        1980
   Thr Gln Tyr
<210> 12
   <211> 659
   <212> PRT
   <213> Gossypium hirsutum
<400> 12
   Met Ala Ala Ala Thr Ser Asn Ser Ala Leu Pro Lys Leu Ser Thr Leu
   1               5                   10                  15
   Thr Ser Ser Phe Lys Ser Ser Ile Pro Ile Ser Lys Ser Ser Leu Pro
               20                  25                  30
   Phe Ser Thr Thr Pro Gln Lys Pro Thr Pro Tyr Arg Ser Phe Asp Val
           35                  40                  45
   Ser Cys Ser Leu Ser His Ala Ser Ser Asn Pro Arg Ser Ala Ala Ala
       50                  55                  60
   Ser Val Thr Gln Lys Thr Ala Pro Pro His Tyr Phe Ile Ser Arg Tyr
   65                  70                  75                  80
   Ala Asp Asp Glu Pro Arg Lys Gly Ala Asp Ile Leu Val Glu Ala Leu
                   85                  90                  95
   Glu Arg Glu Gly Val Lys Asp Val Phe Ala Tyr Pro Gly Gly Ala Ser
               100                 105                 110
   Met Glu Ile His Gln Ala Leu Thr Arg Ser Lys Ile Ile Arg Asn Val
           115                 120                 125
   Leu Pro Arg His Glu Gln Gly Gly Val Phe Ala Ala Glu Gly Tyr Ala
       130                 135                 140
   Arg Ser Ser Gly Ile Ser Gly Val Cys Ile Ala Thr Ser Gly Pro Gly
   145                 150                 155                 160
   Ala Thr Asn Leu Val Ser Gly Leu Ala Asp Ala Met Leu Asp Ser Ile
                   165                 170                 175
   Pro Leu Val Ala Ile Thr Gly Gln Val Pro Arg Arg Met Ile Gly Thr
               180                 185                 190
   Asp Ala Phe Gln Glu Thr Pro Ile Val Glu Val Thr Arg Ser Ile Thr
           195                 200                 205
   Lys His Asn Tyr Leu Val Leu Asp Val Asp Asp Ile Pro Arg Ile Val
       210                 215                 220
   Ser Glu Ala Phe Phe Leu Ala Ser Ser Gly Arg Pro Gly Pro Val Leu
   225                 230                 235                 240
   Ile Asp Val Pro Lys Asp Ile Gln Gln Gln Leu Ala Val Pro Lys Trp
                   245                 250                 255
   Asn His Ser Leu Arg Leu Pro Gly Tyr Leu Ser Arg Leu Pro Lys Ala
               260                 265                 270
   Pro Ala Glu Ala His Leu Glu Gln Ile Val Arg Leu Val Ser Glu Ser
   275 280 285
   Lys Lys Pro Val Leu Tyr Val Gly Gly Gly Cys Leu Asn Ser Ser Glu
   290 295 300
   Glu Leu Lys Arg Phe Val Glu Leu Thr Gly Ile Pro Val Ala Ser Thr
   305 310 315 320
   Leu Met Gly Leu Gly Ala Phe Pro Ile Ser Asp Glu Leu Ser Leu Gln
   325 330 335
   Met Leu Gly Met His Gly Thr Val Tyr Ala Asn Tyr Ala Val Asp Lys
   340 345 350
   Ser Asp Leu Leu Leu Ala Phe Gly Val Arg Phe Asp Asp Arg Val Thr
   355 360 365
   Gly Lys Leu Glu Ala Phe Ala Ser Arg Ala Lys Ile Val His Ile Asp
   370 375 380
   Ile Asp Ser Ala Glu Ile Gly Lys Asn Lys Gln Pro His Met Ser Val
   385 390 395 400
   Cys Ser Asp Val Lys Leu Ala Leu Gln Gly Ile Asn Lys Ile Leu Glu
   405 410 415
   Thr Thr Gly Ala Lys Leu Asn Leu Asp Tyr Ser Glu Trp Arg Gln Glu
   420 425 430
   Leu Asn Glu Gln Lys Leu Lys Phe Pro Leu Ser Tyr Lys Thr Phe Gly
   435 440 445
   Glu Ala Ile Pro Pro Gln Tyr Ala Ile Gln Val Leu Asp Glu Leu Thr
   450 455 460
   Gly Gly Asn Ala Ile Ile Ser Thr Gly Val Gly Gln His Gln Met Trp
   465 470 475 480
   Ala Ala Gln Phe Tyr Lys Tyr Lys Lys Pro Arg Gln Trp Leu Thr Ser
   485 490 495
   Gly Gly Leu Gly Ala Met Gly Phe Gly Leu Pro Ala Ala Ile Gly Ala
   500 505 510
   Ala Val Ala Asn Pro Glu Ala Val Val Val Asp Ile Asp Gly Asp Gly
   515 520 525
   Ser Phe Ile Met Asn Val Gln Glu Leu Ala Thr Met Arg Val Glu Asn
       530                 535                 540
   Leu Pro Val Lys Ile Leu Leu Leu Asn Asn Gln His Leu Gly Met Val
   545                 550                 555                 560
   Val Gln Trp Glu Asp Arg Phe Tyr Lys Ala Asn Arg Ala His Thr Tyr
                   565                 570                 575
   Leu Gly Asp Pro Ser Asn Glu Ser Glu Ile Phe Pro Asn Met Leu Lys
               580                 585                 590
   Phe Ala Glu Ala Cys Gly Ile Pro Ala Ala Arg Val Thr Lys Lys Glu
           595                 600                 605
   Asp Leu Lys Ala Ala Ile Gln Lys Met Leu Asp Thr Pro Gly Pro Tyr
       610                 615                 620
   Leu Leu Asp Val Ile Val Pro His Gln Glu His Val Leu Pro Met Ile
   625                 630                 635                 640
   Pro Ser Gly Gly Ala Phe Lys Asp Val Ile Thr Glu Gly Asp Gly Arg
                   645                 650                 655
   Thr Gln Tyr
<210> 13
   <211> 1980
   <212> DNA
   <213> Gossypium hirsutum
<220>
   <221> CDS
   <222> (1)..(1980)
<400> 13
   atg gcg gct gcc act gcg aac tcc gct ttg cct aag ctt tct acg tta          48
   Met Ala Ala Ala Thr Ala Asn Ser Ala Leu Pro Lys Leu Ser Thr Leu
   1               5                   10                  15
   act tcc tcc ttc aaa tct tcc ata ccc att tcc aaa tcc agc ctt ccc          96
   Thr Ser Ser Phe Lys Ser Ser Ile Pro Ile Ser Lys Ser Ser Leu Pro
               20                  25                  30
   ttc tcc aca acc cct caa aag ccc acc cct tac cgc tcc ttc gac gtt         144
   Phe Ser Thr Thr Pro Gln Lys Pro Thr Pro Tyr Arg Ser Phe Asp Val
           35                  40                  45
   tcc tgc tct ctt tct cat gca agc tct aac ccc aga tcc gcc gcc aca         192
   Ser Cys Ser Leu Ser His Ala Ser Ser Asn Pro Arg Ser Ala Ala Thr
       50                  55                  60
   tcc gtt acc cca aaa aat gct cct ccc cat gat ttc att tcc agg tat        240
   Ser Val Thr Pro Lys Asn Ala Pro Pro His Asp Phe Ile Ser Arg Tyr
   65                  70                  75                  80
   gcg gat gat gag ccc cgg aaa ggc gct gat atc ctg gtg gaa gcc ctg        288
   Ala Asp Asp Glu Pro Arg Lys Gly Ala Asp Ile Leu Val Glu Ala Leu
                   85                  90                  95
   gtt cgt gaa ggg gtc aag gat gtg ttt gcc tac cca ggt gga gct tca        336
   Val Arg Glu Gly Val Lys Asp Val Phe Ala Tyr Pro Gly Gly Ala Ser
               100                 105                 110
   atg gag atc cac cag gct tta acc cgc tca aaa atc atc cga aat gtc        384
   Met Glu Ile His Gln Ala Leu Thr Arg Ser Lys Ile Ile Arg Asn Val
           115                 120                 125
   ctt ccg cga cac gag caa ggt ggg gtc ttt gcc gcc gag ggc tac gcg        432
   Leu Pro Arg His Glu Gln Gly Gly Val Phe Ala Ala Glu Gly Tyr Ala
       130                 135                 140
   cgc tcc tct ggc att ccc ggc gtt tgc att gcg acg tct ggc cct ggg        480
   Arg Ser Ser Gly Ile Pro Gly Val Cys Ile Ala Thr Ser Gly Pro Gly
   145                 150                 155                 160
   gca acc aac ttg gtg agt ggt ctc gct gat gca atg ctc gat agt atc        528
   Ala Thr Asn Leu Val Ser Gly Leu Ala Asp Ala Met Leu Asp Ser Ile
                   165                 170                 175
   cct ctc gtg gcg atc act ggt caa gtc cct cgt cgg atg atc ggt acc        576
   Pro Leu Val Ala Ile Thr Gly Gln Val Pro Arg Arg Met Ile Gly Thr
               180                 185                 190
   gat gct ttc cag gaa act cca att gtt gag gta aca agg tct att acg        624
   Asp Ala Phe Gln Glu Thr Pro Ile Val Glu Val Thr Arg Ser Ile Thr
           195                 200                 205
   aag cat aat tat ctt gtt ctt gat gtg gat gat att cct agg att gtt        672
   Lys His Asn Tyr Leu Val Leu Asp Val Asp Asp Ile Pro Arg Ile Val
       210                 215                 220
   agt gag gct ttc ttt tta gct tcc tcg ggc agg ccg gga cct gtt ctg        720
   Ser Glu Ala Phe Phe Leu Ala Ser Ser Gly Arg Pro Gly Pro Val Leu
   225                 230                 235                 240
   att gat gtt cct aag gat ata caa cag caa ctt gct gtt cct aaa tgg        768
   Ile Asp Val Pro Lys Asp Ile Gln Gln Gln Leu Ala Val Pro Lys Trp
                   245                 250                 255
   aac cat tct ctt aga ttg cca ggg tat ttg tct agg ttg cct aag gct        816
   Asn His Ser Leu Arg Leu Pro Gly Tyr Leu Ser Arg Leu Pro Lys Ala
               260                 265                 270
   ccc ggt gag gct cat ctc gaa cag att gtt aga ttg gtt tct gag tct        864
   Pro Gly Glu Ala His Leu Glu Gln Ile Val Arg Leu Val Ser Glu Ser
           275                 280                 285
   aag aag cct gtt tta tat gtt ggt ggt ggg tgt ttg aac tct agt gag        912
   Lys Lys Pro Val Leu Tyr Val Gly Gly Gly Cys Leu Asn Ser Ser Glu
       290                 295                 300
   gag ttg aag agg ttt gtt gag ctt aca ggg ata cct gtt gca agt act        960
   Glu Leu Lys Arg Phe Val Glu Leu Thr Gly Ile Pro Val Ala Ser Thr
   305                 310                 315                 320
   ttg atg ggt ctt gga gcc ttt ccg att tcg gat gac ttg tcg tta caa        1008
   Leu Met Gly Leu Gly Ala Phe Pro Ile Ser Asp Asp Leu Ser Leu Gln
                   325                 330                 335
   atg ctt ggg atg cac gga act gtg tat gcc aat tat gct gtt gat aag        1056
   Met Leu Gly Met His Gly Thr Val Tyr Ala Asn Tyr Ala Val Asp Lys
               340                 345                 350
   agt gat ttg ttg ctt gct ttt gga gtg aga ttt gat gat agg gtg acg        1104
   Ser Asp Leu Leu Leu Ala Phe Gly Val Arg Phe Asp Asp Arg Val Thr
           355                 360                 365
   gga aaa ctt gag gct ttt gcc agc cgg gca aag att gtg cat atc gat        1152
   Gly Lys Leu Glu Ala Phe Ala Ser Arg Ala Lys Ile Val His Ile Asp
       370                 375                 380
   atc gat tct gcc gag att ggg aag aac aag cag cct cat gtg tca gtg        1200
   Ile Asp Ser Ala Glu Ile Gly Lys Asn Lys Gln Pro His Val Ser Val
   385                 390                 395                 400
   tgt tcc gat gtg aaa ttg gca ttg cag ggg ata aat aag ata ttg gag        1248
   Cys Ser Asp Val Lys Leu Ala Leu Gln Gly Ile Asn Lys Ile Leu Glu
                   405                 410                 415
   acc aag gta gca aag ctg aat ctt gat tat tcg gaa tgg agg cag gag        1296
   Thr Lys Val Ala Lys Leu Asn Leu Asp Tyr Ser Glu Trp Arg Gln Glu
               420                 425                 430
   tta aac gag cag aag ctg aaa ttc cct ttg agt tac aag acc ttt ggt        1344
   Leu Asn Glu Gln Lys Leu Lys Phe Pro Leu Ser Tyr Lys Thr Phe Gly
           435                 440                 445
   gaa gct att cca cct caa tat gca att cag gtt ctt gat gaa tta act        1392
   Glu Ala Ile Pro Pro Gln Tyr Ala Ile Gln Val Leu Asp Glu Leu Thr
       450                 455                 460
   ggc ggg aat gca att ata agt act ggt gtt ggt cag cat caa atg tgg        1440
   Gly Gly Asn Ala Ile Ile Ser Thr Gly Val Gly Gln His Gln Met Trp
   465                 470                 475                 480
   gct gct caa ttt tac aag tat aag aag cct cgt caa tgg tta aca tct        1488
   Ala Ala Gln Phe Tyr Lys Tyr Lys Lys Pro Arg Gln Trp Leu Thr Ser
                   485                 490                 495
   ggg gga ttg ggt gct atg gga ttt gga ttg cct gct gct att gga gct        1536
   Gly Gly Leu Gly Ala Met Gly Phe Gly Leu Pro Ala Ala Ile Gly Ala
               500                 505                 510
   gcc gtt gca aac cca gag gca gtc gtt gta gac atc gat ggt gat gga        1584
   Ala Val Ala Asn Pro Glu Ala Val Val Val Asp Ile Asp Gly Asp Gly
           515                 520                 525
   agt ttt atc atg aac gtg caa gag ttg gcg act atc cgt gtg gaa aat        1632
   Ser Phe Ile Met Asn Val Gln Glu Leu Ala Thr Ile Arg Val Glu Asn
       530                 535                 540
   ctt ccg gtt aag ata tta ttg ttg aat aat cag cat ttg ggt atg gtt        1680
   Leu Pro Val Lys Ile Leu Leu Leu Asn Asn Gln His Leu Gly Met Val
   545                 550                 555                 560
   gtt caa tgg gag gac cgg ttt tac aag gca aac agg gct cat aca tac        1728
   Val Gln Trp Glu Asp Arg Phe Tyr Lys Ala Asn Arg Ala His Thr Tyr
                   565                 570                 575
   ttg gga gac cca tcc aac gag tcg gaa ata ttc cca aat atg ttg aaa      1776
   Leu Gly Asp Pro Ser Asn Glu Ser Glu Ile Phe Pro Asn Met Leu Lys
               580                 585                 590
   ttt gct gaa gca tgc ggg ata cca gct gcc cgg gtg aca aag aaa gaa      1824
   Phe Ala Glu Ala Cys Gly Ile Pro Ala Ala Arg Val Thr Lys Lys Glu
           595                 600                 605
   gat cta aaa gca gca atg cag aaa atg ttg gac act cct gga cct tac      1872
   Asp Leu Lys Ala Ala Met Gln Lys Met Leu Asp Thr Pro Gly Pro Tyr
       610                 615                 620
   ttg ttg gat gtg att gtc cca cat caa gaa cat gtc ctg cct atg atc      1920
   Leu Leu Asp Val Ile Val Pro His Gln Glu His Val Leu Pro Met Ile
   625                 630                 635                 640
   ccc agt gga ggg gct ttc aaa gat gtg atc aca gag ggt gat gga aga      1968
   Pro Ser Gly Gly Ala Phe Lys Asp Val Ile Thr Glu Gly Asp Gly Arg
                   645                 650                 655
   aca caa tat tga                                                      1980
   Thr Gln Tyr
<210> 14
   <211> 659
   <212> PRT
   <213> Gossypium hirsutum
<400> 14
   Met Ala Ala Ala Thr Ala Asn Ser Ala Leu Pro Lys Leu Ser Thr Leu
   1               5                   10                  15
   Thr Ser Ser Phe Lys Ser Ser Ile Pro Ile Ser Lys Ser Ser Leu Pro
               20                  25                  30
   Phe Ser Thr Thr Pro Gln Lys Pro Thr Pro Tyr Arg Ser Phe Asp Val
           35                  40                  45
   Ser Cys Ser Leu Ser His Ala Ser Ser Asn Pro Arg Ser Ala Ala Thr
       50                  55                  60
   Ser Val Thr Pro Lys Asn Ala Pro Pro His Asp Phe Ile Ser Arg Tyr
   65                  70                  75                  80
   Ala Asp Asp Glu Pro Arg Lys Gly Ala Asp Ile Leu Val Glu Ala Leu
                   85                  90                  95
   Val Arg Glu Gly Val Lys Asp Val Phe Ala Tyr Pro Gly Gly Ala Ser
               100                 105                 110
   Met Glu Ile His Gln Ala Leu Thr Arg Ser Lys Ile Ile Arg Asn Val
   115 120 125
   Leu Pro Arg His Glu Gln Gly Gly Val Phe Ala Ala Glu Gly Tyr Ala
   130 135 140
   Arg Ser Ser Gly Ile Pro Gly Val Cys Ile Ala Thr Ser Gly Pro Gly
   145 150 155 160
   Ala Thr Asn Leu Val Ser Gly Leu Ala Asp Ala Met Leu Asp Ser Ile
   165 170 175
   Pro Leu Val Ala Ile Thr Gly Gln Val Pro Arg Arg Met Ile Gly Thr
   180 185 190
   Asp Ala Phe Gln Glu Thr Pro Ile Val Glu Val Thr Arg Ser Ile Thr
   195 200 205
   Lys His Asn Tyr Leu Val Leu Asp Val Asp Asp Ile Pro Arg Ile Val
   210 215 220
   Ser Glu Ala Phe Phe Leu Ala Ser Ser Gly Arg Pro Gly Pro Val Leu
   225 230 235 240
   Ile Asp Val Pro Lys Asp Ile Gln Gln Gln Leu Ala Val Pro Lys Trp
   245 250 255
   Asn His Ser Leu Arg Leu Pro Gly Tyr Leu Ser Arg Leu Pro Lys Ala
   260 265 270
   Pro Gly Glu Ala His Leu Glu Gln Ile Val Arg Leu Val Ser Glu Ser
   275 280 285
   Lys Lys Pro Val Leu Tyr Val Gly Gly Gly Cys Leu Asn Ser Ser Glu
   290 295 300
   Glu Leu Lys Arg Phe Val Glu Leu Thr Gly Ile Pro Val Ala Ser Thr
   305 310 315 320
   Leu Met Gly Leu Gly Ala Phe Pro Ile Ser Asp Asp Leu Ser Leu Gln
   325 330 335
   Met Leu Gly Met His Gly Thr Val Tyr Ala Asn Tyr Ala Val Asp Lys
   340 345 350
   Ser Asp Leu Leu Leu Ala Phe Gly Val Arg Phe Asp Asp Arg Val Thr
   355 360 365
   Gly Lys Leu Glu Ala Phe Ala Ser Arg Ala Lys Ile Val His Ile Asp
       370                 375                 380
   Ile Asp Ser Ala Glu Ile Gly Lys Asn Lys Gln Pro His Val Ser Val
   385                 390                 395                 400
   Cys Ser Asp Val Lys Leu Ala Leu Gln Gly Ile Asn Lys Ile Leu Glu
                   405                 410                 415
   Thr Lys Val Ala Lys Leu Asn Leu Asp Tyr Ser Glu Trp Arg Gln Glu
               420                 425                 430
   Leu Asn Glu Gln Lys Leu Lys Phe Pro Leu Ser Tyr Lys Thr Phe Gly
           435                 440                 445
   Glu Ala Ile Pro Pro Gln Tyr Ala Ile Gln Val Leu Asp Glu Leu Thr
       450                 455                 460
   Gly Gly Asn Ala Ile Ile Ser Thr Gly Val Gly Gln His Gln Met Trp
   465                 470                 475                 480
   Ala Ala Gln Phe Tyr Lys Tyr Lys Lys Pro Arg Gln Trp Leu Thr Ser
                   485                 490                 495
   Gly Gly Leu Gly Ala Met Gly Phe Gly Leu Pro Ala Ala Ile Gly Ala
               500                 505                 510
   Ala Val Ala Asn Pro Glu Ala Val Val Val Asp Ile Asp Gly Asp Gly
           515                 520                 525
   Ser Phe Ile Met Asn Val Gln Glu Leu Ala Thr Ile Arg Val Glu Asn
       530                 535                 540
   Leu Pro Val Lys Ile Leu Leu Leu Asn Asn Gln His Leu Gly Met Val
   545                 550                 555                 560
   Val Gln Trp Glu Asp Arg Phe Tyr Lys Ala Asn Arg Ala His Thr Tyr
                   565                 570                 575
   Leu Gly Asp Pro Ser Asn Glu Ser Glu Ile Phe Pro Asn Met Leu Lys
               580                 585                 590
   Phe Ala Glu Ala Cys Gly Ile Pro Ala Ala Arg Val Thr Lys Lys Glu
           595                 600                 605
   Asp Leu Lys Ala Ala Met Gln Lys Met Leu Asp Thr Pro Gly Pro Tyr
       610                 615                 620
   Leu Leu Asp Val Ile Val Pro His Gln Glu His Val Leu Pro Met Ile
   625                 630                 635                 640
   Pro Ser Gly Gly Ala Phe Lys Asp Val Ile Thr Glu Gly Asp Gly Arg
                   645                 650                 655
   Thr Gln Tyr
<210> 15
   <211> 1938
   <212> DNA
   <213> Glycine max
<220>
   <221> CDS
   <222> (1)..(1938)
<400> 15
   atg gcg gct ata act gcc cca aaa gct gcg ttc agc gtc ctc cct tca         48
   Met Ala Ala Ile Thr Ala Pro Lys Ala Ala Phe Ser Val Leu Pro Ser
   1               5                   10                  15
   tca tcc cat tcc ccg aat ccc ttt gtg cgt ttc gca atc cca tac agc         96
   Ser Ser His Ser Pro Asn Pro Phe Val Arg Phe Ala Ile Pro Tyr Ser
               20                  25                  30
   ccc cat cac tcc cag cgc cgt tct ctc aga atc tcc agc gcc cta tcc        144
   Pro His His Ser Gln Arg Arg Ser Leu Arg Ile Ser Ser Ala Leu Ser
           35                  40                  45
   gat gca acc acg aaa tcc tcc acc gcc gcc gct gaa gct ttc gcc tcc        192
   Asp Ala Thr Thr Lys Ser Ser Thr Ala Ala Ala Glu Ala Phe Ala Ser
       50                  55                  60
   cga ttc ggc ctc gat gag ccc cgc aag ggc gcc gac atc ctc gtg gag        240
   Arg Phe Gly Leu Asp Glu Pro Arg Lys Gly Ala Asp Ile Leu Val Glu
   65                  70                  75                  80
   gcg ctg gag cgg cag ggc gtg acg gac gtc ttc gcc tac ccc gga ggc        288
   Ala Leu Glu Arg Gln Gly Val Thr Asp Val Phe Ala Tyr Pro Gly Gly
                   85                  90                  95
   gcc tcc atg gag atc cac cag gcg ctc act cgc tca tcc tcc atc cgc        336
   Ala Ser Met Glu Ile His Gln Ala Leu Thr Arg Ser Ser Ser Ile Arg
               100                 105                 110
   aac gtc ctc cct cgc cac gaa cag ggc ggc gtc ttc gcc gcc gag ggc        384
   Asn Val Leu Pro Arg His Glu Gln Gly Gly Val Phe Ala Ala Glu Gly
           115                 120                 125
   tac gcc cgc tct tcc ggc ctc ccc ggc gtc tgc atc gcc acc tcc ggc        432
   Tyr Ala Arg Ser Ser Gly Leu Pro Gly Val Cys Ile Ala Thr Ser Gly
       130                 135                 140
   ccc ggc gcc acc aac ctc gtc tcc ggc ctc gcc gac gcc ttg ctt gac        480
   Pro Gly Ala Thr Asn Leu Val Ser Gly Leu Ala Asp Ala Leu Leu Asp
   145                 150                 155                 160
   agc gtc ccc ctc gtc gcc atc acc ggc cag gtc ccc cgc cgc atg atc        528
   Ser Val Pro Leu Val Ala Ile Thr Gly Gln Val Pro Arg Arg Met Ile
                   165                 170                 175
   ggc aca gac gcc ttc caa gaa acc ccc atc gtc gag gta aca cgt tcc        576
   Gly Thr Asp Ala Phe Gln Glu Thr Pro Ile Val Glu Val Thr Arg Ser
               180                 185                 190
   atc act aag cat aac tat ctc gtt ctc gat gtt gat gac att cct aga        624
   Ile Thr Lys His Asn Tyr Leu Val Leu Asp Val Asp Asp Ile Pro Arg
           195                 200                 205
   atc gtt aat gaa gcg ttt ttc tta gcc act tcg ggt aga cct ggc cct        672
   Ile Val Asn Glu Ala Phe Phe Leu Ala Thr Ser Gly Arg Pro Gly Pro
       210                 215                 220
   gtg tta att gat ata cct aaa gat att cag caa cag ctt gcg att cct        720
   Val Leu Ile Asp Ile Pro Lys Asp Ile Gln Gln Gln Leu Ala Ile Pro
   225                 230                 235                 240
   aat tgg gat caa cca att agg tta cct ggt tac acg tct agg ttg cct        768
   Asn Trp Asp Gln Pro Ile Arg Leu Pro Gly Tyr Thr Ser Arg Leu Pro
                   245                 250                 255
   aag tct cct aat gag aaa cat ttg gag ctt att gtg agg ttg gtt atg        816
   Lys Ser Pro Asn Glu Lys His Leu Glu Leu Ile Val Arg Leu Val Met
               260                 265                 270
   gaa tct aag aaa ccg gtt ttg tat gtt ggt ggt ggt tgt ttg aat tct        864
   Glu Ser Lys Lys Pro Val Leu Tyr Val Gly Gly Gly Cys Leu Asn Ser
           275                 280                 285
   agt gag gaa ttg cgg cgt ttt gtc gag ctc acc ggt gtt cct gtt gct        912
   Ser Glu Glu Leu Arg Arg Phe Val Glu Leu Thr Gly Val Pro Val Ala
       290                 295                 300
   agc act ttg atg ggt ctt ggt gct tat ccc att gct gat gat aat tct        960
   Ser Thr Leu Met Gly Leu Gly Ala Tyr Pro Ile Ala Asp Asp Asn Ser
   305                 310                 315                 320
   ctt cag atg ctt ggg atg cat ggg act gtt tat gct aat tat gct gtg       1008
   Leu Gln Met Leu Gly Met His Gly Thr Val Tyr Ala Asn Tyr Ala Val
                   325                 330                 335
   gat agg gct gat ctt ttg ctt gcg ttt ggg gtg agg ttt gat gac cgt       1056
   Asp Arg Ala Asp Leu Leu Leu Ala Phe Gly Val Arg Phe Asp Asp Arg
               340                 345                 350
   gtc acc ggg aag ctt gag gct ttt gcg agc cgg gct aag att gtt cac       1104
   Val Thr Gly Lys Leu Glu Ala Phe Ala Ser Arg Ala Lys Ile Val His
           355                 360                 365
   att gat att gat tca gct gag att ggg aag aac aag cag ccg cat gtg       1152
   Ile Asp Ile Asp Ser Ala Glu Ile Gly Lys Asn Lys Gln Pro His Val
       370                 375                 380
   tcg gtt tgt gcg gat ttg aag ttg gca ttg aag ggg att aat cgc gtg       1200
   Ser Val Cys Ala Asp Leu Lys Leu Ala Leu Lys Gly Ile Asn Arg Val
   385                 390                 395                 400
   tta gag agc aga ggg gtt gca ggg aag ctt gat ttt aga ggt tgg agg       1248
   Leu Glu Ser Arg Gly Val Ala Gly Lys Leu Asp Phe Arg Gly Trp Arg
                   405                 410                 415
   gaa gaa ctg aat gag cag aag cga agg ttt cct ttg agt tat aag act       1296
   Glu Glu Leu Asn Glu Gln Lys Arg Arg Phe Pro Leu Ser Tyr Lys Thr
               420                 425                 430
   ttt gag aaa gag att tct cct cag tat gct ata cag gtt ctt gac gag       1344
   Phe Glu Lys Glu Ile Ser Pro Gln Tyr Ala Ile Gln Val Leu Asp Glu
           435                 440                 445
   tta acc aat ggg gaa gct att gtg agt acc gga gtt gga cag cac cag       1392
   Leu Thr Asn Gly Glu Ala Ile Val Ser Thr Gly Val Gly Gln His Gln
       450                 455                 460
   atg tgg gct gct cag ttt tac aag tac aag agg cct agg cag tgg tta       1440
   Met Trp Ala Ala Gln Phe Tyr Lys Tyr Lys Arg Pro Arg Gln Trp Leu
   465                 470                 475                 480
   aca tcc ggt ggt ctt ggt gct atg ggg ttt gga ttg ccg gct gcc att       1488
   Thr Ser Gly Gly Leu Gly Ala Met Gly Phe Gly Leu Pro Ala Ala Ile
                   485                 490                 495
   ggt gcg gct gtg gct aat ccc ggt gcg gtt gtg gtt gac att gat ggt       1536
   Gly Ala Ala Val Ala Asn Pro Gly Ala Val Val Val Asp Ile Asp Gly
               500                 505                 510
   gat ggg agt ttt atg atg aat gtt cag gag ctg gcc acc atc aag gtg       1584
   Asp Gly Ser Phe Met Met Asn Val Gln Glu Leu Ala Thr Ile Lys Val
           515                 520                 525
   gag aag ctc cct gtt aag ata ttg ttg ttg aat aat caa cac ttg ggt       1632
   Glu Lys Leu Pro Val Lys Ile Leu Leu Leu Asn Asn Gln His Leu Gly
       530                 535                 540
   atg gtt gtt cag tgg gag gat cgg ttc tat aag tct aac agg gct cac       1680
   Met Val Val Gln Trp Glu Asp Arg Phe Tyr Lys Ser Asn Arg Ala His
   545                 550                 555                 560
   aca tat ctg gga gac ccc tct aat gag aat gcc ata tac ccc aat atg       1728
   Thr Tyr Leu Gly Asp Pro Ser Asn Glu Asn Ala Ile Tyr Pro Asn Met
                   565                 570                 575
   ttg aag ttt gct gat gct tgt ggg ata ccg gca gct cgt gtg acc aag       1776
   Leu Lys Phe Ala Asp Ala Cys Gly Ile Pro Ala Ala Arg Val Thr Lys
               580                 585                 590
   aaa gaa gac ctt aga gcg gca att cag aaa atg ctg gag acc cct ggc       1824
   Lys Glu Asp Leu Arg Ala Ala Ile Gln Lys Met Leu Glu Thr Pro Gly
           595                 600                 605
   ccc tat ctt ctt gat gtc att gta ccc cat caa gag cat gtc ttg ccc       1872
   Pro Tyr Leu Leu Asp Val Ile Val Pro His Gln Glu His Val Leu Pro
       610                 615                 620
   atg att cct agc aat gga acc ttc cag gac gtg ata act gag ggt gat       1920
   Met Ile Pro Ser Asn Gly Thr Phe Gln Asp Val Ile Thr Glu Gly Asp
   625                 630                 635                 640
   ggc agg aca agt tac tga                                               1938
   Gly Arg Thr Ser Tyr
                   645
<210> 16
   <211> 645
   <212> PRT
   <213> Glycine max
<400> 16
   Met Ala Ala Ile Thr Ala Pro Lys Ala Ala Phe Ser Val Leu Pro Ser
   1               5                   10                  15
   Ser Ser His Ser Pro Asn Pro Phe Val Arg Phe Ala Ile Pro Tyr Ser
               20                  25                  30
   Pro His His Ser Gln Arg Arg Ser Leu Arg Ile Ser Ser Ala Leu Ser
           35                  40                  45
   Asp Ala Thr Thr Lys Ser Ser Thr Ala Ala Ala Glu Ala Phe Ala Ser
       50                  55                  60
   Arg Phe Gly Leu Asp Glu Pro Arg Lys Gly Ala Asp Ile Leu Val Glu
   65                  70                  75                  80
   Ala Leu Glu Arg Gln Gly Val Thr Asp Val Phe Ala Tyr Pro Gly Gly
                   85                  90                  95
   Ala Ser Met Glu Ile His Gln Ala Leu Thr Arg Ser Ser Ser Ile Arg
               100                 105                 110
   Asn Val Leu Pro Arg His Glu Gln Gly Gly Val Phe Ala Ala Glu Gly
           115                 120                 125
   Tyr Ala Arg Ser Ser Gly Leu Pro Gly Val Cys Ile Ala Thr Ser Gly
       130                 135                 140
   Pro Gly Ala Thr Asn Leu Val Ser Gly Leu Ala Asp Ala Leu Leu Asp
   145                 150                 155                 160
   Ser Val Pro Leu Val Ala Ile Thr Gly Gln Val Pro Arg Arg Met Ile
                   165                 170                 175
   Gly Thr Asp Ala Phe Gln Glu Thr Pro Ile Val Glu Val Thr Arg Ser
               180                 185                 190
   Ile Thr Lys His Asn Tyr Leu Val Leu Asp Val Asp Asp Ile Pro Arg
           195                 200                 205
   Ile Val Asn Glu Ala Phe Phe Leu Ala Thr Ser Gly Arg Pro Gly Pro
       210                 215                 220
   Val Leu Ile Asp Ile Pro Lys Asp Ile Gln Gln Gln Leu Ala Ile Pro
   225 230 235 240
   Asn Trp Asp Gln Pro Ile Arg Leu Pro Gly Tyr Thr Ser Arg Leu Pro
   245 250 255
   Lys Ser Pro Asn Glu Lys His Leu Glu Leu Ile Val Arg Leu Val Met
   260 265 270
   Glu Ser Lys Lys Pro Val Leu Tyr Val Gly Gly Gly Cys Leu Asn Ser
   275 280 285
   Ser Glu Glu Leu Arg Arg Phe Val Glu Leu Thr Gly Val Pro Val Ala
   290 295 300
   Ser Thr Leu Met Gly Leu Gly Ala Tyr Pro Ile Ala Asp Asp Asn Ser
   305 310 315 320
   Leu Gln Met Leu Gly Met His Gly Thr Val Tyr Ala Asn Tyr Ala Val
   325 330 335
   Asp Arg Ala Asp Leu Leu Leu Ala Phe Gly Val Arg Phe Asp Asp Arg
   340 345 350
   Val Thr Gly Lys Leu Glu Ala Phe Ala Ser Arg Ala Lys Ile Val His
   355 360 365
   Ile Asp Ile Asp Ser Ala Glu Ile Gly Lys Asn Lys Gln Pro His Val
   370 375 380
   Ser Val Cys Ala Asp Leu Lys Leu Ala Leu Lys Gly Ile Asn Arg Val
   385 390 395 400
   Leu Glu Ser Arg Gly Val Ala Gly Lys Leu Asp Phe Arg Gly Trp Arg
   405 410 415
   Glu Glu Leu Asn Glu Gln Lys Arg Arg Phe Pro Leu Ser Tyr Lys Thr
   420 425 430
   Phe Glu Lys Glu Ile Ser Pro Gln Tyr Ala Ile Gln Val Leu Asp Glu
   435 440 445
   Leu Thr Asn Gly Glu Ala Ile Val Ser Thr Gly Val Gly Gln His Gln
   450 455 460
   Met Trp Ala Ala Gln Phe Tyr Lys Tyr Lys Arg Pro Arg Gln Trp Leu
   465 470 475 480
   Thr Ser Gly Gly Leu Gly Ala Met Gly Phe Gly Leu Pro Ala Ala Ile
                   485                 490                 495
   Gly Ala Ala Val Ala Asn Pro Gly Ala Val Val Val Asp Ile Asp Gly
               500                 505                 510
   Asp Gly Ser Phe Met Met Asn Val Gln Glu Leu Ala Thr Ile Lys Val
           515                 520                 525
   Glu Lys Leu Pro Val Lys Ile Leu Leu Leu Asn Asn Gln His Leu Gly
       530                 535                 540
   Met Val Val Gln Trp Glu Asp Arg Phe Tyr Lys Ser Asn Arg Ala His
   545                 550                 555                 560
   Thr Tyr Leu Gly Asp Pro Ser Asn Glu Asn Ala Ile Tyr Pro Asn Met
                   565                 570                 575
   Leu Lys Phe Ala Asp Ala Cys Gly Ile Pro Ala Ala Arg Val Thr Lys
               580                 585                 590
   Lys Glu Asp Leu Arg Ala Ala Ile Gln Lys Met Leu Glu Thr Pro Gly
           595                 600                 605
   Pro Tyr Leu Leu Asp Val Ile Val Pro His Gln Glu His Val Leu Pro
       610                 615                 620
   Met Ile Pro Ser Asn Gly Thr Phe Gln Asp Val Ile Thr Glu Gly Asp
   625                 630                 635                 640
   Gly Arg Thr Ser Tyr
                   645
<210> 17
   <211> 1962
   <212> DNA
   <213> Glycine max
<220>
   <221> CDS
   <222> (1)..(1962)
<400> 17
   atg gcg gca acc act gcc cca aaa cct gcg ttc acc gcc ctc cct tct         48
   Met Ala Ala Thr Thr Ala Pro Lys Pro Ala Phe Thr Ala Leu Pro Ser
   1               5                   10                  15
   tca tca tca tca tct tcc cag aag ccc ttt ctg cgt ctc gcc ctc caa         96
   Ser Ser Ser Ser Ser Ser Gln Lys Pro Phe Leu Arg Leu Ala Leu Gln
               20                  25                  30
   ttt ccc tcc ctc cca aac agt tcc tat cac tcc caa cgc cct tct ctc        144
   Phe Pro Ser Leu Pro Asn Ser Ser Tyr His Ser Gln Arg Pro Ser Leu
           35                  40                  45
   aaa atc tcc agc gcc ctc tcc gat gca acc gcg aaa acc acc acc gcc        192
   Lys Ile Ser Ser Ala Leu Ser Asp Ala Thr Ala Lys Thr Thr Thr Ala
       50                  55                  60
   gcc gcc gcc gaa gat ttt gtc tcc cga ttc ggc ctg gag gag ccc cgc        240
   Ala Ala Ala Glu Asp Phe Val Ser Arg Phe Gly Leu Glu Glu Pro Arg
   65                  70                  75                  80
   aag ggc gcc gac atc ctc gtg gag gcg ctg gag cgg cag ggc gtg acg        288
   Lys Gly Ala Asp Ile Leu Val Glu Ala Leu Glu Arg Gln Gly Val Thr
                   85                  90                  95
   gac gtg ttc gcc tac ccc ggt ggt gct tcc atg gag atc cac cag gca        336
   Asp Val Phe Ala Tyr Pro Gly Gly Ala Ser Met Glu Ile His Gln Ala
               100                 105                 110
   ctc acc cgc tcc gcc tcc atc cgc aac gtc ctc ccc cgc cac gag cag        384
   Leu Thr Arg Ser Ala Ser Ile Arg Asn Val Leu Pro Arg His Glu Gln
           115                 120                 125
   ggt ggc gtc ttc gcc gcc gag ggc tac gcc cgt tcc tcc ggc atc ccc        432
   Gly Gly Val Phe Ala Ala Glu Gly Tyr Ala Arg Ser Ser Gly Ile Pro
       130                 135                 140
   ggc gtc tgc atc gcc acc tcc ggc ccc ggc gcc acc aac ctc gtc agc        480
   Gly Val Cys Ile Ala Thr Ser Gly Pro Gly Ala Thr Asn Leu Val Ser
   145                 150                 155                 160
   ggc ctc gcc gac gcc atg ctc gac agc gtc ccc ctc gtc gcc atc acc        528
   Gly Leu Ala Asp Ala Met Leu Asp Ser Val Pro Leu Val Ala Ile Thr
                   165                 170                 175
   ggc cag gtc ccc cgc cgc atg atc ggc acc gac gcc ttc caa gaa acc        576
   Gly Gln Val Pro Arg Arg Met Ile Gly Thr Asp Ala Phe Gln Glu Thr
               180                 185                 190
   ccc atc gtc gag gta acg cgt tcc gtc aca aag cat aac tat ctc gtt        624
   Pro Ile Val Glu Val Thr Arg Ser Val Thr Lys His Asn Tyr Leu Val
           195                 200                 205
   cta gat gtt gat gat att cct aga atc gtt aat gaa gcg ttt ttc tta        672
   Leu Asp Val Asp Asp Ile Pro Arg Ile Val Asn Glu Ala Phe Phe Leu
       210                 215                 220
   gcc act tcg ggt aga cct ggc cct gtg tta att gat ata ccc aaa gat        720
   Ala Thr Ser Gly Arg Pro Gly Pro Val Leu Ile Asp Ile Pro Lys Asp
   225                 230                 235                 240
   att cag caa cag ttt gcg att cct aat tgg gat caa cca att agg tta        768
   Ile Gln Gln Gln Phe Ala Ile Pro Asn Trp Asp Gln Pro Ile Arg Leu
                   245                 250                 255
   cct ggt tac atg tct agg ttg cct aag tct ccc aat gag aat cat ttg        816
   Pro Gly Tyr Met Ser Arg Leu Pro Lys Ser Pro Asn Glu Asn His Leu
               260                 265                 270
   gag ctt att gtg agg ttg gtt atg gaa tct aag aaa cct gtt ttg tat        864
   Glu Leu Ile Val Arg Leu Val Met Glu Ser Lys Lys Pro Val Leu Tyr
           275                 280                 285
   gtt ggt ggt ggt tgt ttg aat tct agt gaa gaa ttg cgg cgt ttt gtc          912
   Val Gly Gly Gly Cys Leu Asn Ser Ser Glu Glu Leu Arg Arg Phe Val
       290                 295                 300
   gag ctc act ggt gtt cct gtt gct agt act ttg atg ggt ctt ggt gct          960
   Glu Leu Thr Gly Val Pro Val Ala Ser Thr Leu Met Gly Leu Gly Ala
   305                 310                 315                 320
   tat ccc att gct gat gag aat tct ctt cag atg ctt ggg atg cac ggg         1008
   Tyr Pro Ile Ala Asp Glu Asn Ser Leu Gln Met Leu Gly Met His Gly
                   325                 330                 335
   act gtt tat gct aat tat gct gtg gac aag gct gat att ttg ctt gcg         1056
   Thr Val Tyr Ala Asn Tyr Ala Val Asp Lys Ala Asp Ile Leu Leu Ala
               340                 345                 350
   ttt ggg gtg agg ttt gat gac cgt gtc acg ggg aag ctc gag gct ttt         1104
   Phe Gly Val Arg Phe Asp Asp Arg Val Thr Gly Lys Leu Glu Ala Phe
           355                 360                 365
   gcg agc cgg gct aag ata gtt cac att gat att gat tcg gca gag att         1152
   Ala Ser Arg Ala Lys Ile Val His Ile Asp Ile Asp Ser Ala Glu Ile
       370                 375                 380
   gga aag aac aag cag ccg cat gtt tcg gtt tgc gct gat ttg aag ctg         1200
   Gly Lys Asn Lys Gln Pro His Val Ser Val Cys Ala Asp Leu Lys Leu
   385                 390                 395                 400
   gcg ttg aag ggg att aat cac atg ttg gag agc aga ggg gtt ggg ggg         1248
   Ala Leu Lys Gly Ile Asn His Met Leu Glu Ser Arg Gly Val Gly Gly
                   405                 410                 415
   aag ctt gat ttt aga ggt tgg agg gaa gag ctg aat gag cag aag cga         1296
   Lys Leu Asp Phe Arg Gly Trp Arg Glu Glu Leu Asn Glu Gln Lys Arg
               420                 425                 430
   agg ttt cct ttg agc tat aag act ttt gag gat gag att tct cct cag         1344
   Arg Phe Pro Leu Ser Tyr Lys Thr Phe Glu Asp Glu Ile Ser Pro Gln
           435                 440                 445
   tat gct ata cag gtt ctt gat gag ttg act aat ggg gat gct att gtg         1392
   Tyr Ala Ile Gln Val Leu Asp Glu Leu Thr Asn Gly Asp Ala Ile Val
       450                 455                 460
   agt act gga gtt gga cag cac cag atg tgg gct gct cag ttt tac aag         1440
   Ser Thr Gly Val Gly Gln His Gln Met Trp Ala Ala Gln Phe Tyr Lys
   465                 470                 475                 480
   tac aag agg cct agg cag tgg tta aca tcc ggt ggt ctt ggt gct atg         1488
   Tyr Lys Arg Pro Arg Gln Trp Leu Thr Ser Gly Gly Leu Gly Ala Met
                   485                 490                 495
   ggg ttt gga ttg ccg gct gcc atc ggt gcg gct gtg gct aat ccg ggt         1536
   Gly Phe Gly Leu Pro Ala Ala Ile Gly Ala Ala Val Ala Asn Pro Gly
               500                 505                 510
   gcg gtt gtg gtt gac att gat ggt gat ggg agt ttt ata atg aat gtt         1584
   Ala Val Val Val Asp Ile Asp Gly Asp Gly Ser Phe Ile Met Asn Val
           515                 520                 525
   cag gag ctg gcc act atc aag gta gag aag ctc ccg gtt aag ata ttg         1632
   Gln Glu Leu Ala Thr Ile Lys Val Glu Lys Leu Pro Val Lys Ile Leu
       530                 535                 540
   ttg ttg aat aat caa cac ttg ggt atg gtt gtt cag tgg gag gat cgg      1680
   Leu Leu Asn Asn Gln His Leu Gly Met Val Val Gln Trp Glu Asp Arg
   545                 550                 555                 560
   ttc tat aag tct aac agg gct cac acg tat ctg gga gac ccc tct aat      1728
   Phe Tyr Lys Ser Asn Arg Ala His Thr Tyr Leu Gly Asp Pro Ser Asn
                   565                 570                 575
   gag aat gcc ata ttt cct gat atg ttg aag ttt gct gat gct tgc ggg      1776
   Glu Asn Ala Ile Phe Pro Asp Met Leu Lys Phe Ala Asp Ala Cys Gly
               580                 585                 590
   ata ccg gca gct cgt gtg acc aag aaa gaa gac ctt aga gcg gca att      1824
   Ile Pro Ala Ala Arg Val Thr Lys Lys Glu Asp Leu Arg Ala Ala Ile
           595                 600                 605
   cag aaa atg ttg gac acc cct ggc ccc tat ctt ctt gat gtc att gta      1872
   Gln Lys Met Leu Asp Thr Pro Gly Pro Tyr Leu Leu Asp Val Ile Val
       610                 615                 620
   ccc cat caa gag cat gtc ttg ccc atg att cct agc aat gga acc ttc      1920
   Pro His Gln Glu His Val Leu Pro Met Ile Pro Ser Asn Gly Thr Phe
   625                 630                 635                 640
   cag gac gtg ata act gag ggt gat ggc agg aca agt tac tga              1962
   Gln Asp Val Ile Thr Glu Gly Asp Gly Arg Thr Ser Tyr
                   645                 650
<210> 18
   <211> 653
   <212> PRT
   <213> Glycine max
<400> 18
   Met Ala Ala Thr Thr Ala Pro Lys Pro Ala Phe Thr Ala Leu Pro Ser
   1               5                   10                  15
   Ser Ser Ser Ser Ser Ser Gln Lys Pro Phe Leu Arg Leu Ala Leu Gln
               20                  25                  30
   Phe Pro Ser Leu Pro Asn Ser Ser Tyr His Ser Gln Arg Pro Ser Leu
           35                  40                  45
   Lys Ile Ser Ser Ala Leu Ser Asp Ala Thr Ala Lys Thr Thr Thr Ala
       50                  55                  60
   Ala Ala Ala Glu Asp Phe Val Ser Arg Phe Gly Leu Glu Glu Pro Arg
   65                  70                  75                  80
   Lys Gly Ala Asp Ile Leu Val Glu Ala Leu Glu Arg Gln Gly Val Thr
                   85                  90                  95
   Asp Val Phe Ala Tyr Pro Gly Gly Ala Ser Met Glu Ile His Gln Ala
               100                 105                 110
   Leu Thr Arg Ser Ala Ser Ile Arg Asn Val Leu Pro Arg His Glu Gln
           115                 120                 125
   Gly Gly Val Phe Ala Ala Glu Gly Tyr Ala Arg Ser Ser Gly Ile Pro
       130                 135                 140
   Gly Val Cys Ile Ala Thr Ser Gly Pro Gly Ala Thr Asn Leu Val Ser
   145                 150                 155                 160
   Gly Leu Ala Asp Ala Met Leu Asp Ser Val Pro Leu Val Ala Ile Thr
                   165                 170                 175
   Gly Gln Val Pro Arg Arg Met Ile Gly Thr Asp Ala Phe Gln Glu Thr
               180                 185                 190
   Pro Ile Val Glu Val Thr Arg Ser Val Thr Lys His Asn Tyr Leu Val
           195                 200                 205
   Leu Asp Val Asp Asp Ile Pro Arg Ile Val Asn Glu Ala Phe Phe Leu
       210                 215                 220
   Ala Thr Ser Gly Arg Pro Gly Pro Val Leu Ile Asp Ile Pro Lys Asp
   225                 230                 235                 240
   Ile Gln Gln Gln Phe Ala Ile Pro Asn Trp Asp Gln Pro Ile Arg Leu
                   245                 250                 255
   Pro Gly Tyr Met Ser Arg Leu Pro Lys Ser Pro Asn Glu Asn His Leu
               260                 265                 270
   Glu Leu Ile Val Arg Leu Val Met Glu Ser Lys Lys Pro Val Leu Tyr
           275                 280                 285
   Val Gly Gly Gly Cys Leu Asn Ser Ser Glu Glu Leu Arg Arg Phe Val
       290                 295                 300
   Glu Leu Thr Gly Val Pro Val Ala Ser Thr Leu Met Gly Leu Gly Ala
   305                 310                 315                 320
   Tyr Pro Ile Ala Asp Glu Asn Ser Leu Gln Met Leu Gly Met His Gly
                   325                 330                 335
   Thr Val Tyr Ala Asn Tyr Ala Val Asp Lys Ala Asp Ile Leu Leu Ala
               340                 345                 350
   Phe Gly Val Arg Phe Asp Asp Arg Val Thr Gly Lys Leu Glu Ala Phe
   355 360 365
   Ala Ser Arg Ala Lys Ile Val His Ile Asp Ile Asp Ser Ala Glu Ile
   370 375 380
   Gly Lys Asn Lys Gln Pro His Val Ser Val Cys Ala Asp Leu Lys Leu
   385 390 395 400
   Ala Leu Lys Gly Ile Asn His Met Leu Glu Ser Arg Gly Val Gly Gly
   405 410 415
   Lys Leu Asp Phe Arg Gly Trp Arg Glu Glu Leu Asn Glu Gln Lys Arg
   420 425 430
   Arg Phe Pro Leu Ser Tyr Lys Thr Phe Glu Asp Glu Ile Ser Pro Gln
   435 440 445
   Tyr Ala Ile Gln Val Leu Asp Glu Leu Thr Asn Gly Asp Ala Ile Val
   450 455 460
   Ser Thr Gly Val Gly Gln His Gln Met Trp Ala Ala Gln Phe Tyr Lys
   465 470 475 480
   Tyr Lys Arg Pro Arg Gln Trp Leu Thr Ser Gly Gly Leu Gly Ala Met
   485 490 495
   Gly Phe Gly Leu Pro Ala Ala Ile Gly Ala Ala Val Ala Asn Pro Gly
   500 505 510
   Ala Val Val Val Asp Ile Asp Gly Asp Gly Ser Phe Ile Met Asn Val
   515 520 525
   Gln Glu Leu Ala Thr Ile Lys Val Glu Lys Leu Pro Val Lys Ile Leu
   530 535 540
   Leu Leu Asn Asn Gln His Leu Gly Met Val Val Gln Trp Glu Asp Arg
   545 550 555 560
   Phe Tyr Lys Ser Asn Arg Ala His Thr Tyr Leu Gly Asp Pro Ser Asn
   565 570 575
   Glu Asn Ala Ile Phe Pro Asp Met Leu Lys Phe Ala Asp Ala Cys Gly
   580 585 590
   Ile Pro Ala Ala Arg Val Thr Lys Lys Glu Asp Leu Arg Ala Ala Ile
   595 600 605
   Gln Lys Met Leu Asp Thr Pro Gly Pro Tyr Leu Leu Asp Val Ile Val
       610                 615                 620
   Pro His Gln Glu His Val Leu Pro Met Ile Pro Ser Asn Gly Thr Phe
   625                 630                 635                 640
   Gln Asp Val Ile Thr Glu Gly Asp Gly Arg Thr Ser Tyr
                   645                 650
<210> 19
   <211> 1971
   <212> DNA
   <213> Glycine max
<220>
   <221> CDS
   <222> (1)..(1971)
<400> 19
   atg gcg gcc aca gct tcc aga acc acc cca ttc tct tct ccc tct tcc        48
   Met Ala Ala Thr Ala Ser Arg Thr Thr Pro Phe Ser Ser Pro Ser Ser
   1               5                   10                  15
   tct tca cac ccc acc ttc ccc aaa cgc att gct aga tcc acc ctc cct        96
   Ser Ser His Pro Thr Phe Pro Lys Arg Ile Ala Arg Ser Thr Leu Pro
               20                  25                  30
   ctc tct cat caa acc ttc att aaa ccc aac cac gcg ctc aaa atc aaa       144
   Leu Ser His Gln Thr Phe Ile Lys Pro Asn His Ala Leu Lys Ile Lys
           35                  40                  45
   tgt tcc atc tcc aaa ccc ccc tcg ccg ccg gcc acc acc acc act gca       192
   Cys Ser Ile Ser Lys Pro Pro Ser Pro Pro Ala Thr Thr Thr Thr Ala
       50                  55                  60
   gcg ccg gca aca tca gag ccc ttc gtg tcg cgg ttc gcc tcc gct gaa       240
   Ala Pro Ala Thr Ser Glu Pro Phe Val Ser Arg Phe Ala Ser Ala Glu
   65                  70                  75                  80
   ccg cgc aag ggc tcg gac atc ctt gtg gag gcc ctt gag cgg ctg ggc       288
   Pro Arg Lys Gly Ser Asp Ile Leu Val Glu Ala Leu Glu Arg Leu Gly
                   85                  90                  95
   gtg act aat gtc ttc gcc tac ccc ggc ggc gcg tcg atg gag atc cac       336
   Val Thr Asn Val Phe Ala Tyr Pro Gly Gly Ala Ser Met Glu Ile His
               100                 105                 110
   cag gct ctc acg cgc tcc tcc acc atc cgc aac gtc ctc cct cgc cac       384
   Gln Ala Leu Thr Arg Ser Ser Thr Ile Arg Asn Val Leu Pro Arg His
           115                 120                 125
   gag cag ggc ggc atc ttc gcc gcc gag ggc tac gcg cgc tcc tct ggc       432
   Glu Gln Gly Gly Ile Phe Ala Ala Glu Gly Tyr Ala Arg Ser Ser Gly
       130                 135                 140
   ctc ccc ggc gtc tgc atc gcc acc tcc ggc ccc ggc gcc acc aac ctc       480
   Leu Pro Gly Val Cys Ile Ala Thr Ser Gly Pro Gly Ala Thr Asn Leu
   145                 150                 155                 160
   gtc agc ggc ctc gcc gac gcc tta atg gac agc gtc ccc gtc gtc gcc       528
   Val Ser Gly Leu Ala Asp Ala Leu Met Asp Ser Val Pro Val Val Ala
                   165                 170                 175
   atc acc ggc cag gtc ccc cgg cgc atg att ggc acc gac gcc ttc caa       576
   Ile Thr Gly Gln Val Pro Arg Arg Met Ile Gly Thr Asp Ala Phe Gln
               180                 185                 190
   gaa acc ccc atc gtc gaa gtc acc aga tcc atc acc aag cac aac tac       624
   Glu Thr Pro Ile Val Glu Val Thr Arg Ser Ile Thr Lys His Asn Tyr
           195                 200                 205
   ctc atc ctc gac gtc gac gac att ccc cgc atc gtc gcc gaa gcc ttc       672
   Leu Ile Leu Asp Val Asp Asp Ile Pro Arg Ile Val Ala Glu Ala Phe
       210                 215                 220
   ttc gtc gcc acc tcc ggc cgc ccc ggc ccg gtc ctc atc gac atc ccc       720
   Phe Val Ala Thr Ser Gly Arg Pro Gly Pro Val Leu Ile Asp Ile Pro
   225                 230                 235                 240
   aaa gac gtc caa cag caa ctc gcc gtg cct aat tgg gac gag ccc atc       768
   Lys Asp Val Gln Gln Gln Leu Ala Val Pro Asn Trp Asp Glu Pro Ile
                   245                 250                 255
   aac ctc ccc ggg tac ctc gcc agg ctt ccc agg ccc ccc acc gag gcc       816
   Asn Leu Pro Gly Tyr Leu Ala Arg Leu Pro Arg Pro Pro Thr Glu Ala
               260                 265                 270
   caa tta gag cac att gtc aga ctc ata acc gag gcc caa aag ccc gtc       864
   Gln Leu Glu His Ile Val Arg Leu Ile Thr Glu Ala Gln Lys Pro Val
           275                 280                 285
   ctc tac gtc gga ggt ggc agt ttg aat tcc agt gat gaa ttg aga cgc       912
   Leu Tyr Val Gly Gly Gly Ser Leu Asn Ser Ser Asp Glu Leu Arg Arg
       290                 295                 300
   ttt gtg gaa ctg act ggt att ccc gtt gct agc act tta atg ggc ctt       960
   Phe Val Glu Leu Thr Gly Ile Pro Val Ala Ser Thr Leu Met Gly Leu
   305                 310                 315                 320
   gga act tat cct att ggg gat gaa tat tcc ctt caa atg ttg ggt atg      1008
   Gly Thr Tyr Pro Ile Gly Asp Glu Tyr Ser Leu Gln Met Leu Gly Met
                   325                 330                 335
   cat ggt act gtt tat gct aac tat gct gtt gac aat agt gat ttg ttg      1056
   His Gly Thr Val Tyr Ala Asn Tyr Ala Val Asp Asn Ser Asp Leu Leu
               340                 345                 350
   ctt gcc ttt ggg gta agg ttt gat gac cgt gtt act ggg aag ctt gag      1104
   Leu Ala Phe Gly Val Arg Phe Asp Asp Arg Val Thr Gly Lys Leu Glu
           355                 360                 365
   gct ttt gct agt agg gct aag att gtt cac att gat att gat tct gct      1152
   Ala Phe Ala Ser Arg Ala Lys Ile Val His Ile Asp Ile Asp Ser Ala
       370                 375                 380
   gag att gga aag aat aag cag gcg cat gtg tcg gtt tgc gcg gat ttg      1200
   Glu Ile Gly Lys Asn Lys Gln Ala His Val Ser Val Cys Ala Asp Leu
   385                 390                 395                 400
   aag ctg gcc ttg cag ggg att aat atg att ttg gag aaa aaa aga gtg      1248
   Lys Leu Ala Leu Gln Gly Ile Asn Met Ile Leu Glu Lys Lys Arg Val
                   405                 410                 415
   ggg ggt ggt aag ctt gat ctt gga ggc tgg agg gaa gag att aat gtg      1296
   Gly Gly Gly Lys Leu Asp Leu Gly Gly Trp Arg Glu Glu Ile Asn Val
               420                 425                 430
   cag aaa cac aag ttt cca ttg ggt tac aaa aca ttc cag gac gcc att      1344
   Gln Lys His Lys Phe Pro Leu Gly Tyr Lys Thr Phe Gln Asp Ala Ile
           435                 440                 445
   tcc ccg cag cat gct att gag gtt ctt gat gag ttg aca aat ggg gat      1392
   Ser Pro Gln His Ala Ile Glu Val Leu Asp Glu Leu Thr Asn Gly Asp
       450                 455                 460
   gct att gtt agt act ggg gtt ggg cag cat caa atg tgg gct gca cag      1440
   Ala Ile Val Ser Thr Gly Val Gly Gln His Gln Met Trp Ala Ala Gln
   465                 470                 475                 480
   ttt tac aag tac aag aga ccg agg cag tgg ttg acc tcg ggg ggt ctt      1488
   Phe Tyr Lys Tyr Lys Arg Pro Arg Gln Trp Leu Thr Ser Gly Gly Leu
                   485                 490                 495
   gga gcc atg ggt ttt gga ttg cct gcg gct att ggc gct gct gtt gct      1536
   Gly Ala Met Gly Phe Gly Leu Pro Ala Ala Ile Gly Ala Ala Val Ala
               500                 505                 510
   aac ccc ggc gct gtt gtg gtt gac att gat gga gat ggt agt ttc atc      1584
   Asn Pro Gly Ala Val Val Val Asp Ile Asp Gly Asp Gly Ser Phe Ile
           515                 520                 525
   atg aat gtt caa gaa ttg gca act ata aga gtg gag aat ctt cct gtt      1632
   Met Asn Val Gln Glu Leu Ala Thr Ile Arg Val Glu Asn Leu Pro Val
       530                 535                 540
   aag ata ttg ttg ttg aat aat cag cat ttg ggc atg gtg gtt cag tgg      1680
   Lys Ile Leu Leu Leu Asn Asn Gln His Leu Gly Met Val Val Gln Trp
   545                 550                 555                 560
   gag gat cgg ttc tac aag tcc aat aga gct cac acc tat ctt gga gac      1728
   Glu Asp Arg Phe Tyr Lys Ser Asn Arg Ala His Thr Tyr Leu Gly Asp
                   565                 570                 575
   ccg tct agc gag aat gag ata ttc cca aac atg ctc aag ttt gct gac      1776
   Pro Ser Ser Glu Asn Glu Ile Phe Pro Asn Met Leu Lys Phe Ala Asp
               580                 585                 590
   gct tgt ggg ata ccg gca gcg cgt gtg acg aag aag gaa gag ctt aga      1824
   Ala Cys Gly Ile Pro Ala Ala Arg Val Thr Lys Lys Glu Glu Leu Arg
           595                 600                 605
   gca gaa att cag aaa atg ttg gac acc cct ggc ccc tac ctt ctt gat      1872
   Ala Glu Ile Gln Lys Met Leu Asp Thr Pro Gly Pro Tyr Leu Leu Asp
       610                 615                 620
   gtc att gtg cca cat cag gag cat gtg ttg ccg atg att ccc agt aat      1920
   Val Ile Val Pro His Gln Glu His Val Leu Pro Met Ile Pro Ser Asn
   625                 630                 635                 640
   gga tcc ttc aag gat gtg ata acg gag gga gac ggt aga acg agg tac      1968
   Gly Ser Phe Lys Asp Val Ile Thr Glu Gly Asp Gly Arg Thr Arg Tyr
                   645                 650                 655
   tga 1971
<210> 20
   <211> 656
   <212> PRT
   <213> Glycine max
<400> 20
   Met Ala Ala Thr Ala Ser Arg Thr Thr Pro Phe Ser Ser Pro Ser Ser
   1               5                   10                  15
   Ser Ser His Pro Thr Phe Pro Lys Arg Ile Ala Arg Ser Thr Leu Pro
               20                  25                  30
   Leu Ser His Gln Thr Phe Ile Lys Pro Asn His Ala Leu Lys Ile Lys
           35                  40                  45
   Cys Ser Ile Ser Lys Pro Pro Ser Pro Pro Ala Thr Thr Thr Thr Ala
       50                  55                  60
   Ala Pro Ala Thr Ser Glu Pro Phe Val Ser Arg Phe Ala Ser Ala Glu
   65                  70                  75                  80
   Pro Arg Lys Gly Ser Asp Ile Leu Val Glu Ala Leu Glu Arg Leu Gly
                   85                  90                  95
   Val Thr Asn Val Phe Ala Tyr Pro Gly Gly Ala Ser Met Glu Ile His
               100                 105                 110
   Gln Ala Leu Thr Arg Ser Ser Thr Ile Arg Asn Val Leu Pro Arg His
           115                 120                 125
   Glu Gln Gly Gly Ile Phe Ala Ala Glu Gly Tyr Ala Arg Ser Ser Gly
       130                 135                 140
   Leu Pro Gly Val Cys Ile Ala Thr Ser Gly Pro Gly Ala Thr Asn Leu
   145                 150                 155                 160
   Val Ser Gly Leu Ala Asp Ala Leu Met Asp Ser Val Pro Val Val Ala
                   165                 170                 175
   Ile Thr Gly Gln Val Pro Arg Arg Met Ile Gly Thr Asp Ala Phe Gln
               180                 185                 190
   Glu Thr Pro Ile Val Glu Val Thr Arg Ser Ile Thr Lys His Asn Tyr
           195                 200                 205
   Leu Ile Leu Asp Val Asp Asp Ile Pro Arg Ile Val Ala Glu Ala Phe
       210                 215                 220
   Phe Val Ala Thr Ser Gly Arg Pro Gly Pro Val Leu Ile Asp Ile Pro
   225                 230                 235                 240
   Lys Asp Val Gln Gln Gln Leu Ala Val Pro Asn Trp Asp Glu Pro Ile
                   245                 250                 255
   Asn Leu Pro Gly Tyr Leu Ala Arg Leu Pro Arg Pro Pro Thr Glu Ala
               260                 265                 270
   Gln Leu Glu His Ile Val Arg Leu Ile Thr Glu Ala Gln Lys Pro Val
           275                 280                 285
   Leu Tyr Val Gly Gly Gly Ser Leu Asn Ser Ser Asp Glu Leu Arg Arg
       290                 295                 300
   Phe Val Glu Leu Thr Gly Ile Pro Val Ala Ser Thr Leu Met Gly Leu
   305                 310                 315                 320
   Gly Thr Tyr Pro Ile Gly Asp Glu Tyr Ser Leu Gln Met Leu Gly Met
                   325                 330                 335
   His Gly Thr Val Tyr Ala Asn Tyr Ala Val Asp Asn Ser Asp Leu Leu
               340                 345                 350
   Leu Ala Phe Gly Val Arg Phe Asp Asp Arg Val Thr Gly Lys Leu Glu
           355                 360                 365
   Ala Phe Ala Ser Arg Ala Lys Ile Val His Ile Asp Ile Asp Ser Ala
       370                 375                 380
   Glu Ile Gly Lys Asn Lys Gln Ala His Val Ser Val Cys Ala Asp Leu
   385                 390                 395                 400
   Lys Leu Ala Leu Gln Gly Ile Asn Met Ile Leu Glu Lys Lys Arg Val
                   405                 410                 415
   Gly Gly Gly Lys Leu Asp Leu Gly Gly Trp Arg Glu Glu Ile Asn Val
               420                 425                 430
   Gln Lys His Lys Phe Pro Leu Gly Tyr Lys Thr Phe Gln Asp Ala Ile
           435                 440                 445
   Ser Pro Gln His Ala Ile Glu Val Leu Asp Glu Leu Thr Asn Gly Asp
       450                 455                 460
   Ala Ile Val Ser Thr Gly Val Gly Gln His Gln Met Trp Ala Ala Gln
   465                 470                 475                 480
   Phe Tyr Lys Tyr Lys Arg Pro Arg Gln Trp Leu Thr Ser Gly Gly Leu
                   485                 490                 495
   Gly Ala Met Gly Phe Gly Leu Pro Ala Ala Ile Gly Ala Ala Val Ala
               500                 505                 510
   Asn Pro Gly Ala Val Val Val Asp Ile Asp Gly Asp Gly Ser Phe Ile
           515                 520                 525
   Met Asn Val Gln Glu Leu Ala Thr Ile Arg Val Glu Asn Leu Pro Val
       530                 535                 540
   Lys Ile Leu Leu Leu Asn Asn Gln His Leu Gly Met Val Val Gln Trp
   545                 550                 555                 560
   Glu Asp Arg Phe Tyr Lys Ser Asn Arg Ala His Thr Tyr Leu Gly Asp
                   565                 570                 575
   Pro Ser Ser Glu Asn Glu Ile Phe Pro Asn Met Leu Lys Phe Ala Asp
               580                 585                 590
   Ala Cys Gly Ile Pro Ala Ala Arg Val Thr Lys Lys Glu Glu Leu Arg
           595                 600                 605
   Ala Glu Ile Gln Lys Met Leu Asp Thr Pro Gly Pro Tyr Leu Leu Asp
       610                 615                 620
   Val Ile Val Pro His Gln Glu His Val Leu Pro Met Ile Pro Ser Asn
   625                 630                 635                 640
   Gly Ser Phe Lys Asp Val Ile Thr Glu Gly Asp Gly Arg Thr Arg Tyr
                   645                 650                 655
<210> 21
   <211> 1902
   <212> DNA
   <213> Glycine max
<220>
   <221> CDS
   <222> (1)..(1902)
<400> 21
   atg gcg gcc acc gct tcc aga acc acc cga ttc tct tct tcc tct tca       48
   Met Ala Ala Thr Ala Ser Arg Thr Thr Arg Phe Ser Ser Ser Ser Ser
   1               5                   10                  15
   cac ccc acc ttc ccc aaa cgc att act aga tcc acc ctc cct ctc tct      96
   His Pro Thr Phe Pro Lys Arg Ile Thr Arg Ser Thr Leu Pro Leu Ser
               20                  25                  30
   cat caa acc ctc acc aaa ccc aac cac gct ctc aaa atc aaa tgt tcc     144
   His Gln Thr Leu Thr Lys Pro Asn His Ala Leu Lys Ile Lys Cys Ser
           35                  40                  45
   atc tcc aaa ccc ccc acg gcg gcg ccc ttc acc aag gaa gcg ccg acc     192
   Ile Ser Lys Pro Pro Thr Ala Ala Pro Phe Thr Lys Glu Ala Pro Thr
       50                  55                  60
   acg gag ccc ttc gtg tca cgg ttc gcc tcc ggc gaa cct cgc aag ggc     240
   Thr Glu Pro Phe Val Ser Arg Phe Ala Ser Gly Glu Pro Arg Lys Gly
   65                  70                  75                  80
   gcg gac atc ctt gtg gag gcg ctg gag agg cag ggc gtg acg acg gtg     288
   Ala Asp Ile Leu Val Glu Ala Leu Glu Arg Gln Gly Val Thr Thr Val
                   85                  90                  95
   ttc gcg tac ccc ggc ggt gcg tcg atg gag atc cac cag gcg ctc acg     336
   Phe Ala Tyr Pro Gly Gly Ala Ser Met Glu Ile His Gln Ala Leu Thr
               100                 105                 110
   cgc tcc gcc gcc atc cgc aac gtg ctc ccg cgc cac gag cag ggc ggc     384
   Arg Ser Ala Ala Ile Arg Asn Val Leu Pro Arg His Glu Gln Gly Gly
           115                 120                 125
   gtc ttc gcc gcc gaa ggc tac gcg cgt tcc tcc ggc ctc ccc ggc gtc     432
   Val Phe Ala Ala Glu Gly Tyr Ala Arg Ser Ser Gly Leu Pro Gly Val
       130                 135                 140
   tgc att gcc acc tcc ggc ccc ggc gcc acc aac ctc gtg agc ggc ctc     480
   Cys Ile Ala Thr Ser Gly Pro Gly Ala Thr Asn Leu Val Ser Gly Leu
   145                 150                 155                 160
   gcc gac gct tta atg gac agc gtc cca gtc gtc gcc atc acc ggc cag     528
   Ala Asp Ala Leu Met Asp Ser Val Pro Val Val Ala Ile Thr Gly Gln
                   165                 170                 175
   gtg agc aga tcc atc acg aag cac aac tac ctc atc ctc gac gtc gac     576
   Val Ser Arg Ser Ile Thr Lys His Asn Tyr Leu Ile Leu Asp Val Asp
               180                 185                 190
   gac atc ccc cgc gtc gtc gcc gag gct ttc ttc gtc gcc acc tcc ggc     624
   Asp Ile Pro Arg Val Val Ala Glu Ala Phe Phe Val Ala Thr Ser Gly
           195                 200                 205
   cgc ccc ggt ccg gtc ctc atc gac att ccc aaa gac gtt cag cag caa     672
   Arg Pro Gly Pro Val Leu Ile Asp Ile Pro Lys Asp Val Gln Gln Gln
       210                 215                 220
   ctc gcc gtg cct aat tgg gac gag ccc gtt aac ctc ccc ggt tac ctc     720
   Leu Ala Val Pro Asn Trp Asp Glu Pro Val Asn Leu Pro Gly Tyr Leu
   225                 230                 235                 240
   gcc agg ctg ccc agg ccc ccc gcc gag gcc caa ttg gaa cac att gtc     768
   Ala Arg Leu Pro Arg Pro Pro Ala Glu Ala Gln Leu Glu His Ile Val
                   245                 250                 255
   aga ctc atc atg gag gcc caa aag ccc gtt ctc tac gtc ggc ggt ggc     816
   Arg Leu Ile Met Glu Ala Gln Lys Pro Val Leu Tyr Val Gly Gly Gly
               260                 265                 270
   agt ttg aat tcc agt gct gaa ttg agg cgc ttt gtt gaa ctc act ggt        864
   Ser Leu Asn Ser Ser Ala Glu Leu Arg Arg Phe Val Glu Leu Thr Gly
           275                 280                 285
   att ccc gtt gct agc act tta atg ggt ctt gga act ttt cct att ggt        912
   Ile Pro Val Ala Ser Thr Leu Met Gly Leu Gly Thr Phe Pro Ile Gly
       290                 295                 300
   gat gaa tat tcc ctt cag atg ctg ggt atg cat ggt act gtt tat gct        960
   Asp Glu Tyr Ser Leu Gln Met Leu Gly Met His Gly Thr Val Tyr Ala
   305                 310                 315                 320
   aac tat gct gtt gac aat agt gat ttg ttg ctt gcc ttt ggg gta agg       1008
   Asn Tyr Ala Val Asp Asn Ser Asp Leu Leu Leu Ala Phe Gly Val Arg
                   325                 330                 335
   ttt gat gac cgt gtt act ggg aag ctt gag gct ttt gct agt agg gct       1056
   Phe Asp Asp Arg Val Thr Gly Lys Leu Glu Ala Phe Ala Ser Arg Ala
               340                 345                 350
   aag att gtt cac att gat att gat tct gcc gag att ggg aag aac aag       1104
   Lys Ile Val His Ile Asp Ile Asp Ser Ala Glu Ile Gly Lys Asn Lys
           355                 360                 365
   cag gcg cac gtg tcg gtt tgc gcg gat ttg aag ttg gcc ttg aag gga       1152
   Gln Ala His Val Ser Val Cys Ala Asp Leu Lys Leu Ala Leu Lys Gly
       370                 375                 380
   att aat atg att ttg gag gag aaa gga gtg gag ggt aag ttt gat ctt       1200
   Ile Asn Met Ile Leu Glu Glu Lys Gly Val Glu Gly Lys Phe Asp Leu
   385                 390                 395                 400
   gga ggt tgg aga gaa gag att aat gtg cag aaa cac aag ttt cca ttg       1248
   Gly Gly Trp Arg Glu Glu Ile Asn Val Gln Lys His Lys Phe Pro Leu
                   405                 410                 415
   ggt tac aag aca ttc cag gac gcg att tct ccg cag cat gct atc gag       1296
   Gly Tyr Lys Thr Phe Gln Asp Ala Ile Ser Pro Gln His Ala Ile Glu
               420                 425                 430
   gtt ctt gat gag ttg act aat gga gat gct att gtt agt act ggg gtt       1344
   Val Leu Asp Glu Leu Thr Asn Gly Asp Ala Ile Val Ser Thr Gly Val
           435                 440                 445
   ggg cag cat caa atg tgg gct gcg cag ttt tac aag tac aag aga ccg       1392
   Gly Gln His Gln Met Trp Ala Ala Gln Phe Tyr Lys Tyr Lys Arg Pro
       450                 455                 460
   agg cag tgg ttg acc tca ggg ggt ctt gga gcc atg ggt ttt gga ttg       1440
   Arg Gln Trp Leu Thr Ser Gly Gly Leu Gly Ala Met Gly Phe Gly Leu
   465                 470                 475                 480
   cct gcg gct att ggt gct gct gtt gct aac cct ggg gct gtt gtg gtt       1488
   Pro Ala Ala Ile Gly Ala Ala Val Ala Asn Pro Gly Ala Val Val Val
                   485                 490                 495
   gac att gat ggg gat ggt agt ttc atc atg aat gtt cag gag ttg gcc       1536
   Asp Ile Asp Gly Asp Gly Ser Phe Ile Met Asn Val Gln Glu Leu Ala
               500                 505                 510
   act ata aga gtg gag aat ctc cca gtt aag ata ttg ttg ttg aac aat       1584
   Thr Ile Arg Val Glu Asn Leu Pro Val Lys Ile Leu Leu Leu Asn Asn
           515                 520                 525
   cag cat ttg ggt atg gtg gtt cag tgg gag gat agg ttc tac aag tcc      1632
   Gln His Leu Gly Met Val Val Gln Trp Glu Asp Arg Phe Tyr Lys Ser
       530                 535                 540
   aat aga gct cac acc tat ctt gga gat ccg tct agc gag agc gag ata      1680
   Asn Arg Ala His Thr Tyr Leu Gly Asp Pro Ser Ser Glu Ser Glu Ile
   545                 550                 555                 560
   ttc cca aac atg ctc aag ttt gct gat gct tgt ggg ata ccg gca gcg      1728
   Phe Pro Asn Met Leu Lys Phe Ala Asp Ala Cys Gly Ile Pro Ala Ala
                   565                 570                 575
   cga gtg acg aag aag gaa gag ctt aga gcg gca att cag aga atg ttg      1776
   Arg Val Thr Lys Lys Glu Glu Leu Arg Ala Ala Ile Gln Arg Met Leu
               580                 585                 590
   gac acc cct ggc ccc tac ctt ctt gat gtc att gtg ccc cat cag gag      1824
   Asp Thr Pro Gly Pro Tyr Leu Leu Asp Val Ile Val Pro His Gln Glu
           595                 600                 605
   cat gtg ttg ccg atg att ccc agt aat gga tcc ttc aag gat gtg ata      1872
   His Val Leu Pro Met Ile Pro Ser Asn Gly Ser Phe Lys Asp Val Ile
       610                 615                 620
   act gag ggt gat ggt aga acg agg tac tga                              1902
   Thr Glu Gly Asp Gly Arg Thr Arg Tyr
   625                 630
<210> 22
   <211> 633
   <212> PRT
   <213> Glycine max
<400> 22
   Met Ala Ala Thr Ala Ser Arg Thr Thr Arg Phe Ser Ser Ser Ser Ser
   1               5                   10                  15
   His Pro Thr Phe Pro Lys Arg Ile Thr Arg Ser Thr Leu Pro Leu Ser
               20                  25                  30
   His Gln Thr Leu Thr Lys Pro Asn His Ala Leu Lys Ile Lys Cys Ser
           35                  40                  45
   Ile Ser Lys Pro Pro Thr Ala Ala Pro Phe Thr Lys Glu Ala Pro Thr
       50                  55                  60
   Thr Glu Pro Phe Val Ser Arg Phe Ala Ser Gly Glu Pro Arg Lys Gly
   65                  70                  75                  80
   Ala Asp Ile Leu Val Glu Ala Leu Glu Arg Gln Gly Val Thr Thr Val
                   85                  90                  95
   Phe Ala Tyr Pro Gly Gly Ala Ser Met Glu Ile His Gln Ala Leu Thr
   100 105 110
   Arg Ser Ala Ala Ile Arg Asn Val Leu Pro Arg His Glu Gln Gly Gly
   115 120 125
   Val Phe Ala Ala Glu Gly Tyr Ala Arg Ser Ser Gly Leu Pro Gly Val
   130 135 140
   Cys Ile Ala Thr Ser Gly Pro Gly Ala Thr Asn Leu Val Ser Gly Leu
   145 150 155 160
   Ala Asp Ala Leu Met Asp Ser Val Pro Val Val Ala Ile Thr Gly Gln
   165 170 175
   Val Ser Arg Ser Ile Thr Lys His Asn Tyr Leu Ile Leu Asp Val Asp
   180 185 190
   Asp Ile Pro Arg Val Val Ala Glu Ala Phe Phe Val Ala Thr Ser Gly
   195 200 205
   Arg Pro Gly Pro Val Leu Ile Asp Ile Pro Lys Asp Val Gln Gln Gln
   210 215 220
   Leu Ala Val Pro Asn Trp Asp Glu Pro Val Asn Leu Pro Gly Tyr Leu
   225 230 235 240
   Ala Arg Leu Pro Arg Pro Pro Ala Glu Ala Gln Leu Glu His Ile Val
   245 250 255
   Arg Leu Ile Met Glu Ala Gln Lys Pro Val Leu Tyr Val Gly Gly Gly
   260 265 270
   Ser Leu Asn Ser Ser Ala Glu Leu Arg Arg Phe Val Glu Leu Thr Gly
   275 280 285
   Ile Pro Val Ala Ser Thr Leu Met Gly Leu Gly Thr Phe Pro Ile Gly
   290 295 300
   Asp Glu Tyr Ser Leu Gln Met Leu Gly Met His Gly Thr Val Tyr Ala
   305 310 315 320
   Asn Tyr Ala Val Asp Asn Ser Asp Leu Leu Leu Ala Phe Gly Val Arg
   325 330 335
   Phe Asp Asp Arg Val Thr Gly Lys Leu Glu Ala Phe Ala Ser Arg Ala
   340 345 350
   Lys Ile Val His Ile Asp Ile Asp Ser Ala Glu Ile Gly Lys Asn Lys
   355 360 365
   Gln Ala His Val Ser Val Cys Ala Asp Leu Lys Leu Ala Leu Lys Gly
   370 375 380
   Ile Asn Met Ile Leu Glu Glu Lys Gly Val Glu Gly Lys Phe Asp Leu
   385 390 395 400
   Gly Gly Trp Arg Glu Glu Ile Asn Val Gln Lys His Lys Phe Pro Leu
   405 410 415
   Gly Tyr Lys Thr Phe Gln Asp Ala Ile Ser Pro Gln His Ala Ile Glu
   420 425 430
   Val Leu Asp Glu Leu Thr Asn Gly Asp Ala Ile Val Ser Thr Gly Val
   435 440 445
   Gly Gln His Gln Met Trp Ala Ala Gln Phe Tyr Lys Tyr Lys Arg Pro
   450 455 460
   Arg Gln Trp Leu Thr Ser Gly Gly Leu Gly Ala Met Gly Phe Gly Leu
   465 470 475 480
   Pro Ala Ala Ile Gly Ala Ala Val Ala Asn Pro Gly Ala Val Val Val
   485 490 495
   Asp Ile Asp Gly Asp Gly Ser Phe Ile Met Asn Val Gln Glu Leu Ala
   500 505 510
   Thr Ile Arg Val Glu Asn Leu Pro Val Lys Ile Leu Leu Leu Asn Asn
   515 520 525
   Gln His Leu Gly Met Val Val Gln Trp Glu Asp Arg Phe Tyr Lys Ser
   530 535 540
   Asn Arg Ala His Thr Tyr Leu Gly Asp Pro Ser Ser Glu Ser Glu Ile
   545 550 555 560
   Phe Pro Asn Met Leu Lys Phe Ala Asp Ala Cys Gly Ile Pro Ala Ala
   565 570 575
   Arg Val Thr Lys Lys Glu Glu Leu Arg Ala Ala Ile Gln Arg Met Leu
   580 585 590
   Asp Thr Pro Gly Pro Tyr Leu Leu Asp Val Ile Val Pro His Gln Glu
   595 600 605
   His Val Leu Pro Met Ile Pro Ser Asn Gly Ser Phe Lys Asp Val Ile
   610 615 620
   Thr Glu Gly Asp Gly Arg Thr Arg Tyr
   625 630
<210> 23
   <211> 1995
   <212> DNA
   <213> Nicotiana tabacum
<220>
   <221> CDS
   <222> (1)..(1995)
<400> 23
   atg gcg gcg gct gcg gcg gct cca tct ccc tct ttc tcc aaa acc cta          48
   Met Ala Ala Ala Ala Ala Ala Pro Ser Pro Ser Phe Ser Lys Thr Leu
   1               5                   10                  15
   tcg tcc tcc tcc tcc aaa tcc tcc acc ctc ctc cct aga tcc acc ttc          96
   Ser Ser Ser Ser Ser Lys Ser Ser Thr Leu Leu Pro Arg Ser Thr Phe
               20                  25                  30
   cct ttc ccc cac cac ccc cac aaa acc acc cca cca ccc ctc cac ctc         144
   Pro Phe Pro His His Pro His Lys Thr Thr Pro Pro Pro Leu His Leu
           35                  40                  45
   acc ccc acc cac att cac agc caa cgc cgt cgt ttc acc atc tcc aat         192
   Thr Pro Thr His Ile His Ser Gln Arg Arg Arg Phe Thr Ile Ser Asn
       50                  55                  60
   gtc att tcc act acc caa aaa gtt tcc gag acc caa aaa gcc gaa act         240
   Val Ile Ser Thr Thr Gln Lys Val Ser Glu Thr Gln Lys Ala Glu Thr
   65                  70                  75                  80
   ttc gtt tcc cgt ttt gcc cct gac gaa ccc aga aag ggt tcc gac gtt         288
   Phe Val Ser Arg Phe Ala Pro Asp Glu Pro Arg Lys Gly Ser Asp Val
                   85                  90                  95
   ctc gtg gag gcc ctc gaa aga gaa ggg gtt acg gac gtt ttt gcg tac         336
   Leu Val Glu Ala Leu Glu Arg Glu Gly Val Thr Asp Val Phe Ala Tyr
               100                 105                 110
   cca ggc ggc gct tcc atg gag att cac caa gct ttg acg cgc tca agc         384
   Pro Gly Gly Ala Ser Met Glu Ile His Gln Ala Leu Thr Arg Ser Ser
           115                 120                 125
   atc atc cgc aac gtg cta cca cgt cac gag cag ggt ggt gtc ttc gcc         432
   Ile Ile Arg Asn Val Leu Pro Arg His Glu Gln Gly Gly Val Phe Ala
       130                 135                 140
   gct gag ggt tac gca cgc gcc acc ggc ttc ccc ggc gtt tgc att gcc         480
   Ala Glu Gly Tyr Ala Arg Ala Thr Gly Phe Pro Gly Val Cys Ile Ala
   145                 150                 155                 160
   acc tcc ggc cct ggc gcc acc aat ctc gtc agt ggc ctc gcg gac gcc         528
   Thr Ser Gly Pro Gly Ala Thr Asn Leu Val Ser Gly Leu Ala Asp Ala
                   165                 170                 175
   cta ctg gat agc gtc ccc att gtt gct ata acc ggt caa gtg cca cgt      576
   Leu Leu Asp Ser Val Pro Ile Val Ala Ile Thr Gly Gln Val Pro Arg
               180                 185                 190
   agg atg atc ggt act gat gct ttt cag gaa act ccg att gtt gag gta      624
   Arg Met Ile Gly Thr Asp Ala Phe Gln Glu Thr Pro Ile Val Glu Val
           195                 200                 205
   act aga tcg att acc aag cat aat tat ctc gtt atg gac gta gag gat      672
   Thr Arg Ser Ile Thr Lys His Asn Tyr Leu Val Met Asp Val Glu Asp
       210                 215                 220
   att cct agg gtt gta cgt gag gct ttt ttc ctt gcg aga tcg ggc cgg      720
   Ile Pro Arg Val Val Arg Glu Ala Phe Phe Leu Ala Arg Ser Gly Arg
   225                 230                 235                 240
   cct ggc cct gtt ttg att gat gta cct aag gat att cag caa caa ttg      768
   Pro Gly Pro Val Leu Ile Asp Val Pro Lys Asp Ile Gln Gln Gln Leu
                   245                 250                 255
   gtg ata cct gac tgg gat cag cca atg agg ttg cct ggt tac atg tct      816
   Val Ile Pro Asp Trp Asp Gln Pro Met Arg Leu Pro Gly Tyr Met Ser
               260                 265                 270
   agg tta cct aaa ttg ccc aat gag atg ctt tta gaa caa att gtt agg      864
   Arg Leu Pro Lys Leu Pro Asn Glu Met Leu Leu Glu Gln Ile Val Arg
           275                 280                 285
   ctt att tct gag tca aag aag cct gtt ttg tat gtg ggg ggt ggg tgt      912
   Leu Ile Ser Glu Ser Lys Lys Pro Val Leu Tyr Val Gly Gly Gly Cys
       290                 295                 300
   tcg caa tcg agt gag gag ttg aga cga ttc gtg gag ctc acc ggt atc      960
   Ser Gln Ser Ser Glu Glu Leu Arg Arg Phe Val Glu Leu Thr Gly Ile
   305                 310                 315                 320
   ccc gtg gca agt act ttg atg ggt ctt gga gct ttt cca act ggg gat     1008
   Pro Val Ala Ser Thr Leu Met Gly Leu Gly Ala Phe Pro Thr Gly Asp
                   325                 330                 335
   gag ctt tcc ctt tca atg ttg ggt atg cat ggt act gtt tat gct aat     1056
   Glu Leu Ser Leu Ser Met Leu Gly Met His Gly Thr Val Tyr Ala Asn
               340                 345                 350
   tat gct gtg gac agt agt gat tta ttg ctc gca ttt ggg gtg agg ttt     1104
   Tyr Ala Val Asp Ser Ser Asp Leu Leu Leu Ala Phe Gly Val Arg Phe
           355                 360                 365
   gat gat aga gtt act gga aag tta gaa gct ttt gct agc cga gcg aaa     1152
   Asp Asp Arg Val Thr Gly Lys Leu Glu Ala Phe Ala Ser Arg Ala Lys
       370                 375                 380
   att gtt cac att gat att gat tca gct gag att gga aag aac aag cag     1200
   Ile Val His Ile Asp Ile Asp Ser Ala Glu Ile Gly Lys Asn Lys Gln
   385                 390                 395                 400
   cct cat gtt tcc att tgt gcg gat atc aag ttg gcg tta cag ggt ttg     1248
   Pro His Val Ser Ile Cys Ala Asp Ile Lys Leu Ala Leu Gln Gly Leu
                   405                 410                 415
   aat tcg ata ttg gag agt aag gaa ggt aaa ctg aag ttg gat ttt tct     1296
   Asn Ser Ile Leu Glu Ser Lys Glu Gly Lys Leu Lys Leu Asp Phe Ser
               420                 425                 430
   gct tgg agg cag gag ttg acg gtg cag aaa gtg aag tac ccg ttg aat        1344
   Ala Trp Arg Gln Glu Leu Thr Val Gln Lys Val Lys Tyr Pro Leu Asn
           435                 440                 445
   ttt aaa act ttt ggt gat gct att cct ccg caa tat gct atc cag gtt        1392
   Phe Lys Thr Phe Gly Asp Ala Ile Pro Pro Gln Tyr Ala Ile Gln Val
       450                 455                 460
   cta gat gag tta act aat ggg agt gct att ata agt acc ggt gtt ggg        1440
   Leu Asp Glu Leu Thr Asn Gly Ser Ala Ile Ile Ser Thr Gly Val Gly
   465                 470                 475                 480
   cag cac cag atg tgg gct gct caa tat tat aag tac aga aag cca cgc        1488
   Gln His Gln Met Trp Ala Ala Gln Tyr Tyr Lys Tyr Arg Lys Pro Arg
                   485                 490                 495
   caa tgg ttg aca tct ggt gga tta gga gcg atg gga ttt ggt ttg ccc        1536
   Gln Trp Leu Thr Ser Gly Gly Leu Gly Ala Met Gly Phe Gly Leu Pro
               500                 505                 510
   gct gct att ggt gcg gct gtt gga aga cct gat gaa gtt gtg gtt gac        1584
   Ala Ala Ile Gly Ala Ala Val Gly Arg Pro Asp Glu Val Val Val Asp
           515                 520                 525
   att gat ggt gat ggc agt ttc atc atg aat gtg cag gag cta gca act        1632
   Ile Asp Gly Asp Gly Ser Phe Ile Met Asn Val Gln Glu Leu Ala Thr
       530                 535                 540
   att aag gtg gag aat ctc cca gtt aag att atg tta ctg aat aat caa        1680
   Ile Lys Val Glu Asn Leu Pro Val Lys Ile Met Leu Leu Asn Asn Gln
   545                 550                 555                 560
   cac ttg gga atg gtg gtt caa tgg gag gat cgg ttc tat aag gct aac        1728
   His Leu Gly Met Val Val Gln Trp Glu Asp Arg Phe Tyr Lys Ala Asn
                   565                 570                 575
   aga gca cac aca tac ctg ggg aat cct tct aat gag gcg gag atc ttt        1776
   Arg Ala His Thr Tyr Leu Gly Asn Pro Ser Asn Glu Ala Glu Ile Phe
               580                 585                 590
   cct aat atg ttg aaa ttt gca gag gct tgt ggc gta cct gct gcg aga        1824
   Pro Asn Met Leu Lys Phe Ala Glu Ala Cys Gly Val Pro Ala Ala Arg
           595                 600                 605
   gtg aca cac agg gat gat ctt aga gcg gct att caa aag atg tta gac        1872
   Val Thr His Arg Asp Asp Leu Arg Ala Ala Ile Gln Lys Met Leu Asp
       610                 615                 620
   act cct ggg cca tac ttg ttg gat gtg att gta cct cat cag gaa cat        1920
   Thr Pro Gly Pro Tyr Leu Leu Asp Val Ile Val Pro His Gln Glu His
   625                 630                 635                 640
   gtt cta cct atg att ccc agt ggc ggg gct ttc aaa gat gtg atc aca        1968
   Val Leu Pro Met Ile Pro Ser Gly Gly Ala Phe Lys Asp Val Ile Thr
                   645                 650                 655
   gag ggt gac ggg aga agt tcc tat tga                                    1995
   Glu Gly Asp Gly Arg Ser Ser Tyr
               660
<210> 24
   <211> 664
   <212> PRT
   <213> Nicotiana tabacum
<400> 24
   Met Ala Ala Ala Ala Ala Ala Pro Ser Pro Ser Phe Ser Lys Thr Leu
   1               5                   10                  15
   Ser Ser Ser Ser Ser Lys Ser Ser Thr Leu Leu Pro Arg Ser Thr Phe
               20                  25                  30
   Pro Phe Pro His His Pro His Lys Thr Thr Pro Pro Pro Leu His Leu
           35                  40                  45
   Thr Pro Thr His Ile His Ser Gln Arg Arg Arg Phe Thr Ile Ser Asn
       50                  55                  60
   Val Ile Ser Thr Thr Gln Lys Val Ser Glu Thr Gln Lys Ala Glu Thr
   65                  70                  75                  80
   Phe Val Ser Arg Phe Ala Pro Asp Glu Pro Arg Lys Gly Ser Asp Val
                   85                  90                  95
   Leu Val Glu Ala Leu Glu Arg Glu Gly Val Thr Asp Val Phe Ala Tyr
               100                 105                 110
   Pro Gly Gly Ala Ser Met Glu Ile His Gln Ala Leu Thr Arg Ser Ser
           115                 120                 125
   Ile Ile Arg Asn Val Leu Pro Arg His Glu Gln Gly Gly Val Phe Ala
       130                 135                 140
   Ala Glu Gly Tyr Ala Arg Ala Thr Gly Phe Pro Gly Val Cys Ile Ala
   145                 150                 155                 160
   Thr Ser Gly Pro Gly Ala Thr Asn Leu Val Ser Gly Leu Ala Asp Ala
                   165                 170                 175
   Leu Leu Asp Ser Val Pro Ile Val Ala Ile Thr Gly Gln Val Pro Arg
               180                 185                 190
   Arg Met Ile Gly Thr Asp Ala Phe Gln Glu Thr Pro Ile Val Glu Val
           195                 200                 205
   Thr Arg Ser Ile Thr Lys His Asn Tyr Leu Val Met Asp Val Glu Asp
       210                 215                 220
   Ile Pro Arg Val Val Arg Glu Ala Phe Phe Leu Ala Arg Ser Gly Arg
   225                 230                 235                 240
   Pro Gly Pro Val Leu Ile Asp Val Pro Lys Asp Ile Gln Gln Gln Leu
                   245                 250                 255
   Val Ile Pro Asp Trp Asp Gln Pro Met Arg Leu Pro Gly Tyr Met Ser
               260                 265                 270
   Arg Leu Pro Lys Leu Pro Asn Glu Met Leu Leu Glu Gln Ile Val Arg
           275                 280                 285
   Leu Ile Ser Glu Ser Lys Lys Pro Val Leu Tyr Val Gly Gly Gly Cys
       290                 295                 300
   Ser Gln Ser Ser Glu Glu Leu Arg Arg Phe Val Glu Leu Thr Gly Ile
   305                 310                 315                 320
   Pro Val Ala Ser Thr Leu Met Gly Leu Gly Ala Phe Pro Thr Gly Asp
                   325                 330                 335
   Glu Leu Ser Leu Ser Met Leu Gly Met His Gly Thr Val Tyr Ala Asn
               340                 345                 350
   Tyr Ala Val Asp Ser Ser Asp Leu Leu Leu Ala Phe Gly Val Arg Phe
           355                 360                 365
   Asp Asp Arg Val Thr Gly Lys Leu Glu Ala Phe Ala Ser Arg Ala Lys
       370                 375                 380
   Ile Val His Ile Asp Ile Asp Ser Ala Glu Ile Gly Lys Asn Lys Gln
   385                 390                 395                 400
   Pro His Val Ser Ile Cys Ala Asp Ile Lys Leu Ala Leu Gln Gly Leu
                   405                 410                 415
   Asn Ser Ile Leu Glu Ser Lys Glu Gly Lys Leu Lys Leu Asp Phe Ser
               420                 425                 430
   Ala Trp Arg Gln Glu Leu Thr Val Gln Lys Val Lys Tyr Pro Leu Asn
           435                 440                 445
   Phe Lys Thr Phe Gly Asp Ala Ile Pro Pro Gln Tyr Ala Ile Gln Val
       450                 455                 460
   Leu Asp Glu Leu Thr Asn Gly Ser Ala Ile Ile Ser Thr Gly Val Gly
   465                 470                 475                 480
   Gln His Gln Met Trp Ala Ala Gln Tyr Tyr Lys Tyr Arg Lys Pro Arg
                   485                 490                 495
   Gln Trp Leu Thr Ser Gly Gly Leu Gly Ala Met Gly Phe Gly Leu Pro
               500                 505                 510
   Ala Ala Ile Gly Ala Ala Val Gly Arg Pro Asp Glu Val Val Val Asp
           515                 520                 525
   Ile Asp Gly Asp Gly Ser Phe Ile Met Asn Val Gln Glu Leu Ala Thr
       530                 535                 540
   Ile Lys Val Glu Asn Leu Pro Val Lys Ile Met Leu Leu Asn Asn Gln
   545                 550                 555                 560
   His Leu Gly Met Val Val Gln Trp Glu Asp Arg Phe Tyr Lys Ala Asn
                   565                 570                 575
   Arg Ala His Thr Tyr Leu Gly Asn Pro Ser Asn Glu Ala Glu Ile Phe
               580                 585                 590
   Pro Asn Met Leu Lys Phe Ala Glu Ala Cys Gly Val Pro Ala Ala Arg
           595                 600                 605
   Val Thr His Arg Asp Asp Leu Arg Ala Ala Ile Gln Lys Met Leu Asp
       610                 615                 620
   Thr Pro Gly Pro Tyr Leu Leu Asp Val Ile Val Pro His Gln Glu His
   625                 630                 635                 640
   Val Leu Pro Met Ile Pro Ser Gly Gly Ala Phe Lys Asp Val Ile Thr
                   645                 650                 655
   Glu Gly Asp Gly Arg Ser Ser Tyr
               660
<210> 25
   <211> 2004
   <212> DNA
   <213> Nicotiana tabacum
<220>
   <221> CDS
   <222> (1)..(2004)
<400> 25
   atg gcg gcg gcg gct cca tct ccc tct tct tcc gct ttc tcc aaa acc        48
   Met Ala Ala Ala Ala Pro Ser Pro Ser Ser Ser Ala Phe Ser Lys Thr
   1               5                   10                  15
   cta tcg cct tcc tcc tcc aca tcc tcc acc ctc ctc cct aga tca acc        96
   Leu Ser Pro Ser Ser Ser Thr Ser Ser Thr Leu Leu Pro Arg Ser Thr
               20                  25                  30
   ttc cct ttc ccc cac cac ccc cac aag acc acc cca cca ccc ctc cac       144
   Phe Pro Phe Pro His His Pro His Lys Thr Thr Pro Pro Pro Leu His
           35                  40                  45
   ctc acc cac act cac att cac att cac agc caa cgc cgt cgt ttc acc       192
   Leu Thr His Thr His Ile His Ile His Ser Gln Arg Arg Arg Phe Thr
       50                  55                  60
   ata tcc aat gtc att tcc act aac caa aaa gtt tcc cag acc gaa aaa       240
   Ile Ser Asn Val Ile Ser Thr Asn Gln Lys Val Ser Gln Thr Glu Lys
   65                  70                  75                  80
   acc gaa act ttc gtt tcc cgt ttt gct cct gac gaa ccc aga aag ggt       288
   Thr Glu Thr Phe Val Ser Arg Phe Ala Pro Asp Glu Pro Arg Lys Gly
                   85                  90                  95
   tcc gac gtt ctc gtg gag gct ctc gaa aga gaa ggg gtt acg gac gtc       336
   Ser Asp Val Leu Val Glu Ala Leu Glu Arg Glu Gly Val Thr Asp Val
               100                 105                 110
   ttt gcg tac cca ggt ggc gct tcc atg gag att cac caa gct ttg acc       384
   Phe Ala Tyr Pro Gly Gly Ala Ser Met Glu Ile His Gln Ala Leu Thr
           115                 120                 125
   cgt tca agc atc atc cgc aac gtg ctg cca cgt cac gag cag ggc ggt       432
   Arg Ser Ser Ile Ile Arg Asn Val Leu Pro Arg His Glu Gln Gly Gly
       130                 135                 140
   gtc ttc gcc gct gag ggt tac gca cgc gcc acc gga ttt ccc ggc gtt       480
   Val Phe Ala Ala Glu Gly Tyr Ala Arg Ala Thr Gly Phe Pro Gly Val
   145                 150                 155                 160
   tgc att gcc acc tct ggc ccc ggc gcc acc aat ctc gtc agc ggc ctc       528
   Cys Ile Ala Thr Ser Gly Pro Gly Ala Thr Asn Leu Val Ser Gly Leu
                   165                 170                 175
   gct gac gcg cta ctg gat agc gtc ccc att gtt gct ata aca ggt caa       576
   Ala Asp Ala Leu Leu Asp Ser Val Pro Ile Val Ala Ile Thr Gly Gln
               180                 185                 190
   gtg cca cgt agg atg ata ggt act gat gct ttt cag gaa act cct att       624
   Val Pro Arg Arg Met Ile Gly Thr Asp Ala Phe Gln Glu Thr Pro Ile
           195                 200                 205
   gtt gag gta act aga tcg att acc aag cat aat tat ctc gtt atg gac       672
   Val Glu Val Thr Arg Ser Ile Thr Lys His Asn Tyr Leu Val Met Asp
       210                 215                 220
   gta gag gat att cct agg gtt gta cgt gaa gct ttt ttc ctc gcg aga       720
   Val Glu Asp Ile Pro Arg Val Val Arg Glu Ala Phe Phe Leu Ala Arg
   225                 230                 235                 240
   tcg ggc cgg cct ggc cct att ttg att gat gta cct aag gat att cag       768
   Ser Gly Arg Pro Gly Pro Ile Leu Ile Asp Val Pro Lys Asp Ile Gln
                   245                 250                 255
   caa caa ttg gtg ata cct gac tgg gat cag cca atg agg tta cct ggt       816
   Gln Gln Leu Val Ile Pro Asp Trp Asp Gln Pro Met Arg Leu Pro Gly
                   260                 265                 270
   tac atg tct agg tta cct aaa ttg ccc aat gag atg ctt tta gaa caa         864
   Tyr Met Ser Arg Leu Pro Lys Leu Pro Asn Glu Met Leu Leu Glu Gln
               275                 280                 285
   att gtt agg ctt att tct gag tca aag aag cct gtt ttg tat gtg ggg         912
   Ile Val Arg Leu Ile Ser Glu Ser Lys Lys Pro Val Leu Tyr Val Gly
           290                 295                 300
   ggt ggg tgt tcg caa tcg agt gag gac ttg aga cga ttc gtg gag ctc         960
   Gly Gly Cys Ser Gln Ser Ser Glu Asp Leu Arg Arg Phe Val Glu Leu
   305                 310                 315                 320
   acg ggt atc ccc gtg gca agt act ttg atg ggt ctt gga gct ttt cca        1008
   Thr Gly Ile Pro Val Ala Ser Thr Leu Met Gly Leu Gly Ala Phe Pro
                   325                 330                 335
   act ggg gat gag ctt tcc ctt tca atg ttg ggt atg cat ggt act gtt        1056
   Thr Gly Asp Glu Leu Ser Leu Ser Met Leu Gly Met His Gly Thr Val
               340                 345                 350
   tat gct aat tat gct gtg gac agt agt gat ttg ttg ctc gca ttt ggg        1104
   Tyr Ala Asn Tyr Ala Val Asp Ser Ser Asp Leu Leu Leu Ala Phe Gly
           355                 360                 365
   gtg agg ttt gat gat aga gtt act gga aag tta gaa gct ttt gct agc        1152
   Val Arg Phe Asp Asp Arg Val Thr Gly Lys Leu Glu Ala Phe Ala Ser
       370                 375                 380
   cga gca aaa att gtt cac att gat att gat tca gct gag att gga aag        1200
   Arg Ala Lys Ile Val His Ile Asp Ile Asp Ser Ala Glu Ile Gly Lys
   385                 390                 395                 400
   aac aag cag cct cat gtt tcc att tgt gca gat atc aag ttg gcg tta        1248
   Asn Lys Gln Pro His Val Ser Ile Cys Ala Asp Ile Lys Leu Ala Leu
                   405                 410                 415
   cag ggt ttg aat tcg ata ctg gag agt aag gaa ggt aaa ctg aag ttg        1296
   Gln Gly Leu Asn Ser Ile Leu Glu Ser Lys Glu Gly Lys Leu Lys Leu
               420                 425                 430
   gat ttt tct gct tgg agg cag gag ttg acg gag cag aaa gtg aag cac        1344
   Asp Phe Ser Ala Trp Arg Gln Glu Leu Thr Glu Gln Lys Val Lys His
           435                 440                 445
   cca ttg aac ttt aaa act ttt ggt gat gca att cct ccg caa tat gct        1392
   Pro Leu Asn Phe Lys Thr Phe Gly Asp Ala Ile Pro Pro Gln Tyr Ala
       450                 455                 460
   atc cag gtt cta gat gag tta act aat ggg aat gct att ata agt act        1440
   Ile Gln Val Leu Asp Glu Leu Thr Asn Gly Asn Ala Ile Ile Ser Thr
   465                 470                 475                 480
   ggt gtg ggg caa cac cag atg tgg gct gct caa tac tat aag tac aga        1488
   Gly Val Gly Gln His Gln Met Trp Ala Ala Gln Tyr Tyr Lys Tyr Arg
                   485                 490                 495
   aag cca cgc caa tgg ttg aca tct ggt gga tta gga gca atg gga ttt        1536
   Lys Pro Arg Gln Trp Leu Thr Ser Gly Gly Leu Gly Ala Met Gly Phe
               500                 505                 510
   ggt ttg ccc gct gct att ggt gcg gct gtt gga aga ccg gat gaa gtt        1584
   Gly Leu Pro Ala Ala Ile Gly Ala Ala Val Gly Arg Pro Asp Glu Val
           515                 520                 525
   gtg gtt gac att gat ggt gat ggc agt ttc atc atg aat gtg cag gag      1632
   Val Val Asp Ile Asp Gly Asp Gly Ser Phe Ile Met Asn Val Gln Glu
       530                 535                 540
   ctt gca aca att aag gtg gag aat ctc cca gtt aag att atg tta ctg      1680
   Leu Ala Thr Ile Lys Val Glu Asn Leu Pro Val Lys Ile Met Leu Leu
   545                 550                 555                 560
   aat aat caa cac ttg gga atg gtg gtt caa tgg gag gat cgg ttc tat      1728
   Asn Asn Gln His Leu Gly Met Val Val Gln Trp Glu Asp Arg Phe Tyr
                   565                 570                 575
   aag gct aac aga gca cac aca tac ctg ggg aat cct tct aat gag gcg      1776
   Lys Ala Asn Arg Ala His Thr Tyr Leu Gly Asn Pro Ser Asn Glu Ala
               580                 585                 590
   gag atc ttt cct aat atg ctg aaa ttt gca gag gct tgt ggc gta cct      1824
   Glu Ile Phe Pro Asn Met Leu Lys Phe Ala Glu Ala Cys Gly Val Pro
           595                 600                 605
   gct gca aga gtg aca cat agg gat gat ctt aga gct gcc att cag aag      1872
   Ala Ala Arg Val Thr His Arg Asp Asp Leu Arg Ala Ala Ile Gln Lys
       610                 615                 620
   atg tta gac act cct ggg cca tac ttg ttg gat gtg att gta cct cat      1920
   Met Leu Asp Thr Pro Gly Pro Tyr Leu Leu Asp Val Ile Val Pro His
   625                 630                 635                 640
   cag gaa cat gtt tta cct atg att ccc agt ggc gga gct ttc aaa gat      1968
   Gln Glu His Val Leu Pro Met Ile Pro Ser Gly Gly Ala Phe Lys Asp
                   645                 650                 655
   gtg atc aca gag ggt gac ggg aga agt tcc tat tga                      2004
   Val Ile Thr Glu Gly Asp Gly Arg Ser Ser Tyr
               660                 665
<210> 26
   <211> 667
   <212> PRT
   <213> Nicotiana tabacum
<400> 26
   Met Ala Ala Ala Ala Pro Ser Pro Ser Ser Ser Ala Phe Ser Lys Thr
   1               5                   10                  15
   Leu Ser Pro Ser Ser Ser Thr Ser Ser Thr Leu Leu Pro Arg Ser Thr
               20                  25                  30
   Phe Pro Phe Pro His His Pro His Lys Thr Thr Pro Pro Pro Leu His
           35                  40                  45
   Leu Thr His Thr His Ile His Ile His Ser Gln Arg Arg Arg Phe Thr
       50                  55                  60
   Ile Ser Asn Val Ile Ser Thr Asn Gln Lys Val Ser Gln Thr Glu Lys
   65                  70                  75                  80
   Thr Glu Thr Phe Val Ser Arg Phe Ala Pro Asp Glu Pro Arg Lys Gly
                   85                  90                  95
   Ser Asp Val Leu Val Glu Ala Leu Glu Arg Glu Gly Val Thr Asp Val
               100                 105                 110
   Phe Ala Tyr Pro Gly Gly Ala Ser Met Glu Ile His Gln Ala Leu Thr
           115                 120                 125
   Arg Ser Ser Ile Ile Arg Asn Val Leu Pro Arg His Glu Gln Gly Gly
       130                 135                 140
   Val Phe Ala Ala Glu Gly Tyr Ala Arg Ala Thr Gly Phe Pro Gly Val
   145                 150                 155                 160
   Cys Ile Ala Thr Ser Gly Pro Gly Ala Thr Asn Leu Val Ser Gly Leu
                   165                 170                 175
   Ala Asp Ala Leu Leu Asp Ser Val Pro Ile Val Ala Ile Thr Gly Gln
               180                 185                 190
   Val Pro Arg Arg Met Ile Gly Thr Asp Ala Phe Gln Glu Thr Pro Ile
           195                 200                 205
   Val Glu Val Thr Arg Ser Ile Thr Lys His Asn Tyr Leu Val Met Asp
       210                 215                 220
   Val Glu Asp Ile Pro Arg Val Val Arg Glu Ala Phe Phe Leu Ala Arg
   225                 230                 235                 240
   Ser Gly Arg Pro Gly Pro Ile Leu Ile Asp Val Pro Lys Asp Ile Gln
                   245                 250                 255
   Gln Gln Leu Val Ile Pro Asp Trp Asp Gln Pro Met Arg Leu Pro Gly
               260                 265                 270
   Tyr Met Ser Arg Leu Pro Lys Leu Pro Asn Glu Met Leu Leu Glu Gln
           275                 280                 285
   Ile Val Arg Leu Ile Ser Glu Ser Lys Lys Pro Val Leu Tyr Val Gly
       290                 295                 300
   Gly Gly Cys Ser Gln Ser Ser Glu Asp Leu Arg Arg Phe Val Glu Leu
   305                 310                 315                 320
   Thr Gly Ile Pro Val Ala Ser Thr Leu Met Gly Leu Gly Ala Phe Pro
                   325                 330                 335
   Thr Gly Asp Glu Leu Ser Leu Ser Met Leu Gly Met His Gly Thr Val
               340                 345                 350
   Tyr Ala Asn Tyr Ala Val Asp Ser Ser Asp Leu Leu Leu Ala Phe Gly
           355                 360                 365
   Val Arg Phe Asp Asp Arg Val Thr Gly Lys Leu Glu Ala Phe Ala Ser
       370                 375                 380
   Arg Ala Lys Ile Val His Ile Asp Ile Asp Ser Ala Glu Ile Gly Lys
   385                 390                 395                 400
   Asn Lys Gln Pro His Val Ser Ile Cys Ala Asp Ile Lys Leu Ala Leu
                   405                 410                 415
   Gln Gly Leu Asn Ser Ile Leu Glu Ser Lys Glu Gly Lys Leu Lys Leu
               420                 425                 430
   Asp Phe Ser Ala Trp Arg Gln Glu Leu Thr Glu Gln Lys Val Lys His
           435                 440                 445
   Pro Leu Asn Phe Lys Thr Phe Gly Asp Ala Ile Pro Pro Gln Tyr Ala
       450                 455                 460
   Ile Gln Val Leu Asp Glu Leu Thr Asn Gly Asn Ala Ile Ile Ser Thr
   465                 470                 475                 480
   Gly Val Gly Gln His Gln Met Trp Ala Ala Gln Tyr Tyr Lys Tyr Arg
                   485                 490                 495
   Lys Pro Arg Gln Trp Leu Thr Ser Gly Gly Leu Gly Ala Met Gly Phe
               500                 505                 510
   Gly Leu Pro Ala Ala Ile Gly Ala Ala Val Gly Arg Pro Asp Glu Val
           515                 520                 525
   Val Val Asp Ile Asp Gly Asp Gly Ser Phe Ile Met Asn Val Gln Glu
       530                 535                 540
   Leu Ala Thr Ile Lys Val Glu Asn Leu Pro Val Lys Ile Met Leu Leu
   545                 550                 555                 560
   Asn Asn Gln His Leu Gly Met Val Val Gln Trp Glu Asp Arg Phe Tyr
                   565                 570                 575
   Lys Ala Asn Arg Ala His Thr Tyr Leu Gly Asn Pro Ser Asn Glu Ala
                   580                 585                 590
   Glu Ile Phe Pro Asn Met Leu Lys Phe Ala Glu Ala Cys Gly Val Pro
               595                 600                 605
   Ala Ala Arg Val Thr His Arg Asp Asp Leu Arg Ala Ala Ile Gln Lys
       610                 615                 620
   Met Leu Asp Thr Pro Gly Pro Tyr Leu Leu Asp Val Ile Val Pro His
   625                 630                 635                 640
   Gln Glu His Val Leu Pro Met Ile Pro Ser Gly Gly Ala Phe Lys Asp
                   645                 650                 655
   Val Ile Thr Glu Gly Asp Gly Arg Ser Ser Tyr
                   660                 665
<210> 27
   <211> 1980
   <212> DNA
   <213> Solanum tuberosum
<220>
   <221> CDS
   <222> (1)..(1980)
<400> 27
   atg gcg gct gct gcc tca cca tct cca tgt ttc tcc aaa acc cta cct        48
   Met Ala Ala Ala Ala Ser Pro Ser Pro Cys Phe Ser Lys Thr Leu Pro
   1               5                   10                  15
   cca tct tcc tcc aaa tct tcc acc att ctt cct aga tct acc ttc cct        96
   Pro Ser Ser Ser Lys Ser Ser Thr Ile Leu Pro Arg Ser Thr Phe Pro
               20                  25                  30
   ttc cac aat cac cct caa aaa gcc tca ccc ctt cat ctc acc cac acc       144
   Phe His Asn His Pro Gln Lys Ala Ser Pro Leu His Leu Thr His Thr
           35                  40                  45
   cat cat cat cgt cgt ggt ttc gcc gtt tcc aat gtc gtc ata tcc act       192
   His His His Arg Arg Gly Phe Ala Val Ser Asn Val Val Ile Ser Thr
       50                  55                  60
   acc acc cat aac gac gtt tct gaa cct gaa aca ttc gtt tcc cgt ttc       240
   Thr Thr His Asn Asp Val Ser Glu Pro Glu Thr Phe Val Ser Arg Phe
   65                  70                  75                  80
   gcc cct gac gaa ccc aga aag ggt tgt gat gtt ctt gtg gag gca ctt       288
   Ala Pro Asp Glu Pro Arg Lys Gly Cys Asp Val Leu Val Glu Ala Leu
                   85                  90                  95
   gaa agg gag ggg gtt acg gat gta ttt gcg tac cca gga ggt gct tct       336
   Glu Arg Glu Gly Val Thr Asp Val Phe Ala Tyr Pro Gly Gly Ala Ser
               100                 105                 110
   atg gag att cat cag gct ttg aca cgt tcg aat att att cgt aat gtg        384
   Met Glu Ile His Gln Ala Leu Thr Arg Ser Asn Ile Ile Arg Asn Val
           115                 120                 125
   ctg cca cgt cat gag caa ggt ggt gtg ttt gct gca gag ggt tac gca        432
   Leu Pro Arg His Glu Gln Gly Gly Val Phe Ala Ala Glu Gly Tyr Ala
       130                 135                 140
   cgg gcg act ggg ttc cct ggt gtt tgc att gct acc tct ggt ccg gga        480
   Arg Ala Thr Gly Phe Pro Gly Val Cys Ile Ala Thr Ser Gly Pro Gly
   145                 150                 155                 160
   gct acg aat ctt gtt agt ggt ctt gcg gat gct ttg ttg gat agt att        528
   Ala Thr Asn Leu Val Ser Gly Leu Ala Asp Ala Leu Leu Asp Ser Ile
                   165                 170                 175
   ccg att gtt gct att acg ggt caa gtg ccg agg agg atg att ggt act        576
   Pro Ile Val Ala Ile Thr Gly Gln Val Pro Arg Arg Met Ile Gly Thr
               180                 185                 190
   gat gcg ttt cag gaa acg cct att gtt gag gta acg aga tct att acg        624
   Asp Ala Phe Gln Glu Thr Pro Ile Val Glu Val Thr Arg Ser Ile Thr
           195                 200                 205
   aag cat aat tat ctt gtt atg gat gta gag gat att cct agg gtt gtt        672
   Lys His Asn Tyr Leu Val Met Asp Val Glu Asp Ile Pro Arg Val Val
       210                 215                 220
   cgt gaa gcg ttt ttt cta gcg aaa tcg gga cgg cct ggg ccg gtt ttg        720
   Arg Glu Ala Phe Phe Leu Ala Lys Ser Gly Arg Pro Gly Pro Val Leu
   225                 230                 235                 240
   att gat gta cct aag gat att cag caa caa ttg gtg ata cct aat tgg        768
   Ile Asp Val Pro Lys Asp Ile Gln Gln Gln Leu Val Ile Pro Asn Trp
                   245                 250                 255
   gat cag cca atg agg ttg cct ggt tac atg tct agg tta cct aaa ttg        816
   Asp Gln Pro Met Arg Leu Pro Gly Tyr Met Ser Arg Leu Pro Lys Leu
               260                 265                 270
   cct aat gag atg ctt ttg gaa caa att att agg ctg att tcg gag tcg        864
   Pro Asn Glu Met Leu Leu Glu Gln Ile Ile Arg Leu Ile Ser Glu Ser
           275                 280                 285
   aag aag cct gtt ttg tat gtg ggt ggt ggg tgt ttg caa tca agt gag        912
   Lys Lys Pro Val Leu Tyr Val Gly Gly Gly Cys Leu Gln Ser Ser Glu
       290                 295                 300
   gag ctg aga cga ttt gtg gag ctt acg ggt att cct gtg gcg agt act        960
   Glu Leu Arg Arg Phe Val Glu Leu Thr Gly Ile Pro Val Ala Ser Thr
   305                 310                 315                 320
   ttg atg ggt ctt gga gct ttt cca act ggg gat gag ctt tcc ctt caa       1008
   Leu Met Gly Leu Gly Ala Phe Pro Thr Gly Asp Glu Leu Ser Leu Gln
                   325                 330                 335
   atg ttg ggt atg cat ggg act gtg tat gct aat tat gct gtg gat ggt       1056
   Met Leu Gly Met His Gly Thr Val Tyr Ala Asn Tyr Ala Val Asp Gly
               340                 345                 350
   agt gat ttg ttg ctt gca ttt ggg gtg agg ttt gat gat cga gtt act       1104
   Ser Asp Leu Leu Leu Ala Phe Gly Val Arg Phe Asp Asp Arg Val Thr
           355                 360                 365
   ggt aaa ttg gaa gct ttt gct agc cga gcg aaa att gtc cac att gat      1152
   Gly Lys Leu Glu Ala Phe Ala Ser Arg Ala Lys Ile Val His Ile Asp
       370                 375                 380
   att gat tcg gct gag att gga aag aac aag caa cct cat gtt tcc att      1200
   Ile Asp Ser Ala Glu Ile Gly Lys Asn Lys Gln Pro His Val Ser Ile
   385                 390                 395                 400
   tgt gca gat atc aag ttg gca tta cag ggt ttg aat tcc ata ttg gag      1248
   Cys Ala Asp Ile Lys Leu Ala Leu Gln Gly Leu Asn Ser Ile Leu Glu
                   405                 410                 415
   ggt aaa gaa ggt aag ctg aag ttg gac ttt tct gct tgg aga cag gag      1296
   Gly Lys Glu Gly Lys Leu Lys Leu Asp Phe Ser Ala Trp Arg Gln Glu
               420                 425                 430
   tta acg gaa cag aag gtg aag tac cca ttg agt ttt aag act ttt ggt      1344
   Leu Thr Glu Gln Lys Val Lys Tyr Pro Leu Ser Phe Lys Thr Phe Gly
           435                 440                 445
   gaa gcc atc cct cca caa tat gct att cag gtt ctt gat gag tta act      1392
   Glu Ala Ile Pro Pro Gln Tyr Ala Ile Gln Val Leu Asp Glu Leu Thr
       450                 455                 460
   aac gga aat gcc att att agt act ggt gtg ggg caa cac cag atg tgg      1440
   Asn Gly Asn Ala Ile Ile Ser Thr Gly Val Gly Gln His Gln Met Trp
   465                 470                 475                 480
   gct gcc caa tac tat aag tac aaa aag cca cac caa tgg ttg aca tct      1488
   Ala Ala Gln Tyr Tyr Lys Tyr Lys Lys Pro His Gln Trp Leu Thr Ser
                   485                 490                 495
   ggt gga tta gga gca atg gga ttt ggt ttg cct gct gca ata ggt gcg      1536
   Gly Gly Leu Gly Ala Met Gly Phe Gly Leu Pro Ala Ala Ile Gly Ala
               500                 505                 510
   gct gtt gga aga ccg ggt gag att gtg gtt gac att gat ggt gac ggg      1584
   Ala Val Gly Arg Pro Gly Glu Ile Val Val Asp Ile Asp Gly Asp Gly
           515                 520                 525
   agt ttt atc atg aat gtg cag gag tta gca aca att aag gtg gag aat      1632
   Ser Phe Ile Met Asn Val Gln Glu Leu Ala Thr Ile Lys Val Glu Asn
       530                 535                 540
   ctc cca gtt aag att atg ttg ctg aat aat caa cac ttg gga atg gtg      1680
   Leu Pro Val Lys Ile Met Leu Leu Asn Asn Gln His Leu Gly Met Val
   545                 550                 555                 560
   gtt caa tgg gag gat cga ttc tat aag gct aac aga gca cac act tac      1728
   Val Gln Trp Glu Asp Arg Phe Tyr Lys Ala Asn Arg Ala His Thr Tyr
                   565                 570                 575
   ttg ggt gat cct gct aat gag gaa gag atc ttc cct aat atg ttg aaa      1776
   Leu Gly Asp Pro Ala Asn Glu Glu Glu Ile Phe Pro Asn Met Leu Lys
               580                 585                 590
   ttc gca gag gct tgt ggc gta cct gct gca aga gtg tca cac agg gat      1824
   Phe Ala Glu Ala Cys Gly Val Pro Ala Ala Arg Val Ser His Arg Asp
           595                 600                 605
   gat ctt aga gct gcc att caa aag atg tta gac act cct ggg cca tac       1872
   Asp Leu Arg Ala Ala Ile Gln Lys Met Leu Asp Thr Pro Gly Pro Tyr
       610                 615                 620
   ttg ttg gat gtg att gta cct cat cag gag cac gtt cta cct atg att       1920
   Leu Leu Asp Val Ile Val Pro His Gln Glu His Val Leu Pro Met Ile
   625                 630                 635                 640
   ccc agt ggc ggt gct ttc aaa gat gtg atc aca gag ggt gat ggg aga       1968
   Pro Ser Gly Gly Ala Phe Lys Asp Val Ile Thr Glu Gly Asp Gly Arg
                   645                 650                 655
   cgt tca tat tga                                                       1980
   Arg Ser Tyr
<210> 28
   <211> 659
   <212> PRT
   <213> Solanum tuberosum
<400> 28
   Met Ala Ala Ala Ala Ser Pro Ser Pro Cys Phe Ser Lys Thr Leu Pro
   1               5                   10                  15
   Pro Ser Ser Ser Lys Ser Ser Thr Ile Leu Pro Arg Ser Thr Phe Pro
               20                  25                  30
   Phe His Asn His Pro Gln Lys Ala Ser Pro Leu His Leu Thr His Thr
           35                  40                  45
   His His His Arg Arg Gly Phe Ala Val Ser Asn Val Val Ile Ser Thr
       50                  55                  60
   Thr Thr His Asn Asp Val Ser Glu Pro Glu Thr Phe Val Ser Arg Phe
   65                  70                  75                  80
   Ala Pro Asp Glu Pro Arg Lys Gly Cys Asp Val Leu Val Glu Ala Leu
                   85                  90                  95
   Glu Arg Glu Gly Val Thr Asp Val Phe Ala Tyr Pro Gly Gly Ala Ser
               100                 105                 110
   Met Glu Ile His Gln Ala Leu Thr Arg Ser Asn Ile Ile Arg Asn Val
           115                 120                 125
   Leu Pro Arg His Glu Gln Gly Gly Val Phe Ala Ala Glu Gly Tyr Ala
       130                 135                 140
   Arg Ala Thr Gly Phe Pro Gly Val Cys Ile Ala Thr Ser Gly Pro Gly
   145                 150                 155                 160
   Ala Thr Asn Leu Val Ser Gly Leu Ala Asp Ala Leu Leu Asp Ser Ile
   165 170 175
   Pro Ile Val Ala Ile Thr Gly Gln Val Pro Arg Arg Met Ile Gly Thr
   180 185 190
   Asp Ala Phe Gln Glu Thr Pro Ile Val Glu Val Thr Arg Ser Ile Thr
   195 200 205
   Lys His Asn Tyr Leu Val Met Asp Val Glu Asp Ile Pro Arg Val Val
   210 215 220
   Arg Glu Ala Phe Phe Leu Ala Lys Ser Gly Arg Pro Gly Pro Val Leu
   225 230 235 240
   Ile Asp Val Pro Lys Asp Ile Gln Gln Gln Leu Val Ile Pro Asn Trp
   245 250 255
   Asp Gln Pro Met Arg Leu Pro Gly Tyr Met Ser Arg Leu Pro Lys Leu
   260 265 270
   Pro Asn Glu Met Leu Leu Glu Gln Ile Ile Arg Leu Ile Ser Glu Ser
   275 280 285
   Lys Lys Pro Val Leu Tyr Val Gly Gly Gly Cys Leu Gln Ser Ser Glu
   290 295 300
   Glu Leu Arg Arg Phe Val Glu Leu Thr Gly Ile Pro Val Ala Ser Thr
   305 310 315 320
   Leu Met Gly Leu Gly Ala Phe Pro Thr Gly Asp Glu Leu Ser Leu Gln
   325 330 335
   Met Leu Gly Met His Gly Thr Val Tyr Ala Asn Tyr Ala Val Asp Gly
   340 345 350
   Ser Asp Leu Leu Leu Ala Phe Gly Val Arg Phe Asp Asp Arg Val Thr
   355 360 365
   Gly Lys Leu Glu Ala Phe Ala Ser Arg Ala Lys Ile Val His Ile Asp
   370 375 380
   Ile Asp Ser Ala Glu Ile Gly Lys Asn Lys Gln Pro His Val Ser Ile
   385 390 395 400
   Cys Ala Asp Ile Lys Leu Ala Leu Gln Gly Leu Asn Ser Ile Leu Glu
   405 410 415
   Gly Lys Glu Gly Lys Leu Lys Leu Asp Phe Ser Ala Trp Arg Gln Glu
   420 425 430
   Leu Thr Glu Gln Lys Val Lys Tyr Pro Leu Ser Phe Lys Thr Phe Gly
   435 440 445
   Glu Ala Ile Pro Pro Gln Tyr Ala Ile Gln Val Leu Asp Glu Leu Thr
   450 455 460
   Asn Gly Asn Ala Ile Ile Ser Thr Gly Val Gly Gln His Gln Met Trp
   465 470 475 480
   Ala Ala Gln Tyr Tyr Lys Tyr Lys Lys Pro His Gln Trp Leu Thr Ser
   485 490 495
   Gly Gly Leu Gly Ala Met Gly Phe Gly Leu Pro Ala Ala Ile Gly Ala
   500 505 510
   Ala Val Gly Arg Pro Gly Glu Ile Val Val Asp Ile Asp Gly Asp Gly
   515 520 525
   Ser Phe Ile Met Asn Val Gln Glu Leu Ala Thr Ile Lys Val Glu Asn
   530 535 540
   Leu Pro Val Lys Ile Met Leu Leu Asn Asn Gln His Leu Gly Met Val
   545 550 555 560
   Val Gln Trp Glu Asp Arg Phe Tyr Lys Ala Asn Arg Ala His Thr Tyr
   565 570 575
   Leu Gly Asp Pro Ala Asn Glu Glu Glu Ile Phe Pro Asn Met Leu Lys
   580 585 590
   Phe Ala Glu Ala Cys Gly Val Pro Ala Ala Arg Val Ser His Arg Asp
   595 600 605
   Asp Leu Arg Ala Ala Ile Gln Lys Met Leu Asp Thr Pro Gly Pro Tyr
   610 615 620
   Leu Leu Asp Val Ile Val Pro His Gln Glu His Val Leu Pro Met Ile
   625 630 635 640
   Pro Ser Gly Gly Ala Phe Lys Asp Val Ile Thr Glu Gly Asp Gly Arg
   645 650 655
   Arg Ser Tyr
<210> 29
   <211> 1974
   <212> DNA
   <213> Solanum tuberosum
<220>
   <221> CDS
   <222> (1)..(1974)
<400> 29
   atg gcg gct gca tct cca tct cct tgt ttt tcc aaa aac cta cct cca       48
   Met Ala Ala Ala Ser Pro Ser Pro Cys Phe Ser Lys Asn Leu Pro Pro
   1               5                   10                  15
   tct tca tca aaa tct tcc atc ctt ctt ccc aaa tct acc ttt act ttc       96
   Ser Ser Ser Lys Ser Ser Ile Leu Leu Pro Lys Ser Thr Phe Thr Phe
               20                  25                  30
   cac aat cac ccc aaa aat acc tca ccc ctt cac ctt acc cac acc caa      144
   His Asn His Pro Lys Asn Thr Ser Pro Leu His Leu Thr His Thr Gln
           35                  40                  45
   cat cat agc cgt ttc act gtc tca aat gtc atc cta tca acc acg acc      192
   His His Ser Arg Phe Thr Val Ser Asn Val Ile Leu Ser Thr Thr Thr
       50                  55                  60
   cat aac gac gtt tct gaa ccc gaa atc ttc gtt tca cgt ttc gcc cct      240
   His Asn Asp Val Ser Glu Pro Glu Ile Phe Val Ser Arg Phe Ala Pro
   65                  70                  75                  80
   gac gaa ccc aga aag ggt tgt gat gtt ctt gtg gag gca ctt gaa agg      288
   Asp Glu Pro Arg Lys Gly Cys Asp Val Leu Val Glu Ala Leu Glu Arg
                   85                  90                  95
   gaa ggg gtt aag gat gta ttt gca tac cca gga ggt gct tcc atg gag      336
   Glu Gly Val Lys Asp Val Phe Ala Tyr Pro Gly Gly Ala Ser Met Glu
               100                 105                 110
   att cat cag gct ttg aca cgt tcc aat att att cgt aat gtg ctg cca      384
   Ile His Gln Ala Leu Thr Arg Ser Asn Ile Ile Arg Asn Val Leu Pro
           115                 120                 125
   cgt cat gaa cag ggt ggt gtg ttt gct gca gag ggt tac gca cgg gcc      432
   Arg His Glu Gln Gly Gly Val Phe Ala Ala Glu Gly Tyr Ala Arg Ala
       130                 135                 140
   act ggg ttc cct ggt gtt tgc att gct aca tct ggt ccg gga gct acg      480
   Thr Gly Phe Pro Gly Val Cys Ile Ala Thr Ser Gly Pro Gly Ala Thr
   145                 150                 155                 160
   aat ctt gtt agc ggt ctt gcg gat gct ttg ttg gat agt att ccg att      528
   Asn Leu Val Ser Gly Leu Ala Asp Ala Leu Leu Asp Ser Ile Pro Ile
                   165                 170                 175
   gtt gct att acg ggt caa gtg ccg agg agg atg att ggt act gat gcg      576
   Val Ala Ile Thr Gly Gln Val Pro Arg Arg Met Ile Gly Thr Asp Ala
               180                 185                 190
   ttt cag gaa act cct att gtt gag gta acg aga tcc att acg aag cat      624
   Phe Gln Glu Thr Pro Ile Val Glu Val Thr Arg Ser Ile Thr Lys His
           195                 200                 205
   aat tat ctt gtt atg gat gta gag gat att cct agg gtt gtt cgt gaa       672
   Asn Tyr Leu Val Met Asp Val Glu Asp Ile Pro Arg Val Val Arg Glu
       210                 215                 220
   gcg ttt ttt cta gcg aaa tcg gga cgg cct gga ccg gtt ctg att gat       720
   Ala Phe Phe Leu Ala Lys Ser Gly Arg Pro Gly Pro Val Leu Ile Asp
   225                 230                 235                 240
   gtt cct aag gat att cag caa caa ttg gtg ata cct aat tgg gat cag       768
   Val Pro Lys Asp Ile Gln Gln Gln Leu Val Ile Pro Asn Trp Asp Gln
                   245                 250                 255
   cca atg agg ttg cct ggt tac atc tct agg ttg cct aaa ttg cct aat       816
   Pro Met Arg Leu Pro Gly Tyr Ile Ser Arg Leu Pro Lys Leu Pro Asn
               260                 265                 270
   gag atg ctt ttg gaa caa att gtt agg ctg att tcg gag tcg aag aag       864
   Glu Met Leu Leu Glu Gln Ile Val Arg Leu Ile Ser Glu Ser Lys Lys
           275                 280                 285
   cct gtt ttg tat gtg ggt ggt ggg tgt aca caa tcg agt gag gag ctg       912
   Pro Val Leu Tyr Val Gly Gly Gly Cys Thr Gln Ser Ser Glu Glu Leu
       290                 295                 300
   aga cga ttt gtg gag ctt acg ggt att cct gtg gcg agt act ttg atg       960
   Arg Arg Phe Val Glu Leu Thr Gly Ile Pro Val Ala Ser Thr Leu Met
   305                 310                 315                 320
   ggt ctt gga act ttt cca tgt ggg gat gag ctt tct ctt caa atg ttg      1008
   Gly Leu Gly Thr Phe Pro Cys Gly Asp Glu Leu Ser Leu Gln Met Leu
                   325                 330                 335
   ggt atg cat ggg act gtg tat gct aat tat gcg gtg gat agt agt gat      1056
   Gly Met His Gly Thr Val Tyr Ala Asn Tyr Ala Val Asp Ser Ser Asp
               340                 345                 350
   ttg ttg ctt gca ttt ggg gtg agg ttt gat gat cga gtt act ggt aaa      1104
   Leu Leu Leu Ala Phe Gly Val Arg Phe Asp Asp Arg Val Thr Gly Lys
           355                 360                 365
   ttg gaa gct ttt gct agc cga gcg aaa att gtc cac att gat att gat      1152
   Leu Glu Ala Phe Ala Ser Arg Ala Lys Ile Val His Ile Asp Ile Asp
       370                 375                 380
   tcg gct gag att gga aag aac aag caa cct cat gtt tcc att tgt gca      1200
   Ser Ala Glu Ile Gly Lys Asn Lys Gln Pro His Val Ser Ile Cys Ala
   385                 390                 395                 400
   gat atc aag ttg gca tta cag ggt ttg aat tcc ata ttg gag ggt aaa      1248
   Asp Ile Lys Leu Ala Leu Gln Gly Leu Asn Ser Ile Leu Glu Gly Lys
                   405                 410                 415
   gaa ggt aag ctg aag ttg gac ttt tct gct tgg agg cag gag tta acg      1296
   Glu Gly Lys Leu Lys Leu Asp Phe Ser Ala Trp Arg Gln Glu Leu Thr
               420                 425                 430
   gag cag aag gtg aag tac cca ttg aat tat aag act ttt ggt gaa gcc      1344
   Glu Gln Lys Val Lys Tyr Pro Leu Asn Tyr Lys Thr Phe Gly Glu Ala
           435                 440                 445
   atc cct cca caa tat gct att cag gtt ctt gat gag tta act aac gga       1392
   Ile Pro Pro Gln Tyr Ala Ile Gln Val Leu Asp Glu Leu Thr Asn Gly
       450                 455                 460
   aat gcc att att agt act ggt gtg ggg caa cac caa atg tgg gct gcc       1440
   Asn Ala Ile Ile Ser Thr Gly Val Gly Gln His Gln Met Trp Ala Ala
   465                 470                 475                 480
   caa tac tat aag tac aaa aag cca cgc caa tgg ttg aca tct ggt gga       1488
   Gln Tyr Tyr Lys Tyr Lys Lys Pro Arg Gln Trp Leu Thr Ser Gly Gly
                   485                 490                 495
   tta gga gca atg gga ttt ggt ttg cct gct gct ata ggt gcg gct gtt       1536
   Leu Gly Ala Met Gly Phe Gly Leu Pro Ala Ala Ile Gly Ala Ala Val
               500                 505                 510
   gga aga ccg ggt gag att gtg gtt gac att gat ggt gac ggg agt ttt       1584
   Gly Arg Pro Gly Glu Ile Val Val Asp Ile Asp Gly Asp Gly Ser Phe
           515                 520                 525
   atc atg aat gtg cag gag tta gca aca att aag gtg gag aat ctc cca       1632
   Ile Met Asn Val Gln Glu Leu Ala Thr Ile Lys Val Glu Asn Leu Pro
       530                 535                 540
   gtt aag att atg tta ctg aat aat caa cac ttg gga atg gtg gtt cag       1680
   Val Lys Ile Met Leu Leu Asn Asn Gln His Leu Gly Met Val Val Gln
   545                 550                 555                 560
   tgg gag gat cga ttc tat aag gct aac aga gca cac act tac ttg ggt       1728
   Trp Glu Asp Arg Phe Tyr Lys Ala Asn Arg Ala His Thr Tyr Leu Gly
                   565                 570                 575
   aat cct gct aat gag gaa gaa atc ttc cct aat atg ctg aaa ttt gca       1776
   Asn Pro Ala Asn Glu Glu Glu Ile Phe Pro Asn Met Leu Lys Phe Ala
               580                 585                 590
   gag gct tgt ggc gta cct gct gca aga gtg tca cac agg gat gat ctt       1824
   Glu Ala Cys Gly Val Pro Ala Ala Arg Val Ser His Arg Asp Asp Leu
           595                 600                 605
   aga gct gcc att caa aag atg tta gac act cct ggg cca tac ttg ttg       1872
   Arg Ala Ala Ile Gln Lys Met Leu Asp Thr Pro Gly Pro Tyr Leu Leu
       610                 615                 620
   gat gtg att gta cct cat cag gag cac gtt cta cct atg att ccc agt       1920
   Asp Val Ile Val Pro His Gln Glu His Val Leu Pro Met Ile Pro Ser
   625                 630                 635                 640
   ggt ggc gct ttc aaa gat gtg att acg gag ggt gat ggg aga cgt tcc       1968
   Gly Gly Ala Phe Lys Asp Val Ile Thr Glu Gly Asp Gly Arg Arg Ser
                   645                 650                 655
   tat tga                                                               1974
   Tyr
<210> 30
   <211> 657
   <212> PRT
   <213> Solanum tuberosum
<400> 30
   Met Ala Ala Ala Ser Pro Ser Pro Cys Phe Ser Lys Asn Leu Pro Pro
   1               5                   10                  15
   Ser Ser Ser Lys Ser Ser Ile Leu Leu Pro Lys Ser Thr Phe Thr Phe
               20                  25                  30
   His Asn His Pro Lys Asn Thr Ser Pro Leu His Leu Thr His Thr Gln
           35                  40                  45
   His His Ser Arg Phe Thr Val Ser Asn Val Ile Leu Ser Thr Thr Thr
       50                  55                  60
   His Asn Asp Val Ser Glu Pro Glu Ile Phe Val Ser Arg Phe Ala Pro
   65                  70                  75                  80
   Asp Glu Pro Arg Lys Gly Cys Asp Val Leu Val Glu Ala Leu Glu Arg
                   85                  90                  95
   Glu Gly Val Lys Asp Val Phe Ala Tyr Pro Gly Gly Ala Ser Met Glu
               100                 105                 110
   Ile His Gln Ala Leu Thr Arg Ser Asn Ile Ile Arg Asn Val Leu Pro
           115                 120                 125
   Arg His Glu Gln Gly Gly Val Phe Ala Ala Glu Gly Tyr Ala Arg Ala
       130                 135                 140
   Thr Gly Phe Pro Gly Val Cys Ile Ala Thr Ser Gly Pro Gly Ala Thr
   145                 150                 155                 160
   Asn Leu Val Ser Gly Leu Ala Asp Ala Leu Leu Asp Ser Ile Pro Ile
                   165                 170                 175
   Val Ala Ile Thr Gly Gln Val Pro Arg Arg Met Ile Gly Thr Asp Ala
               180                 185                 190
   Phe Gln Glu Thr Pro Ile Val Glu Val Thr Arg Ser Ile Thr Lys His
           195                 200                 205
   Asn Tyr Leu Val Met Asp Val Glu Asp Ile Pro Arg Val Val Arg Glu
       210                 215                 220
   Ala Phe Phe Leu Ala Lys Ser Gly Arg Pro Gly Pro Val Leu Ile Asp
   225                 230                 235                 240
   Val Pro Lys Asp Ile Gln Gln Gln Leu Val Ile Pro Asn Trp Asp Gln
                   245                 250                 255
   Pro Met Arg Leu Pro Gly Tyr Ile Ser Arg Leu Pro Lys Leu Pro Asn
   260 265 270
   Glu Met Leu Leu Glu Gln Ile Val Arg Leu Ile Ser Glu Ser Lys Lys
   275 280 285
   Pro Val Leu Tyr Val Gly Gly Gly Cys Thr Gln Ser Ser Glu Glu Leu
   290 295 300
   Arg Arg Phe Val Glu Leu Thr Gly Ile Pro Val Ala Ser Thr Leu Met
   305 310 315 320
   Gly Leu Gly Thr Phe Pro Cys Gly Asp Glu Leu Ser Leu Gln Met Leu
   325 330 335
   Gly Met His Gly Thr Val Tyr Ala Asn Tyr Ala Val Asp Ser Ser Asp
   340 345 350
   Leu Leu Leu Ala Phe Gly Val Arg Phe Asp Asp Arg Val Thr Gly Lys
   355 360 365
   Leu Glu Ala Phe Ala Ser Arg Ala Lys Ile Val His Ile Asp Ile Asp
   370 375 380
   Ser Ala Glu Ile Gly Lys Asn Lys Gln Pro His Val Ser Ile Cys Ala
   385 390 395 400
   Asp Ile Lys Leu Ala Leu Gln Gly Leu Asn Ser Ile Leu Glu Gly Lys
   405 410 415
   Glu Gly Lys Leu Lys Leu Asp Phe Ser Ala Trp Arg Gln Glu Leu Thr
   420 425 430
   Glu Gln Lys Val Lys Tyr Pro Leu Asn Tyr Lys Thr Phe Gly Glu Ala
   435 440 445
   Ile Pro Pro Gln Tyr Ala Ile Gln Val Leu Asp Glu Leu Thr Asn Gly
   450 455 460
   Asn Ala Ile Ile Ser Thr Gly Val Gly Gln His Gln Met Trp Ala Ala
   465 470 475 480
   Gln Tyr Tyr Lys Tyr Lys Lys Pro Arg Gln Trp Leu Thr Ser Gly Gly
   485 490 495
   Leu Gly Ala Met Gly Phe Gly Leu Pro Ala Ala Ile Gly Ala Ala Val
                   500                 505                 510
   Gly Arg Pro Gly Glu Ile Val Val Asp Ile Asp Gly Asp Gly Ser Phe
           515                 520                 525
   Ile Met Asn Val Gln Glu Leu Ala Thr Ile Lys Val Glu Asn Leu Pro
       530                 535                 540
   Val Lys Ile Met Leu Leu Asn Asn Gln His Leu Gly Met Val Val Gln
   545                 550                 555                 560
   Trp Glu Asp Arg Phe Tyr Lys Ala Asn Arg Ala His Thr Tyr Leu Gly
                   565                 570                 575
   Asn Pro Ala Asn Glu Glu Glu Ile Phe Pro Asn Met Leu Lys Phe Ala
               580                 585                 590
   Glu Ala Cys Gly Val Pro Ala Ala Arg Val Ser His Arg Asp Asp Leu
           595                 600                 605
   Arg Ala Ala Ile Gln Lys Met Leu Asp Thr Pro Gly Pro Tyr Leu Leu
       610                 615                 620
   Asp Val Ile Val Pro His Gln Glu His Val Leu Pro Met Ile Pro Ser
   625                 630                 635                 640
   Gly Gly Ala Phe Lys Asp Val Ile Thr Glu Gly Asp Gly Arg Arg Ser
                   645                 650                 655
   Tyr
<210> 31
   <211> 1971
   <212> DNA
   <213> Solanum tuberosum
<220>
   <221> CDS
   <222> (1)..(1971)
<400> 31
   atg gcg gca tct tct cca aat cct tcc tct tcc act ttc ctc aac aat        48
   Met Ala Ala Ser Ser Pro Asn Pro Ser Ser Ser Thr Phe Leu Asn Asn
   1               5                   10                  15
   cct ttt aca tca tct tct tat tat tcc tct aag ctt ctt tcc aga acc        96
   Pro Phe Thr Ser Ser Ser Tyr Tyr Ser Ser Lys Leu Leu Ser Arg Thr
               20                  25                  30
   ttc ttt cct ttt cca cac act cca aca att att cct cat aaa tct ctt       144
   Phe Phe Pro Phe Pro His Thr Pro Thr Ile Ile Pro His Lys Ser Leu
           35                  40                  45
   acc atc acc atc gac gtt tcc aca acc cca aat ggt gct cca gta cca      192
   Thr Ile Thr Ile Asp Val Ser Thr Thr Pro Asn Gly Ala Pro Val Pro
       50                  55                  60
   tta atg gag acg aca ttc act tct cga ttc ctc ccc gat gaa ccc cga      240
   Leu Met Glu Thr Thr Phe Thr Ser Arg Phe Leu Pro Asp Glu Pro Arg
   65                  70                  75                  80
   aaa gga tgt gac atc ttg gtt gaa gcc ctt gag cgt gaa ggc gtt aac      288
   Lys Gly Cys Asp Ile Leu Val Glu Ala Leu Glu Arg Glu Gly Val Asn
                   85                  90                  95
   aat gta ttt gct tat cca ggc ggt act act ctg gaa att cac cag gct      336
   Asn Val Phe Ala Tyr Pro Gly Gly Thr Thr Leu Glu Ile His Gln Ala
               100                 105                 110
   ctt acc cgt tct cca acc ata agg aat ata ttg cca cgt cat gaa cag      384
   Leu Thr Arg Ser Pro Thr Ile Arg Asn Ile Leu Pro Arg His Glu Gln
           115                 120                 125
   ggt ggc gtt ttc gcg gct gaa ggt tat gca cgc gcc acc ggg ttc cct      432
   Gly Gly Val Phe Ala Ala Glu Gly Tyr Ala Arg Ala Thr Gly Phe Pro
       130                 135                 140
   ggt gtt tgt atg gct acc tct ggc cct gga gct act aat ctt att agt      480
   Gly Val Cys Met Ala Thr Ser Gly Pro Gly Ala Thr Asn Leu Ile Ser
   145                 150                 155                 160
   ggt ctt gct gat gct atg gct gat agt att cca att gtt gct att aca      528
   Gly Leu Ala Asp Ala Met Ala Asp Ser Ile Pro Ile Val Ala Ile Thr
                   165                 170                 175
   ggt caa gtt cct cgt aga atg att gga aca gac gca ttt caa gaa att      576
   Gly Gln Val Pro Arg Arg Met Ile Gly Thr Asp Ala Phe Gln Glu Ile
               180                 185                 190
   cca att att gag gta act aga tca att acc aag cat aat tat cta gtc      624
   Pro Ile Ile Glu Val Thr Arg Ser Ile Thr Lys His Asn Tyr Leu Val
           195                 200                 205
   atg gac gtt cac gat att ccc agg att gtc cga gag gcg ttt ttc cta      672
   Met Asp Val His Asp Ile Pro Arg Ile Val Arg Glu Ala Phe Phe Leu
       210                 215                 220
   gct cta tca gga cga cct gga ccg gtt tta att gat gtt cct aaa gat      720
   Ala Leu Ser Gly Arg Pro Gly Pro Val Leu Ile Asp Val Pro Lys Asp
   225                 230                 235                 240
   gtt caa caa caa atg gat att cct aat tgg gat cag ccc atg aag ttg      768
   Val Gln Gln Gln Met Asp Ile Pro Asn Trp Asp Gln Pro Met Lys Leu
                   245                 250                 255
   cca ggt tat atg tct aga ctc ccg tta cca cca aaa aag acg cta ttg      816
   Pro Gly Tyr Met Ser Arg Leu Pro Leu Pro Pro Lys Lys Thr Leu Leu
               260                 265                 270
   gaa caa att gtt aga tta ttt tcc gag tca aag aag cct gtt ttg tat      864
   Glu Gln Ile Val Arg Leu Phe Ser Glu Ser Lys Lys Pro Val Leu Tyr
           275                 280                 285
   gtt ggt gga ggg tgc gta caa tcc agc aat gag tta aga cgt ttt gtt        912
   Val Gly Gly Gly Cys Val Gln Ser Ser Asn Glu Leu Arg Arg Phe Val
       290                 295                 300
   cag ctc aca ggt att cct gtg gcg agt act ttg atg gga cta gga gcg        960
   Gln Leu Thr Gly Ile Pro Val Ala Ser Thr Leu Met Gly Leu Gly Ala
   305                 310                 315                 320
   ttt cca aca ggg gat gag ctt tcg ctt caa atg cta ggg atg cat ggt       1008
   Phe Pro Thr Gly Asp Glu Leu Ser Leu Gln Met Leu Gly Met His Gly
                   325                 330                 335
   act gtt tat tca aat tac gcg gtg gat agt agt gat ttg ctt cta gca       1056
   Thr Val Tyr Ser Asn Tyr Ala Val Asp Ser Ser Asp Leu Leu Leu Ala
               340                 345                 350
   ttt gga gtg agg ttt gat gat cgt gtc acg ggt aaa tta gaa aca ttt       1104
   Phe Gly Val Arg Phe Asp Asp Arg Val Thr Gly Lys Leu Glu Thr Phe
           355                 360                 365
   gca agc aga gca aaa att gtt cac att gat atc gac tcc aaa gag att       1152
   Ala Ser Arg Ala Lys Ile Val His Ile Asp Ile Asp Ser Lys Glu Ile
       370                 375                 380
   ggg aaa aac aag aag cct cat gta tct att tgt act gat att aag ctg       1200
   Gly Lys Asn Lys Lys Pro His Val Ser Ile Cys Thr Asp Ile Lys Leu
   385                 390                 395                 400
   gcg tta cag ggg ttg aat ttg ata tta atg gag agg gaa aat aca aga       1248
   Ala Leu Gln Gly Leu Asn Leu Ile Leu Met Glu Arg Glu Asn Thr Arg
                   405                 410                 415
   aac gcg ttg aag ctg aat ttc tcg cct tgg agg aaa gag tta aca gag       1296
   Asn Ala Leu Lys Leu Asn Phe Ser Pro Trp Arg Lys Glu Leu Thr Glu
               420                 425                 430
   caa aaa ttg aag tat cct ttg aaa tac aag ttt tat ggt gga tct att       1344
   Gln Lys Leu Lys Tyr Pro Leu Lys Tyr Lys Phe Tyr Gly Gly Ser Ile
           435                 440                 445
   cct cca caa tat gca att gaa gtc ctt gat gag tta act aat gga aat       1392
   Pro Pro Gln Tyr Ala Ile Glu Val Leu Asp Glu Leu Thr Asn Gly Asn
       450                 455                 460
   gct att ata aca agt ggt gta ggg caa cac caa atg tgg tct gct caa       1440
   Ala Ile Ile Thr Ser Gly Val Gly Gln His Gln Met Trp Ser Ala Gln
   465                 470                 475                 480
   tac tat aag tac aaa aat cca atg cag tgt ttg aca tct ggt gga ttc       1488
   Tyr Tyr Lys Tyr Lys Asn Pro Met Gln Cys Leu Thr Ser Gly Gly Phe
                   485                 490                 495
   gga gcg atg ggg ttt gga tta cct gct gca ata gga gca gca ata gca       1536
   Gly Ala Met Gly Phe Gly Leu Pro Ala Ala Ile Gly Ala Ala Ile Ala
               500                 505                 510
   aga cca gat gca att gtt gtg gac atc gat ggg gat ggt agt ttc atg       1584
   Arg Pro Asp Ala Ile Val Val Asp Ile Asp Gly Asp Gly Ser Phe Met
           515                 520                 525
   atg aat gtg caa gag tta gca aca gtt aga gtc gag aat ctt cct gtt       1632
   Met Asn Val Gln Glu Leu Ala Thr Val Arg Val Glu Asn Leu Pro Val
       530                 535                 540
   aag atg atg att tta aat aat caa cat ttg gga atg gcg aca cag tgg       1680
   Lys Met Met Ile Leu Asn Asn Gln His Leu Gly Met Ala Thr Gln Trp
   545                 550                 555                 560
   gag gat agg ttc tat aag gcg aat aga gca gat tct tat ctc gga aat       1728
   Glu Asp Arg Phe Tyr Lys Ala Asn Arg Ala Asp Ser Tyr Leu Gly Asn
                   565                 570                 575
   cct tct aac aag gca cgg att ttc cct aac atg ttg aaa ttc gcg gag       1776
   Pro Ser Asn Lys Ala Arg Ile Phe Pro Asn Met Leu Lys Phe Ala Glu
               580                 585                 590
   gct tgt gat ata cct gct gcc cga gtg att aat aat aat gat gtt aga       1824
   Ala Cys Asp Ile Pro Ala Ala Arg Val Ile Asn Asn Asn Asp Val Arg
           595                 600                 605
   gat gct ata cga aag atg tta gat act cct ggg cct tac ttg ttg gat       1872
   Asp Ala Ile Arg Lys Met Leu Asp Thr Pro Gly Pro Tyr Leu Leu Asp
       610                 615                 620
   gtg att gtg tct cat cag gag cat gtt ttg cct atg att ccc agt gat       1920
   Val Ile Val Ser His Gln Glu His Val Leu Pro Met Ile Pro Ser Asp
   625                 630                 635                 640
   ggg gct ttt aag gat gtt atc acg gag ggc gat ggg aga tgt tcc tac       1968
   Gly Ala Phe Lys Asp Val Ile Thr Glu Gly Asp Gly Arg Cys Ser Tyr
                   645                 650                 655
   tga                                                                   1971
<210> 32
   <211> 656
   <212> PRT
   <213> Solanum tuberosum
<400> 32
   Met Ala Ala Ser Ser Pro Asn Pro Ser Ser Ser Thr Phe Leu Asn Asn
   1               5                   10                  15
   Pro Phe Thr Ser Ser Ser Tyr Tyr Ser Ser Lys Leu Leu Ser Arg Thr
               20                  25                  30
   Phe Phe Pro Phe Pro His Thr Pro Thr Ile Ile Pro His Lys Ser Leu
           35                  40                  45
   Thr Ile Thr Ile Asp Val Ser Thr Thr Pro Asn Gly Ala Pro Val Pro
       50                  55                  60
   Leu Met Glu Thr Thr Phe Thr Ser Arg Phe Leu Pro Asp Glu Pro Arg
   65                  70                  75                  80
   Lys Gly Cys Asp Ile Leu Val Glu Ala Leu Glu Arg Glu Gly Val Asn
                   85                  90                  95
   Asn Val Phe Ala Tyr Pro Gly Gly Thr Thr Leu Glu Ile His Gln Ala
   100 105 110
   Leu Thr Arg Ser Pro Thr Ile Arg Asn Ile Leu Pro Arg His Glu Gln
   115 120 125
   Gly Gly Val Phe Ala Ala Glu Gly Tyr Ala Arg Ala Thr Gly Phe Pro
   130 135 140
   Gly Val Cys Met Ala Thr Ser Gly Pro Gly Ala Thr Asn Leu Ile Ser
   145 150 155 160
   Gly Leu Ala Asp Ala Met Ala Asp Ser Ile Pro Ile Val Ala Ile Thr
   165 170 175
   Gly Gln Val Pro Arg Arg Met Ile Gly Thr Asp Ala Phe Gln Glu Ile
   180 185 190
   Pro Ile Ile Glu Val Thr Arg Ser Ile Thr Lys His Asn Tyr Leu Val
   195 200 205
   Met Asp Val His Asp Ile Pro Arg Ile Val Arg Glu Ala Phe Phe Leu
   210 215 220
   Ala Leu Ser Gly Arg Pro Gly Pro Val Leu Ile Asp Val Pro Lys Asp
   225 230 235 240
   Val Gln Gln Gln Met Asp Ile Pro Asn Trp Asp Gln Pro Met Lys Leu
   245 250 255
   Pro Gly Tyr Met Ser Arg Leu Pro Leu Pro Pro Lys Lys Thr Leu Leu
   260 265 270
   Glu Gln Ile Val Arg Leu Phe Ser Glu Ser Lys Lys Pro Val Leu Tyr
   275 280 285
   Val Gly Gly Gly Cys Val Gln Ser Ser Asn Glu Leu Arg Arg Phe Val
   290 295 300
   Gln Leu Thr Gly Ile Pro Val Ala Ser Thr Leu Met Gly Leu Gly Ala
   305 310 315 320
   Phe Pro Thr Gly Asp Glu Leu Ser Leu Gln Met Leu Gly Met His Gly
   325 330 335
   Thr Val Tyr Ser Asn Tyr Ala Val Asp Ser Ser Asp Leu Leu Leu Ala
   340 345 350
   Phe Gly Val Arg Phe Asp Asp Arg Val Thr Gly Lys Leu Glu Thr Phe
           355                 360                 365
   Ala Ser Arg Ala Lys Ile Val His Ile Asp Ile Asp Ser Lys Glu Ile
       370                 375                 380
   Gly Lys Asn Lys Lys Pro His Val Ser Ile Cys Thr Asp Ile Lys Leu
   385                 390                 395                 400
   Ala Leu Gln Gly Leu Asn Leu Ile Leu Met Glu Arg Glu Asn Thr Arg
                   405                 410                 415
   Asn Ala Leu Lys Leu Asn Phe Ser Pro Trp Arg Lys Glu Leu Thr Glu
               420                 425                 430
   Gln Lys Leu Lys Tyr Pro Leu Lys Tyr Lys Phe Tyr Gly Gly Ser Ile
           435                 440                 445
   Pro Pro Gln Tyr Ala Ile Glu Val Leu Asp Glu Leu Thr Asn Gly Asn
       450                 455                 460
   Ala Ile Ile Thr Ser Gly Val Gly Gln His Gln Met Trp Ser Ala Gln
   465                 470                 475                 480
   Tyr Tyr Lys Tyr Lys Asn Pro Met Gln Cys Leu Thr Ser Gly Gly Phe
                   485                 490                 495
   Gly Ala Met Gly Phe Gly Leu Pro Ala Ala Ile Gly Ala Ala Ile Ala
               500                 505                 510
   Arg Pro Asp Ala Ile Val Val Asp Ile Asp Gly Asp Gly Ser Phe Met
           515                 520                 525
   Met Asn Val Gln Glu Leu Ala Thr Val Arg Val Glu Asn Leu Pro Val
       530                 535                 540
   Lys Met Met Ile Leu Asn Asn Gln His Leu Gly Met Ala Thr Gln Trp
   545                 550                 555                 560
   Glu Asp Arg Phe Tyr Lys Ala Asn Arg Ala Asp Ser Tyr Leu Gly Asn
                   565                 570                 575
   Pro Ser Asn Lys Ala Arg Ile Phe Pro Asn Met Leu Lys Phe Ala Glu
               580                 585                 590
   Ala Cys Asp Ile Pro Ala Ala Arg Val Ile Asn Asn Asn Asp Val Arg
           595                 600                 605
   Asp Ala Ile Arg Lys Met Leu Asp Thr Pro Gly Pro Tyr Leu Leu Asp
   610 615 620
   Val Ile Val Ser His Gln Glu His Val Leu Pro Met Ile Pro Ser Asp
   625 630 635 640
   Gly Ala Phe Lys Asp Val Ile Thr Glu Gly Asp Gly Arg Cys Ser Tyr
   645 650 655
<210> 33
   <211> 1797
   <212> DNA
   <213> Triticum aestivum
<400> 33
   gccgcgtccc ccgccgccac ctccgtcgcg cctcccgcca ccgcgctccg gccgtggggc 60
   ccctccgagc cccgcaaggg cgccgacatc ctcgtcgagg cgctggagcg ctgcggcatc 120
   gtcgacgtct tcgcctaccc tggcggcgcg tccatggaga tccaccaggc gctgacgcgc 180
   tcgccagtca tcaccaacca cctcttccgc cacgagcagg gggaggcgtt cgcggcgtcc 240
   gggtacgccc gcgcgtccgg ccgcgtcggc gtctgcgtcg ccacctccgg cccgggggcc 300
   accaacctcg tctccgcgct cgccgacgct ctcctcgact ccatccccat ggtcgccatc 360
   acgggccagg tcccccgccg catgatcggc acggatgcgt tccaggagac gcccatcgtg 420
   gaggtcacgc gctccatcac caagcacaac tacctggtcc ttgacgtgga ggatatcccc 480
   cgcgtcatcc aggaagcctt cttcctcgca tcctctggcc gcccggggcc ggtgctggtt 540
   gatatcccca aggacatcca gcagcagatg gctgtgcctg tctgggacac gccgatgagt 600
   ttgccagggt acatcgcccg cctgcccaag ccaccatcta ctgaatcgct tgagcaggtc 660
   ctgcgtctgg ttggcgagtc acggcgccca attctgtatg ttggtggtgg ctgcgctgca 720
   tctggtgagg agttgcgccg ctttgttgag ctcactggga ttccagttac aactactctt 780
   atgggccttg gcaacttccc cagtgacgac ccactgtctc tgcgcatgct ggggatgcat 840
   ggcactgtgt atgcaaatta tgcagtagat aaggctgacc tgttgcttgc atttggtgtg 900
   cggtttgatg atcgtgtgac cgggaaaatc gaggcttttg caagcaggtc caagattgtg 960
   cacattgaca ttgacccagc tgagattggc aagaacaagc agccacatgt ctccatttgt 1020
   gcagatgtta agcttgcttt acaggggttg aatgctctat taaatgggag caaagcacaa 1080
   cagggtctgg attttggtcc atggcacaag gagttggatc agcagaagag ggagtttcct 1140
   ctaggattca agacttttgg tgaggccatc ccgccgcaat atgctatcca ggtactggat 1200
   gagctgacaa aaggggaggc gatcattgcc accggtgttg ggcagcatca gatgtgggcg 1260
   gctcagtatt acacttacaa gcggccacgg cagtggctgt cttcatccgg tttgggtgca 1320
   atgggatttg ggttgccagc tgcagctggc gctgctgtgg ccaacccagg tgttacagtt 1380
   gttgacattg atggggatgg tagtttcctc atgaacattc aggagttggc gttgatccgt 1440
   attgagaacc tcccagtgaa ggtgatgata ttgaacaacc agcatctggg aatggtggtg      1500
   cagtgggagg ataggtttta caaggccaac cgggcgcaca cataccttgg caacccagaa      1560
   aatgagagtg agatatatcc agattttgtg acgattgcta aaggattcaa cgttccggca 1620
   gttcgtgtga cgaagaagag cgaagtcact gcagcaatca agaagatgct tgagacccca 1680
   gggccatact tgttggatat cattgtcccg catcaggagc acgtgctgcc tatgatccca 1740
   agcggtggtg cttttaagga catgatcatg gagggtgatg gcaggacctc gtactga 1797
<210> 34
   <211> 598
   <212> PRT
   <213> Triticum aestivum
<400> 34
   Ala Ala Ser Pro Ala Ala Thr Ser Val Ala Pro Pro Ala Thr Ala Leu
   1               5                   10                  15
   Arg Pro Trp Gly Pro Ser Glu Pro Arg Lys Gly Ala Asp Ile Leu Val
               20                  25                  30
   Glu Ala Leu Glu Arg Cys Gly Ile Val Asp Val Phe Ala Tyr Pro Gly
           35                  40                  45
   Gly Ala Ser Met Glu Ile His Gln Ala Leu Thr Arg Ser Pro Val Ile
       50                  55                  60
   Thr Asn His Leu Phe Arg His Glu Gln Gly Glu Ala Phe Ala Ala Ser
   65                  70                  75                  80
   Gly Tyr Ala Arg Ala Ser Gly Arg Val Gly Val Cys Val Ala Thr Ser
                   85                  90                  95
   Gly Pro Gly Ala Thr Asn Leu Val Ser Ala Leu Ala Asp Ala Leu Leu
               100                 105                 110
   Asp Ser Ile Pro Met Val Ala Ile Thr Gly Gln Val Pro Arg Arg Met
           115                 120                 125
   Ile Gly Thr Asp Ala Phe Gln Glu Thr Pro Ile Val Glu Val Thr Arg
       130                 135                 140
   Ser Ile Thr Lys His Asn Tyr Leu Val Leu Asp Val Glu Asp Ile Pro
   145                 150                 155                 160
   Arg Val Ile Gln Glu Ala Phe Phe Leu Ala Ser Ser Gly Arg Pro Gly
                   165                 170                 175
   Pro Val Leu Val Asp Ile Pro Lys Asp Ile Gln Gln Gln Met Ala Val
               180                 185                 190
   Pro Val Trp Asp Thr Pro Met Ser Leu Pro Gly Tyr Ile Ala Arg Leu
           195                 200                 205
   Pro Lys Pro Pro Ser Thr Glu Ser Leu Glu Gln Val Leu Arg Leu Val
       210                 215                 220
   Gly Glu Ser Arg Arg Pro Ile Leu Tyr Val Gly Gly Gly Cys Ala Ala
   225                 230                 235                 240
   Ser Gly Glu Glu Leu Arg Arg Phe Val Glu Leu Thr Gly Ile Pro Val
                   245                 250                 255
   Thr Thr Thr Leu Met Gly Leu Gly Asn Phe Pro Ser Asp Asp Pro Leu
               260                 265                 270
   Ser Leu Arg Met Leu Gly Met His Gly Thr Val Tyr Ala Asn Tyr Ala
           275                 280                 285
   Val Asp Lys Ala Asp Leu Leu Leu Ala Phe Gly Val Arg Phe Asp Asp
       290                 295                 300
   Arg Val Thr Gly Lys Ile Glu Ala Phe Ala Ser Arg Ser Lys Ile Val
   305                 310                 315                 320
   His Ile Asp Ile Asp Pro Ala Glu Ile Gly Lys Asn Lys Gln Pro His
                   325                 330                 335
   Val Ser Ile Cys Ala Asp Val Lys Leu Ala Leu Gln Gly Leu Asn Ala
               340                 345                 350
   Leu Leu Asn Gly Ser Lys Ala Gln Gln Gly Leu Asp Phe Gly Pro Trp
           355                 360                 365
   His Lys Glu Leu Asp Gln Gln Lys Arg Glu Phe Pro Leu Gly Phe Lys
       370                 375                 380
   Thr Phe Gly Glu Ala Ile Pro Pro Gln Tyr Ala Ile Gln Val Leu Asp
   385                 390                 395                 400
   Glu Leu Thr Lys Gly Glu Ala Ile Ile Ala Thr Gly Val Gly Gln His
   405 410 415
   Gln Met Trp Ala Ala Gln Tyr Tyr Thr Tyr Lys Arg Pro Arg Gln Trp
   420 425 430
   Leu Ser Ser Ser Gly Leu Gly Ala Met Gly Phe Gly Leu Pro Ala Ala
   435 440 445
   Ala Gly Ala Ala Val Ala Asn Pro Gly Val Thr Val Val Asp Ile Asp
   450 455 460
   Gly Asp Gly Ser Phe Leu Met Asn Ile Gln Glu Leu Ala Leu Ile Arg
   465 470 475 480
   Ile Glu Asn Leu Pro Val Lys Val Met Ile Leu Asn Asn Gln His Leu
   485 490 495
   Gly Met Val Val Gln Trp Glu Asp Arg Phe Tyr Lys Ala Asn Arg Ala
   500 505 510
   His Thr Tyr Leu Gly Asn Pro Glu Asn Glu Ser Glu Ile Tyr Pro Asp
   515 520 525
   Phe Val Thr Ile Ala Lys Gly Phe Asn Val Pro Ala Val Arg Val Thr
   530 535 540
   Lys Lys Ser Glu Val Thr Ala Ala Ile Lys Lys Met Leu Glu Thr Pro
   545 550 555 560
   Gly Pro Tyr Leu Leu Asp Ile Ile Val Pro His Gln Glu His Val Leu
   565 570 575
   Pro Met Ile Pro Ser Gly Gly Ala Phe Lys Asp Met Ile Met Glu Gly
   580 585 590
   Asp Gly Arg Thr Ser Tyr
   595
<210> 35
   <211> 1797
   <212> DNA
   <213> Triticum aestivum
<400> 35
   gccgcgtccc ccgccgccac ctccgccgcg cctcccgcaa ccgcgctccg gccctggggc 60
   ccgtccgagc cccgcaaggg cgccgacatc ctcgtcgagg cgctcgagcg ctgcggcatc 120
   gtcgacgtct tcgcctaccc cggcggcgcc tccatggaga tccaccaggc gctgacgcgc 180
   tcgcccgtca tcaccaacca cctcttccgc cacgagcagg gggaggcgtt cgcggcgtcc 240
   ggctacgccc gcgcgtccgg ccgcgtcggc gtctgcgtcg ccacctccgg cccgggggcc 300
   accaacctcg tctccgcgct cgccgacgcc ctcctcgact ccatccccat ggtcgccatc 360
   acgggccagg tcccccgccg catgatcggc acggacgcgt tccaggagac gcccatagtg 420
   gaggtcacgc gctccatcac caagcacaac tacctggtcc ttgacgtgga ggatatcccc 480
   cgcgtcatcc aggaagcctt cttccttgca tcctctggcc gcccggggcc ggtgctagtt 540
   gatatcccca aggacatcca gcagcagatg gctgtgcccg tctgggacac tccaatgagt 600
   ttgccagggt acatcgcccg cctgcccaag ccaccatcta ctgaatcgct tgagcaggtc 660
   ctgcgtctgg ttggcgagtc acggcgccca attctgtatg ttggtggtgg ctgcgctgcg 720
   tctggcgagg agttgcgccg ctttgttgag cttactggga ttccagttac aactactctg 780
   atgggccttg gcaacttccc cagcgacgac ccactgtctc tgcgcatgct tgggatgcat 840
   ggcactgtgt atgcaaatta tgcagtagat aaggctgacc tgttgctcgc atttggtgtg 900
   cggtttgatg atcgtgtgac tgggaaaatc gaggcttttg caagcaggtc caagattgtg 960
   cacattgaca ttgacccagc tgagattggc aagaacaagc agccacatgt ctccatttgt 1020
   gcagatgtta agcttgcttt acaggggttg aatgatctat taaatgggag caaagcacaa 1080
   cagggtctgg attttggtcc atggcacaag gagttggatc agcagaagag ggagtttcct 1140
   ctaggattca agacttttgg cgaggccatc ccgccgcaat atgctatcca ggtactggat 1200
   gagctgacaa aaggggaggc gatcattgcc actggtgttg ggcagcacca gatgtgggcg 1260
   gctcagtatt acacttacaa gcggccacgg cagtggctgt cttcgtctgg tttgggggca 1320
   atgggatttg ggttaccagc tgcagctggc gctgctgtgg ccaacccagg tgttacagtt 1380
   gttgacattg atggtgatgg tagtttcctc atgaacattc aggagttggc gttgatccgc 1440
   attgagaacc tcccagtgaa ggtgatgata ttgaacaacc agcatctggg aatggtggtg 1500
   cagtgggagg ataggtttta caaggccaat cgggcgcaca cataccttgg caacccagaa 1560
   aatgagagtg agatatatcc agattttgtg acgattgcta aaggattcaa cgttccagca 1620
   gttcgagtga cgaagaagag cgaagtcact gcagcaatca agaagatgct tgagacccca 1680
   gggccatact tgttggatat catagtcccg catcaggagc acgtgctgcc tatgatccca 1740
   agcggtggtg ctttcaagga catgatcatg gagggtgatg gcaggacctc gtactga 1797
<210> 36
   <211> 598
   <212> PRT
   <213> Triticum aestivum
<400> 36
   Ala Ala Ser Pro Ala Ala Thr Ser Ala Ala Pro Pro Ala Thr Ala Leu
   1               5                   10                  15
   Arg Pro Trp Gly Pro Ser Glu Pro Arg Lys Gly Ala Asp Ile Leu Val
               20                  25                  30
   Glu Ala Leu Glu Arg Cys Gly Ile Val Asp Val Phe Ala Tyr Pro Gly
           35                  40                  45
   Gly Ala Ser Met Glu Ile His Gln Ala Leu Thr Arg Ser Pro Val Ile
       50                  55                  60
   Thr Asn His Leu Phe Arg His Glu Gln Gly Glu Ala Phe Ala Ala Ser
   65                  70                  75                  80
   Gly Tyr Ala Arg Ala Ser Gly Arg Val Gly Val Cys Val Ala Thr Ser
                   85                  90                  95
   Gly Pro Gly Ala Thr Asn Leu Val Ser Ala Leu Ala Asp Ala Leu Leu
               100                 105                 110
   Asp Ser Ile Pro Met Val Ala Ile Thr Gly Gln Val Pro Arg Arg Met
           115                 120                 125
   Ile Gly Thr Asp Ala Phe Gln Glu Thr Pro Ile Val Glu Val Thr Arg
       130                 135                 140
   Ser Ile Thr Lys His Asn Tyr Leu Val Leu Asp Val Glu Asp Ile Pro
   145                 150                 155                 160
   Arg Val Ile Gln Glu Ala Phe Phe Leu Ala Ser Ser Gly Arg Pro Gly
                   165                 170                 175
   Pro Val Leu Val Asp Ile Pro Lys Asp Ile Gln Gln Gln Met Ala Val
               180                 185                 190
   Pro Val Trp Asp Thr Pro Met Ser Leu Pro Gly Tyr Ile Ala Arg Leu
           195                 200                 205
   Pro Lys Pro Pro Ser Thr Glu Ser Leu Glu Gln Val Leu Arg Leu Val
       210                 215                 220
   Gly Glu Ser Arg Arg Pro Ile Leu Tyr Val Gly Gly Gly Cys Ala Ala
   225                 230                 235                 240
   Ser Gly Glu Glu Leu Arg Arg Phe Val Glu Leu Thr Gly Ile Pro Val
                   245                 250                 255
   Thr Thr Thr Leu Met Gly Leu Gly Asn Phe Pro Ser Asp Asp Pro Leu
               260                 265                 270
   Ser Leu Arg Met Leu Gly Met His Gly Thr Val Tyr Ala Asn Tyr Ala
           275                 280                 285
   Val Asp Lys Ala Asp Leu Leu Leu Ala Phe Gly Val Arg Phe Asp Asp
       290                 295                 300
   Arg Val Thr Gly Lys Ile Glu Ala Phe Ala Ser Arg Ser Lys Ile Val
   305                 310                 315                 320
   His Ile Asp Ile Asp Pro Ala Glu Ile Gly Lys Asn Lys Gln Pro His
                   325                 330                 335
   Val Ser Ile Cys Ala Asp Val Lys Leu Ala Leu Gln Gly Leu Asn Asp
               340                 345                 350
   Leu Leu Asn Gly Ser Lys Ala Gln Gln Gly Leu Asp Phe Gly Pro Trp
           355                 360                 365
   His Lys Glu Leu Asp Gln Gln Lys Arg Glu Phe Pro Leu Gly Phe Lys
       370                 375                 380
   Thr Phe Gly Glu Ala Ile Pro Pro Gln Tyr Ala Ile Gln Val Leu Asp
   385                 390                 395                 400
   Glu Leu Thr Lys Gly Glu Ala Ile Ile Ala Thr Gly Val Gly Gln His
                   405                 410                 415
   Gln Met Trp Ala Ala Gln Tyr Tyr Thr Tyr Lys Arg Pro Arg Gln Trp
               420                 425                 430
   Leu Ser Ser Ser Gly Leu Gly Ala Met Gly Phe Gly Leu Pro Ala Ala
           435                 440                 445
   Ala Gly Ala Ala Val Ala Asn Pro Gly Val Thr Val Val Asp Ile Asp
       450                 455                 460
   Gly Asp Gly Ser Phe Leu Met Asn Ile Gln Glu Leu Ala Leu Ile Arg
   465                 470                 475                 480
   Ile Glu Asn Leu Pro Val Lys Val Met Ile Leu Asn Asn Gln His Leu
                   485                 490                 495
   Gly Met Val Val Gln Trp Glu Asp Arg Phe Tyr Lys Ala Asn Arg Ala
               500                 505                 510
   His Thr Tyr Leu Gly Asn Pro Glu Asn Glu Ser Glu Ile Tyr Pro Asp
   515 520 525
   Phe Val Thr Ile Ala Lys Gly Phe Asn Val Pro Ala Val Arg Val Thr
   530 535 540
   Lys Lys Ser Glu Val Thr Ala Ala Ile Lys Lys Met Leu Glu Thr Pro
   545 550 555 560
   Gly Pro Tyr Leu Leu Asp Ile Ile Val Pro His Gln Glu His Val Leu
   565 570 575
   Pro Met Ile Pro Ser Gly Gly Ala Phe Lys Asp Met Ile Met Glu Gly
   580 585 590
   Asp Gly Arg Thr Ser Tyr
   595
<210> 37
   <211> 455
   <212> DNA
   <213> Saccharum officinarum
<400> 37
   ctggtctggg ggcaatggga tttggtttgc cggctgctgc tggcgctgct gtggccaacc 60
   caggtgtcac tgttgttgac atcgacggag atggtagctt cctcatgaac attcaggagc 120
   tagctatgat ccgaattgag aacctcccag tgaaggtctt tgcgctaaac aaccagcacc 180
   tggggatggt ggtgcagtgg gaggacaggt tctataaggc caacagagca cacacatact 240
   tgggaaaccc agagaatgaa agtgagatat atccagattt cgtgacaatt gccaaagggt 300
   tcaacattcc agcagtccgt gtgacaaaga agagcgaagt ccatgcagca atcaagaaga 360
   tgcttgagac tccagggccg tacctcttgg atataatcgt cccgcaccag gagcatgtgt 420
   tgcctatgat cccgagtggt ggagctttca aggac 455
<210> 38
   <211> 151
   <212> PRT
   <213> Saccharum officinarum
<400> 38
   Gly Leu Gly Ala Met Gly Phe Gly Leu Pro Ala Ala Ala Gly Ala Ala
   1 5 10 15
   Val Ala Asn Pro Gly Val Thr Val Val Asp Ile Asp Gly Asp Gly Ser
   20 25 30
   Phe Leu Met Asn Ile Gln Glu Leu Ala Met Ile Arg Ile Glu Asn Leu
           35                  40                  45
   Pro Val Lys Val Phe Ala Leu Asn Asn Gln His Leu Gly Met Val Val
       50                  55                  60
   Gln Trp Glu Asp Arg Phe Tyr Lys Ala Asn Arg Ala His Thr Tyr Leu
   65                  70                  75                  80
   Gly Asn Pro Glu Asn Glu Ser Glu Ile Tyr Pro Asp Phe Val Thr Ile
                   85                  90                  95
   Ala Lys Gly Phe Asn Ile Pro Ala Val Arg Val Thr Lys Lys Ser Glu
               100                 105                 110
   Val His Ala Ala Ile Lys Lys Met Leu Glu Thr Pro Gly Pro Tyr Leu
           115                 120                 125
   Leu Asp Ile Ile Val Pro His Gln Glu His Val Leu Pro Met Ile Pro
       130                 135                 140
   Ser Gly Gly Ala Phe Lys Asp
   145                 150

## Description

### FIELD OF THE INVENTION

This invention relates to herbicide-resistant allotetraploid *Brassica* plants, such as *Brassica napus* or *Brassica juncea* plants, seed of such plants, parts thereof, progeny thereof as well as a method for their manufacture, and methods using such plants, and to crop protection by using ALS (acetolactate synthase; also known as AHAS (acetohydroxyacid synthase; EC 2.2.1.6; formerly EC 4.1.3.18)) inhibitor herbicides against unwanted vegetation in areas of growing such herbicide-resistant plants.

### BACKGROUND OF THE INVENTION

Since more than 40 years, herbicides are the preferred tools to control weeds in *B. napus.* The products used for this purpose, namely Metazachlor, Dimethachlor, Quinmerac, Clomazone, Metolachlor, Napropamide, Clopyralid, Propyzamide, Propaquizafop, Fluazifop and others allow suppressing weeds in *B. napus* fields without damaging the crop. Nevertheless, under adverse environmental conditions the efficacy of these products leaves room for improvements, especially if noxious weeds like *Geranium dissectum, Centaurea cyanus, Sinapis arvensis* and/or *Alopecurus myosuroides* germinate over an extended period of time.

Acetohydroxyacid synthase (AHAS), also known as "acetolactate synthase" (ALS [EC 2.2.1.6; formerly EC 4.1.3.18]) is the first enzyme that catalyzes the biochemical synthesis of the branched chain amino acids valine, leucine and isoleucine (Singh (1999) "Biosynthesis of valine, leucine and isoleucine," in Plant Amino Acid, Singh, B.K., ed., Marcel Dekker Inc. New York, New York, pp. 227-247).

The ALS/AHAS enzyme is present in bacteria, fungi, and plants and from various organisms protein isolates have been obtained and their corresponding amino acid/nucleic acid sequences as well as their biochemical characteristics have been determined/characterized (see, e.g., Umbarger et al., Annu. Rev. Biochem. (1978), 47, 533-606; Chiman et al., Biochim. Biophys. Acta (1998), 1385, 401-419; Duggleby and Pang, J. Biochem. Mol. Biol. (2000), 33, 1-36; Duggleby: Structure and Properties of Acetohydroxyacid Synthase in Thiamine: Catalytic Mechanisms in Normal and Disease States, Vol 11, Marcel Dekker, New York, 2004, 251-274).

ALS is the target of five structurally diverse herbicide families belonging to the class of ALS inhibitor herbicides, like (a) sulfonylurea herbicides (Beyer E.M et al. (1988), Sulfonylureas in Herbicides: Chemistry, Degradation, and Mode of Action; Marcel Dekker, New York, 1988, 117-189), (b) sulfonylaminocarbonyltriazolinone herbicides (Pontzen, R., Pflanz.-Nachrichten Bayer, 2002, 55, 37-52), (c) imidazolinone herbicides (Shaner, D.L., et al., Plant Physiol., 1984, 76, 545-546; Shaner, D.L., and O'Connor, S.L. (Eds.) The Imidazolinone Herbicides, CRC Press, Boca Raton, FL, 1991), (d) triazolopyrimidine herbicides (Kleschick, W.A. et al., Agric. Food Chem., 1992, 40, 1083-1085), and (e) pyrimidinyl(thio)benzoate herbicides (Shimizu, T.J., Pestic. Sci.,1997, 22, 245-256; Shimizu, T. et al., Acetolactate Syntehase Inhibitors in Herbicide Classes in Development, Böger, P., Wakabayashi, K., Hirai, K., (Eds.), Springer Verlag, Berlin, 2002, 1-41).

Inhibitors of the ALS interrupt the biosynthesis of valine, leucine and isoleucine in plants. The consequence is an immediate depletion of the respective amino acid pools causing a stop of protein biosynthesis leading to a cessation of plant growth and eventually the plant dies, or - at least - is damaged.

ALS inhibitor herbicides such as imidazolinone and sulfonylurea herbicides are widely used in modern agriculture due to their effectiveness at moderate application rates and relative non-toxicity in animals. By inhibiting ALS activity, these families of herbicides prevent further growth and development of susceptible plants including many weed species.

Various mutants in ALS in various plants have been described that confer resistance to one or more ALS inhibitor herbicides. Plants conferring mutant ALS alleles show different levels of tolerance to ALS inhibitor herbicides, depending on the chemical structure of the ALS inhibitor herbicide and the site of the point mutation(s) in the ALS gene and the hereby encoded ALS protein.

Several mutants (naturally occurring in weeds but also artificially induced in crops by either mutation or transgenic approaches) of the ALS conferring tolerance to one or more chemicals defined under the above given ALS inhbitor herbicide classes/groups are known at various parts of the enzyme (i.e. in the α-, β-, and γ-domain of the ALS) are known and have been identified in various organisms, including plants (US Patent No. 5,378,82; Duggleby, R.G. et al., (2008), Plant Physiol. and Biochem., pp 309-324; Siyuan, T. et al. (2005), Pest Management Sci., 61, pp 246-257; Jung, S. (2004) Biochem J., pp 53-61; Kolkman, J.M. (2004), Theor. Appl. Genet., 109, pp 1147-1159; Duggleby, R.G. et al (2003), Eur. J. Biochem., 270, pp 1295-2904; Pang, S.S., et al. (2003), J. Biol. Chem., pp 7639-7644); Yadav, N. et al., (1986), Proc. Natl. Acad. Sci., 83, pp 4418-4422), Jander G. et al. (2003), Plant Physiol., 131, pp. 139-146); Tranel, P.J., and Wright, T.R. (2002), Weed Science, 50, pp 700-712); Chang, A.K., and Duggleby, R.G. (1998), Biochem J., 333, pp. 765-777).

Among the artificially obtained various mutants, it has already been described that these are tolerant against various classes of ALS inhibitor herbicides, such as against certain sulfonylureas or representative compounds of the class of imidazolinones.

EP-A-0360750 describes the production of ALS inhibtor herbicide tolerant plants by producing an increased amount of the targeted ALS inside the plant. Such plants show an increased tolerance against certain sulfonyureas, like chlorsulfuron, sulfometuron-methyl, and triasulfuron.

US 5,198,599 describes sulfonylurea and imidazolinone tolerant plants that have been obtained via a selection process and which show a tolerance against chlorsulfuron, bensulfuron, chlorimuron, thifensulfuron and sulfometuron.

WO09/046334 describes mutated acetohydroxyacid synthase (*AHAS*) nucleic acids and the proteins encoded by the mutated nucleic acids, as well as canola plants, cells, and seeds comprising the mutated genes, whereby the plants display increased tolerance to imidazolinones and sulfonylureas.

WO09/031031 discloses herbicide-resistant *Brassica* plants and novel polynucleotide sequences that encode wild-type and imidazolinone-resistant *Brassica* acetohydroxyacid synthase large subunit proteins, seeds, and methods using such plants.

US patent application 09/0013424 describes improved imidazolinone herbicide resistant *Brassica* lines, including *Brassica juncea,* methods for generation of such lines, and methods for selection of such lines, as well as *Brassica AHAS* genes and sequences and a gene allele bearing a point mutation that gives rise to imidazolinone herbicide resistance.

WO08/124495 discloses nucleic acids encoding mutants of the acetohydroxyacid synthase (*AHAS*) large subunit comprising at least two mutations, for example double and triple mutants, which are useful for producing transgenic or non-transgenic plants with improved levels of tolerance to AHAS-inhibiting herbicides. The invention also provides expression vectors, cells, plants comprising the polynucleotides encoding the *AHAS* large subunit double and triple mutants, plants comprising two or more *AHAS* large subunit single mutant polypeptides, and methods for making and using the same.

WO 2010/037061 describes transgenic and non-transgenic plants with improved tolerance to AHAS-inhibiting herbicides such as an oilseed rape which is tolerant towards one specific class of ALS inhibitors, the Imidazolinone herbicides.

WO2011/114232 describes herbicide-tolerant winter-type *Brassica* plants which express an AHAS enzyme that is tolerant to the action of one or more AHAS enzyme inhibitors.

Tan et al. (Pest.Manag. Sci (2005), 61: 246-257) inter alia refers to imidazolinone-tolerant oilseed rape.

WO2012/049268 and WO2012/049266 describe an ALS inhibitor herbicide tolerant *Beta vulgaris* plant comprising a mutation of an endogenous ALS gene encoding an ALS polypeptide containing a leucine instead of a tryptophan at a position 569 of the ALS polypeptide. WO2010/014007 describes a sunflower plant comprising an AHASL1 gene encoding an AHASL1 protein with a tryptophan to leucine substitution at a position corresponding to Trp574 of the Arabidopsis protein. WO2007/149069 describes herbicide tolerant *Coreopsis tinctoria* comprising an AHAS allele with both a P197L and a W574L mutation.

In order to provide plants with an increased tolerance to even high concentrations of ALS inhibitor herbicides and to mixtures of herbicidal compounds that may be required for sufficient weed control, additional ALS-inhibiting herbicide-resistant breeding lines and varieties of crop plants, as well as methods and compositions for the production and use of ALS inhibiting herbicide-resistant breeding lines and varieties, are needed.

Thus, the technical problem is to comply with this need.

The present invention addresses this need and thus provides as a solution to the technical problem of obtaining ALS inhibitor herbicide tolerant polyploid plants, such as *Brassica napus* plants and *Brassica juncea* plants and parts thereof according to the present invention.

By applying various breeding methods, high yielding commercial varieties highly competitive in all specific markets with the add-on of a robust ALS inhibitor herbicide tolerance can be developed subsequently by using the originally obtained mutant plants.

### SUMMARY OF THE INVENTION

In one aspect, the present invention provides an allotetraploid ALS inhibitor herbicide tolerant *Brassica* plant or parts thereof, comprising at least two ALS genes, wherein all endogenous ALS genes encode an ALS polypeptide comprising at a position corresponding to position 574 of SEQ ID NO: 10 instead of the naturally encoded amino acid tryptophan the amino acid leucine, and wherein at least one of the ALS genes encodes an ALS polypeptide which further comprises at a position corresponding to position 197 of SEQ ID NO: 10 instead of the naturally encoded amino acid proline the amino acid serine, characterized in that said plant or parts thereof is homozygous for said ALS genes encoding an ALS polypeptide comprising at a position corresponding to position 574 of SEQ ID NO: 10 instead of the naturally encoded amino acid tryptophan the amino acid leucine, and is homozygous for said ALS genes encoding an ALS polypeptide comprising at a position corresponding to position 197 of SEQ ID NO: 10 instead of the naturally encoded amino acid proline the amino acid serine and comprising at a position corresponding to position 574 of SEQ ID NO: 10 instead of the naturally encoded amino acid tryptophan the amino acid leucine. In another aspect, all ALS genes of said plants or parts thereof encode an ALS polypeptide comprising at a position corresponding to position 574 of SEQ ID NO: 10 instead of the naturally encoded amino acid tryptophan the amino acid leucine, and comprising at a position corresponding to position 197 of SEQ ID NO: 10 instead of the naturally encoded amino acid proline the amino acid serine. In another embodiment, said allotetraploid ALS inhibitor herbicide tolerant *Brassica* plant or parts thereof is selected from the group consisting of:
a. *Brassica napus* comprising an ALS I gene encoding an ALS I polypeptide comprising at a position corresponding to position 182 of SEQ ID NO: 2 instead of the naturally encoded amino acid proline the amino acid serine and comprising at a position corresponding to position 559 of SEQ ID NO: 2 instead of the naturally encoded amino acid tryptophan the amino acid leucine, and an ALS III gene encoding an ALS III polypeptide comprising at a position corresponding to position 556 of SEQ ID NO: 4 instead of the naturally encoded amino acid tryptophan the amino acid leucine;
b. *Brassica napus* comprising an ALS I gene encoding an ALS I polypeptide comprising at a position corresponding to position 559 of SEQ ID NO: 2 instead of the naturally encoded amino acid tryptophan the amino acid leucine, and an ALS III gene encoding an ALS III polypeptide comprising at a position corresponding to position 179 of SEQ ID NO: 4 instead of the naturally encoded amino acid proline the amino acid serine and comprising at a position corresponding to position 556 of SEQ ID NO: 4 instead of the naturally encoded amino acid tryptophan the amino acid leucine;
c. *Brassica napus* comprising an ALS I gene encoding an ALS I polypeptide comprising at a position corresponding to position 182 of SEQ ID NO: 2 instead of the naturally encoded amino acid proline the amino acid serine and comprising at a position corresponding to position 559 of SEQ ID NO: 2 instead of the naturally encoded amino acid tryptophan the amino acid leucine, and an ALS III gene encoding an ALS III polypeptide comprising at a position corresponding to position 179 of SEQ ID NO: 4 instead of the naturally encoded amino acid proline the amino acid serine and comprising at a position corresponding to position 556 of SEQ ID NO: 4 instead of the naturally encoded amino acid tryptophan the amino acid leucine;
d. *Brassica juncea* compring an ALS-A gene encoding an ALS-A polypeptide comprising at a position corresponding to position 179 of SEQ ID NO: 6 instead of the naturally encoded amino acid proline the amino acid serine and comprising at a position corresponding to position 556 of SEQ ID NO: 6 instead of the naturally encoded amino acid tryptophan the amino acid leucine, and an ALS-B gene encoding an ALS-B polypeptide comprising at a position corresponding to position 559 of SEQ ID NO: 8 instead of the naturally encoded amino acid tryptophan the amino acid leucine;
e. *Brassica juncea* compring an ALS-A gene encoding an ALS-A polypeptide comprising at a position corresponding to position 556 of SEQ ID NO: 6 instead of the naturally encoded amino acid tryptophan the amino acid leucine, and an ALS-B gene encoding an ALS-B polypeptide polypeptide comprising at a position corresponding to position 182 of SEQ ID NO: 8 instead of the naturally encoded amino acid proline the amino acid serine and comprising at a position corresponding to position 559 of SEQ ID NO: 8 instead of the naturally encoded amino acid tryptophan the amino acid leucine; and
f. *Brassica juncea* compring an ALS-A gene encoding an ALS-A polypeptide comprising at a position corresponding to position 179 of SEQ ID NO: 6 instead of the naturally encoded amino acid proline the amino acid serine and comprising at a position corresponding to position 556 of SEQ ID NO: 6 instead of the naturally encoded amino acid tryptophan the amino acid leucine, and an ALS-B gene encoding an ALS-B polypeptide comprising at a position corresponding to position 182 of SEQ ID NO: 8 instead of the naturally encoded amino acid proline the amino acid serine and comprising at a position corresponding to position 559 of SEQ ID NO: 8 instead of the naturally encoded amino acid tryptophan the amino acid leucine.

In another embodiment, said *B. napus* plants or parts thereof comprise an ALS I gene encoding an ALS I polypeptide which is at least 90% identical to SEQ ID NO: 2 of which the proline at position 182 is substituted with a serine, and of which the tryptophan at position 559 is substituted with a leucine, and an ALS III gene encoding an ALS III polypeptide which is at least 90% identical to SEQ ID NO: 4 of which the tryptophan at position 556 is substituted with a leucine, such as an ALS I gene encoding an ALS I polypeptide which is identical to SEQ ID NO: 2 of which the proline at position 182 is substituted with a serine, and of which the tryptophan at position 559 is substituted with a leucine, and an ALS III gene encoding an ALS III polypeptide which is identical to SEQ ID NO: 4 of which the tryptophan at position 556 is substituted with a leucine, or such as an ALS I gene comprising the nucleotide sequence corresponding to SEQ ID NO: 1 of which the C at position 544 is substituted with T and of which the G at position 1676 is substituted with T, and an ALS III gene comprising the nucleotide sequence corresponding to SEQ ID NO: 3 of which the G at position 1667 is substituted with T and which is obtainable from seeds deposited at NCIMB under accession numbers NCIMB 42145, NCIMB 42235, and/or NCIMB 42260 or wherein reference seed of said plant has been deposited at NCIMB under accession numbers NCIMB 42145, NCIMB 42235, and/or NCIMB 42260, or said *B. napus* plants or parts thereof comprise an ALS I gene encoding an ALS I polypeptide which is at least 90% identical to SEQ ID NO: 2 of which the tryptophan at position 559 is substituted with a leucine, and an ALS III gene encoding an ALS III polypeptide which is at least 90% identical to SEQ ID NO: 4 of which the proline at position 179 is substituted with a serine and of which the tryptophan at position 556 is substituted with a leucine, such as an ALS I gene encoding an ALS I polypeptide which is identical to SEQ ID NO: 2 of which the tryptophan at position 559 is substituted with a leucine, and an ALS III gene encoding an ALS III polypeptide which is identical to SEQ ID NO: 4 of which the proline at position 179 is substituted with a serine, and of which the tryptophan at position 556 is substituted with a leucine, or such as an ALS I gene comprising the nucleotide sequence corresponding to SEQ ID NO: 1 of which the G at position 1676 is substituted with T, and an ALS III gene comprising the nucleotide sequence corresponding to SEQ ID NO: 3 of which the C at position 535 is substituted with T and of which the G at position 1667 is substituted with T and which is obtainable from seeds deposited at NCIMB under accession numbers NCIMB 42145, NCIMB 42235, NCIMB 42153, and/or NCIMB 42259 or wherein reference seed of said plant has been deposited at NCIMB under accession numbers NCIMB 42145, NCIMB 42235, NCIMB 42153, and/or NCIMB 42259, or said *B. napus* plants or parts thereof comprise an ALS I gene encoding an ALS I polypeptide which is at least 90% identical to SEQ ID NO: 2 of which the proline at position 182 is substituted with a serine, and of which the tryptophan at position 559 is substituted with a leucine, and an ALS III gene encoding an ALS III polypeptide which is at least 90% identical to SEQ ID NO: 4 of which the proline at position 179 is substituted with a serine and of which the tryptophan at position 556 is substituted with a leucine, such as an ALS I gene encoding an ALS I polypeptide which is identical to SEQ ID NO: 2 of which the proline at position 182 is substituted with a serine, and of which the tryptophan at position 559 is substituted with a leucine, and an ALS III gene encoding an ALS III polypeptide which is identical to SEQ ID NO: 4 of which the proline at position 179 is substituted with a serine and of which the tryptophan at position 556 is substituted with a leucine, or such as an ALS I gene comprising the nucleotide sequence corresponding to SEQ ID NO: 1 of which the C at position 544 is substituted with T and of which the G at position 1676 is substituted with T, and an ALS III gene comprising the nucleotide sequence corresponding to SEQ ID NO: 3 of which the C at position 535 is substituted with T and of which the G at position 1667 is substituted with T and which is obtainable from seeds deposited at NCIMB under accession numbers NCIMB 42153, NCIMB 42259, and/or NCIMB 42260 or wherein reference seed of said plant has been deposited at NCIMB under accession numbers NCIMB 42153, NCIMB 42259, and/or NCIMB 42260, or said *B*. *juncea* plants or parts thereof comprise an ALS-A gene encoding an ALS-A polypeptide which is at least 90% identical to SEQ ID NO: 6 of which the proline at position 179 is substituted with a serine and of which the tryptophan at position 556 is substituted with a leucine, and an ALS-B gene encoding an ALS-B polypeptide which is at least 90% identical to SEQ ID NO: 8 of which the tryptophan at position 559 is substituted with a leucine, or such as an ALS-A gene encoding an ALS-A polypeptide which is identical to SEQ ID NO: 6 of which the proline at position 179 is substituted with a serine and of which the tryptophan at position 556 is substituted with a leucine, and an ALS-B gene encoding an ALS-B polypeptide which is identical to SEQ ID NO: 8 of which the tryptophan at position 559 is substituted with a leucine, or said *B. juncea* plants or parts thereof comprise an ALS-A gene encoding an ALS-A polypeptide which is at least 90% identical to SEQ ID NO: 6 of which the tryptophan at position 556 is substituted with a leucine, and an ALS-B gene encoding an ALS-B polypeptide which is at least 90% identical to SEQ ID NO: 8 of which the proline at position 182 is substituted with a serine, and of which the tryptophan at position 559 is substituted with a leucine, or such as an ALS-A gene encoding an ALS-A polypeptide which is identical to SEQ ID NO: 6 of which the tryptophan at position 556 is substituted with a leucine, and an ALS-B gene encoding an ALS-B polypeptide which is identical to SEQ ID NO: 8 of which the proline at position 182 is substituted with a serine, and of which the tryptophan at position 559 is substituted with a leucine, or said *B. juncea* plants or parts thereof comprise an ALS-A gene encoding an ALS-A polypeptide which is at least 90% identical to SEQ ID NO: 6 of which the proline at position 179 is substituted with a serine and of which the tryptophan at position 556 is substituted with a leucine, and an ALS-B gene encoding an ALS-B polypeptide which is at least 90% identical to SEQ ID NO: 8 of which the proline at position 182 is substituted with a serine, and of which the tryptophan at position 559 is substituted with a leucine, such as an ALS-A gene encoding an ALS-A polypeptide which is identical to SEQ ID NO: 6 of which the proline at position 179 is substituted with a serine and of which the tryptophan at position 556 is substituted with a leucine, and an ALS-B gene encoding an ALS-B polypeptide which is identical to SEQ ID NO: 8 of which the proline at position 182 is substituted with a serine, and of which the tryptophan at position 559 is substituted with a leucine.

Suitable are Brassica plants or parts thereof which are *Brassica napus* winter oilseed rape.

Yet another embodiment refers to a plant or parts thereof according to the present invention, which are tolerant to one or more ALS-inhibitor herbicides belonging to the group consisting of sulfonylurea herbicides, sulfonylaminocarbonyltriazolinone herbicides, imidazolinone herbicides, triazolopyrimidine herbicides, and pyrimidinyl(thio)benzoate herbicides.

Also described is food, feed, or an industrial product obtainable from a plant according to the invention, such as oil, meal, grain, starch, flour or protein, or the industrial product is biofuel, fiber, industrial chemicals, a pharmaceutical or a nutraceutical.

Yet another aspect refers to progeny of a plant according to the present invention obtained by further breeding with said plant according to the present invention, wherein wherein all ALS genes of said progeny encode an ALS polypeptide comprising at a position corresponding to position 574 of SEQ ID NO: 10 instead of the naturally encoded amino acid tryptophan the amino acid leucine and wherein at least one of the ALS genes encodes an ALS polypeptide which further comprises at a position corresponding to position 197 of SEQ ID NO: 10 instead of the naturally encoded amino acid proline the amino acid serine.

The plants according to the invention can be used in a method for producing a hybrid seed, comprising crossing a parent plant according to the present invention with a second parent plant.

Yet another aspect refers to a hybrid plant produced from crossing a parent plant according to the present invention with a second parent plant and harvesting a resultant hybrid seed and growing said seed, wherein all ALS genes of said hybrid plant encode an ALS polypeptide comprising at a position corresponding to position 574 of SEQ ID NO: 10 instead of the naturally encoded amino acid tryptophan the amino acid leucine and wherein at least one of the ALS genes encodes an ALS polypeptide which further comprises at a position corresponding to position 197 of SEQ ID NO: 10 instead of the naturally encoded amino acid proline the amino acid serine.

Suitable is a method for producing food, feed, or an industrial product, such as oil, meal, grain, starch, flour, protein, biofuel, fiber, industrial chemicals, a pharmaceutical or a nutraceutical, comprising obtaining the plant according to the present invention or a part thereof, and preparing the food, feed, or industrial product from the plant or part thereof.

Described is a method to increase the tolerance to ALS inhibitor herbicide(s) of polyploid plants, said method comprising introducing at least two ALS genes, wherein all ALS genes encode an ALS polypeptide comprising at a position corresponding to position 574 of SEQ ID NO: 10 instead of the naturally encoded amino acid tryptophan the amino acid leucine, and wherein one ALS gene encodes an ALS polypeptide which further comprises at a position corresponding to position 197 of SEQ ID NO: 10 instead of the naturally encoded amino acid proline the amino acid serine.

Also described is the use of one or more ALS inhibitor herbicide(s) for controlling unwanted vegetation in a growing area, such as a *Brassica* growing area, or such as *B. napus* growing area which plants, such as *Brassica* plants, or such as *B. napus* plants, comprise at least two ALS genes, wherein all ALS genes encode an ALS polypeptide comprising at a position corresponding to position 574 of SEQ ID NO: 10 instead of the naturally encoded amino acid tryptophan the amino acid leucine, and wherein at least one of the ALS genes encodes an ALS polypeptide which further comprises at a position corresponding to position 197 of SEQ ID NO: 10 instead of the naturally encoded amino acid proline the amino acid serine.

ALS inhibitor herbicide(s) that can be used can belong to:
the group of the (sulfon)amides (group (A)) consisting of:
the subgroup (A1) of the sulfonylureas, consisting of: amidosulfuron [CAS RN 120923-37-7] (= A1-1); azimsulfuron [CAS RN 120162-55-2] (= A1-2); bensulfuron-methyl [CAS RN 83055-99-6] (= A1-3); chlorimuron-ethyl [CAS RN 90982-32-4] (= A1-4); chlorsulfuron [CAS RN 64902-72-3] (= A1-5); cinosulfuron [CAS RN 94593-91-6] (= A1-6); cyclosulfamuron [CAS RN 136849-15-5] (= A1-7); ethametsulfuron-methyl [CAS RN 97780-06-8] (= A1-8); ethoxysulfuron [CAS RN 126801-58-9] (= A1-9); flazasulfuron [CAS RN 104040-78-0] (= A1-10); flucetosulfuron [CAS RN 412928-75-7] (= A1-11); flupyrsulfuron-methyl-sodium [CAS RN 144740-54-5] (= A1-12); foramsulfuron [CAS RN 173159-57-4] (= A1-13); halosulfuron-methyl [CAS RN 100784-20-1] (= A1-14); imazosulfuron [CAS RN 122548-33-8] (= A1-15); iodosulfuron-methyl-sodium [CAS RN 144550-36-7] (= A1-16); mesosulfuron-methyl [CAS RN 208465-21-8] (= A1-17); metsulfuron-methyl [CAS RN 74223-64-6] (= A1-18); monosulfuron [CAS RN 155860-63-2] (= A1-19); nicosulfuron [CAS RN 111991-09-4] (=A1-20); orthosulfamuron [CAS RN 213464-77-8] (= A1-21); oxasulfuron [CAS RN 144651-06-9] (= A1-22); primisulfuron-methyl [CAS RN 86209-51-0] (= A1-23); prosulfuron [CAS RN 94125-34-5] (= A1-24); pyrazosulfuron-ethyl [CAS RN 93697-74-6] (= A1-25); rimsulfuron [CAS RN 122931-48-0] (= A1-26); sulfometuron-methyl [CAS RN 74222-97-2] (= A1-27); sulfosulfuron [CAS RN 141776-32-1] (= A1-28); thifensulfuron-methyl [CAS RN 79277-27-3] (= A1-29); triasulfuron [CAS RN 82097-50-5] (= A1-30); tribenuron-methyl [CAS RN 101200-48-0] (= A1-31); trifloxysulfuron [CAS RN 145099-21-4] (sodium) (= A1-32); triflusulfuron-methyl [CAS RN 126535-15-7] (= A1-33); tritosulfuron [CAS RN 142469-14-5] (= A1-34); NC-330 [CAS RN 104770-29-8] (= A1-35); NC-620 [CAS RN 868680-84-6] (= A1-36); TH-547 [CAS RN 570415-88-2] (= A1-37); monosulfuron-methyl [CAS RN 175076-90-1] (= A1-38); metazosulfuron [CAS RN 868680-84-6] (=A1-39) ; methiopyrsulfuron [CAS RN 441050-97-1] (=A1-40) ; iofensulfuron-sodium [CAS RN 1144097-30-2] (= A1-41) ; propyrisulfuron [CAS RN 570415-88-2] (=A1-42);
the subgroup of the sulfonylaminocarbonyltriazolinones (subgroup ((A2)), consisting of: flucarbazone-sodium [CAS RN 181274-17-9] (= A2-1); propoxycarbazone-sodium [CAS RN 181274-15-7] (= A2-2); thiencarbazone-methyl [CAS RN 317815-83-1] (= A2-3);
the subgroup of the triazolopyrimidines (subgroup (A2)), consisting of: cloransulam-methyl [147150-35-4] (= A3-1); diclosulam [CAS RN 145701-21-9] (= A3-2); florasulam [CAS RN 145701-23-1] (= A3-3); flumetsulam [CAS RN 98967-40-9] (= A3-4); metosulam [CAS RN 139528-85-1] (= A3-5); penoxsulam [CAS RN 219714-96-2] (= A3-6); pyroxsulam [CAS RN 422556-08-9] (= A3-7);
the subgroup of the sulfonanilides (subgroup (A4)), consisting of: compounds or salts thereof, and racemates and enantiomers thereof, from the group described by the general formula (I): 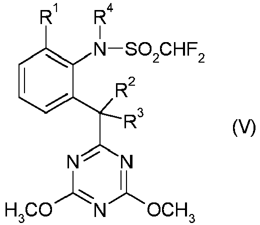 in which
   R¹ is halogen, preferably fluorine or chlorine,
   R² is hydrogen and R³ is hydroxyl or
   R² and R³ together with the carbon atom to which they are attached are a carbonyl group C=O and
   R⁴ is hydrogen or methyl;
and more especially compounds of the below given chemical structure (A4-1) to (A4-8) 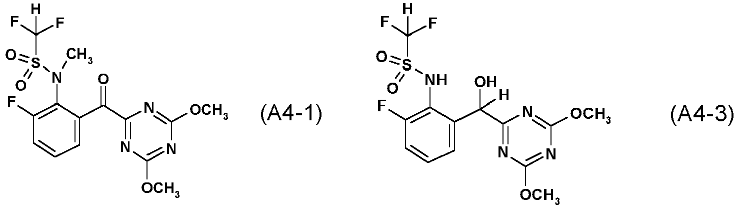 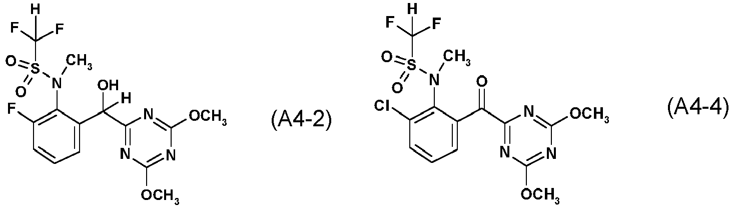 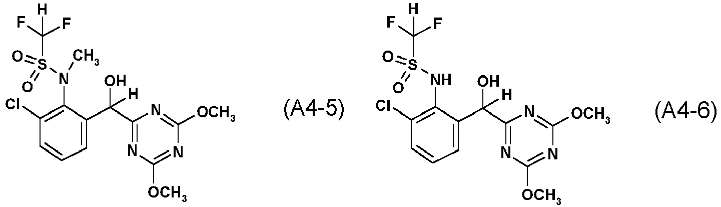 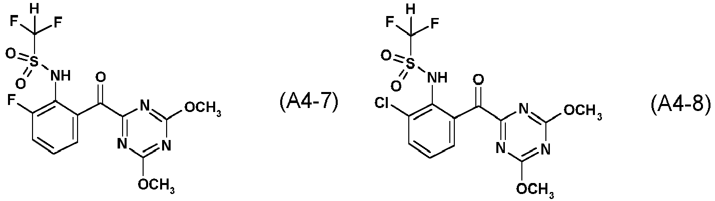
the group of the imidazolinones (group (B)), consisting of:
   imazamethabenzmethyl [CAS RN 81405-85-8] (= B1-1); imazamox [CAS RN 114311-32-9] (= B1-2); imazapic [CAS RN 104098-48-8] (= B1-3); imazapyr [CAS RN 81334-34-1] (= B1-4); imazaquin [CAS RN 81335-37-7] (= B1-5); imazethapyr [CAS RN 81335-77-5] (= B1-6); SYP-298 [CAS RN 557064-77-4] (= B1-7); and SYP-300 [CAS RN 374718-10-2] (= B1-8);
   the group of the pyrimidinyl(thio)benzoates (group (C)), consisting of:
      the subgroup of the pyrimidinyloxybenzoeacids (subgroup (C1)) consisting of: bispyribac-sodium [CAS RN 125401-92-5] (= C1-1); pyribenzoxim [CAS RN 168088-61-7] (= C1-2); pyriminobac-methyl [CAS RN 136191-64-5] (= C1-3); pyribambenz-isopropyl [CAS RN 420138-41-6] (= C1-4); and
      pyribambenz-propyl [CAS RN 420138-40-5] (= C1-5);
the subgroup of the pyrimidinylthiobenzoeacids (subgroup (C2)), consisting of: pyriftalid [CAS RN 135186-78-6] (= C2-1); and pyrithiobac-sodium [CAS RN 123343-16-8] (= C2-2).

ALS inhibitor herbicide(s) that can be used can belong(s) to the group consisting of: amidosulfuron [CAS RN 120923-37-7] (= A1-1); chlorimuron-ethyl [CAS RN 90982-32-4] (= A1-4); chlorsulfuron [CAS RN 64902-72-3] (=A1-5); ethametsulfuron-methyl [CAS RN 97780-06-8] (= A1-8); ethoxysulfuron [CAS RN 126801-58-9] (= A1-9); flupyrsulfuron-methyl-sodium [CAS RN 144740-54-5] (= A1-12); foramsulfuron [CAS RN 173159-57-4] (= A1-13); iodosulfuron-methyl-sodium [CAS RN 144550-36-7] (= A1-16); mesosulfuron-methyl [CAS RN 208465-21-8] (= A1-17); metsulfuron-methyl [CAS RN 74223-64-6] (= A1-18); monosulfuron [CAS RN 155860-63-2] (= A1-19); nicosulfuron [CAS RN 111991-09-4] (= A1-20); rimsulfuron [CAS RN 122931-48-0] (= A1-26); sulfosulfuron [CAS RN 141776-32-1] (= A1-28); thifensulfuron-methyl [CAS RN 79277-27-3] (= A1-29); tribenuron-methyl [CAS RN 101200-48-0] (= A1-31); triflusulfuron-methyl [CAS RN 126535-15-7] (= A1-33); iofensulfuron-sodium [CAS RN 1144097-30-2] (= A1-41); flucarbazone-sodium [CAS RN 181274-17-9] (= A2-1); propoxycarbazone-sodium [CAS RN 181274-15-7] (= A2-2); thiencarbazone-methyl [CAS RN 317815-83-1] (= A2-3); florasulam [CAS RN 145701-23-1] (= A3-3); metosulam [CAS RN 139528-85-1] (= A3-5); pyroxsulam [CAS RN 422556-08-9] (= A3-7); (A4-1); (A4-2); (A4-3); imazamox [CAS RN 114311-32-9] (= B1-2); and bispyribac-sodium [CAS RN 125401-92-5] (= C1-1).

ALS inhibitor herbicide(s) that can be used can belong(s) to the group consisting of: amidosulfuron [CAS RN 120923-37-7] (= A1-1); foramsulfuron [CAS RN 173159-57-4] (= A1-13); iofensulfuron-sodium [CAS RN 1144097-30-2] (= A1-41) ; thiencarbazone-methyl [CAS RN 317815-83-1] (= A2-3); imazamox [CAS RN 114311-32-9] (= B1-2); and bispyribac-sodium [CAS RN 125401-92-5] (= C1-1).

Also disclosed is the use of one or more ALS inhibitor herbicide(s) for controlling unwanted vegetation in a growing area as described herein, wherein the *Brassica* plants are selected from the group consisting of:
a. *B. napus* plants comprising an ALS I *B. napus* gene encoding an ALS I polypeptide comprising at a position corresponding to position 182 of SEQ ID NO: 2 instead of the naturally encoded amino acid proline the amino acid serine and at a position corresponding to position 559 of SEQ ID NO: 2 instead of the naturally encoded amino acid tryptophan the amino acid leucine, and wherein an ALS III *B. napus* gene encodes an ALS III polypeptide comprising at a position corresponding to position 556 of SEQ ID NO: 4 instead of the naturally encoded amino acid tryptophan the amino acid leucine; and
b. *B. napus* plants comprising an ALS I *B. napus* gene encoding an ALS I polypeptide comprising at a position corresponding to position 559 of SEQ ID NO: 2 instead of the naturally encoded amino acid tryptophan the amino acid leucine, and wherein an ALS III *B. napus* gene encodes an ALS III polypeptide comprising at a position corresponding to position 179 of SEQ ID NO: 4 instead of the naturally encoded amino acid proline the amino acid serine and at a position corresponding to position 556 of SEQ ID NO: 4 instead of the naturally encoded amino acid tryptophan the amino acid leucine.

Also disclosed is the use of one or more ALS inhibitor herbicide(s) for controlling unwanted vegetation in a growing area as described herein, wherein the ALS inhibitor herbicide(s) are used in combination with non-ALS inhibitor herbicides (i.e. herbicides showing a mode of action that is different to the inhibition of the ALS enzyme [acetohydroxyacid synthase; EC 2.2.1.6] (group B herbicides according to the HRAC classification on mode of action), and wherein the non ALS inhibitor herbicide(s) is/are selected form the group consisting of: acetochlor (= D1), carbetamide (= D56), fenoxaprop-P-ethyl (= D164), fluazifop-P-butyl (= D174), haloxyfop-P-methyl (= D222), metolachlor (= D275), dimethenamid (= D132), napropamide (= D290), pethoxamid (= D317), propaquizafop (= D341), propisochlor (= D344), propyzamide (= D345), quinmerac (= D363), propachlor (D 427), clomazone (= D83), clopyralid (= D86), dimethachlor (= D130), metazachlor (= D265), picloram (= D321), and quizalofop-P-ethyl (= D368).

Also disclosed is the use of one or more ALS inhibitor herbicide(s) for controlling unwanted vegetation in a growing area as described herein, wherein the ALS inhibitor herbicide(s) are used in combination with non-ALS inhibitor herbicide(s) is/are selected form the group consisting of: clomazone (= D83), clopyralid (= D86), dimethachlor (= D130), metazachlor (= D265), picloram (= D321), and quizalofop-P-ethyl (= D368).

Further described is a method for controlling unwanted vegetation in plant growing areas, such as *Brassica* growing areas, or such as *B. napus* growing areas, by applying one or more ALS inhibitor herbicide(s) alone or in combination with one or more herbicide(s) that do(es) not belong to the class of ALS inhibitor herbicides for weed control in growing areas, such as *Brassica* growing areas, or such as *B. napus* growing areas, which plants, such as *Brassica* plants, or such as *B. napus* plants comprise at least two ALS genes, wherein all ALS genes encode an ALS polypeptide comprising at a position corresponding to position 574 of SEQ ID NO: 10 instead of the naturally encoded amino acid tryptophan the amino acid leucine, and wherein at least one of the ALS genes encodes an ALS polypeptide which further comprises at a position corresponding to position 197 of SEQ ID NO: 10 instead of the naturally encoded amino acid proline the amino acid serine.

In the method as described herein for controlling unwanted vegetation, the ALS inhibitor herbicide(s) can be taken from the groups as defined in [39].

In the method as described herein, the ALS inhibitor herbicide(s) can be taken from the groups as defined in [40].

In the method as described herein, the non ALS inhibitor herbicide(s) can be taken from the group as defined in [43].

In the method as described herein, the non ALS inhibitor herbicide(s) can be taken from the group as defined in [44].

### BRIEF DESCRIPTION OF THE DRAWINGS

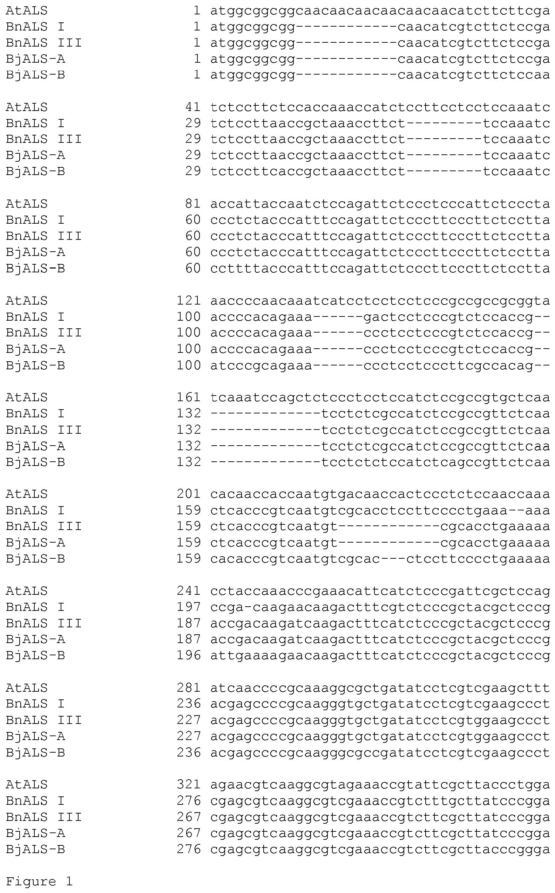

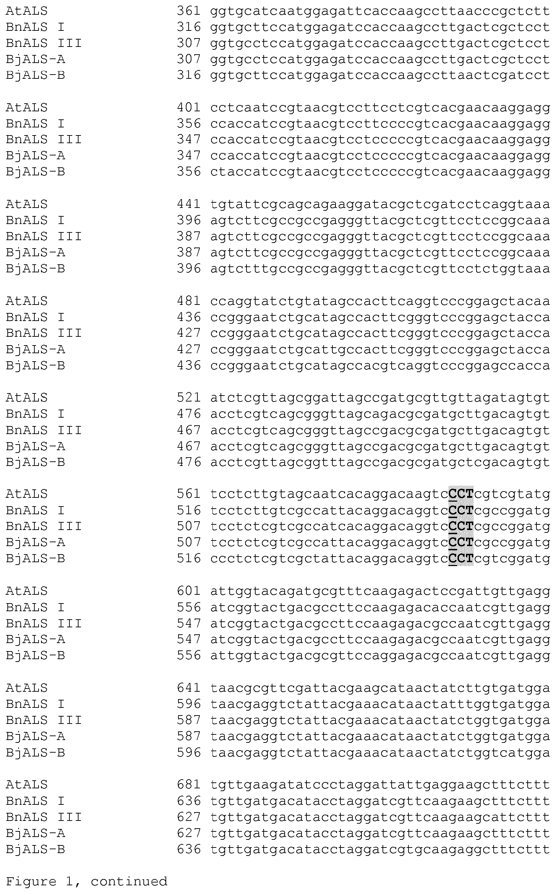

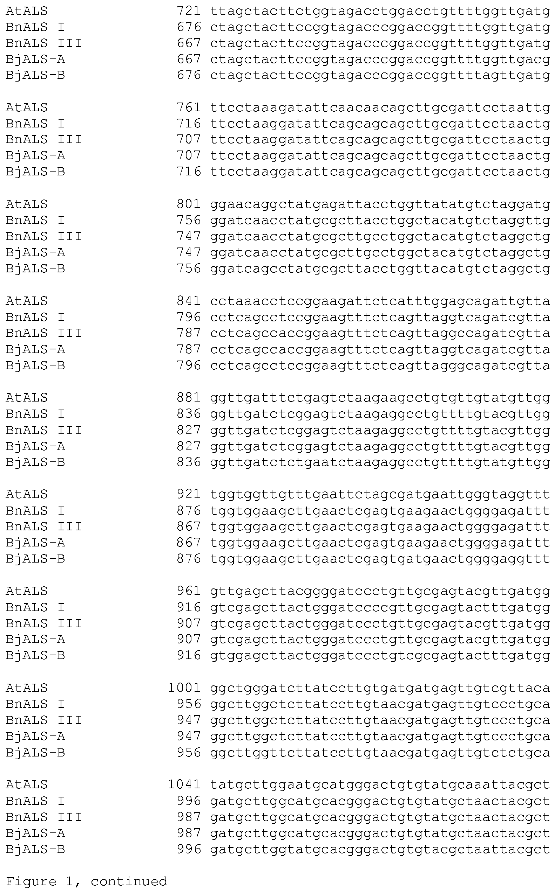

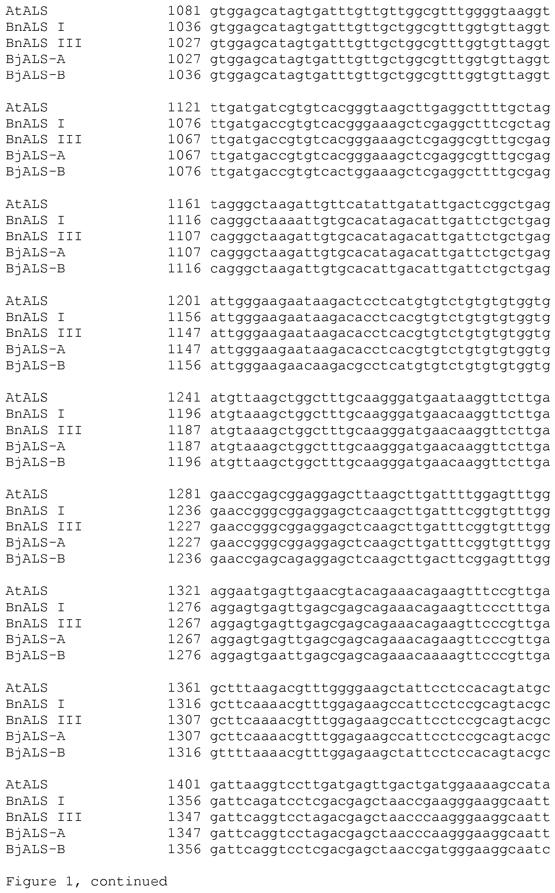

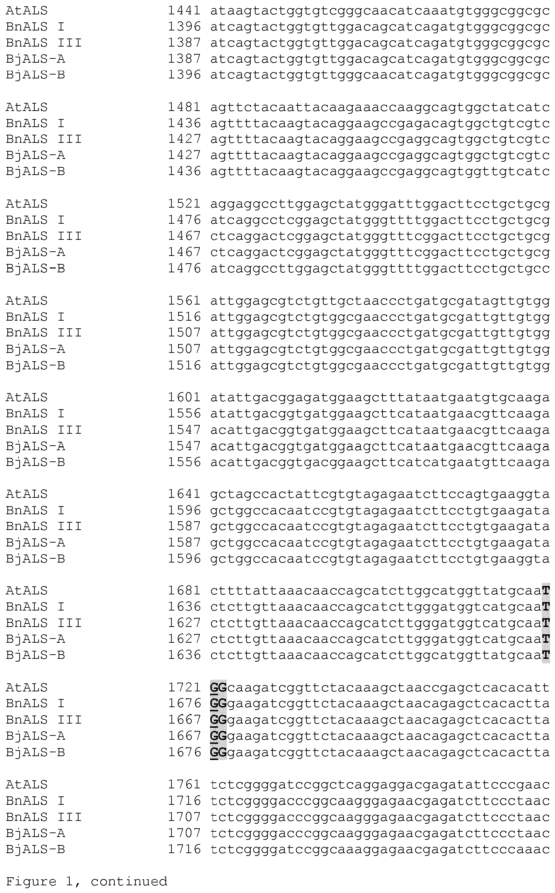

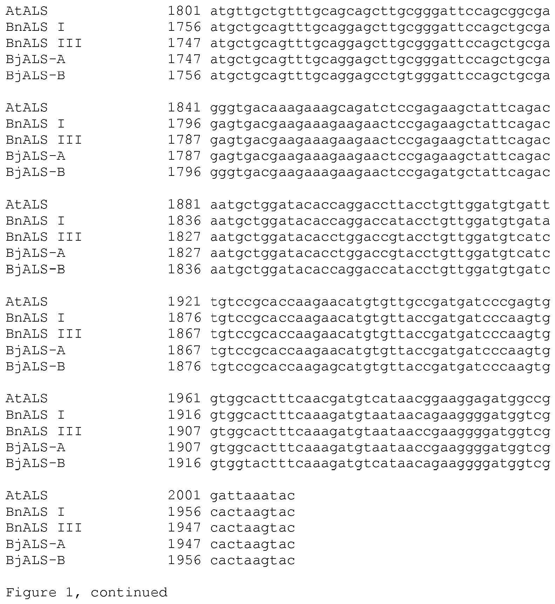

**Figure 1****:** Alignment of SEQ ID NOs: 9 (AtALS), 1 (BnALS I), 3 (BnALS III), 5 (BjALS-A), 7 (BjALS-B). The codon encoding the Proline at a position corresponding to position 197 of SEQ ID NO: 10, and the codon encoding the Tryptophan at a position corresponding to position 574 of SEQ ID NO: 10 are indicated with bold capitals on gray background.

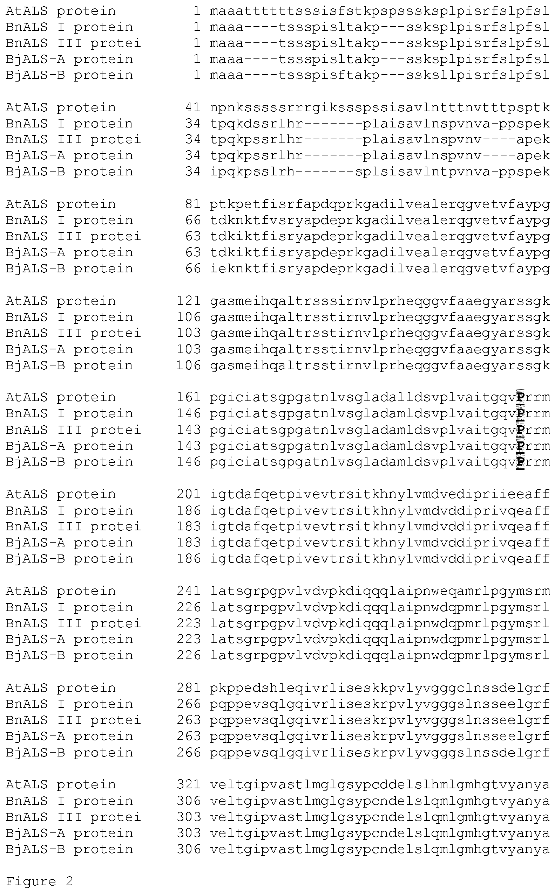

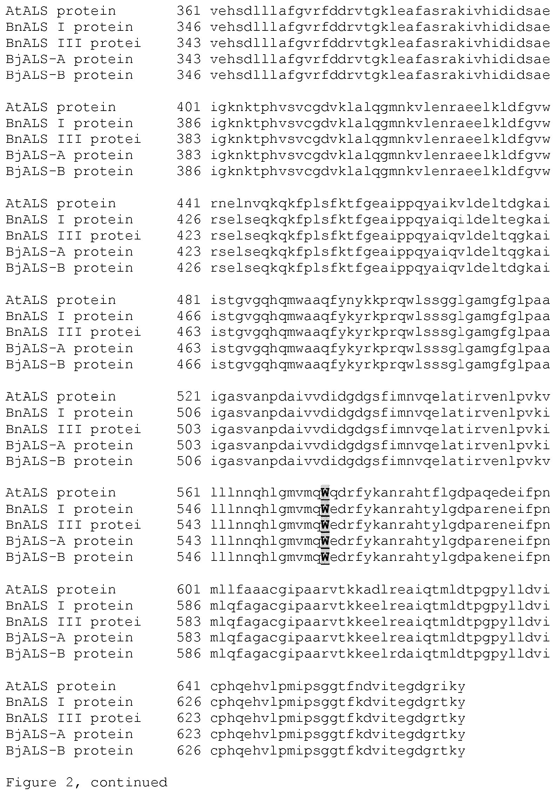

**Figure 2****:** Alignment of SEQ ID NOs: 10 (AtALS protein), 2 (BnALS I protein), 4 (BnALS III protein), 6 (BjALS-A protein), 8 (BjALS-B protein). The Proline (P) at a position corresponding to position 197 of SEQ ID NO: 10 and the Tryptophan (W) at a position corresponding to position 574 of SEQ ID NO: 10 are indicated with bold underlined capitals on gray background.

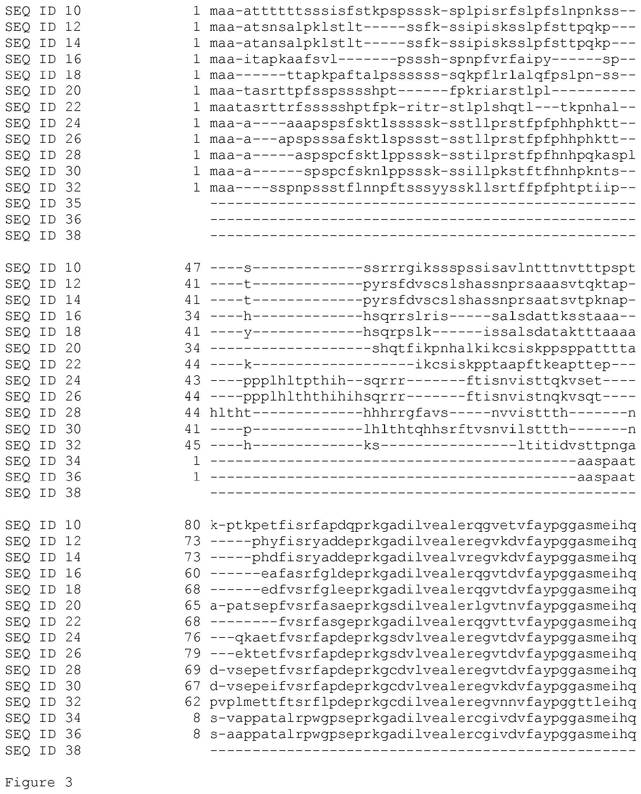

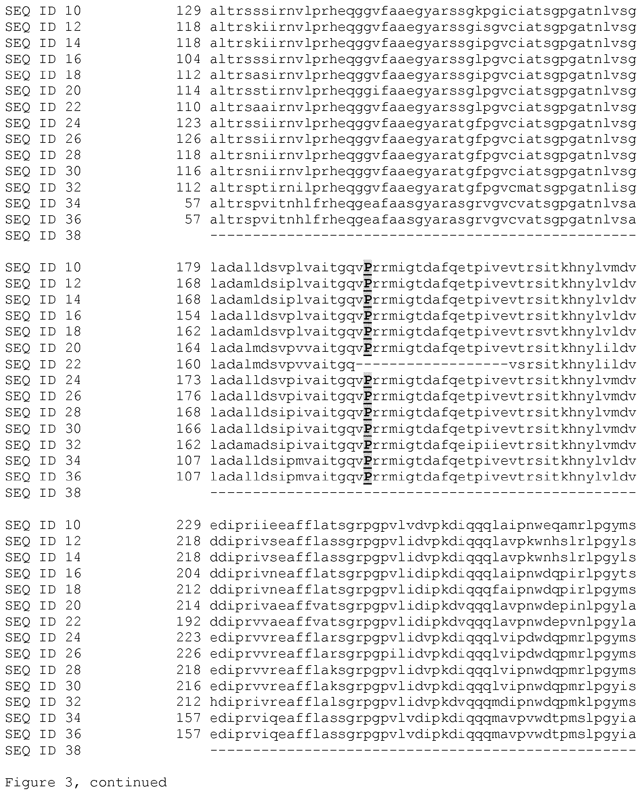

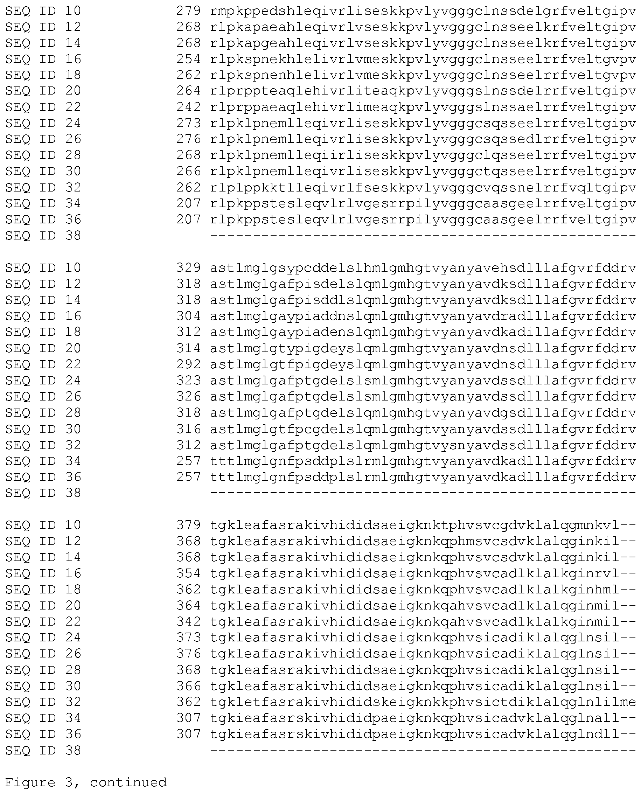

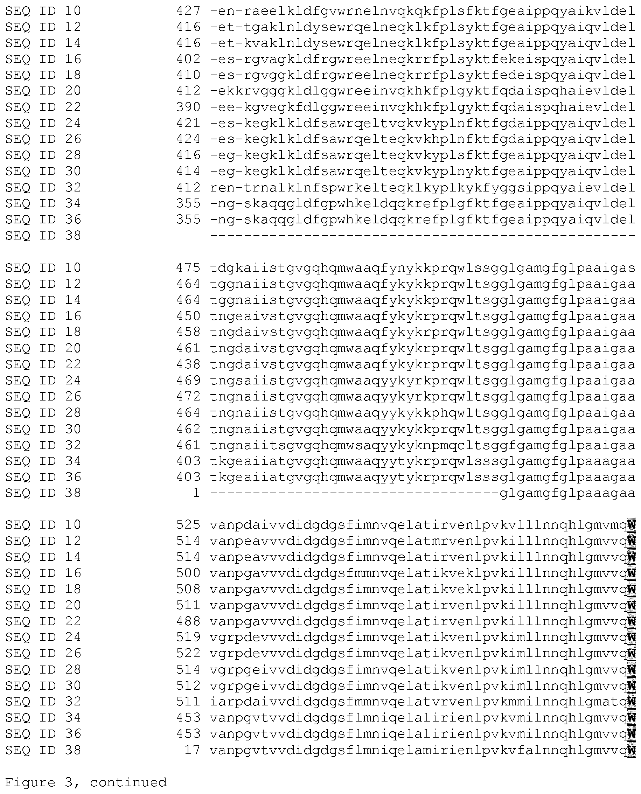

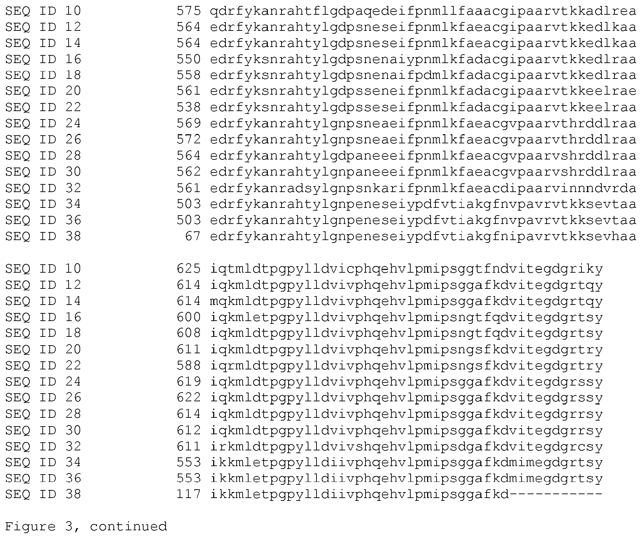

**Figure 3****:** Alignment of SEQ ID NOs: 10, 12, 14, 16, 18, 20, 22, 24, 26, 28, 30, 32, 34, 36, and 38. The Proline (P) at a position corresponding to position 197 of SEQ ID NO: 10 and the Tryptophan (W) at a position corresponding to position 574 of SEQ ID NO: 10 is indicated with bold underlined capitals on a gray background. The P at a position corresponding to position 197 of SEQ ID NO: 10 is absent from SEQ ID NO: 22, but this might be due to an error in the annotation of the gene, in which the sequence encoding the amino acids corresponding to amino acids 196-213 of SEQ ID NO: 10 are annotated as an intron (see EnsemblPlants number GLYMA04G37270.1). The P at a position corresponding to position 197 of SEQ ID NO: 10 is absent from SEQ ID NO: 38 as this concerns only a partial sequence of the Saccharum officinarum ALS gene (see GenBank number EU243998.1).

### DETAILED DESCRIPTION

### General definitions

It must be noted that as used herein, the terms "a", "an", and "the", include singular and plural references unless the context clearly indicates otherwise, i.e., such terms may refer to "one", "one or more" or "at least one". Thus, for example, reference to "a reagent" includes one or more of such different reagents and reference to "the method" includes reference to equivalent steps and methods known to those of ordinary skill in the art that could be modified or substituted for the methods described herein.

To the extent publications and patents cited in this disclosure contradict or are inconsistent with this specification, the specification will supersede any such material.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

### Plant

When used herein the term "polyploid plant" refers to a plant containing more than two paired sets of chromosomes. A polyploid plant can be an autopolyploid plant, which contains multiple chromosome sets from a single species. A polyploid plant can further be an allopolyploid plant, which contains multiple chromosome sets derived from different species, such as an allotetraploid plant, which contains four sets of chromosomes derived from two different species. Such polyploid plants can be, for example, triploid plants, comprising three sets of chromosomes, or can be tetraploid plants, comprising four sets of chromosomes, or can be pentaploid plants, comprising five sets of chromosomes, or can be hexaploid plants, comprising six sets of chromosomes, or can be octaploid plants, comprising eight sets of chromosomes, or can be decaploid plants, comprising ten sets of chromosomes, or can be dodecaploid plants, comprising twelve sets of chromosomes. Examples of polyploid plants include *Brasssica napus, Brassica juncea, Brassica carinata,* wheat, cotton (*Gossypium hirsutum*), potato, alfalfa, sugar cane, soybeans, leek, tobacco, peanut, kinnow, pelargonium, chrysanthemum, triticale, oat, kiwifruit, strawberry, dahlia, pansies, oca, tulips, lilies, daylilies, apple, banana, citrus, coffee and watermelon.

When used herein the term *"Brassica napus"* is abbreviated as *"B. napus".* Furthermore, the term "oilseed rape" is used herein. Said three terms are interchangeably used and should be understood to fully comprise the cultivated forms *of B. napus,* e.g., as defined in Tang et al, Plant Breeding, Volume 116, Issue 5, pages 471-474, October 1997 and Jesske et al., Tagung der Vereinigung der Pflanzenzüchter und Saatgutkaufleute Österreichs, 2009, 171-172, ISBN: 978-3-902559-37-1). Similarly, for example, the term *"Brassica juncea"* is abbreviated as *"B. juncea* ", and the term *"Arabidopsis thaliana"* is abbreviated as *"A. thaliana".* Both terms are interchangeably used herein.

When used herein "winter-type *Brassica* plant" can be winter-type *Brassica juncea,* or winter-type *Brassica napus.* Winter-type *Brassica napus* as used herein is also referred to as winter oilseed rape (WOSR). The term 'winter-type' refers to plant species that require cold treatment, or vernalization, before it will flower. In nature such plant species are mainly biennal species. In the first year the biennal plant grows vegetative (leafs, stems, roots) as rozet, and after a cold period between first and second year (winter season) the plant will elongate and start to flower in the second year. Winter oilseed rape is planted right after the harvest, typically from September to November in the Northern Hemisphere, sprouting before freezing occurs, then becomes dormant until the soil warms in the spring and is ready to be harvested in summer.

The term "wild-type" as used herein refers to a plant, a nucleic acid molecule or protein that can be found in nature as distinct from being artificially produced or mutated by man. Thus, a "wild type" plant does not produce or comprise ALS proteins with an amino acid different from tryptophan 574 (the numbers behind the amino acids indicate the positions corresponding to these positions of SEQ ID NO: 10, which is the ALS protein as derived from *A. thaliana*).

A "wild-type" *B. napus* plant can refer to a *B. napus* plant having at least one ALS nucleic acid sequence containing at least 60%, or 70%, or 80%, or 90%, or 95%, or 97%, or 98%, or 99% sequence identity, or is identical to SEQ ID NO: 1 and at least one ALS nucleic acid sequence containing at least 60%, or 70%, or 80%, or 90%, or 95%, or 97%, or 98%, or 99% sequence identity, or is identical to SEQ ID NO: 3, provided that said plant does not carry an ALS I gene carrying a mutation in the Trp574 codon yielding an amino acid different from Trp or an ALS I carrying a mutation in the P197 codon yielding an amino acid different from Pro and a mutation in the Trp574 codon yielding an amino acid different from Trp, and does not carry an ALS III gene carrying a mutation in the Trp574 codon yielding an amino acid different from Trp or an ALS III gene carrying a mutation in the P197 codon yielding an amino acid different from Pro and a mutation in the Trp574 codon yielding an amino acid different from Trp, wherein the amino acid position referred to is the position in the reference *A. thaliana* sequence (SEQ ID NO: 10). The use of the term "wild-type" is not intended to necessarily imply that a plant, plant tissue, plant cell, or other host cell lacks recombinant DNA in its genome, and/or does not possess herbicide resistant characteristics that are different from those disclosed herein.

A "wild-type" *B. juncea* plant can refer to a *B. juncea* plant having at least one ALS nucleic acid sequence containing at least 60%, or 70%, or 80%, or 90%, or 95%, or 97%, or 98%, or 99% sequence identity, or is identical to SEQ ID NO: 5 and at least one ALS nucleic acid sequence containing at least 60%, or 70%, or 80%, or 90%, or 95%, or 97%, or 98%, or 99% sequence identity, or is identical to SEQ ID NO: 7, provided that said plant does not carry an ALS-A gene carrying a mutation in the Trp574 codon yielding an amino acid different from Trp or an ALS-A gene carrying a mutation in the P197 codon yielding an amino acid different from Pro and a mutation in the Trp574 codon yielding an amino acid different from Trp, and does not carry an ALS-B gene carrying a mutation in the Trp574 codon yielding an amino acid different from Trp or an ALS-B gene carrying a mutation in the P197 codon yielding an amino acid different from Pro and a mutation in the Trp574 codon yielding an amino acid different from Trp, wherein the amino acid position referred to is the position in the reference A. *thaliana* sequence (SEQ ID NO: 10). The use of the term "wild-type" is not intended to necessarily imply that a plant, plant tissue, plant cell, or other host cell lacks recombinant DNA in its genome, and/or does not possess herbicide resistant characteristics that are different from those disclosed herein.

Due to the fact that the plants of the present invention which are herbicide resistant were generated by "random evolution", i.e., methods preferably leading to fertile plants having two point mutation as described herein in more detail without exogenous genetic manipulation, they are non-transgenic as far as the ALS gene in its endogenous gene locus is concerned.

Mutant ALS alleles as described herein can also be provided to plant cells as transgene. Accordingly, plants may contain a mutant ALS gene as described herein as transgene.

Moreover, the plants of the present invention and their offspring are fertile and thus useful for breeding purposes in order to generate varieties conferring agronomically useful levels of tolerance to ALS inhibitor herbicides, thus allowing innovative weed control measures plant growing areas.

The term *"Brassica* plant" as used herein refers to the genus of plants in the mustard family (*Brassicaceae*). The members of the genus may be collectively known either as cabbages, or as mustards. The genus *"Brassica"* encompasses, e.g., *B. carinata, B. elongata, B. fruticulosa, B. juncea, B. napus, B. narinosa, B. nigra, B. oleracea, B. perviridis, B. rapa, B. rupestris, B. septiceps,* and *B. tournefortii.* The skilled person will understand that the term not only encompasses *B. napus* but also other hybrids which have at least one parent plant of the genus *"Brassica".*

As used herein unless clearly indicated otherwise, the term "plant" intends to mean a plant at any developmental stage. Moreover, the term also encompasses "parts of a plant". The term "plant" encompasses a plant as described herein, or progeny of the plants which retain the distinguishing characteristics of the parents, such as seed obtained by selfing or crossing, e.g. hybrid seed (obtained by crossing two inbred parental lines), hybrid plants and plant parts derived there from are encompassed herein, unless otherwise indicated.

Parts of (a) plant(s) may be attached to or separate from a whole intact plant. Such parts of a plant include, but are not limited to, cells of a plant, tissues or organs, seeds, severed parts such as roots, leaves, flowers, pollen, etc.

The obtained plants according to the invention can be used in a conventional breeding scheme to produce more plants with the same characteristics or to introduce the ALS alleles according to the invention in other varieties of the same or related plant species, or in hybrid plants. The obtained plants can further be used for creating propagating material. Plants according to the invention can further be used to produce gametes, seeds (including crushed seeds and seed cakes), seed oil, embryos, either zygotic or somatic, progeny or hybrids of plants obtained by methods of the invention.

"Creating propagating material", as used herein, relates to any means know in the art to produce further plants, plant parts or seeds and includes inter alia vegetative reproduction methods (e.g. air or ground layering, division, (bud) grafting, micropropagation, striking or cutting), sexual reproduction (crossing with another plant) and asexual reproduction (e.g. apomixis, somatic hybridization).

In one embodiment, a plant of the invention comprises at least two ALS genes wherein all ALS genes encode an ALS polypeptide wherein Trp at a position corresponding to position 574 of SEQ ID NO: 10 is substituted by Leu, and wherein at least one of the ALS genes encodes an ALS polypeptide which further comprises at a position corresponding to position 197 of SEQ ID NO: 10 instead of the naturally encoded amino acid proline the amino acid serine. An ALS gene encoding an ALS polypeptide wherein Trp at a position corresponding to position 574 of SEQ ID NO: 10 is substituted by Leu may, or may not, further comprise at a position corresponding to position 197 of SEQ ID NO: 10 instead of the naturally encoded amino acid proline the amino acid serine. The plant according to the invention may comprise at least two ALS genes, wherein all ALS genes encode an ALS polypeptide wherein Trp at a position corresponding to position 574 of SEQ ID NO: 10 is substituted by Leu, and wherein one of the ALS genes encodes an ALS polypeptide which further comprises at a position corresponding to position 197 of SEQ ID NO: 10 instead of the naturally encoded amino acid proline the amino acid serine. In another embodiment, said plant comprises at least two ALS genes, wherein all ALS genes encode an ALS polypeptide comprising at a position corresponding to position 574 of SEQ ID NO: 10 instead of the naturally encoded amino acid tryptophan the amino acid leucine, and comprising at a position corresponding to position 197 of SEQ ID NO: 10 instead of the naturally encoded amino acid proline the amino acid serine.

In one embodiment, a *B. napus* plant of the invention comprises an ALS I protein wherein Pro at a position corresponding to position 182 of SEQ ID NO: 2 is substituted by Ser, and wherein Trp at a position corresponding to position 559 of SEQ ID NO: 2 is substituted by Leu and an ALS III protein wherein Trp at a position corresponding to position 556 of SEQ ID NO: 4 is substituted by Leu, or a *B. napus* plant of the invention comprises an ALS I protein wherein Trp at a position corresponding to position 559 of SEQ ID NO: 2 is substituted by Leu and an ALS III protein wherein Pro at a position corresponding to position 179 of SEQ ID NO: 4 is substituted by Ser, and wherein Trp at a position corresponding to position 556 of SEQ ID NO: 4 is substituted by Leu, or a *B. napus* of the invention comprises an ALS I protein wherein Pro at a position corresponding to position 182 of SEQ ID NO: 2 is substituted by Ser, and wherein Trp at a position corresponding to position 559 of SEQ ID NO: 2 is substituted by Leu and an ALS III protein wherein Pro at a position corresponding to position 179 of SEQ ID NO: 4 is substituted by Ser, and wherein Trp at a position corresponding to position 556 of SEQ ID NO: 4 is substituted by Leu.

In a further embodiment, a *B. napus* plant of the invention comprises an ALS I protein wherein Pro at a position corresponding to position 182 of SEQ ID NO: 2 is substituted by Ser, and wherein Trp at a position corresponding to position 559 of SEQ ID NO: 2 is substituted by Leu and an ALS III protein wherein Trp at a position corresponding to position 556 of SEQ ID NO: 4 is substituted by Leu, and does neither comprise a wild type ALS I protein nor a wild type ALS III protein, or a *B. napus* plant of the invention comprisesan ALS I protein wherein Trp at a position corresponding to position 559 of SEQ ID NO: 2 is substituted by Leu and an ALS III protein wherein Pro at a position corresponding to position 179 of SEQ ID NO: 4 is substituted by Ser, and wherein Trp at a position corresponding to position 556 of SEQ ID NO: 4 is substituted by Leu and does neither comprise a wild type ALS I protein nor a wild type ALS III protein, or a *B. napus* plant of the invention comprises an ALS I protein wherein Pro at a position corresponding to position 182 of SEQ ID NO: 2 is substituted by Ser, and wherein Trp at a position corresponding to position 559 of SEQ ID NO: 2 is substituted by Leu and an ALS III protein wherein Pro at a position corresponding to position 179 of SEQ ID NO: 4 is substituted by Ser, and wherein Trp at a position corresponding to position 556 of SEQ ID NO: 4 is substituted by Leu and does neither comprise a wild type ALS I protein nor a wild type ALS III protein.

The *B. napus* plant of the invention may comprise an ALS I gene of SEQ ID NO: 1 of which the C at position 544 is substituted with T and of which the G at position 1676 is substituted with T and an ALS III gene of SEQ ID NO: 3 of which the G at position 1667 is substituted with T, or a *B. napus* plant of the invention may comprise an ALS I gene of SEQ ID NO: 1 of which the G at position 1676 is substituted with T, and an ALS III gene of SEQ ID NO: 3 of which the C at position 535 is substituted with T and of which the G at position 1667 is substituted with T, or a *B. napus* plant of the invention may comprise an ALS I gene of SEQ ID NO: 1 of which the C at position 544 is substituted with T and of which the G at position 1676 is substituted with T and an ALS III gene of SEQ ID NO: 3 of which the C at position 535 is substituted with T and of which the G at position 1667 is substituted with T.

In one embodiment, a *B. juncea* plant of the invention comprises an ALS-A protein wherein Pro at a position corresponding to position 179 of SEQ ID NO: 6 is substituted by Ser, and wherein Trp at a position corresponding to position 556 of SEQ ID NO: 6 is substituted by Leu and an ALS-B protein wherein Trp at a position corresponding to position 559 of SEQ ID NO: 8 is substituted by Leu, or a *B. juncea* plant of the invention comprises an ALS-A protein wherein Trp at a position corresponding to position 556 of SEQ ID NO: 6 is substituted by Leu and an ALS-B protein wherein Pro at a position corresponding to position 182 of SEQ ID NO: 8 is substituted by Ser, and wherein Trp at a position corresponding to position 559 of SEQ ID NO: 8 is substituted by Leu, or a *B. juncea* plant of the invention comprises an ALS-A protein wherein Pro at a position corresponding to position 179 of SEQ ID NO: 6 is substituted by Ser, and wherein Trp at a position corresponding to position 556 of SEQ ID NO: 6 is substituted by Leu and an ALS-B protein wherein Pro at a position corresponding to position 182 of SEQ ID NO: 8 is substituted by Ser, and wherein Trp at a position corresponding to position 559 of SEQ ID NO: 8 is substituted by Leu.

The *B. juncea* plant of the invention may comprise an ALS-A protein wherein Pro at a position corresponding to position 179 of SEQ ID NO: 6 is substituted by Ser, and wherein Trp at a position corresponding to position 556 of SEQ ID NO: 6 is substituted by Leu and an ALS-B protein wherein Trp at a position corresponding to position 559 of SEQ ID NO: 8 is substituted by Leu, and does neither comprise a wild type ALS-A protein nor a wild type ALS-B protein, or a *B. juncea* plant of the invention may comprise an ALS-A protein wherein Trp at a position corresponding to position 556 of SEQ ID NO: 6 is substituted by Leu and an ALS-B protein wherein Pro at a position corresponding to position 182 of SEQ ID NO: 8 is substituted by Ser, and wherein Trp at a position corresponding to position 559 of SEQ ID NO: 8 is substituted by Leu and does neither comprise a wild type ALS-A protein nor a wild type ALS-B protein, or a *B. juncea* plant of the invention may comprise an ALS-A protein wherein Pro at a position corresponding to position 179 of SEQ ID NO: 6 is substituted by Ser, and wherein Trp at a position corresponding to position 556 of SEQ ID NO: 6 is substituted by Leu and an ALS-B protein wherein Pro at a position corresponding to position 182 of SEQ ID NO: 8 is substituted by Ser, and wherein Trp at a position corresponding to position 559 of SEQ ID NO: 8 is substituted by Leu and does neither comprise a wild type ALS-A protein nor a wild type ALS-B protein.

The *B. juncea* plant of the invention may comprise an ALS-A gene of SEQ ID NO: 5 of which the C at position 535 is substituted with T and of which the G at position 1667 is substituted with T and an ALS-B gene of SEQ ID NO: 7 of which the G at position 1676 is substituted with T, or a *B. juncea* plant of the invention may comprise an ALS-A gene of SEQ ID NO: 5 of which the G at position 1667 is substituted with T and an ALS-B gene of SEQ ID NO: 7 of which the C at position 544 is substituted with T and of which the G at position 1676 is substituted with T, or a *B. juncea* plant of the invention may comprise an ALS-A gene of SEQ ID NO: 5 of which the C at position 535 is substituted with T and of which the G at position 1667 is substituted with T and an ALS-B gene of SEQ ID NO: 7 of which the C at position 544 is substituted with T and of which the G at position 1676 is substituted with T.

In one embodiment, a plant in accordance with the present invention is obtainable from or derivable from or can be obtained from or derived from seeds deposited with the NCIMB, Ferguson Building, Craibstone Estate, Bucksburn, Aberdeen, AB 21 9YA UK, under the Budapest Treaty on May 20, 2013, under accession number NCIMB 42145, from seeds deposited with the NCIMB, Ferguson Building, Craibstone Estate, Bucksburn, Aberdeen, AB 21 9YA UK, under the Budapest Treaty on May 8, 2014, under accession number NCIMB 42235, from seeds deposited with the NCIMB, Ferguson Building, Craibstone Estate, Bucksburn, Aberdeen, AB 21 9YA UK, under the Budapest Treaty on July 5, 2013, under accession number NCIMB 42153, from seeds deposited with the NCIMB, Ferguson Building, Craibstone Estate, Bucksburn, Aberdeen, AB 21 9YA UK, under the Budapest Treaty on July 1, 2014, under accession number NCIMB 42259, and/or from seeds deposited with the NCIMB, Ferguson Building, Craibstone Estate, Bucksburn, Aberdeen, AB 21 9YA UK, under the Budapest Treaty on July 1, 2014, under accession number NCIMB 42260.

It will be clear that a *B. napus* plant comprising an ALS I gene encoding an ALS I polypeptide comprising at a position corresponding to position 182 of SEQ ID NO: 2 instead of the naturally encoded amino acid proline the amino acid serine and comprising at a position corresponding to position 559 of SEQ ID NO: 2 instead of the naturally encoded amino acid tryptophan the amino acid leucine, and an ALS III gene encoding an ALS III polypeptide comprising at a position corresponding to position 556 of SEQ ID NO: 4 instead of the naturally encoded amino acid tryptophan the amino acid is obtainable from or derivable from or can be obtained from or derived from seeds deposited with the NCIMB under Number 42145 and Number 42260, or from seeds deposited with the NCIMB under Number 42235 and Number 42260, and that a *B. napus* plant comprising an ALS I gene encoding an ALS I polypeptide comprising at a position corresponding to position 559 of SEQ ID NO: 2 instead of the naturally encoded amino acid tryptophan the amino acid leucine, and an ALS III gene encoding an ALS III polypeptide comprising at a position corresponding to position 179 of SEQ ID NO: 4 instead of the naturally encoded amino acid proline the amino acid serine and comprising at a position corresponding to position 556 of SEQ ID NO: 4 instead of the naturally encoded amino acid tryptophan the amino acid leucine is obtainable from or derivable from or can be obtained from or derived from seeds deposited with the NCIMB under Number 42145 and Number 42153, or from seeds deposited with the NCIMB under Number 42235 and Number 42153, or from seeds deposited with the NCIMB under Number 42145 and Number 42259, or from seeds deposited with the NCIMB under Number 42235 and Number 42259, and that a *B. napus* plant comprising an ALS I gene encoding an ALS I polypeptide comprising at a position corresponding to position 182 of SEQ ID NO: 2 instead of the naturally encoded amino acid proline the amino acid serine and comprising at a position corresponding to position 559 of SEQ ID NO: 2 instead of the naturally encoded amino acid tryptophan the amino acid leucine, and an ALS III gene encoding an ALS III polypeptide comprising at a position corresponding to position 179 of SEQ ID NO: 4 instead of the naturally encoded amino acid proline the amino acid serine and comprising at a position corresponding to position 556 of SEQ ID NO: 4 instead of the naturally encoded amino acid tryptophan the amino acid leucine is obtainable from or derivable from or can be obtained from or derived from seeds deposited with the NCIMB under Number 42153 and Number 42260, or from seeds deposited with the NCIMB under Number 42259 and Number 42260.

Said plant obtainable from or derivable from or can be obtained from or derived from seeds deposited with the NCIMB under Number 42145, from seeds deposited with NCIMB under number 42235, from seeds deposited with the NCIMB under Number 42153 and/or from seeds deposited with the NCIMB under Number 42259 can be a plant directly grown or regenerated from one of said deposited seeds or a plant comprising both mutant alleles described herein, i.e., an ALS I allele coding for an ALS I protein having a mutation at a position corresponding to position 559 of SEQ ID NO:2 as described herein and an ALS III allele coding for an ALS III protein having a mutation at a position corresponding to position 179 and at a position corresponding to position 556 of SEQ ID NO: 4 as described herein. Said plant obtainable from or derivable from or can be obtained from or derived from seeds deposited with the NCIMB under Number 42145, from seeds deposited with NCIMB under number 42235, and/or from seeds deposited with the NCIMB under Number 42260 can be a plant directly grown or regenerated from one of said deposited seeds or a plant comprising both mutant alleles described herein, i.e., an ALS I allele coding for an ALS I protein having a mutation at a position corresponding to position 182 and at a position corresponding to position 559 of SEQ ID NO:2 as described herein and an ALS III allele coding for an ALS III protein having a mutation at a position corresponding to position 556 of SEQ ID NO: 4 as described herein. Said plant obtainable from or derivable from or can be obtained from or derived from seeds deposited with the NCIMB under Number 42153, from seeds deposited with the NCIMB under Number 42259, and/or from seeds deposited with the NCIMB under Number 42260 can be a plant directly grown or regenerated from one of said deposited seeds or a plant comprising both mutant alleles described herein, i.e., an ALS I allele coding for an ALS I protein having a mutation at a position corresponding to position 182 and at a position corresponding to position 559 of SEQ ID NO:2 as described herein and an ALS III allele coding for an ALS III protein having a mutation at a position corresponding to position 179 and at a position corresponding to position 556 of SEQ ID NO: 4 as described herein.

Such a plant obtainable from or derivable from or can be obtained from or derived from seeds deposited with the NCIMB under Number 42145, 42235, 42153, and/or 42259 encompasses also a first, second, third, fourth or higher generation progeny of a plant directly grown or regenerated from said deposited seed or a first, second, third, fourth or higher generation progeny of a plant having at least one ALS I allele decoding for an ALS I protein having a mutation at a position corresponding to position 559 of SEQ ID NO:2 as described herein and at least one ALS III allele decoding for an ALS III protein having a mutation at a position corresponding to position 179 and at a position corresponding to position 556 of SEQ ID NO: 4 as described herein. Such a plant can be homozygous regarding its ALS I and ALS III alleles. A plant obtainable from or derivable from or can be obtained from or derived from seeds deposited with the NCIMB under Number 42145, 42235 and/or 42260 encompasses also a first, second, third, fourth or higher generation progeny of a plant directly grown or regenerated from said deposited seed or a first, second, third, fourth or higher generation progeny of a plant having at least one ALS I allele decoding for an ALS I protein having a mutation at a position corresponding to position 182 and at a position corresponding to position 559 of SEQ ID NO:2 as described herein and at least one ALS III allele decoding for an ALS III protein having a mutation at a position corresponding to position 556 of SEQ ID NO: 4 as described herein. Such a plant can be homozygous regarding its ALS I and ALS III alleles. A plant obtainable from or derivable from or can be obtained from or derived from seeds deposited with the NCIMB under Number 42153, 42259 and/or 42260 encompasses also a first, second, third, fourth or higher generation progeny of a plant directly grown or regenerated from said deposited seed or a first, second, third, fourth or higher generation progeny of a plant having at least one ALS I allele decoding for an ALS I protein having a mutation at a position corresponding to position 182 and at a position corresponding to position 559 of SEQ ID NO:2 as described herein and at least one ALS III allele decoding for an ALS III protein having a mutation at a position corresponding to position 179 and at a position corresponding to position 556 of SEQ ID NO: 4 as described herein. Such a plant may be homozygous regarding its ALS I and ALS III alleles.

In a further embodiment, a plant in accordance with the present invention is provided which comprises an ALS I allele coding for an ALS I protein having a mutation at a position corresponding to position 559 of SEQ ID NO:2 and an ALS III allele coding for an ALS III protein having a mutation at a position corresponding to position 179 and at a position corresponding to position 556 of SEQ ID NO: 4 as present in reference seeds deposited with the NCIMB, Ferguson Building, Craibstone Estate, Bucksburn, Aberdeen, AB 21 9YA UK, under the Budapest Treaty on May 20, 2013, under accession number NCIMB 42145, on May 8, 2014 under accession number NCIMB 42235, on July 5, 2013 under accession number NCIMB 42153, and on July 1, 2014 under accession number NCIMB 42259. In yet a further embodiment, a plant in accordance with the present invention is provided which comprises an ALS I allele coding for an ALS I protein having a mutation at a position corresponding to position 182 and at a position corresponding to position 559 of SEQ ID NO:2 and an ALS III allele coding for an ALS III protein having a mutation at a position corresponding to position 556 of SEQ ID NO: 4 as present in reference seeds deposited with the NCIMB, Ferguson Building, Craibstone Estate, Bucksburn, Aberdeen, AB 21 9YA UK, under the Budapest Treaty on May 20, 2013, under accession number NCIMB 42145, on May 8, 2014, under accession number NCIMB 42235, and on July 1, 2014, under accession number NCIMB 42260. In another embodiment, a plant in accordance with the present invention is provided which comprises an ALS I allele coding for an ALS I protein having a mutation at a position corresponding to position 182 and at a position corresponding to position 559 of SEQ ID NO:2 and an ALS III allele coding for an ALS III protein having a mutation at a position corresponding to position 179 and at a position corresponding to position 556 of SEQ ID NO: 4 as present in reference seeds deposited with the NCIMB, Ferguson Building, Craibstone Estate, Bucksburn, Aberdeen, AB 21 9YA UK, under the Budapest Treaty on July 5, 2013, under accession number NCIMB 42153, on July 1, 2014, under accession number NCIMB 42259, and on July 1, 2014, under accession number NCIMB 42260.

Moreover, also plant cells are obtainable from or are derivable from or are obtained from or are derived from said deposited seeds; or plant cells are obtainable from or are derivable from or are obtained from or are derived from plants which were grown from said deposited seeds.

Accordingly, one embodiment of the present invention is also directed to reference seeds comprising the mutant alleles described herein having been deposited under Number NCIMB 42145, NCIMB 42235, NCIMB 42153, NCIMB 42259 or NCIMB 42260.

One embodiment of the present invention refers to progeny of an allotetraploid ALS inhibitor herbicide tolerant *Brassica* plant or parts thereof according to the invention comprising at least two ALS genes, wherein all endogenous ALS genes encode an ALS polypeptide comprising at a position corresponding to position 574 of SEQ ID NO: 10 instead of the naturally encoded amino acid tryptophan the amino acid leucine and wherein at least one of the ALS genes encodes an ALS polypeptide which further comprises at a position corresponding to position 197 of SEQ ID NO: 10 instead of the naturally encoded amino acid proline the amino acid serine, such as an ALS inhibitor herbicide tolerant *B. napus* plant or parts thereof comprising an ALS I polypeptide comprising at a position corresponding to position 182 of SEQ ID NO: 2 instead of the naturally encoded amino acid proline the amino acid serine and at a position corresponding to position 559 of SEQ ID NO: 2 instead of the naturally encoded amino acid tryptophan the amino acid leucine, and an ALS III polypeptide comprising at a position corresponding to position 556 of SEQ ID NO: 4 instead of the naturally encoded amino acid tryptophan the amino acid leucine, or such as an ALS inhibitor herbicide tolerant *B. napus* plant or parts thereof comprising an ALS I gene encoding an ALS I polypeptide comprising at a position corresponding to position 559 of SEQ ID NO: 2 instead of the naturally encoded amino acid tryptophan the amino acid leucine, and an ALS III gene encoding an ALS III polypeptide comprising at a position corresponding to position 179 of SEQ ID NO: 4 instead of the naturally encoded amino acid proline the amino acid serine and comprising at a position corresponding to position 556 of SEQ ID NO: 4 instead of the naturally encoded amino acid tryptophan the amino acid leucine, or such as an ALS inhibitor herbicide tolerant *B*. *napus* plant or parts thereof comprising an ALS I gene encoding an ALS I polypeptide comprising at a position corresponding to position 182 of SEQ ID NO: 2 instead of the naturally encoded amino acid proline the amino acid serine and at a position corresponding to position 559 of SEQ ID NO: 2 instead of the naturally encoded amino acid tryptophan the amino acid leucine, and an ALS III gene encoding an ALS III polypeptide comprising at a position corresponding to position 179 of SEQ ID NO: 4 instead of the naturally encoded amino acid proline the amino acid serine and comprising at a position corresponding to position 556 of SEQ ID NO: 4 instead of the naturally encoded amino acid tryptophan the amino acid leucine, or such as an ALS inhibitor herbicide tolerant *B. juncea* plant or parts thereof comprising an ALS-A gene encoding an ALS-A polypeptide comprising at a position corresponding to position 179 of SEQ ID NO: 6 instead of the naturally encoded amino acid proline the amino acid serine and comprising at a position corresponding to position 556 of SEQ ID NO: 6 instead of the naturally encoded amino acid tryptophan the amino acid leucine, and an ALS-B gene encoding an ALS-B polypeptide comprising at a position corresponding to position 559 of SEQ ID NO: 8 instead of the naturally encoded amino acid tryptophan the amino acid leucine, or such as an ALS inhibitor herbicide tolerant *B. juncea* plant or parts thereof comprising an ALS-A gene encoding an ALS-A polypeptide comprising at a position corresponding to position 556 of SEQ ID NO: 6 instead of the naturally encoded amino acid tryptophan the amino acid leucine, and an ALS-B gene encoding an ALS-B polypeptide comprising at a position corresponding to position 182 of SEQ ID NO: 8 instead of the naturally encoded amino acid proline the amino acid serine and comprising at a position corresponding to position 559 of SEQ ID NO: 8 instead of the naturally encoded amino acid tryptophan the amino acid leucine, or such as an ALS inhibitor herbicide tolerant *B. juncea* plant or parts thereof comprising an ALS-A gene encoding an ALS-A polypeptide comprising at a position corresponding to position 179 of SEQ ID NO: 6 instead of the naturally encoded amino acid proline the amino acid serine and comprising at a position corresponding to position 556 of SEQ ID NO: 6 instead of the naturally encoded amino acid tryptophan the amino acid leucine, and an ALS-B gene encoding an ALS-B polypeptide comprising at a position corresponding to position 182 of SEQ ID NO: 8 instead of the naturally encoded amino acid proline the amino acid serine and comprising at a position corresponding to position 559 of SEQ ID NO: 8 instead of the naturally encoded amino acid tryptophan the amino acid leucine.

"Progeny" as used herein refers to plants derived from a polyploid ALS inhibitor herbicide tolerant plant comprising at least two ALS genes, wherein all ALS genes encode an ALS polypeptide comprising at a position corresponding to position 574 of SEQ ID NO: 10 instead of the naturally encoded amino acid tryptophan the amino acid leucine, and wherein at least one of the ALS genes encodes an ALS polypeptide which further comprises at a position corresponding to position 197 of SEQ ID NO: 10 instead of the naturally encoded amino acid proline the amino acid serine, such as a *Brassica juncea* plant or parts thereof comprising an ALS-A polypeptide comprising at a position corresponding to position 179 of SEQ ID NO: 6 instead of the naturally encoded amino acid proline the amino acid serine and comprising at a position corresponding to position 556 of SEQ ID NO: 6 instead of the naturally encoded amino acid tryptophan the amino acid leucine, and an ALS-B polypeptide comprising at a position corresponding to position 559 of SEQ ID NO: 8 instead of the naturally encoded amino acid tryptophan the amino acid leucine, or such as a *Brassica juncea* plant or parts thereof comprising an ALS-A polypeptide comprising at a position corresponding to position 556 of SEQ ID NO: 6 instead of the naturally encoded amino acid tryptophan the amino acid leucine , and an ALS-B polypeptide comprising at a position corresponding to position 182 of SEQ ID NO: 8 instead of the naturally encoded amino acid proline the amino acid serine and comprising at a position corresponding to position 559 of SEQ ID NO: 8 instead of the naturally encoded amino acid tryptophan the amino acid leucine, or such as a *Brassica juncea* plant or parts thereof comprising an ALS-A polypeptide comprising at a position corresponding to position 179 of SEQ ID NO: 6 instead of the naturally encoded amino acid proline the amino acid serine and comprising at a position corresponding to position 556 of SEQ ID NO: 6 instead of the naturally encoded amino acid tryptophan the amino acid leucine, and an ALS-B polypeptide comprising at a position corresponding to position 182 of SEQ ID NO: 8 instead of the naturally encoded amino acid proline the amino acid serine and comprising at a position corresponding to position 559 of SEQ ID NO: 8 instead of the naturally encoded amino acid tryptophan the amino acid leucine, or such as a *Brassica napus* plant or parts thereof comprising an ALS I polypeptide comprising at a position corresponding to position 182 of SEQ ID NO: 2 instead of the naturally encoded amino acid proline the amino acid serine and comprising at a position corresponding to position 559 of SEQ ID NO: 2 instead of the naturally encoded amino acid tryptophan the amino acid leucine, and an ALS III polypeptide comprising at a position corresponding to position 556 of SEQ ID NO: 4 instead of the naturally encoded amino acid tryptophan the amino acid leucine, e.g., a plant obtainable from or derivable from or obtained from or derived from seeds deposited with the NCIMB, Ferguson Building, Craibstone Estate, Bucksburn, Aberdeen, AB 21 9YA UK, under the Budapest Treaty on May 20, 2013, under accession number NCIMB 42145, from seeds deposited with the NCIMB, Ferguson Building, Craibstone Estate, Bucksburn, Aberdeen, AB 21 9YA UK, under the Budapest Treaty on 8 May, 2014, under accession number NCIMB 42235, and/or from seeds deposited with the NCIMB, Ferguson Building, Craibstone Estate, Bucksburn, Aberdeen, AB 21 9YA UK, under the Budapest Treaty on July 1, 2014, under accession number NCIMB 42260, or such as a *Brassica napus* plant or parts thereof comprising an ALS I polypeptide comprising at a position corresponding to position 559 of SEQ ID NO: 2 instead of the naturally encoded amino acid tryptophan the amino acid leucine, and an ALS III polypeptide comprising at a position corresponding to position 179 of SEQ ID NO: 4 instead of the naturally encoded amino acid proline the amino acid serine and comprising at a position corresponding to position 556 of SEQ ID NO: 4 instead of the naturally encoded amino acid tryptophan the amino acid leucine, e.g., a plant obtainable from or derivable from or obtained from or derived from seeds deposited with the NCIMB, Ferguson Building, Craibstone Estate, Bucksburn, Aberdeen, AB 21 9YA UK, under the Budapest Treaty on May 20, 2013, under accession number NCIMB 42145, from seeds deposited with the NCIMB, Ferguson Building, Craibstone Estate, Bucksburn, Aberdeen, AB 21 9YA UK, under the Budapest Treaty on 8 May, 2014, under accession number NCIMB 42235, from seeds deposited with the NCIMB, Ferguson Building, Craibstone Estate, Bucksburn, Aberdeen, AB 21 9YA UK, under the Budapest Treaty on July 5, 2013, under accession number NCIMB 42153, and/or from seeds deposited with the NCIMB, Ferguson Building, Craibstone Estate, Bucksburn, Aberdeen, AB 21 9YA UK, under the Budapest Treaty on July 1, 2014, under accession number NCIMB 42259, or such as a *Brassica napus* plant or parts thereof comprising an ALS I polypeptide comprising at a position corresponding to position 182 of SEQ ID NO: 2 instead of the naturally encoded amino acid proline the amino acid serine and comprising at a position corresponding to position 559 of SEQ ID NO: 2 instead of the naturally encoded amino acid tryptophan the amino acid leucine, and an ALS III polypeptide comprising at a position corresponding to position 179 of SEQ ID NO: 4 instead of the naturally encoded amino acid proline the amino acid serine and comprising at a position corresponding to position 556 of SEQ ID NO: 4 instead of the naturally encoded amino acid tryptophan the amino acid leucine, e.g., a plant obtainable from or derivable from or obtained from or derived from seeds deposited with the NCIMB, Ferguson Building, Craibstone Estate, Bucksburn, Aberdeen, AB 21 9YA UK, under the Budapest Treaty on July 5, 2013, under accession number NCIMB 42153, from seeds deposited with the NCIMB, Ferguson Building, Craibstone Estate, Bucksburn, Aberdeen, AB 21 9YA UK, under the Budapest Treaty on July 1, 2014, under accession number NCIMB 42259, and/or from seeds deposited with the NCIMB, Ferguson Building, Craibstone Estate, Bucksburn, Aberdeen, AB 21 9YA UK, under the Budapest Treaty on July 1, 2014, under accession number NCIMB 42260. Progeny may be derived by regeneration of cell or tissue culture or parts of a plant in accordance with the present invention or selfing of a plant in accordance with the present invention or by growing seeds of a plant in accordance with the present invention. Progeny may also encompass plants derived from crossing of at least a plant in accordance with the present invention with another *B. napus* or *Brassica* plant, backcrossing, inserting of a locus into a plant or further mutation(s). Progeny may be, e.g., a first generation plant such as a hybrid plant (F1) of a crossing of a plant according to the present invention with another plant, such as *B. napus, B. juncea* or *Brassica* plant, or a progeny is regenerated from a plant part of a plant according to the present invention or is the result of self pollination. Progeny may be, e.g., a first, second, third, fourth, fifth, or sixth or higher generation plant derived from, derivable from, obtained from or obtainable from a plant, such as a *Brassica* plant or a *B. napus* plant or a *B. juncea* plant in accordance with the present invention.

Provided hierein is an Essentially Derived Variety having at least an ALS I polypeptide comprising at a position corresponding to position 182 of SEQ ID NO: 2 instead of the naturally encoded amino acid proline the amino acid serine and at a position corresponding to position 559 of SEQ ID NO: 2 instead of the naturally encoded amino acid tryptophan the amino acid leucine, and an ALS III polypeptide comprising at a position corresponding to position 556 of SEQ ID NO: 4 instead of the naturally encoded amino acid tryptophan the amino acid leucine, or having at least an ALS I polypeptide comprising at a position corresponding to position 559 of SEQ ID NO: 2 instead of the naturally encoded amino acid tryptophan the amino acid leucine, and an ALS III polypeptide comprising comprising at a position corresponding to position 179 of SEQ ID NO: 4 instead of the naturally encoded amino acid proline the amino acid serine and at a position corresponding to position 556 of SEQ ID NO: 4 instead of the naturally encoded amino acid tryptophan the amino acid leucine, or having at least an ALS I polypeptide comprising at a position corresponding to position 182 of SEQ ID NO: 2 instead of the naturally encoded amino acid proline the amino acid serine and at a position corresponding to position 559 of SEQ ID NO: 2 instead of the naturally encoded amino acid tryptophan the amino acid leucine, and an ALS III polypeptide comprising comprising at a position corresponding to position 179 of SEQ ID NO: 4 instead of the naturally encoded amino acid proline the amino acid serine and at a position corresponding to position 556 of SEQ ID NO: 4 instead of the naturally encoded amino acid tryptophan the amino acid leucine.

An "Essentially Derived Variety" (EDV) shall be deemed to be essentially derived from another variety, "the initial variety", under the following circumstances and in the case that the Initial Variety is a plant which is derived from seeds deposited with the NCIMB, Ferguson Building, Craibstone Estate, Bucksburn, Aberdeen, AB 21 9YA UK, under the Budapest Treaty on May 20, 2013, under accession number NCIMB 42145, from seeds deposited with the NCIMB, Ferguson Building, Craibstone Estate, Bucksburn, Aberdeen, AB 21 9YA UK, under the Budapest Treaty on May 8, 2014, under accession number NCIMB 42235, from seeds deposited with the NCIMB, Ferguson Building, Craibstone Estate, Bucksburn, Aberdeen, AB 21 9YA UK, under the Budapest Treaty on July 5, 2013, under accession number NCIMB 42153, from seeds deposited with the NCIMB, Ferguson Building, Craibstone Estate, Bucksburn, Aberdeen, AB 21 9YA UK, under the Budapest Treaty on July 1, 2014, under accession number NCIMB 42259, and/or from seeds deposited with the NCIMB, Ferguson Building, Craibstone Estate, Bucksburn, Aberdeen, AB 21 9YA UK, under the Budapest Treaty on July 1, 2014, under accession number NCIMB 42260: (i) it is predominantly derived from the initial variety, or from a variety that is itself predominantly derived from the initial variety, while retaining the expression of the essential characteristics that result from the genotype or combination of genotypes of the initial variety, comprising an ALS I polypeptide comprising at a position corresponding to position 559 of SEQ ID NO: 2 instead of the naturally encoded amino acid tryptophan the amino acid leucine, and an ALS III polypeptide comprising at a position corresponding to position 179 of SEQ ID NO: 4 instead of the naturally encoded amino acid proline the amino acid serine and at a position corresponding to position 556 of SEQ ID NO: 4 instead of the naturally encoded amino acid tryptophan the amino acid leucine, or comprising an ALS I polypeptide comprising at a position corresponding to position 182 of SEQ ID NO: 2 instead of the naturally encoded amino acid proline the amino acid serine and at a position corresponding to position 559 of SEQ ID NO: 2 instead of the naturally encoded amino acid tryptophan the amino acid leucine, and an ALS III polypeptide comprising at a position corresponding to position 556 of SEQ ID NO: 4 instead of the naturally encoded amino acid tryptophan the amino acid leucine, or or comprising an ALS I polypeptide comprising at a position corresponding to position 182 of SEQ ID NO: 2 instead of the naturally encoded amino acie proline the amino acid serine and at a position corresponding to position 559 of SEQ ID NO: 2 instead of the naturally encoded amino acid tryptophan the amino acid leucine, and an ALS III polypeptide comprising at a position corresponding to position 179 of SEQ ID NO: 4 instead of the naturally encoded amino acid proline the amino acid serine and at a position corresponding to position 556 of SEQ ID NO: 4 instead of the naturally encoded amino acid tryptophan the amino acid leucine; (ii) it is clearly distinguishable from the initial variety (e.g., by its phenotype or genotype); and (iii) except for the differences which result from the act of derivation, it conforms to the initial variety in the expression of the essential characteristics that result from the genotype or combination of genotypes of the initial variety. Thus, an EDV may be obtained for example by the selection of a natural or induced mutant, or of a somaclonal variant, the selection of a variant individual from plants of the initial variety, backcrossing, or transformation by genetic engineering.

"Plant line" is for example a breeding line which can be used to develop one or more varieties. One embodiment of the present invention refers to an allotetraploid ALS inhibitor herbicide tolerant *Brassica* line comprising at least two ALS genes, wherein all endogenous ALS genes encode an ALS polypeptide comprising at a position corresponding to position 574 of SEQ ID NO: 10 instead of the naturally encoded amino acid tryptophan the amino acid leucine, and wherein at least one of the ALS genes encodes an ALS polypeptide which further comprises at a position corresponding to position 197 of SEQ ID NO: 10 instead of the naturally encoded amino acid proline the amino acid serine, such as a *B. napus* plant line comprising an ALS I polypeptide comprising at a position corresponding to position 182 of SEQ ID NO: 2 instead of the naturally encoded amino acid proline the amino acid serine and at a position corresponding to position 559 of SEQ ID NO: 2 instead of the naturally encoded amino acid tryptophan the amino acid leucine, and an ALS III polypeptide comprising at a position corresponding to position 556 of SEQ ID NO: 4 instead of the naturally encoded amino acid tryptophan the amino acid leucine, or such as a *B. napus* plant line comprising an ALS I polypeptide comprising at a position corresponding to position 559 of SEQ ID NO: 2 instead of the naturally encoded amino acid tryptophan the amino acid leucine, and an ALS III polypeptide comprising at a position corresponding to position 179 of SEQ ID NO: 4 instead of the naturally encoded amino acid proline the amino acid serine and at a position corresponding to position 556 of SEQ ID NO: 4 instead of the naturally encoded amino acid tryptophan the amino acid leucine, or such as a *B. napus* plant line comprising an ALS I polypeptide comprising at a position corresponding to position 182 of SEQ ID NO: 2 instead of the naturally encoded amino acid proline the amino acid serine and at a position corresponding to position 559 of SEQ ID NO: 2 instead of the naturally encoded amino acid tryptophan the amino acid leucine, and an ALS III polypeptide comprising at a position corresponding to position 179 of SEQ ID NO: 4 instead of the naturally encoded amino acid proline the amino acid serine and at a position corresponding to position 556 of SEQ ID NO: 4 instead of the naturally encoded amino acid tryptophan the amino acid leucine, or such as a *B. juncea* plant line comprising an ALS-A polypeptide comprising at a position corresponding to position 179 of SEQ ID NO: 6 instead of the naturally encoded amino acid proline the amino acid serine and at a position corresponding to position 556 of SEQ ID NO: 6 instead of the naturally encoded amino acid tryptophan the amino acid leucine, and an ALS-B polypeptide comprising at a position corresponding to position 559 of SEQ ID NO: 8 instead of the naturally encoded amino acid tryptophan the amino acid leucine, or such as a *B. juncea* plant line comprising an ALS-A polypeptide comprising at a position corresponding to position 556 of SEQ ID NO: 6 instead of the naturally encoded amino acid tryptophan the amino acid leucine, and an ALS-B polypeptide comprising at a position corresponding to position 182 of SEQ ID NO: 8 instead of the naturally encoded amino acid proline the amino acid serine and at a position corresponding to position 559 of SEQ ID NO: 8 instead of the naturally encoded amino acid tryptophan the amino acid leucine, or such as a *B. juncea* plant line comprising an ALS-A polypeptide comprising at a position corresponding to position 179 of SEQ ID NO: 6 instead of the naturally encoded amino acid proline the amino acid serine and at a position corresponding to position 556 of SEQ ID NO: 6 instead of the naturally encoded amino acid tryptophan the amino acid leucine, and an ALS-B polypeptide comprising at a position corresponding to position 182 of SEQ ID NO: 8 instead of the naturally encoded amino acid proline the amino acid serine and at a position corresponding to position 559 of SEQ ID NO: 8 instead of the naturally encoded amino acid tryptophan the amino acid leucine.

A "variety" is used herein in conformity with the UPOV convention and refers to a plant grouping within a single botanical taxon of the lowest known rank, which grouping can be defined by the expression of the characteristics resulting from a given genotype or combination of genotypes, can be distinguished from any other plant grouping by the expression of at least one of the said characteristics and is considered as a unit with regard to its suitability for being propagated unchanged (stable).

"Hybrid" refers to the seeds harvested from crossing one plant line or variety with another plant line or variety.

"F₁ Hybrid" refers to the first generation progeny of the cross of two genetically divergent plants. In one embodiment, such a F₁ Hybrid is homozygous in the essential feature, i.e., said F₁ Hybrid being a hybrid of a polyploid plant comprising at least two ALS genes, wherein all ALS genes encode an ALS polypeptide comprising at a position corresponding to position 574 of SEQ ID NO: 10 instead of the naturally encoded amino acid tryptophan the amino acid leucine and wherein at least one of the ALS genes encodes an ALS polypeptide which further comprises at a position corresponding to position 197 of SEQ ID NO: 10 instead of the naturally encoded amino acid proline the amino acid serine, such as an F1 *B. napus* hybrid comprising ALS I alleles encoding an ALS I polypeptide comprising at a position corresponding to position 182 of SEQ ID NO: 2 instead of the naturally encoded amino acid proline the amino acid serine and at a position corresponding to position 559 of SEQ ID NO: 2 instead of the naturally encoded amino acid tryptophan the amino acid leucine and comprising ALS III alleles encoding an ALS III polypeptide comprising at a position corresponding to position 556 of SEQ ID NO: 4 instead of the naturally encoded amino acid tryptophan the amino acid leucine, or such as an F1 *B. napus* hybrid comprising ALS I alleles encoding an ALS I polypeptide comprising at a position corresponding to position 559 of SEQ ID NO: 2 instead of the naturally encoded amino acid tryptophan the amino acid leucine and comprising ALS III alleles encoding an ALS III polypeptide comprising at a position corresponding to position 179 of SEQ ID NO: 4 instead of the naturally encoded amino acid proline the amino acid serine and at a position corresponding to position 556 of SEQ ID NO: 4 instead of the naturally encoded amino acid tryptophan the amino acid leucine, or such as an F1 *B. napus* hybrid comprising ALS I alleles encoding an ALS I polypeptide comprising at a position corresponding to position 182 of SEQ ID NO: 2 instead of the naturally encoded amino acid proline the amino acid serine and at a position corresponding to position 559 of SEQ ID NO: 2 instead of the naturally encoded amino acid tryptophan the amino acid leucine and comprising ALS III alleles encoding an ALS III polypeptide comprising at a position corresponding to position 179 of SEQ ID NO: 4 instead of the naturally encoded amino acid proline the amino acid serine and at a position corresponding to position 556 of SEQ ID NO: 4 instead of the naturally encoded amino acid tryptophan the amino acid leucine, or such as an F1 *B. juncea* hybrid comprising ALS-A alleles encoding an ALS-A polypeptide comprising at a position corresponding to position 179 of SEQ ID NO: 6 instead of the naturally encoded amino acid proline the amino acid serine and at a position corresponding to position 556 of SEQ ID NO: 6 instead of the naturally encoded amino acid tryptophan the amino acid leucine and comprising ALS-B alleles encoding an ALS-B polypeptide comprising at a position corresponding to position 559 of SEQ ID NO: 8 instead of the naturally encoded amino acid tryptophan the amino acid leucine, or such as an F1 *B*. *juncea* hybrid comprising ALS-A alleles encoding an ALS-A polypeptide comprising at a position corresponding to position 556 of SEQ ID NO: 6 instead of the naturally encoded amino acid tryptophan the amino acid leucine and comprising ALS-B alleles encoding an ALS-B polypeptide comprising at a position corresponding to position 182 of SEQ ID NO: 8 instead of the naturally encoded amino acid proline the amino acid serine and at a position corresponding to position 559 of SEQ ID NO: 8 instead of the naturally encoded amino acid tryptophan the amino acid leucine, or such as an F1 *B. juncea* hybrid comprising ALS-A alleles encoding an ALS-A polypeptide comprising at a position corresponding to position 179 of SEQ ID NO: 6 instead of the naturally encoded amino acid proline the amino acid serine and at a position corresponding to position 556 of SEQ ID NO: 6 instead of the naturally encoded amino acid tryptophan the amino acid leucine and comprising ALS-B alleles encoding an ALS-B polypeptide comprising at a position corresponding to position 182 of SEQ ID NO: 8 instead of the naturally encoded amino acid proline the amino acid serine and at a position corresponding to position 559 of SEQ ID NO: 8 instead of the naturally encoded amino acid tryptophan the amino acid leucine.

"Crossing" refers to the mating of two parent plants.

"Backcrossing" refers to a process in which a breeder repeatedly crosses hybrid progeny, for example a first generation hybrid (F₁), back to one of the parents of the hybrid progeny. Backcrossing can be used to introduce one or more single locus conversions from one genetic background into another.

"Cross-pollination" refers to fertilization by the union of two gametes from different plants.

"Regeneration" refers to the development of a plant from tissue culture.

"Selfing" refers to self-pollination of a plant, i.e., the transfer of pollen from the anther to the stigma of the same plant.

Single Locus Converted (Conversion) Plant: Plants which are developed by a plant breeding technique called backcrossing, wherein essentially all of the desired morphological and physiological characteristics of a oilseed rape variety are recovered in addition to the characteristics of the single locus transferred into the variety via the backcrossing technique and/or by genetic transformation.

Plants of the present invention can be identified using any genotypic analysis method. Genotypic evaluation of the plants includes using techniques such as Isozyme Electrophoresis, Restriction Fragment Length Polymorphisms (RFLPs), Randomly Amplified Polymorphic DNAs (RAPDs), Arbitrarily Primed Polymerase Chain Reaction (AP-PCR), Allele-specific PCR (AS-PCR), DNA Amplification Fingerprinting (DAF), Sequence Characterized Amplified Regions (SCARs), Amplified Fragment Length Polymorphisms (AFLPs), Simple Sequence Repeats (SSRs) which are also referred to as "Microsatellites". Additional compositions and methods for analyzing the genotype of the plants provided herein include those methods disclosed in U.S. Publication No. 2004/0171027, U.S. Publication No. 2005/02080506, and U.S. Publication No. 2005/0283858.

### Sequences/Position

The term "sequence" when used herein relates to nucleotide sequence(s), polynucleotide(s), nucleic acid sequence(s), nucleic acid(s), nucleic acid molecule, peptides, polypeptides and proteins, depending on the context in which the term "sequence" is used.

Generally, the skilled person knows, because of his common general knowledge and the context when the terms ALS, ALSL, AHAS or AHASL are used herein as to whether the nucleotide sequence or nucleic acid, or the amino acid sequence or polypeptide, respectively, is meant. The terms acetohydroxyacid synthase, AHAS, acetolactate synthase and ALS are used as interchangeably throughout this text.

The term *B. napus* "ALS" or "AHAS" gene refers to *B. napus* nucleotide sequences which are at least 60, 70, 80, 90, 95, 97, 98, 99% or 100% identical to the *B. napus* ALS nucleotide sequence of SEQ ID NO: 1 or 3.

The term "ALS I" or "AHAS I" gene refers to a *B*. *napus* ALS gene present on the C genome, wherein the sequence of said gene is at least 60, 70, 80, 90, 95, 97, 98, 99% or 100% identical to the nucleotide sequence of SEQ ID NO: 1.

The term "ALS III" or "ALS III" gene refers to a *B*. *napus* ALS gene present on the A genome, wherein the sequence of said gene is at least 60, 70, 80, 90, 95, 97, 98, 99% or 100% identical to the nucleotide sequence of SEQ ID NO: 3.

The term *B. napus* "ALS" or "AHAS" polypeptide refers to amino acid sequences which are at least 90, 95, 97, 98, 99% or 100% identical to the ALS amino acid sequence of SEQ ID NO: 2 or 4. Said X% identical amino acid sequences retain the activity of ALS as described herein, more preferably the ALS polypeptide is tolerant to ALS inhibitor herbicides as described herein. However, such "ALS" or "AHAS" polypeptides still show ALS enzymatic activity at a level of at least 20%, 30%, 40%, 50%, 60%, 70%, 80%, 90% compared to the level of the ALS enzymatic activity of an protein having the SEQ ID NO: 2 (when referring to an ALS I protein)or 4 (when referring to an ALS III protein).

The term "ALS I" or "AHAS I" protein refers to the protein encoded by the ALS I gene, wherein said ALS I protein contains at least 90, 95, 97, 98, 99 or 100% sequence identity to the ALS amino acid sequence of SEQ ID NO: 2.

The term "ALS III" or "AHAS III" protein refers to the protein encoded by the ALS III gene, wherein said ALS III protein contains at least 90, 95, 97, 98, 99% or 100% sequence identity to the ALS amino acid sequence of SEQ ID NO: 4.

The term *B. juncea* "ALS" or "AHAS" gene refers to *B. juncea* nucleotide sequences which are at least 60, 70, 80, 90, 95, 97, 98, 99% or 100% identical to the *B. juncea* ALS nucleotide sequence of SEQ ID NO: 5 or 7.

The term "ALS-A" or "AHAS-A" gene refers to a *B. juncea* ALS gene present on the A genome, wherein the sequence of said gene is at least 60, 70, 80, 90, 95, 97, 98, 99% or 100% identical to the nucleotide sequence of SEQ ID NO: 3.

The term "ALS-B" or "ALS-B" gene refers to a *B. juncea* ALS gene present on the B genome, wherein the sequence of said gene is at least 60, 70, 80, 90, 95, 97, 98, 99% or 100% identical to the nucleotide sequence of SEQ ID NO: 5.

The term *B. juncea* "ALS" or "AHAS" polypeptide refers to amino acid sequences which are at least 90, 95, 97, 98, 99% or 100% identical to the ALS amino acid sequence of SEQ ID NO: 6 or 8. Said X% identical amino acid sequences retain the activity of ALS as described herein, more preferably the ALS polypeptide is tolerant to ALS inhibitor herbicides as described herein. However, such "ALS" or "AHAS" polypeptides still show ALS enzymatic activity at a level of at least 20%, 30%, 40%, 50%, 60%, 70%, 80%, 90% compared to the level of the ALS enzymatic activity of an protein having the SEQ ID NO: 6 (when referring to an ALS-A protein) or 8 (when referring to an ALS-B protein).

The term "ALS-A" or "AHAS-A" protein refers to the protein encoded by the ALS-A gene, wherein said ALS-A protein contains at least 90, 95, 97, 98, 99 or 100% sequence identity to the ALS amino acid sequence of SEQ ID NO: 6.

The term "ALS-B" or "AHAS-B" protein refers to the protein encoded by the ALS-B gene, wherein said ALS-B protein contains at least 90, 95, 97, 98, 99% or 100% sequence identity to the ALS amino acid sequence of SEQ ID NO: 8.

It is well known to the skilled person that the genomes of three allotetraploid, or amphidiploid *Brassica* species *B. napus, B. juncea* and *B. carinata* are derived from three ancestral genomes, denoted by the letters AA (derived from *B. rapa);* BB (derived from *B. nigra),* and CC (derived from *B. oleracea). B. n*apus contains an A genome and a C genome; *B. juncea* contains an A genome and a B genome, and *B. carinata* contains a B genome and a C genome. The ALS gene on a given genome is therefore essentially similar when present in different species. Thus, the ALS gene from the A genome (ALS III from *B. napus* or ALS-A from *B. juncea)* is therefore essentially similar in *B. napus, B. juncea* and *B. rapa.* The ALS gene from the B genome (ALS-B from *B. juncea)* is essentially similar in *B. juncea, B. carinata,* and *B. nigra.* The ALS gene from the C genome (ALS I from *B. napus)* is essentially similar in *B. napus, B. carinata* and *B. oleracea.* Also provided are therefore *B. napus* plants comprising ALS genes essentially similar to ALS-A and to ALS I, *B. juncea* plants comprising ALS genes essentially similar to ALS III and ALS-B, and *B. carinata* plants comprising ALS genes essentially similar to ALS-B and ALS I.

Essentially similar as used herein refers to having at least 90, 95, 97, 98, 99% or 100% sequence identity to the sequence referred to.

The term "position" when used in accordance with the present invention means the position of either an amino acid within an amino acid sequence depicted herein or the position of a nucleotide within a nucleotide sequence depicted herein. The term "corresponding" as used herein also includes that a position is not only determined by the number of the preceding nucleotides/amino acids.

The position of a given nucleotide in accordance with the present invention which may be substituted may vary due to deletions or additional nucleotides elsewhere in the ALS 5'-untranslated region (UTR) including the promoter and/or any other regulatory sequences or gene (including exons and introns). Similarly, the position of a given amino acid in accordance with the present invention which may be substituted may vary due to deletion or addition of amino acids elsewhere in the ALS polypeptide.

Thus, under a "corresponding position" or "a position corresponding to position" in accordance with the present invention it is to be understood that nucleotides/amino acids may differ in the indicated number but may still have similar neighbouring nucleotides/amino acids. Said nucleotides/amino acids which may be exchanged, deleted or added are also comprised by the term "corresponding position".

In order to determine whether a nucleotide residue or amino acid residue in a given ALS nucleotide/amino acid sequence corresponds to a certain position in the nucleotide sequence of SEQ ID NO: 1, 3, 5, 7 or 9, respectively, or their corresponding amino acid sequences of SEQ ID NO: 2, 4, 6, 8 or 10, respectively, the skilled person can use means and methods well-known in the art, e.g., alignments, either manually or by using computer programs such as BLAST (Altschul et al. (1990), Journal of Molecular Biology, 215, 403-410), which stands for Basic Local Alignment Search Tool or ClustalW (Thompson et al. (1994), Nucleic Acid Res., 22, 4673-4680) or any other suitable program which is suitable to generate sequence alignments.

SEQ ID NO: 1 is the nucleotide sequence encoding a *B*. *napus* wild type ALS I, whereas SEQ ID NO: 2 is the *B. napus* amino acid sequence derived from SEQ ID NO: 1. Accordingly, the codon at position 544-546 of the nucleotide sequence of SEQ ID NO: 1 encodes the amino acid at position 182 of SEQ ID NO: 2 (this position, again, corresponds to position 197 of SEQ ID NO: 10), whereas the codon at position 1675-1677 of the nucleotide sequence of SEQ ID NO: 1 encodes the amino acid at position 559 of SEQ ID NO: 2 (this position, again, corresponds to position 574 of SEQ ID NO: 10). In other words, the amino acid proline ("Pro" (three letter code) or "P" (one letter code)) of SEQ ID NO: 2 is encoded by the codon at positions 544-546 of the nucleotide sequence of SEQ ID NO: 1, and the amino acid tryptophan ("Trp" (three letter code) or "W" (one letter code)) of SEQ ID NO: 2 is encoded by the codon at positions 1675-1677 of the nucleotide sequence of SEQ ID NO: 1.

SEQ ID NO: 3 is the nucleotide sequence encoding a *B*. *napus* wild type ALS III, whereas SEQ ID NO: 4 is the *B. napus* amino acid sequence derived from SEQ ID NO: 3. Accordingly, the codon at position 535-537 of the nucleotide sequence of SEQ ID NO: 3 encodes the amino acid at position 179 of SEQ ID NO: 4 (this position, again, corresponds to position 197 of SEQ ID NO: 10), whereas the codon at position 1666-1668 of the nucleotide sequence of SEQ ID NO: 3 encodes the amino acid at position 556 of SEQ ID NO: 4 (this position, again, corresponds to position 574 of SEQ ID NO: 10). In other words, the amino acid proline ("Pro" (three letter code) or "P" (one letter code)) of SEQ ID NO: 4 is encoded by the codon at positions 535-537 of the nucleotide sequence of SEQ ID NO: 3, and the amino acid tryptophan ("Trp" (three letter code) or "W" (one letter code)) of SEQ ID NO: 4 is encoded by the codon at positions 1666-1668 of the nucleotide sequence of SEQ ID NO: 3.

SEQ ID NO: 5 is the nucleotide sequence encoding a *B. juncea* wild type ALS-A, whereas SEQ ID NO: 6 is the *B. juncea* amino acid sequence derived from SEQ ID NO: 5. Accordingly, the codon at position 535-537 of the nucleotide sequence of SEQ ID NO: 5 encodes the amino acid at position 179 of SEQ ID NO: 6 (this position, again, corresponds to position 197 of SEQ ID NO: 10), whereas the codon at position 1666-1668 of the nucleotide sequence of SEQ ID NO: 5 encodes the amino acid at position 556 of SEQ ID NO: 6 (this position, again, corresponds to position 574 of SEQ ID NO: 10). In other words, the amino acid proline ("Pro" (three letter code) or "P" (one letter code)) of SEQ ID NO: 6 is encoded by the codon at positions 535-537 of the nucleotide sequence of SEQ ID NO: 5, and the amino acid tryptophan ("Trp" (three letter code) or "W" (one letter code)) of SEQ ID NO: 6 is encoded by the codon at positions 1666-1668 of the nucleotide sequence of SEQ ID NO: 5.

SEQ ID NO: 7 is the nucleotide sequence encoding a *B. juncea* wild type ALS-B, whereas SEQ ID NO: 8 is the *B. juncea* amino acid sequence derived from SEQ ID NO: 7. Accordingly, the codon at position 544-546 of the nucleotide sequence of SEQ ID NO: 7 encodes the amino acid at position 182 of SEQ ID NO: 8 (this position, again, corresponds to position 197 of SEQ ID NO: 10), whereas the codon at position 1675-1677 of the nucleotide sequence of SEQ ID NO: 7 encodes the amino acid at position 559 of SEQ ID NO: 8 (this position, again, corresponds to position 574 of SEQ ID NO: 10). In other words, the amino acid proline ("Pro" (three letter code) or "P" (one letter code)) of SEQ ID NO: 8 is encoded by the codon at positions 544-546 of the nucleotide sequence of SEQ ID NO: 7, the amino acid tryptophan ("Trp" (three letter code) or "W" (one letter code)) of SEQ ID NO: 8 is encoded by the codon at positions 1675-1677 of the nucleotide sequence of SEQ ID NO: 7.

In the alternative to determine whether a nucleotide residue or amino acid residue in a given ALS nucleotide/amino acid sequence corresponds to a certain position in the nucleotide sequence of SEQ ID NO: 1, 3, 5, or 7, respectively, the nucleotide sequence encoding *A. thaliana* wild type ALS shown in SEQ ID NO: 9 can be used. SEQ ID NO: 10 is the *A. thaliana* amino acid sequence derived from SEQ ID NO: 9.

The codon at position 589-591 of the nucleotide sequence of SEQ ID NO: 9 encodes the amino acid at position 197 of SEQ ID NO: 10, whereby position 197 of SEQ ID NO: 10 corresponds to position 182 of SEQ ID NOs: 2 and 8 and corresponds to position 179 of SEQ ID NOs: 4 and 6, and the codon at position 1720-1722 of the nucleotide sequence of SEQ ID NO: 9 encodes the amino acid at position 574 of SEQ ID NO: 10, whereby position 574 of SEQ ID NO: 10 corresponds to position 559 of SEQ ID NOs: 2 and 8 and corresponds to position 556 of SEQ ID NOs: 4 and 6.

If the *A. thaliana* wild type ALS nucleotide sequence shown in SEQ ID NO: 9 is used as reference sequence (as it is done in most of the relevant literature and, therefore, is used to enable an easier comparison to such known sequences), the codon encoding a serine instead of a proline at position 182 of SEQ ID NO: 2 and SEQ ID NO: 8 is at a position 544-546 of SEQ ID NO: 1 and SEQ ID NO: 7, respectively, which corresponds to position 589-591 of SEQ ID NO: 9, the codon encoding a serine instead of a proline at a position 179 of SEQ ID NO: 4 and SEQ ID NO: 6 is at a position 535-537 of SEQ ID NO: 3 and SEQ ID NO: 5, respectively, which corresponds to position 589-591 of SEQ ID NO: 9. If the *A. thaliana* wild type ALS nucleotide sequence shown in SEQ ID NO: 9 is used as reference sequence, the codon encoding a leucine instead of a tryptophan at position 559 of SEQ ID NO: 2 and SEQ ID NO: 8 is at a position 1675-1677 of SEQ ID NO: 1 and SEQ ID NO: 7, respectively, which corresponds to position 1720-1722 of SEQ ID NO: 9, the codon encoding a leucine instead of a tryptophan at a position 556 of SEQ ID NO: 4 and SEQ ID NO: 6 is at a position 1666-1668 of SEQ ID NO: 3 and SEQ ID NO: 5, respectively, which corresponds to position 1720-1722 of SEQ ID NO: 9.

However, SEQ ID NO: 1 is preferred as the reference nucleotide sequence for mutated ALS I protein encoding sequences, and SEQ ID NO: 2 is preferred as the reference amino acid sequence for mutated sequences in all of the subsequent disclosures.

Similarily, SEQ ID NO: 3 is preferred as the reference nucleotide sequence for mutated ALS III protein encoding sequences and SEQ ID NO: 4 is preferred as the reference amino acid sequence for mutated sequences in all of the subsequent disclosures.

Similarly, SEQ ID NO: 5 is preferred as the reference nucleotide sequence for mutated ALS-A protein encoding sequences, and SEQ ID NO: 6 is preferred as the reference amino acid sequence for mutated sequences in all of the subsequent disclosures.

Similarily, SEQ ID NO: 7 is preferred as the reference nucleotide sequence for mutated ALS-B protein encoding sequences and SEQ ID NO: 8 is preferred as the reference amino acid sequence for mutated sequences in all of the subsequent disclosures.

Thus, in any event, the equivalent position can still be determined through alignment with a reference sequence, such as SEQ ID NO: 1, 3, 5 or 7 (nucleotide sequence) or SEQ ID NO: 2, 4, 6 or 8 (amino acid sequence). Alignments of the various sequences listed above are given in figures 1 and 2.

In view of the difference between the wild-type ALS genes (such as the *B. napus* ALS I and III gene, and the *B. juncea* ALS-A and ALS-B gene) and the mutated ALS genes comprised by a plant of the present invention or progeny thereof, the ALS genes (or polynucleotides or nucleotide sequences) comprised by a plant of the present invention or progeny thereof may also be regarded as a "mutant ALS gene", "mutant ALS allele", "mutant ALS polynucleotide" or the like. Thus, throughout the specification, the terms "mutant allele", "mutant ALS allele", "mutant ALS gene" or "mutant ALS polynucleotide" are used interchangeably.

Unless indicated otherwise herein, these terms refer to a nucleotide sequence encoding an ALS protein that comprises a codon at a position which corresponds to position 1720-1722 of the Arabidopsis ALS gene of SEQ ID NO: 9, and said codon encodes a leucine instead of a tryptophan and, optionally, that further comprises a codon at a position which corresponds to position 589-591 of the Arabidopsis ALS gene of SEQ ID NO: 9, and said codon encodes a serine instead of a proline, such as a nucleotide sequence encoding an ALS I protein that comprises a codon at a position which corresponds to position 1675-1677 of SEQ ID NO: 1 and said codon encodes a leucine instead of a tryptophan and, optionally, further comprises comprises a codon at a position which corresponds to position 544-546 of SEQ ID NO: 1 and said codon encodes a serine instead of a proline; to a nucleotide sequence encoding for an ALS III protein that comprises a codon at a position which corresponds to position 1666-1668 of SEQ ID NO: 3 and said codon of said second nucleotide sequence encodes a leucine instead of a tryptophan and, optionally, further comprises comprises a codon at a position which corresponds to position 535-537 of SEQ ID NO: 3 and said codon encodes a serine instead of a proline; to a nucleotide sequence encoding for an ALS-A protein that comprises a codon at a position which corresponds to position 1666-1668 of SEQ ID NO: 5 and said codon of said second nucleotide sequence encodes a leucine instead of a tryptophan and, optionally, further comprises comprises a codon at a position which corresponds to position 535-537 of SEQ ID NO: 5 and said codon encodes a serine instead of a proline; and to a nucleotide sequence encoding an ALS-B protein that comprises a codon at a position which corresponds to position 1675-1677 of SEQ ID NO: 7 and said codon encodes a leucine instead of a tryptophan and, optionally, further comprises comprises a codon at a position which corresponds to position 544-546 of SEQ ID NO: 7 and said codon encodes a serine instead of a proline.

The term "P197S mutation" refers to a mutation in the codon corresponding to nt 589-591 in *A*. *thaliana* (SEQ ID NO 9) leading to a substitution of the amino acid proline by a serine.

The term "P197S mutation" in ALS I refers to a mutation in the codon corresponding to nt 589-591 in *A. thaliana* (SEQ ID NO 9) or in the codon corresponding to nt 544-546 *of B. napus* ALS I (SEQ ID NO: 1) leading to a substitution of the amino acid proline by a serine.

The term "P197S mutation" in ALS III refers to a mutation in the codon corresponding to nt 589-591 in *A. thaliana* (SEQ ID NO 9) or in the codon corresponding to nt 535-537 *of B. napus* ALS III (SEQ ID NO: 3) leading to a substitution of the amino acid proline by a serine.

The term "P197S mutation" in ALS-A refers to a mutation in the codon corresponding to nt 589-591 in *A. thaliana* (SEQ ID NO 9) or in the codon corresponding to nt 535-537 of *B. juncea* ALS-A (SEQ ID NO: 5) leading to a substitution of the amino acid proline by a serine.

The term "P197S mutation" in ALS-B refers to a mutation in the codon corresponding to nt 589-591 in *A. thaliana* (SEQ ID NO 9) or in the codon corresponding to nt 544-546 of *B. juncea* ALS-A (SEQ ID NO: 7) leading to a substitution of the amino acid proline by a serine.

The term "W574L mutation" refers to a mutation in the codon corresponding to nt 1720-1722 in *A. thaliana* (SEQ ID NO 9) leading to a substitution of the amino acid tryptophan by a leucine.

The term "W574L mutation" in ALS I refers to a mutation in the codon corresponding to nt 1720-1722 in *A. thaliana* (SEQ ID NO 9) or in the codon corresponding to nt 1675-1677 *of B. napus* ALS I (SEQ ID NO: 1) leading to a substitution of the amino acid tryptophan by a leucine.

The term "W574L mutation" in ALS III refers to a mutation in the codon corresponding to nt 1720-1722 in *A. thaliana* (SEQ ID NO 9) or in the codon corresponding to nt 1666-1668 *of B. napus* ALS III (SEQ ID NO: 3) leading to a substitution of the amino acid tryptophan by a leucine.

The term "W574L mutation" in ALS-A refers to a mutation in the codon corresponding to nt 1720-1722 in *A. thaliana* (SEQ ID NO 9) or in the codon corresponding to nt 1666-1668 of *B. juncea* ALS-A (SEQ ID NO: 5) leading to a substitution of the amino acid tryptophan by a leucine.

The term "W574L mutation" in ALS-B refers to a mutation in the codon corresponding to nt 1720-1722 in *A. thaliana* (SEQ ID NO 9) or in the codon corresponding to nt 1675-1677 of *B. juncea* ALS-A (SEQ ID NO: 7) leading to a substitution of the amino acid tryptophan by a leucine.

The terms "nucleotide sequence(s)", "polynucleotide(s)", "nucleic acid sequence(s)", "nucleic acid(s)", "nucleic acid molecule" are used interchangeably herein and refer to nucleotides, either ribonucleotides or deoxyribonucleotides or a combination of both, in a polymeric unbranched form of any length. Nucleic acid sequences include DNA, cDNA, genomic DNA, RNA, synthetic forms and mixed polymers, both sense and antisense strands, or may contain non-natural or derivatized nucleotide bases, as will be readily appreciated by those skilled in the art.

### Homology/identity

In order to determine whether a nucleic acid sequence has a certain degree of identity to the nucleotide sequences of the present invention, the skilled person can use means and methods well-known in the art, e.g., alignments, either manually or by using computer programs such as those mentioned further down below in connection with the definition of the term "hybridization" and degrees of homology.

For the purpose of this invention, the "sequence identity" or "sequence homology" (the terms are used interchangeably herein) of two related nucleotide or amino acid sequences, expressed as a percentage, refers to the number of positions in the two optimally aligned sequences which have identical residues (x100) divided by the number of positions compared. A gap, i.e., a position in an alignment where a residue is present in one sequence but not in the other, is regarded as a position with non-identical residues. The "optimal alignment" of two sequences is found by aligning the two sequences over the entire length according to the Needleman and Wunsch global alignment algorithm (Needleman and Wunsch, 1970, J Mol Biol 48(3):443-53) in The European Molecular Biology Open Software Suite (EMBOSS, Rice et al., 2000, Trends in Genetics 16(6): 276-277; see e.g. http://www.ebi.ac.uk/emboss/align/index.html) using default settings (gap opening penalty = 10 (for nucleotides) / 10 (for proteins) and gap extension penalty = 0.5 (for nucleotides) / 0.5 (for proteins)). For nucleotides the default scoring matrix used is EDNAFULL and for proteins the default scoring matrix is EBLOSUM62.

The term "ALS" or "AHAS" gene also includes nucleotide sequences which are at least 60, 70, 80, 90, 95, 97, 98, 99% or 100% identical to the ALS nucleotide sequences as described herein, wherein these 60, 70, 80, 90, 95, 97, 98, 99, or 100% identical nucleotide sequences comprise at a position corresponding to position 1720-1722 of the nucleotide sequence of SEQ ID NO: 9 a codon encoding Leu instead of Trp (at a position corresponding to position 574 of SEQ ID NO: 10) and, optionally, comprise at a position corresponding to position 589-591 of the nucleotide sequence of SEQ ID NO: 9 a codon encoding Ser instead of Pro (at a position corresponding to position 197 of SEQ ID NO: 10).

The term *B. napus* "ALS" or "AHAS" gene also includes *B. napus* nucleotide sequences which are at least 60, 70, 80, 90, 95, 97, 98, 99% or 100% identical to the *B. napus* ALS nucleotide sequence of SEQ ID NO: 1 or 3, wherein these 60, 70, 80, 90, 95, 97, 98, 99, or 100% identical nucleotide sequences comprise at a position corresponding to position 1675-1677 of the nucleotide sequence of SEQ ID NO: 1 a codon encoding Leu instead of Trp (at position 559 of SEQ ID NO: 2) or at a position corresponding to position 1666-1668 of the nucleotide sequence of SEQ ID NO: 3 a codon encoding Leu instead of Trp (at position 556 of SEQ ID NO: 4) and, optionally, comprise at a position corresponding to position 544-546 of the nucleotide sequence of SEQ ID NO: 1 a codon encoding Ser instead of Pro (at position 182 of SEQ ID NO: 2) or at a position corresponding to position 535-537 of the nucleotide sequence of SEQ ID NO: 3 a codon encoding Ser instead of Pro (at position 179 of SEQ ID NO: 4).

The term *B. juncea* "ALS" or "AHAS" gene also includes *B. juncea* nucleotide sequences which are at least 60, 70, 80, 90, 95, 97, 98, 99% or 100% identical to the *B. juncea* ALS nucleotide sequence of SEQ ID NO: 5 or 7, wherein these 60, 70, 80, 90, 95, 97, 98, 99, or 100% identical nucleotide sequences comprise at a position corresponding to position 1666-1668 of the nucleotide sequence of SEQ ID NO: 5 a codon encoding Leu instead of Trp (at position 556 of SEQ ID NO: 6) or at a position corresponding to position 1675-1677 of the nucleotide sequence of SEQ ID NO: 7 a codon encoding Leu instead of Trp (at position 559 of SEQ ID NO: 8) and, optionally, comprise at a position corresponding to position 535-537 of the nucleotide sequence of SEQ ID NO: 5 a codon encoding Ser instead of Pro (at position 179 of SEQ ID NO: 6) or at a position corresponding to position 544-546 of the nucleotide sequence of SEQ ID NO: 7 a codon encoding Ser instead of Pro (at position 182 of SEQ ID NO: 8).

Likewise, these at least 60, 70, 80, 90, 95, 97, 98, 99, or 100% identical nucleotide sequences include sequences encoding an ALS polypeptide comprising at a position corresponding to position 197 of SEQ ID NO: 10 Ser instead of Pro and at a position corresponding to position 574 of SEQ ID NO: 10 Leu instead of Trp, or at a position corresponding to position 182 of SEQ ID NO: 2 or of SEQ ID NO: 8 Ser instead of Pro and at a position corresponding to position 559 of SEQ ID NO: 2 or of SEQ ID NO: 8 Leu instead of Trp, or at a position corresponding to position 179 of SEQ ID NO: 4 or of SEQ ID NO: 6 Ser instead of Pro and at a position corresponding to position 556 of SEQ ID NO: 4 or of SEQ ID NO: 6 Leu instead of Trp, and include sequences encoding an ALS polypeptide comprising at a position corresponding to position 574 of SEQ ID NO: 10 Leu instead of Trp, or at a position corresponding to position 559 of SEQ ID NO: 2 or of SEQ ID NO: 8 Leu instead of Trp, or at a position corresponding to position 556 of SEQ ID NO: 4 or of SEQ ID NO: 6 Leu instead of Trp. Of course, these nucleotide sequences encode for ALS proteins which retain the activity as described herein, more preferably the thus-encoded ALS polypeptide is tolerant to one or more ALS inhibitor herbicides as described herein. Said term also includes allelic variants and homologs encoding an ALS polypeptide which is preferably tolerant to one or more ALS inhibitor herbicides as described herein.

When used herein, the term "polypeptide" or "protein" (both terms are used interchangeably herein) means a peptide, a protein, or a polypeptide which encompasses amino acid chains of a given length, wherein the amino acid residues are linked by covalent peptide bonds. However, peptidomimetics of such proteins/polypeptides wherein amino acid(s) and/or peptide bond(s) have been replaced by functional analogs are also encompassed by the invention as well as other than the 20 gene-encoded amino acids, such as selenocysteine. Peptides, oligopeptides and proteins may be termed polypeptides. The term polypeptide also refers to, and does not exclude, modifications of the polypeptide, e.g., glycosylation, acetylation, phosphorylation and the like. Such modifications are well described in basic texts and in more detailed monographs, as well as in the research literature. The polypeptide (or protein) that are preferably meant herein have an amino acid sequence that comprises the mutated ALS polypeptides, such as *B. napus* ALS I and III polypeptides (or ALS I and III proteins) of SEQ ID NO: 2 and 4, of which the tryptophan at a position corresponding to position 574 of SEQ ID NO: 10 is substituted with a leucine, and, optionally, of which the proline at a position corresponding to position 197 of SEQ ID NO: 10 is substituted with a serine, respectively, and such as the *B. juncea* ALS-A and -B polypeptides of SEQ ID NO: 6 and 8, of which the tryptophan at a position corresponding to position 574 of SEQ ID NO: 10 is substituted with a leucine, and, optionally, of which the proline at a position corresponding to position 197 of SEQ ID NO: 10 is substituted with a serine, respectively.

The term "ALS" or "AHAS" polypeptide also includes amino acid sequences which comprise an amino acid sequences which is at least 90, 95, 97, 98, 99% or 100% identical to the ALS amino acid sequences as described herein, wherein these at least 90, 95, 97, 98, 99 or 100% identical amino acid sequences comprising at a position corresponding to position 574 of SEQ ID NO: 10 a leucine instead of a tryptophan, or wherein these at least 90, 95, 97, 98, 99 or 100% identical amino acid sequences comprising at a position corresponding to position 197 of SEQ ID NO: 10 a serine instead of a proline and at a position corresponding to position 574 of SEQ ID NO: 10 a leucine instead of a tryptophan. Said X% identical amino acid sequences retain the activity of ALS as described herein, more preferably the ALS polypeptide is tolerant to ALS inhibitor herbicides as described herein. However, such "ALS" or "AHAS" polypeptides still show ALS activity of at least 20%, 30%, 40%, 50%, 60%, 70%, 80%, 90% compared to ALS activity of a protein having the SEQ ID NO: 10.

The term *B. napus* "ALS" or "AHAS" polypeptide also includes amino acid sequences which comprise an amino acid sequences which is at least 90, 95, 97, 98, 99% or 100% identical to the ALS amino acid sequence of SEQ ID NO: 2 or 4, wherein these at least 90, 95, 97, 98, 99 or 100% identical amino acid sequences comprising at a position corresponding to position 559 of SEQ ID NO: 2 a leucine instead of a tryptophan, and at a position corresponding to position 556 of SEQ ID NO: 4 a leucine instead of a tryptophan, or wherein these at least 90, 95, 97, 98, 99 or 100% identical amino acid sequences comprising at a position corresponding to position 182 of SEQ ID NO: 2 a serine instead of a proline and at a position corresponding to position 559 of SEQ ID NO: 2 a leucine instead of a tryptophan, and at a position corresponding to position 179 of SEQ ID NO: 4 a serine instead of a proline and at a position corresponding to position 556 of SEQ ID NO: 4 a leucine instead of a tryptophan . Said X% identical amino acid sequences retain the activity of ALS as described herein, more preferably the ALS polypeptide is tolerant to ALS inhibitor herbicides as described herein. However, such "ALS" or "AHAS" polypeptides still show ALS activity of at least 20%, 30%, 40%, 50%, 60%, 70%, 80%, 90% compared to ALS activity of an protein having the SEQ ID NO: 2 (when referring to an ALS I protein)or 4 (when referring to an ALS III protein).

The term *B. juncea* "ALS" or "AHAS" polypeptide also includes amino acid sequences which comprise an amino acid sequences which is at least 90, 95, 97, 98, 99% or 100% identical to the ALS amino acid sequence of SEQ ID NO: 6 or 8, wherein these at least 90, 95, 97, 98, 99 or 100% identical amino acid sequences comprising at a position corresponding to position 556 of SEQ ID NO: 6 a leucine instead of a tryptophan, and at a position corresponding to position 559 of SEQ ID NO: 8 a leucine instead of a tryptophan, or wherein these at least 90, 95, 97, 98, 99 or 100% identical amino acid sequences comprising at a position corresponding to position 179 of SEQ ID NO: 6 a serine instead of a proline and at a position corresponding to position 556 of SEQ ID NO: 6 a leucine instead of a tryptophan, and at a position corresponding to position 182 of SEQ ID NO: 8 a serine instead of a proline and at a position corresponding to position 559 of SEQ ID NO: 8 a leucine instead of a tryptophan. Said X% identical amino acid sequences retain the activity of ALS as described herein, more preferably the ALS polypeptide is tolerant to ALS inhibitor herbicides as described herein. However, such "ALS" or "AHAS" polypeptides still show ALS activity of at least 20%, 30%, 40%, 50%, 60%, 70%, 80%, 90% compared to ALS activity of an protein having the SEQ ID NO: 6 (when referring to an ALS-A protein)or 8 (when referring to an ALS-B protein).

The same techniques, e.g., BLAST, as described above for the alignment of nucleic acid sequences can be used for alignments of protein sequences as well. For Example, a BLAST search can be perdormed from those skilled in the art using ExPASy (see world wide net: http://expasy.org/tools/).

Sequences encoding AHAS polypeptides from other plant species, in particular from polyploid plant species such as wheat, cotton (*Gossypium hirsutum*), potato, alfalfa, sugar cane, soybeans, leek, tobacco, peanut, kinnow, pelargonium, chrysanthemum, triticale, oat, kiwifruit, strawberry, dahlia, pansies, oca, tulips, lilies, daylilies, apple, banana, citrus, coffee and watermelon, can be identified based on the available sequences as described herein. For example, AHAS sequences from these species can be identified by alignments with sequences from sequence databases, such as by using computer programs such as BLAST (Altschul et al. (1990), Journal of Molecular Biology, 215, 403-410), which stands for Basic Local Alignment Search Tool or ClustalW (Thompson et al. (1994), Nucleic Acid Res., 22, 4673-4680) or any other suitable program which is suitable to generate sequence alignments. Further, the skilled person can identify sequences encoding AHAS polypeptides form other plant species using hybridization using as probes the AHAS nucleotide sequences of parts thereof.

"High stringency conditions" can be provided, for example, by hybridization at 65°C in an aqueous solution containing 6x SSC (20x SSC contains 3.0 M NaCl, 0.3 M Na-citrate, pH 7.0), 5x Denhardt's (100X Denhardt's contains 2% Ficoll, 2% Polyvinyl pyrollidone, 2% Bovine Serum Albumin), 0.5% sodium dodecyl sulphate (SDS), and 20 µg/ml denaturated carrier DNA (single-stranded fish sperm DNA, with an average length of 120 - 3000 nucleotides) as non-specific competitor. Following hybridization, high stringency washing may be done in several steps, with a final wash (about 30 min) at the hybridization temperature in 0.2-0.1 × SSC, 0.1% SDS.

"Moderate stringency conditions" refers to conditions equivalent to hybridization in the above described solution but at about 60-62°C. Moderate stringency washing may be done at the hybridization temperature in 1x SSC, 0.1% SDS.

"Low stringency" refers to conditions equivalent to hybridization in the above described solution at about 50-52°C. Low stringency washing may be done at the hybridization temperature in 2x SSC, 0.1% SDS. See also Sambrook et al. (1989) and Sambrook and Russell (2001).

Other sequences encoding AHAS polypeptides from other plant species may also be obtained by DNA amplification using oligonucleotides specific for genes encoding AHAS as primers, such as but not limited to oligonucleotides comprising or consisting of about 20 to about 50 consecutive nucleotides from the known nucleotide sequences or their complement.

Examples of sequences of AHAS polypeptides and sequences encoding these AHAS polypeptides of polyploid plant species are the *Gossypium hirsutum* AHAS sequences having GenBank number Z46959 (SEQ ID NO: 11 and 12), GenBank number Z46960 (SEQ ID NO: 13 and 14), the *Glycine max* AHAS sequences having GenBank number FJ581423 (SEQ ID NO: 15 and 16), NCBI Reference Sequence XM_003545859 (SEQ ID NO: 17), NCBI Reference Sequence: XP_003545907 (SEQ ID NO: 18), NCBI Reference Sequence XM_003528058 (SEQ ID NO: 19), NCBI Reference Sequence XP_003528106 (SEQ ID NO: 20), EnsemblPlants number GLYMA04G37270.1 (SEQ ID NO: 21 and 22), the *Nicotiana tabacum* AHAS sequences having GenBank number FJ649655 (SEQ ID NO: 23 and 24), ENA number CBV23149 (SEQ ID NO: 25), UniProtKB/Swiss-Prot number P09342.1 (SEQ ID NO: 26), and the *Solanum tuberosum* sequences having GenBank number HM114275 (SEQ ID NO: 27 and 28), EnsemblPlants number PGSC0003DMT400018236 (SEQ ID NO: 29), Uniprot TrEMBL number M1AAA1_SOLTU (SEQ ID NO: 30), EnsemblPlants number PGSC0003DMG400013027 (SEQ ID NO: 31 and 32). Partial AHAS sequences of *Triticum aestivum* are sequences having GenBank number AY210406.1 (SEQ ID NO: 33 and 34) and GenBank number AY210405.1 (SEQ ID NO: 35 and 36). Partial AHAS sequences of *Saccharum officinarum* are the sequence having GenBank number EU243998.1 (SEQ ID NO: 37 and 38).

The plants according to the invention comprise at least two AHAS genes encoding an AHAS polypeptide in which the Tryptophan at a position corresponding to position 574 of SEQ ID NO: 10 is substituted with Leucine and and wherein at least one of the ALS genes encodes an ALS polypeptide which further comprises at a position corresponding to position 197 of SEQ ID NO: 10 instead of the naturally encoded amino acid proline the amino acid serine, such as plants comprising at least two AHAS genes encoding an AHAS polypeptide in which the Tryptophan at a position corresponding to position 574 of SEQ ID NO: 10 is substituted with Leucine and wherein one of the ALS genes encodes an ALS polypeptide which further comprises at a position corresponding to position 197 of SEQ ID NO: 10 instead of the naturally encoded amino acid proline the amino acid serine, or such as plants comprising at least two AHAS genes encoding an AHAS polypeptide in which the Tryptophan at a position corresponding to position 574 of SEQ ID NO: 10 is substituted with Leucine and the Proline at a position corresponding to position 197 of SEQ ID NO: 10 is substituted with Serine. In order to determine whether a nucleotide residue or amino acid residue in a given ALS nucleotide/amino acid sequence corresponds to a certain position in the nucleotide sequence of SEQ ID NO: 9, or the corresponding amino acid sequences of SEQ ID 10, respectively, the skilled person can use means and methods well-known in the art, e.g., alignments, either manually or by using computer programs such as BLAST (Altschul et al. (1990), Journal of Molecular Biology, 215, 403-410), which stands for Basic Local Alignment Search Tool or ClustalW (Thompson et al. (1994), Nucleic Acid Res., 22, 4673-4680) or any other suitable program which is suitable to generate sequence alignments. An alignment of the protein sequences of various AHAS polypeptides or partial AHAS polypeptides with reference to the Arabidopsis AHAS polypeptide of SEQ ID NO: 10 is shown in Figure 3.

### Isolated/purified

An "isolated" nucleic acid sequence (or DNA) is used herein to refer to a nucleic acid sequence (or DNA) that is no longer in its natural environment, for example in an in vitro or in a recombinant bacterial or plant host cell. An "isolated" nucleic acid may be free of nucleotide sequences (preferably protein encoding sequences) that naturally flank the nucleic acid (i.e., sequences located at the 5' and 3' ends of the nucleic acid) in the genomic DNA of the organism from which the nucleic acid is derived. For purposes of the invention, "isolated" when used to refer to nucleic acid molecules excludes isolated chromosomes. For example, the isolated nucleic acid molecule encoding an ALS protein can contain less than about 5 kb, 4 kb, 3 kb, 2 kb, 1 kb, 0.5 kb, or 0.1 kb of nucleotide sequences that naturally flank the nucleic acid molecule in genomic DNA of the cell from which the nucleic acid is derived. An ALS protein that is substantially free of cellular material includes preparations of protein having less than about 30%, 20%, 10%, or 5% (by dry weight) of non-ALS protein (also referred to herein as a "contaminating protein").

### Amino Acid Substitution

Amino acid substitutions encompass amino acid alterations in which an amino acid is replaced with a different naturally-occurring amino acid residue. Such substitutions may be classified as "conservative', in which an amino acid residue contained in the wild-type ALS protein is replaced with another naturally-occurring amino acid of similar character, for example Ala↔Val, Trp↔Leu, Gly↔Asp, Gly↔Ala, Val↔Ile↔Leu, Asp↔Glu, Lys↔Arg, Asn↔Gln or Phe↔Trp↔Tyr. Substitutions may also be "non-conservative", in which an amino acid residue which is present in the wild-type ALS protein is substituted with an amino acid with different properties, such as a naturally-occurring amino acid from a different group. In one embodiment, a plant comprises mutations of its endogenous acetolactate synthase (ALS) genes, wherein an ALS gene encodes an ALS polypeptide comprising at a position corresponding to position 574 of SEQ ID NO: 10 instead of the naturally encoded amino acid tryptophan the amino acid leucine and, optionally, further comprises at a position corresponding to position 197 of SEQ ID NO: 10 instead of the naturally encoded amino acid proline the amino acid serine, such as a *Brassica napus* plant which comprises mutations of its endogenous ALS genes, wherein an ALS I gene encodes an ALS I polypeptide comprising at a position corresponding to position 559 of SEQ ID NO: 2 instead of the naturally encoded amino acid tryptophan the amino acid leucine and, optionally, further comprises at a position corresponding to position 182 of SEQ ID NO: 2 instead of the naturally encoded amino acid proline the amino acid serine, and wherein an ALS III gene encodes an ALS III polypeptide comprising at a position corresponding to position 556 of SEQ ID NO: 4 instead of the naturally encoded amino acid tryptophan the amino acid leucine and, optionally, further comprises at a position corresponding to position 179 of SEQ ID NO: 4 instead of the naturally encoded amino acid proline the amino acid serine, or such as a *Brassica juncea* plant which comprises mutations of its endogenous ALS genes, wherein an ALS-A gene encodes an ALS-A polypeptide comprising at a position corresponding to position 556 of SEQ ID NO: 6 instead of the naturally encoded amino acid tryptophan the amino acid leucine and, optionally, further comprises at a position corresponding to position 179 of SEQ ID NO: 6 instead of the naturally encoded amino acid proline the amino acid serine and wherein an ALS-B gene encodes an ALS-B polypeptide comprising at a position corresponding to position 559 of SEQ ID NO: 8 instead of the naturally encoded amino acid tryptophan the amino acid leucine and, optionally, further comprises at a position corresponding to position 182 of SEQ ID NO: 8 instead of the naturally encoded amino acid proline the amino acid serine. Altered ALS gene sequences, such as gene sequences of ALS I gene sequence SEQ ID NO: 1 and/or ALS III gene sequence SEQ ID NO: 3, or such as ALS-A gene sequence SEQ ID NO: 5 and/or ALS-B gene sequence SEQ ID NO: 7 may contain at least one further mutation.

"Similar amino acids", as used herein, refers to amino acids that have similar amino acid side chains, i.e. amino acids that have polar, non-polar or practically neutral side chains. "Non-similar amino acids", as used herein, refers to amino acids that have different amino acid side chains, for example an amino acid with a polar side chain is non-similar to an amino acid with a non-polar side chain. Polar side chains usually tend to be present on the surface of a protein where they can interact with the aqueous environment found in cells ("hydrophilic" amino acids). On the other hand, "non-polar" amino acids tend to reside within the center of the protein where they can interact with similar non-polar neighbours ("hydrophobic" amino acids"). Examples of amino acids that have polar side chains are arginine, asparagine, aspartate, cysteine, glutamine, glutamate, histidine, lysine, serine, and threonine (all hydrophilic, except for cysteine which is hydrophobic). Examples of amino acids that have non-polar side chains are alanine, glycine, isoleucine, leucine, methionine, phenylalanine, proline, and tryptophan (all hydrophobic, except for glycine which is neutral).

### Genes/Alleles

Unless indicated otherwise, the terms "wild-type allele," "wild-type ALS allele", "wild-type ALS gene" or "wild-type ALS polynucleotide" refer to a nucleotide sequence containing at least 60%, or 70%, or 80%, or 90%, or 95%, or 97%, or 98%, or 99% sequence identity, or is identical to the ALS sequences as described herein, such as a nucleotide sequence containing at least 60%, or 70%, or 80%, or 90%, or 95%, or 97%, or 98%, or 99% sequence identity, or is identical to SEQ ID NO: 1 and or an ALS nucleic acid sequence containing at least 60%, or 70%, or 80%, or 90%, or 95%, or 97%, or 98%, or 99% sequence identity, or is identical to SEQ ID NO: 3, or 70%, or 80%, or 90%, or 95%, or 97%, or 98%, or 99% sequence identity, or is identical to SEQ ID NO: 5, or 70%, or 80%, or 90%, or 95%, or 97%, or 98%, or 99% sequence identity, or is identical to SEQ ID NO: 7, provided that the ALS gene does not carry a mutation in the codon corresponding to the Pro197 codon of SEQ ID NO: 9, such as the ALS I gene, the ALS-III gene, the ALS-A gene and ALS-B gene do not carry a mutation in the Pro197 codon yielding an amino acid different from Pro, wherein the amino acid position referred to is the position in the reference *A. thaliana* sequence (SEQ ID NO: 10), or that that the ALS gene does not carry a mutation in the codon corresponding to the Trp574 codon of SEQ ID NO: 9, such as the ALS I gene, the ALS-III gene, the ALS-A gene and ALS-B gene do not carry a mutation in the Trp574 codon yielding an amino acid different from Trp, wherein the amino acid position referred to is the position in the reference *A. thaliana* sequence (SEQ ID NO: 10).

The terms "wild-type ALS I allele," "wild-type ALS I allele", "wild-type ALS I gene" or "wild-type ALS I polynucleotide" refer to a nucleotide sequence containing at least 60%, or 70%, or 80%, or 90%, or 95%, or 97%, or 98%, or 99% sequence identity, or is identical to SEQ ID NO: 1, provided that it does not carry a mutation in the Pro 197 codon yielding an amino acid different from Pro or a mutation in the Trp574 codon yielding an amino acid different from Trp, wherein the amino acid position referred to is the position in the reference *A. thaliana* sequence (SEQ ID NO: 10).

The terms "wild-type ALS III allele," "wild-type ALS III allele", "wild-type ALS III gene" or "wild-type ALS III polynucleotide" refer to a nucleotide sequence containing at least 60%, or 70%, or 80%, or 90%, or 95%, or 97%, or 98%, or 99% sequence identity, or is identical to SEQ ID NO: 3, provided that it does not carry a mutation in the Pro 197 codon yielding an amino acid different from Pro or a mutation in the Trp574 codon yielding an amino acid different from Trp, wherein the amino acid position referred to is the position in the reference *A. thaliana* sequence (SEQ ID NO: 10).

The terms "wild-type ALS-A allele," "wild-type ALS-A allele", "wild-type ALS-A gene" or "wild-type ALS-A polynucleotide" refer to a nucleotide sequence containing at least 60%, or 70%, or 80%, or 90%, or 95%, or 97%, or 98%, or 99% sequence identity, or is identical to SEQ ID NO: 5, provided that it does not carry a mutation in the Pro 197 codon yielding an amino acid different from Pro or a mutation in the Trp574 codon yielding an amino acid different from Trp, wherein the amino acid position referred to is the position in the reference *A. thaliana* sequence (SEQ ID NO: 10).

The terms "wild-type ALS-B allele," "wild-type ALS-B allele", "wild-type ALS-B gene" or "wild-type ALS-B polynucleotide" refer to a nucleotide sequence containing at least 60%, or 70%, or 80%, or 90%, or 95%, or 97%, or 98%, or 99% sequence identity, or is identical to SEQ ID NO: 7, provided that it does not carry a mutation in the Pro 197 codon yielding an amino acid different from Pro or a mutation in the Trp574 codon yielding an amino acid different from Trp, wherein the amino acid position referred to is the position in the reference *A. thaliana* sequence (SEQ ID NO: 10).

The term "wild type ALS" protein refers to the protein encoded by the ALS gene, wherein said ALS protein contains at least 90, 95, 97, 98, 99, or 100% sequence identity to the ALS amino acid sequence as described herein, provided that the amino acid at the position corresponding to position 197 of SEQ ID NO: 10 is a Pro and that the amino acid at the position corresponding to position 574 of SEQ ID NO: 10 is a Trp.

The term "wild type ALS I" protein refers to the protein encoded by the ALS I gene, wherein said ALS I protein contains at least 90, 95, 97, 98, 99, or 100% sequence identity to the ALS amino acid sequence of SEQ ID NO: 2, provided that the amino acid at the position corresponding to position 197 of SEQ ID NO: 10 is a Pro and that the amino acid at the position corresponding to position 574 of SEQ ID NO: 10 is a Trp.

The term "wild type ALS III" protein refers to the protein encoded by the ALS III gene, wherein said ALS III protein contains at least 90, 95, 97, 98, 99% or 100% sequence identity to the ALS amino acid sequence of SEQ ID NO: 4, provided that the amino acid at the position corresponding to position 197 of SEQ ID NO: 10 is a Pro and that the amino acid at the position corresponding to position 574 of SEQ ID NO: 10 is a Trp.

The term "wild type ALS-A" protein refers to the protein encoded by the ALS-A gene, wherein said ALS-A protein contains at least 90, 95, 97, 98, 99, or 100% sequence identity to the ALS amino acid sequence of SEQ ID NO: 6, provided that the amino acid at the position corresponding to position 197 of SEQ ID NO: 10 is a Pro and that the amino acid at the position corresponding to position 574 of SEQ ID NO: 10 is a Trp.

The term "wild type ALS-B" protein refers to the protein encoded by the ALS-B gene, wherein said ALS-B protein contains at least 90, 95, 97, 98, 99% or 100% sequence identity to the ALS amino acid sequence of SEQ ID NO: 8, provided that the amino acid at the position corresponding to position 197 of SEQ ID NO: 10 is a Pro and that the amino acid at the position corresponding to position 574 of SEQ ID NO: 10 is a Trp.

Such a "wild-type allele", "wild-type ALS allele", "wild-type ALS gene" or "wild-type ALS polynucleotide" may, or may not, comprise mutations, other than the mutation mentioned above. However, SEQ ID NO: 1, SEQ ID NO: 3, SEQ ID NO: 5, SEQ ID NO: 7, and SEQ ID NO: 9 are in any case "wild-type alleles" which can be used as a reference.

The term "gene" when used herein refers to a polymeric form of nucleotides of any length, either ribonucleotides or desoxyribonucleotides. The term includes double- and single-stranded DNA and RNA. It also includes known types of modifications, for example, methylation, "caps", substitutions of one or more of the naturally occurring nucleotides with an analog. Preferably, a gene comprises a coding sequence encoding the herein defined polypeptide. A "coding sequence" is a nucleotide sequence which, when transcribed into mRNA, can be translated into a polypeptide. The boundaries of the coding sequence are determined by a translation start codon at the 5'-terminus and a translation stop codon at the 3'-terminus. A coding sequence can include, but is not limited to mRNA, cDNA, recombinant nucleic acid sequences or genomic DNA, while introns may be present as well under certain circumstances.

In essence, the difference between a wild-type plant, and a plant of the present invention is that all ALS genes of said plant comprise a codon corresponding to position 1720-1722 of SEQ ID NO: 9 encodes a Leu instead of Trp and that at least one ALS gene of said plant comprises in addition a codon corresponding to position 589-591 of SEQ ID NO: 9 encodes a Ser instead of Pro. Correspondingly, the difference between a wild-type *B. napus* plant, and a *B. napus* plant of the present invention is that an ALS I gene comprises a codon - corresponding to position 544-546 of SEQ ID NO: 1 - encodes a Ser instead of Pro and a codon - corresponding to position 1675-1677 of SEQ ID NO: 1 - encodes a Leu instead of Trp; and that an ALS III gene comprises a codon - corresponding to position 1666-1668 of the SEQ ID NO: 3 - encodes Leu instead of Trp, or that an ALS I gene comprises a codon - corresponding to position 1675-1677 of SEQ ID NO: 1 - encodes a Leu instead of Trp; and that an ALS III gene comprises a codon - corresponding to position 535-537 of SEQ ID NO: 3 - encodes a Ser instead of Pro and a codon - corresponding to position 1666-1668 of the SEQ ID NO: 3 - encodes Leu instead of Trp, or that an ALS I gene comprises a codon - corresponding to position 544-546 of SEQ ID NO: 1 - encodes a Ser instead of Pro and a codon - corresponding to position 1675-1677 of SEQ ID NO: 1 - encodes a Leu instead of Trp; and that an ALS III gene comprises a codon - corresponding to position 535-537 of SEQ ID NO: 3 - encodes a Ser instead of Pro and a codon - corresponding to position 1666-1668 of the SEQ ID NO: 3 - encodes Leu instead of Trp; the difference between a wild-type *B. juncea* plant, and a *B. juncea* plant of the present invention is that an ALS-A gene comprises a codon - corresponding to position 535-537 of SEQ ID NO: 5 - encodes a Ser instead of Pro and a codon - corresponding to position 1666-1668 of SEQ ID NO: 5 - encodes a Leu instead of Trp; and that an ALS-B gene comprises a codon - corresponding to position 1675-1677 of the SEQ ID NO: 7 - encodes Leu instead of Trp, or that an ALS-A gene comprises a codon - corresponding to position 1666-1668 of SEQ ID NO: 5 - encodes a Leu instead of Trp; and that an ALS-B gene comprises a codon - corresponding to position 544-546 of SEQ ID NO: 7 - encodes a Ser instead of Pro and a codon - corresponding to position 1675-1677 of the SEQ ID NO: 7 - encodes Leu instead of Trp, or that an ALS-A gene comprises a codon - corresponding to position 535-537 of SEQ ID NO: 5 - encodes a Ser instead of Pro and a codon - corresponding to position 1666-1668 of SEQ ID NO: 5 - encodes a Leu instead of Trp; and that an ALS-B gene comprises a codon - corresponding to position 544-546 of SEQ ID NO: 7 - encodes a Ser instead of Pro and a codon - corresponding to position 1675-1677 of the SEQ ID NO: 7 - encodes Leu instead of Trp.

However, as mentioned above, further differences such as additional mutations may be present between wild-type and the mutant ALS allele as specified herein. Yet, these further differences are not relevant as long as the difference explained before is present.

In one embodiment, a plant according to the present invention comprises at least two ALS genes, wherein all ALS genes encode an ALS protein comprising Leu instead of Trp at a position corresponding to position 574 of SEQ ID NO: 10, and wherein and wherein at least one of the ALS genes encodes an ALS protein further comprising Ser instead of Pro at a position corresponding to position 197 of SEQ ID NO: 10, when comparing said ALS protein with the wild type amino acid sequence of said ALS protein. In a further embodiment, a *B. napus* plant according to the present invention comprises an ALS I gene which encodes an ALS I protein comprising Ser instead of Pro at a position 182 and Leu instead of Trp at a position 559 when comparing said ALS I protein with the wild type amino acid sequence SEQ ID NO: 2; and comprises an ALS III gene which encodes an ALS III protein comprising Leu instead of Trp at a position 556 when comparing said ALS III protein with the wild type amino acid sequence SEQ ID NO: 4, or a *B. napus* plant according to the present invention comprises an ALS I gene which encodes an ALS I protein comprising Leu instead of Trp at a position 559 when comparing said ALS I protein with the wild type amino acid sequence SEQ ID NO: 2; and comprises an ALS III gene which encodes an ALS III protein comprising Ser instead of Pro at a position 179 and Leu instead of Trp at a position 556 when comparing said ALS III protein with the wild type amino acid sequence SEQ ID NO: 4, or a *B. napus* plant according to the present invention comprises an ALS I gene which encodes an ALS I protein comprising Ser instead of Pro at a position 182 and Leu instead of Trp at a position 559 when comparing said ALS I protein with the wild type amino acid sequence SEQ ID NO: 2; and comprises an ALS III gene which encodes an ALS III protein comprising Ser instead of Pro at a position 179 and Leu instead of Trp at a position 556 when comparing said ALS III protein with the wild type amino acid sequence SEQ ID NO: 4. In a further embodiment, a *B. juncea* plant according to the present invention comprises an ALS-A gene which encodes an ALS-A protein comprising Ser instead of Pro at a position 179 and Leu instead of Trp at a position 556 when comparing said ALS-A protein with the wild type amino acid sequence SEQ ID NO: 6; and comprises an ALS-B gene which encodes an ALS-B protein comprising Leu instead of Trp at a position 559 when comparing said ALS-B protein with the wild type amino acid sequence SEQ ID NO: 8, or a *B. juncea* plant according to the present invention comprises an ALS-A gene which encodes an ALS-A protein comprising Leu instead of Trp at a position 556 when comparing said ALS-A protein with the wild type amino acid sequence SEQ ID NO: 6; and comprises an ALS-B gene which encodes an ALS-B protein comprising Ser instead of Pro at a position 182 and Leu instead of Trp at a position 559 when comparing said ALS-B protein with the wild type amino acid sequence SEQ ID NO: 8, or a *B. juncea* plant according to the present invention comprises an ALS-A gene which encodes an ALS-A protein comprising Ser instead of Pro at a position 179 and Leu instead of Trp at a position 556 when comparing said ALS-A protein with the wild type amino acid sequence SEQ ID NO: 6; and comprises an ALS-B gene which encodes an ALS-B protein comprising Ser instead of Pro at a position 182 and Leu instead of Trp at a position 559 when comparing said ALS-B protein with the wild type amino acid sequence SEQ ID NO: 8. The skilled person will understand that such mutated ALS genes, such as ALS I, ALS III, ALS-A and ALS-B genes may comprise further mutations such as one, two or three further mutations.

Consequently, the Pro197Ser substitutions (when the *A. thaliana* ALS amino acid sequence of SEQ ID NO: 10 is used as reference) are a result of an alteration of codons at a position corresponding to position 589-591 of the nucleotide sequence shown in SEQ ID NO: 9, and the Trp574Leu substitutions (when the *A. thaliana* ALS amino acid sequence of SEQ ID NO: 10 is used as reference) are a result of an alteration of codons at a position corresponding to position 1720-1722 of the nucleotide sequence shown in SEQ ID NO: 9.

The substitution at position 197 (when the *A. thaliana* ALS amino acid sequence of SEQ ID NO: 10 is used as reference) may be a P→S substitution, wherein "S" is encoded by any of the codons "TCT", "TCC", "TCA", "TCG", "AGT", "AGC"; said "S" may be encoded by the codon "TCT".

The substitution at position 574 (when the *A. thaliana* ALS amino acid sequence of SEQ ID NO: 10 is used as reference) may be a W→L substitution, wherein "L" is encoded by any of the codons "CTT", "CTC", "CTA", "CTG", "TTA", "TTG"; said "L" may be encoded by the codon "TTG".

Hence, suitable is a plant comprising at least two ALS genes, wherein the nucleotide sequence of all ALS genes in their endogenous gene loci, at least a codon encoding Leu instead of Trp, at a position corresponding to position 1720-1722 of the *A. thaliana* ALS nucleic acid sequence of SEQ ID NO: 9, and where the nucleotide sequence of at least one ALS gene in the endogenous gene locus further comprises at least a codon encoding Ser instead of Pro, at a position corresponding to position 589-591 of SEQ ID NO: 9, such as a *B. napus* plant comprising in the nucleotide sequence of an ALS I gene in its endogenous gene locus, at least a codon encoding Ser instead of Pro, at a position corresponding to position 589-591 and at least a codon encoding Leu instead of Trp, at a position corresponding to position 1720-1722 of the *A. thaliana* ALS nucleic acid sequence of SEQ ID NO: 9 and comprising in the nucleotide sequence of an ALS III gene in its endogenous gene locus, at least a codon encoding Leu instead of Trp at a position corresponding to position 1720-1722 of the *A. thaliana* ALS nucleic acid sequence of SEQ ID NO: 9, or such as a *B. napus* plant comprising in the nucleotide sequence of an ALS I gene in its endogenous gene locus, at least a codon encoding Leu instead of Trp, at a position corresponding to position 1720-1722 of the *A. thaliana* ALS nucleic acid sequence of SEQ ID NO: 9 and comprising in the nucleotide sequence of an ALS III gene in its endogenous gene locus, at least a codon encoding Ser instead of Pro, at a position corresponding to position 589-591 and at least a codon encoding Leu instead of Trp at a position corresponding to position 1720-1722 of the *A. thaliana* ALS nucleic acid sequence of SEQ ID NO: 9, or such as a *B. napus* plant comprising in the nucleotide sequence of an ALS I gene in its endogenous gene locus, at least a codon encoding Ser instead of Pro, at a position corresponding to position 589-591 and at least a codon encoding Leu instead of Trp, at a position corresponding to position 1720-1722 of the *A. thaliana* ALS nucleic acid sequence of SEQ ID NO: 9 and comprising in the nucleotide sequence of an ALS III gene in its endogenous gene locus, at least a codon encoding Ser instead of Pro, at a position corresponding to position 589-591 and at least a codon encoding Leu instead of Trp at a position corresponding to position 1720-1722 of the *A. thaliana* ALS nucleic acid sequence of SEQ ID NO: 9, or such as a *B. juncea* plant comprising in the nucleotide sequence of an ALS-A gene in its endogenous gene locus, at least a codon encoding Ser instead of Pro, at a position corresponding to position 589-591 and at least a codon encoding Leu instead of Trp, at a position corresponding to position 1720-1722 of the *A. thaliana* ALS nucleic acid sequence of SEQ ID NO: 9 and comprising in the nucleotide sequence of an ALS-B gene in its endogenous gene locus, at least a codon encoding Leu instead of Trp at a position corresponding to position 1720-1722 of the *A*. *thaliana* ALS nucleic acid sequence of SEQ ID NO: 9, or such as a *B. juncea* plant comprising in the nucleotide sequence of an ALS-A gene in its endogenous gene locus, at least a codon encoding Leu instead of Trp, at a position corresponding to position 1720-1722 of the *A. thaliana* ALS nucleic acid sequence of SEQ ID NO: 9 and comprising in the nucleotide sequence of an ALS-B gene in its endogenous gene locus, at least a codon encoding Ser instead of Pro, at a position corresponding to position 589-591 and at least a codon encoding Leu instead of Trp at a position corresponding to position 1720-1722 of the *A. thaliana* ALS nucleic acid sequence of SEQ ID NO: 9, or such as a *B. juncea* plant comprising in the nucleotide sequence of an ALS-A gene in its endogenous gene locus, at least a codon encoding Ser instead of Pro, at a position corresponding to position 589-591 and at least a codon encoding Leu instead of Trp, at a position corresponding to position 1720-1722 of the *A. thaliana* ALS nucleic acid sequence of SEQ ID NO: 9 and comprising in the nucleotide sequence of an ALS-B gene in its endogenous gene locus, at least a codon encoding Ser instead of Pro, at a position corresponding to position 589-591 and at least a codon encoding Leu instead of Trp at a position corresponding to position 1720-1722 of the *A. thaliana* ALS nucleic acid sequence of SEQ ID NO: 9.

ALS alleles as described herein or plants comprising ALS alleles according to the invention can be indentified or detected by method known in the art, such as direct sequencing, PCR based assays or hybridization based assays. Alternatively, methods can also be developed using the specific ALS allele specific sequence information provided herein. Such alternative detection methods include linear signal amplification detection methods based on invasive cleavage of particular nucleic acid structures, also known as InvaderTM technology, (as described e.g. in US patent 5,985,557 "Invasive Cleavage of Nucleic Acids", 6,001,567 "Detection of Nucleic Acid sequences by Invader Directed Cleavage), RT-PCR-based detection methods, such as Taqman, or other detection methods, such as SNPlex. Briefly, in the InvaderTM technology, the target mutation sequence may e.g. be hybridized with a labeled first nucleic acid oligonucleotide comprising the nucleotide sequence of the mutation sequence or a sequence spanning the joining region between the 5' flanking region and the mutation region and with a second nucleic acid oligonucleotide comprising the 3' flanking sequence immediately downstream and adjacent to the mutation sequence, wherein the first and second oligonucleotide overlap by at least one nucleotide. The duplex or triplex structure that is produced by this hybridization allows selective probe cleavage with an enzyme (Cleavase®) leaving the target sequence intact. The cleaved labeled probe is subsequently detected, potentially via an intermediate step resulting in further signal amplification.

The present disclosure also relates to the combination of ALS alleles according to the invention in one plant, and to the transfer of ALS alleles according to the invention from one plant to another plant.

### ALS inhibitor herbicide tolerance

The terms "herbicide-tolerant" and "herbicide- resistant" are used interchangeably and are intended to have an equivalent meaning and an equivalent scope. Similarly, the terms "herbicide-tolerance" and "herbicide- resistance" are used interchangeably and are intended to have an equivalent meaning and an equivalent scope.

It is preferred that the plants of the present invention are less sensitive to an ALS inhibitor, such as at least 5 times, or 10 times, or 50 times, or 100 times, or 500 times, or 1000 times, or 2000 times less sensitive as compared to wild type plants having not the substitutions of the present invention, such as wild type *B. napus* plants comprising ALS I polypeptides of SEQ ID NO: 2 and ALS III polypeptides of SEQ ID NO: 4, i.e., wild type plants having not the substitutions of the present invention, or such as wild type *B. juncea* plants comprising ALS-A polypeptides of SEQ ID NO: 6 and ALS-B polypeptides of SEQ ID NO: 8, i.e., wild type plants having not the substitutions of the present invention. Wild type plants wherein all ALS alleles do not comprise the substitutions of the present invention, such as wild type B. napus plants wherein all ALS I alleles are alleles of SEQ ID NO: 1 and all ALS III alleles are alleles of SEQ ID NO: 3, or such as wild type B. juncea plants wherein all ALS-A alleles are alleles of SEQ ID NO: 5 and all ALS-B alleles are alleles of SEQ ID NO: 7 are preferred references when comparing ALS sensitivity. Less sensitive when used herein may, vice versa, be seen as "more tolerable" or "more resistant". Similarly, "more tolerable" or "more resistant" may, vice versa, be seen as "less sensitive".

For example, the *B. napus* plants of the present invention and in particular the *B. napus* plant described in the appended Examples are/is at less sensitive to a combination of the ALS inhibitor herbicides foramsulfuron (a member of the ALS inhibitor subclass "sulfonylurea herbicides") and thiencarbazone-methyl (a member of the ALS inhibitor subclass "sulfonylaminocarbonyltriazolinone herbicides") compared to the wild type enzyme.

An "herbicide-tolerant" or "herbicide-resistant" plant refers to a plant that is tolerant or resistant to at least one AHAS -inhibiting herbicide at a level that would normally kill, or inhibit the growth of a wild-type plant lacking a mutated AHAS nucleic acid molecule. By "herbicide-resistant AHAS nucleic acid molecule" is intended a nucleic acid molecule comprising one or more mutations that results in one or more amino acid substitutions relative to the non-mutated AHAS protein, where the mutations result in the expression of an herbicide-resistant AHAS protein. By "herbicide-tolerant AHAS protein" or "herbicide-resistant AHAS protein", it is intended that such an AHAS protein displays higher AHAS activity, relative to the AHAS activity of a wild-type AHAS protein, when in the presence of at least one herbicide that is known to interfere with AHAS activity and at a concentration or level of the herbicide that is to known to inhibit the AHAS activity of the wild-type AHAS protein. Furthermore, the AHAS activity of such an herbicide-tolerant or herbicide- resistant AHAS protein may be referred to herein as "herbicide-tolerant" or "herbicide-resistant" AHAS activity.

Preferably, the plants of the present invention are less sensitive to various members of ALS inhibitor herbicides, like sulfonylurea herbicides, sulfonylamino-carbonyltriazolinone herbicides, and imidazolinone herbicides. Sulfonylurea herbicides and sulfonylaminocarbonyltriazolinone herbicides against which said plants are less sensitive are preferably selected. The plants of the present invention can be less sensitive to the ALS inhibitor herbicide foramsulfuron (sulfonylurea herbicide) either alone or in combination with one or more further ALS inhibitor herbicides either from the subclass of the sulfonyurea-herbicides or any other sub-class of the ALS inhibitor herbicides.

Hence, the plants of the present invention which are preferably less sensitive to an ALS inhibitor herbicide can likewise also be characterized to be "more tolerant" to an ALS inhibitor" (i.e. an ALS inhibitor tolerant plant).

Thus, an "ALS inhibitor tolerant" plant refers to a plant, preferably a plant according to the present invention or any of its progenies that is more tolerant to at least one ALS inhibitor herbicide at a level that would normally inhibit the growth of a wild-type plant, preferably the ALS inhibitor herbicide controls a wild-type plant. Said wild-type plant does not comprise in the nucleotide sequence of any allele of any endogenous ALS gene a codon encoding a Ser instead of Pro at a position corresponding to position 589-591 of SEQ ID NO: 9 or a codon encoding a Leu instead of Trp a position corresponding to position 1720-1722 of SEQ ID NO: 9, such as a *B. napus* plant which does not comprise the nucleotide sequence of any allele of the endogenous ALS I gene, a codon encoding Ser instead of Pro at a position corresponding to position 544-546 of SEQ ID NO: 1 or a codon encoding Leu instead of Trp at a position corresponding to position 1675-1677 of SEQ ID NO: 1 and does not comprise in the nucleotide sequence of any allele of the endogenous ALS III gene, a codon encoding Ser instead of Pro at a position corresponding to position 535-537 of SEQ ID NO: 3 or a codon encoding Leu instead of Trp at a position corresponding to position 1666-1668 of SEQ ID NO: 3, or such as a *B. juncea* plant which does not comprise the nucleotide sequence of any allele of the endogenous ALS-A gene, a codon encoding Ser instead of Pro at a position corresponding to position 535-537 of SEQ ID NO: 5 or a codon encoding Leu instead of Trp at a position corresponding to position 1666-1668 of SEQ ID NO: 5 and does not comprise in the nucleotide sequence of any allele of the endogenous ALS-B gene, a codon encoding Ser instead of Pro at a position corresponding to position 544-546 of SEQ ID NO: 7 a codon encoding Leu instead of Trp at a position corresponding to position 1675-1677 of SEQ ID NO: 7.

Said nucleotide sequences may generally also be characterized to be "ALS inhibitor herbicide tolerant" nucleotide sequences. By "ALS inhibitor herbicide tolerant nucleotide sequence" is intended a nucleic acid molecule comprising nucleotide sequences encoding for an ALS protein having at least a Leu instead of Trp a position corresponding to position 574 of SEQ ID NO: 10 and, optionally, further comprising a Ser instead of Pro at a position corresponding to position 197 of SEQ ID NO: 10, such as a nucleic acid molecule comprising nucleotide sequences encoding for an ALS I protein having at least a Leu instead of Trp a position corresponding to position 559 of SEQ ID NO: 2 and/or nucleotide sequences encoding for an ALS I protein having at least a Ser instead of Pro a position corresponding to position 182 of SEQ ID NO: 2 and a Leu instead of Trp a position corresponding to position 559 of SEQ ID NO: 2, and/or nucleotide sequences encoding for a ALS III protein having at least a Leu instead of Trp at a position corresponding to position 556 of SEQ ID NO: 4, and/or nucleotide sequences encoding for a ALS III protein having at least a Ser instead of Pro a position corresponding to position 179 of SEQ ID NO: 4 and a Leu instead of Trp at a position corresponding to position 556 of SEQ ID NO: 4, or such as a nucleic acid molecule comprising nucleotide sequences encoding for an ALS-A protein having at least a Leu instead of Trp a position corresponding to position 556 of SEQ ID NO: 6 and/or nucleotide sequences encoding for an ALS-A protein having at least a Ser instead of Pro a position corresponding to position 179 of SEQ ID NO: 6 and a Leu instead of Trp a position corresponding to position 556 of SEQ ID NO: 6 and/or nucleotide sequences encoding for a ALS-B protein having at least a Leu instead of Trp at a position corresponding to position 559 of SEQ ID NO: 8 and/or nucleotide sequences encoding for a ALS-B protein having at least a Ser instead of Pro at a position corresponding to position 182 of SEQ ID NO: 8 a Leu instead of Trp at a position corresponding to position 559 of SEQ ID NO: 8, wherein said at least one mutation or said at least two mutations result in the expression of a less sensitive to an ALS inhibitor herbicide ALS protein.

By "herbicide-tolerant ALS protein", it is intended that such an ALS protein displays higher ALS activity, relative to the ALS activity of a wild-type ALS protein, in the presence of at least one ALS inhibitor herbicide that is known to interfere with ALS activity and at a concentration or level of said herbicide that is known to inhibit the ALS activity of the wild-type ALS protein.

Similarly, the terms "ALS-inhibitor herbicide(s)" or simply "ALS-inhibitor(s)" are used interchangeably. As used herein, an "ALS -inhibitor herbicide" or an "ALS inhibitor" is not meant to be limited to single herbicide that interferes with the activity of the ALS enzyme. Thus, unless otherwise stated or evident from the context, an "ALS-inhibitor herbicide" or an "ALS inhibitor" can be a one herbicide or a mixture of two, three, four, or more herbicides known in the art, preferably as specified herein, each of which interferes with the activity of the ALS enzyme.

"Herbicide resistance" or "herbicide tolerance" can be measured as described in the present application or, e.g., it can be measured by comparison of AHAS activity obtained from cell extracts from plants containing the mutagenized AHAS sequence and from plants lacking the mutagenized AHAS sequence in the presence of an AHAS inhibitor, such as foramsulfuron or imazamox, using the methods disclosed in Singh, et al. Anal. Biochem., (1988), 171 : 173-179. Resistant or tolerant plants may demonstrate greater than 25% uninhibition using the methods disclosed in Singh et al (1988) when assayed, e.g., using 10 µM foramsulfuron or 10µM imazamox.

The activity of specific ALS proteins such as ALS I or ALS III proteins can be measured according to the following method: The coding sequences of wild-type, P197S-W574L-mutant, and W574L-mutant ALS, such as *Brassica* wild-type, P197S-W574L-mutant, and W574L-mutant ALS I, or wild type, P197S-W574L-mutant, and W574L-mutant ALS III, or wild type, P197S-W574L-mutant, and W574L-mutant ALS-A, or wild-type, P197S-W574L-mutant, or W574L-mutant ALS-B genes can be cloned into Novagen pET-32a(+) vectors and the vectors transformed into *Escherichia coli* AD494 according to the instructions of the manufacturer. Bacteria are grown at 37°C in LB-medium containing 100 mg/l carbenicillin and 25 mg/l canamycin, induced with 1 mM isopropyl-P-D-thiogalactopyranoside at an OD₆₀₀ of 0.6, cultivated for 16 hours at 18°C and harvested by, e.g., centrifugation. Bacterial pellets are resuspended in 100 mM sodium phosphate buffer pH 7.0 containing 0.1 mM thiamine-pyrophosphate, 1 mM MgCl₂, and 1 µM FAD at a concentration of 1 gram wet weight per 25 ml of buffer and disrupted by, e.g., sonification. The crude protein extract obtained after centrifugation is used for ALS activity measurements.

P197S-W574L-mutant ALS refers to an ALS protein comprising a serine instead of a proline at a position corresponding to position 197 of SEQ ID NO: 10, and a leucine instead of a tryptophan at a position corresponding to position 574 of SEQ ID NO: 10. W574L-mutant ALS refers to an ALS protein comprising a leucine instead of a tryptophan at a position corresponding to position 574 of SEQ ID NO: 10.

ALS protein can be extracted from leaves or tissue cultures, such as *B. napus* or *B. juncea* leaves, or *B. napus* or *B. juncea* tissue cultures as described by Ray (Plant Physiol, 1984, 75:827-831). An ALS assays can be carried out in 96-well microtiter plates using a modification of the procedure described by Ray (1984): The reaction mixture contains 20 mM potassium phosphate buffer pH 7.0, 20 mM sodium pyruvate, 0.45 mM thiamine-pyrophosphate, 0.45 mM MgCl₂, 9 µM FAD. ALS enzyme and various concentrations of ALS inhibitors can be mixed in a final volume of 90 µl. Assays can be initiated by adding enzyme and the assays can be terminated after 75 min incubation at 30°C by the addition of 40 µl 0.5 M H₂SO₄. After 60 min at room temperature 80 µl of a solution of 1.4% α-naphtol and 0.14% creatine in 0.7 M NaOH can be added and after an additional 45 min incubation at room temperature the absorbance can be determined at 540 nm. pI50-values for inhibition of ALS can be determined as described by Ray (1984), using the XLFit Excel add-in version 4.3.1 curve fitting program of ID Business Solutions Limited.

The ALS nucleotide sequences referred to herein encoding ALS polypeptides preferably confer tolerance to one or more ALS inhibitor herbicides (or, vice versa, less sensitivity to an ALS inhibitor herbicide) as described herein. This is because of the point mutation(s) leading to the amino acid substitution(s) as described herein. The plants of the present invention may show tolerance against a compound of formula (I), e.g.,plants according to the invention show essentially no injury (injury below 5%, 1% or even 0%) when 15 g a.i. / ha are applied whereas injury of wild type is above 90 % .

Surprisingly, it was found that the presence of the W574L mutation in ALS I in combination with the P197S-W574L mutation in ALS III increases herbicide tolerance to ALS inhibitor herbicides of *Brassica* plants.

However, one embodiment of the invention refers to allotetraploid *Brassica* plants and parts thereof which are homozygous regarding the mutation of the ALS genes resulting in a codon encoding Leu instead of Trp at a position corresponding to position 1720-1722 of SEQ ID NO: 9 and which are homozygous regarding the mutation of the ALS genes resulting in a codon encoding Ser instead of Pro at a position corresponding to position 589-591 of SEQ ID NO: 9 and a codon encoding Leu instead of Trp at a position corresponding to position 1720-1722 of SEQ ID NO: 9, such as *B. napus* plants and parts thereof which are homozygous regarding the mutations of ALS-I genes resulting in a codon encoding Ser instead of Pro at a position corresponding to position 544-546 of SEQ ID NO: 1 and a codon encoding Leu instead of Trp at a position corresponding to position 1675-1677 of SEQ ID NO: 1 ; and the mutation of ALS III genes resulting in a codon encoding Leu instead of Trp at a position corresponding to position 1666-1668 of SEQ ID NO: 3; or such as *B. napus* plants and parts thereof which are homozygous regarding the mutation of ALS-I genes resulting in a codon encoding Leu instead of Trp at a position corresponding to position 1675-1677 of SEQ ID NO: 1 and the mutations of ALS III genes resulting in a codon encoding Ser instead of Pro at a position corresponding to position 535-537 of SEQ ID NO: 3 and a codon encoding Leu instead of Trp at a position corresponding to position 1666-1668 of SEQ ID NO: 3; or such as *B. napus* plants and parts thereof which are homozygous regarding the mutations of ALS-I genes resulting in a codon encoding Ser instead of Pro at a position corresponding to position 544-546 of SEQ ID NO: 1 and a codon encoding Leu instead of Trp at a position corresponding to position 1675-1677 of SEQ ID NO: 1 and the mutations of ALS III genes resulting in a codon encoding Ser instead of Pro at a position corresponding to position 535-537 of SEQ ID NO: 3 and a codon encoding Leu instead of Trp at a position corresponding to position 1666-1668 of SEQ ID NO: 3; or such as *B. juncea* plants and parts thereof which are homozygous regarding the mutations of ALS-A genes resulting in a codon encoding Ser instead of Pro at a position corresponding to position 535-537 of SEQ ID NO: 5 and a codon encoding Leu instead of Trp at a position corresponding to position 1666-1668 of SEQ ID NO: 5; and the point mutation of ALS-B genes resulting in a codon encoding Leu instead of Trp at a position corresponding to position 1675-1677 of SEQ ID NO: 7, or such as *B. juncea* plants and parts thereof which are homozygous regarding the mutation of ALS-A genes resulting in a codon encoding Leu instead of Trp at a position corresponding to position 1666-1668 of SEQ ID NO: 5; and the point mutations of ALS-B genes resulting in a codon encoding Ser instead of Pro at a position corresponding to position 544-546 of SEQ ID NO: 7 and a codon encoding Leu instead of Trp at a position corresponding to position 1675-1677 of SEQ ID NO: 7, or such as *B. juncea* plants and parts thereof which are homozygous regarding the mutations of ALS-A genes resulting in a codon encoding Ser instead of Pro at a position corresponding to position 535-537 of SEQ ID NO: 5 and a codon encoding Leu instead of Trp at a position corresponding to position 1666-1668 of SEQ ID NO: 5 and the mutations of ALS-B genes resulting in a codon encoding Ser instead of Pro at a position corresponding to position 544-546 of SEQ ID NO: 7 and a codon encoding Leu instead of Trp at a position corresponding to position 1675-1677 of SEQ ID NO: 7.

As used herein, the term "heterozygous" means a genetic condition existing when (at least) two different alleles reside at a specific locus, but are positioned individually on corresponding pairs of homologous chromosomes in the cell. In other words, (at least) two different ALS alleles, reside at specific loci but are positioned individually on corresponding pairs of homologous chromosomes in the cell.

Conversely, as used herein, the term "homozygous" means a genetic condition existing when two (all) identical alleles reside at a specific locus, but are positioned individually on corresponding pairs of homologous chromosomes in the cell.

As used herein, the term "locus" (loci plural) means a specific place or places or a site on a chromosome where, e.g., a gene or genetic marker is found.

By ALS genes in its "endogenous locus" it is meant that the ALS genes comprised by the plant of the present invention is - when compared to a wild-type plant - located in the same locus, i.e., the ALS genes are positioned (located) on the same chromosome in the same chromosomal context (organization) as they are positioned in a wild-type plant (i.e., without there being any human intervention so as to transfer or re-locate the ALS genes comprised by the plant of the present invention to another location such as to another chromosome or genomic locus (position) different from that where the ALS genes are naturally located). Accordingly, the identical genome-specific satellite markers which surround a wild-type ALS gene also surround an ALS gene comprised by the plant of the present invention.

"Positioned in the same chromosomal context (organization)" means that an ALS gene of the plant of the present invention is located on the same chromosome as it is in a wild-type plant. Accordingly, the same genes as in a wild-type plant are adjacent to the 5'- and 3'-end of an ALS gene comprised by the plant of the present invention. Hence, the same nucleotide sequences which are adjacent to the 5'- and 3'-end of the wild-type ALS gene are adjacent to the 5'- and 3'-end of an ALS gene comprised by the plant of the present invention. The similarity of the chromosomal context between an ALS gene comprised by the plant of the present invention and that of an ALS gene of a wild-type plant can, for example, be tested as follows:

Genome-specific satellite markers which surround a wild-type ALS gene and an ALS gene of the present invention can be used together with sequences from the ALS gene (preferably except for the codon at the position as specified herein which is different between the wild-type ALS gene and an ALS gene comprised by the plant of the present invention) for primer design and subsequent nucleic acid amplification, whereby the amplification product will be identical between a wild-type plant and the plant of the present invention. These genome-specific satellite markers can also be used for a fluorescent in situ hybridization (FISH) in order to check the location of the ALS gene (see Schmidt and Heslop-Harrison (1996), Proc. Natl. Acad. Sci.93:8761-8765 for a FISH protocol of B. napus).

In view of the fact that mutated endogenous ALS genes of the present invention are located at the same chromosome at the same specific location, respectively, the "staining pattern" in FISH of the chromosome on which the wild-type ALS genes are located will be identical to the staining pattern in FISH of the chromosome on which the ALS genes of the present invention are located.

Of course, foreign genes can be transferred to the plant either by genetic engineering or by conventional methods such as crossing. Said genes can be genes conferring herbicide tolerances, preferably conferring herbicide tolerances different from ALS inhibitor herbicide tolerances, genes improving yield, genes improving resistances to biological organisms, and/or genes concerning content modifications.

The plants according to the invention form the basis for the development of commercial varieties including F1 hybrids following procedures known in the breeding community supported by molecular breeding techniques (like marker assisted breeding or marker assisted selection) for speeding up the processes and to secure the correct selection of plants to either obtain the mutation in its homozygous form or in case of comprising one or more mutations at various locations of the ALS encoding endogenous gene to perform the correct selection of heterozygous plants wherein at least at one of the alleles of each ALS gene comprises the Trp574Leu mutation (when referring to SEQ ID NO: 10) according to present invention and at least one of the alleles of one ALS gene comprises thePro197Ser and the Trp574Leu mutation mutation (when referring to SEQ ID NO: 10) according to present invention.

Calli are obtained by means and methods commonly known in the art, e.g., Alexander Dovzhenko, PhD Thesis, Title: "Towards plastid transformation in rapeseed (Brassica napus L.) and sugarbeet (Beta vulgaris L.)", Ludwig-Maximilians-Universitat München, Germany, 2001):

*B. napus* seeds can be immersed for 60 seconds in 70% ethanol, then rinsed twice in sterile water with 0,01 % detergent and then incubated for 1-4 hours in 1% NaOCl bleach. After washing with sterile H₂O at 4°C, the embryos can be isolated using, e.g., forceps and scalpel.

The freshly prepared embryos can be immersed in 0.5 % NaOCl for 30 min and then washed in sterile H₂O. After the last washing step they can be placed on hormone free MS agar medium (Murashige and Skoog (1962), Physiol. Plantarum, 15, 473-497). Those embryos which developed into sterile seedlings can be used for the initiation of regenerable *B. napus* cell cultures.

Cotyledons as well as hypocotyls can be cut into 2-5 mm long segments and then cultivated on agar (0.8 %) solidified MS agar medium containing either 1 mg /l Benzylaminopurin (BAP) or 0.25 mg/l Thidiazuron (TDZ). 4 weeks later the developing shoot cultures can be transferred onto fresh MS agar medium of the same composition and then sub-cultured in monthly intervals. The cultures can be kept at 25°C under dim light at a 12 h/12 h light/dark cycle.

After 7-10 days, subcultures the shoot cultures which were grown on the thidiazuron containing medium formed a distinct callus type, which was fast growing, soft and friable. The colour of this callus type is typically yellowish to light green. Some of these friable calli consistently produced chlorophyll containing shoot primordia from embryo-like structures. These fast growing regenerable calli can be used for the selection of ALS inhibitor herbicide tolerant *B. napus* mutants.

Described herein is a method to increase the tolerance to ALS inhibitor herbicide(s) of polyploid plants, said method comprising introducing at least two ALS genes, wherein said at least two ALS genes encode an ALS polypeptide comprising at a position corresponding to position 574 of SEQ ID NO: 10 instead of the naturally encoded amino acid tryptophan the amino acid leucine, and wherein at least one of the ALS genes encodes an ALS polypeptide which further comprises at a position corresponding to position 197 of SEQ ID NO: 10 instead of the naturally encoded amino acid proline the amino acid serine.

At least two genes which encode an ALS polypeptide comprising at a position corresponding to position 574 of SEQ ID NO: 10 instead of the naturally encoded amino acid tryptophan the amino acid leucine, and at least one gene which encode an ALS polypeptide comprising at a position corresponding to position 197 of SEQ ID NO: 10 instead of the naturally encoded amino acid proline the amino acid serine and at a position corresponding to position 574 of SEQ ID NO: 10 instead of the naturally encoded amino acid tryptophan the amino acid leucine can be introduced by selection methods, such as selection methods described herein in the examples. Upon selection, plants can be identified in which all ALS genes encode an ALS polypeptide comprising at a position corresponding to position 574 of SEQ ID NO: 10 instead of the naturally encoded amino acid tryptophan the amino acid leucine and one ALS gene encodes an ALS polypeptide which further comprises at a position corresponding to position 197 of SEQ ID NO: 10 instead of the naturally encoded amino acid proline the amino acid serine.

Said at genes least two genes which encode an ALS polypeptide comprising at a position corresponding to position 574 of SEQ ID NO: 10 instead of the naturally encoded amino acid tryptophan the amino acid leucine and said at least one gene which encodes an ALS polypeptide comprising at a position corresponding to position 197 of SEQ ID NO: 10 instead of the naturally encoded amino acid proline the amino acid serine and at a position corresponding to position 574 of SEQ ID NO: 10 instead of the naturally encoded amino acid tryptophan the amino acid leucine can also be introduced by crossing a plant comprising at least a first ALS gene encoding an ALS polypeptide comprising at a position corresponding to position 574 of SEQ ID NO: 10 instead of the naturally encoded amino acid tryptophan the amino acid leucine with another plant comprising at least a second ALS gene encoding an ALS polypeptide comprising at a position corresponding to position 197 of SEQ ID NO: 10 instead of the naturally encoded amino acid proline the amino acid serine and at a position corresponding to position 574 of SEQ ID NO: 10 instead of the naturally encoded amino acid tryptophan the amino acid leucine. Said first ALS gene may, optionally, further comprise at a position corresponding to position 197 of SEQ ID NO: 10 instead of the naturally encoded amino acid proline the amino acid serine. Optionally, the progeny plants can be identified using molecular methods as described herein. Alternatively, said at least two genes which encode an ALS polypeptide comprising at a position corresponding to position 574 of SEQ ID NO: 10 instead of the naturally encoded amino acid tryptophan the amino acid leucine and said at least one gene which encodes an ALS polypeptide comprising at a position corresponding to position 197 of SEQ ID NO: 10 instead of the naturally encoded amino acid proline the amino acid serine and at a position corresponding to position 574 of SEQ ID NO: 10 instead of the naturally encoded amino acid tryptophan the amino acid leucine can also be introduced by crossing a plant comprising at least two ALS gene encoding an ALS polypeptide comprising at a position corresponding to position 574 of SEQ ID NO: 10 instead of the naturally encoded amino acid tryptophan the amino acid leucine and at least one gene which encodes an ALS polypeptide comprising at a position corresponding to position 197 of SEQ ID NO: 10 instead of the naturally encoded amino acid proline the amino acid serine and at a position corresponding to position 574 of SEQ ID NO: 10 instead of the naturally encoded amino acid tryptophan the amino acid leucine, with another plant not comprising said at least two ALS genes encoding an ALS polypeptide comprising at a position corresponding to position 574 of SEQ ID NO: 10 instead of the naturally encoded amino acid tryptophan the amino acid leucine, and not comprising said at least one gene which encodes an ALS polypeptide comprising at a position corresponding to position 197 of SEQ ID NO: 10 instead of the naturally encoded amino acid proline the amino acid serine and at a position corresponding to position 574 of SEQ ID NO: 10 instead of the naturally encoded amino acid tryptophan the amino acid leucine. Optionally, the progeny plants can be identified using molecular methods as described herein. It will be clear that the progeny plants contain at least two ALS genes, wherein all ALS genes encode an ALS polypeptide comprising at a position corresponding to position 574 of SEQ ID NO: 10 instead of the naturally encoded amino acid tryptophan the amino acid leucine, and wherein at least one of the ALS genes encodes an ALS polypeptide which further comprises at a position corresponding to position 197 of SEQ ID NO: 10 instead of the naturally encoded amino acid proline the amino acid serine.

Described herein are methods to increase the tolerance to ALS inhibitor herbicide(s) of *Brassica napus* plants, said method comprising introducing an ALS I gene encoding an ALS I polypeptide comprising at a position corresponding to position 182 of SEQ ID NO: 2 instead of the naturally encoded amino acid proline the amino acid serine and at a position corresponding to position 559 of SEQ ID NO: 2 instead of the naturally encoded amino acid tryptophan the amino acid leucine, and introducing an ALS III gene encoding an ALS III polypeptide comprising at a position corresponding to position 556 of SEQ ID NO: 4 instead of the naturally encoded amino acid tryptophan the amino acid leucine, or said method comprising introducing an ALS I gene encoding an ALS I polypeptide comprising at a position corresponding to position 559 of SEQ ID NO: 2 instead of the naturally encoded amino acid tryptophan the amino acid leucine, and introducing an ALS III gene encoding an ALS III polypeptide comprising at a position corresponding to position 179 of SEQ ID NO: 4 instead of the naturally encoded amino acid proline the amino acid serine and at a position corresponding to position 556 of SEQ ID NO: 4 instead of the naturally encoded amino acid tryptophan the amino acid leucine, or said method comprising introducing an ALS I gene encoding an ALS I polypeptide comprising at a position corresponding to position 182 of SEQ ID NO: 2 instead of the naturally encoded amino acid proline the amino acid serine and at a position corresponding to position 559 of SEQ ID NO: 2 instead of the naturally encoded amino acid tryptophan the amino acid leucine, and introducing an ALS III gene encoding an ALS III polypeptide comprising at a position corresponding to position 179 of SEQ ID NO: 4 instead of the naturally encoded amino acid proline the amino acid serine and at a position corresponding to position 556 of SEQ ID NO: 4 instead of the naturally encoded amino acid tryptophan the amino acid leucine, and methods to increase the tolerance to ALS inhibitor herbicide(s) of *Brassica juncea* plants, said method comprising introducing an ALS-A gene encoding an ALS-A polypeptide comprising at a position corresponding to position 179 of SEQ ID NO: 6 instead of the naturally encoded amino acid proline the amino acid serine and at a position corresponding to position 556 of SEQ ID NO: 6 instead of the naturally encoded amino acid tryptophan the amino acid leucine, and introducing an ALS-B gene encoding an ALS-B polypeptide comprising at a position corresponding to position 559 of SEQ ID NO: 8 instead of the naturally encoded amino acid tryptophan the amino acid leucine, or said method comprising introducing an ALS-A gene encoding an ALS-A polypeptide comprising at a position corresponding to position 556 of SEQ ID NO: 6 instead of the naturally encoded amino acid tryptophan the amino acid leucine, and introducing an ALS-B gene encoding an ALS-B polypeptide comprising at a position corresponding to position 182 of SEQ ID NO: 8 instead of the naturally encoded amino acid proline the amino acid serine and at a position corresponding to position 559 of SEQ ID NO: 8 instead of the naturally encoded amino acid tryptophan the amino acid leucine, or said method comprising introducing an ALS-A gene encoding an ALS-A polypeptide comprising at a position corresponding to position 179 of SEQ ID NO: 6 instead of the naturally encoded amino acid proline the amino acid serine and at a position corresponding to position 556 of SEQ ID NO: 6 instead of the naturally encoded amino acid tryptophan the amino acid leucine, and introducing an ALS-B gene encoding an ALS-B polypeptide comprising at a position corresponding to position 182 of SEQ ID NO: 8 instead of the naturally encoded amino acid proline the amino acid serine and at a position corresponding to position 559 of SEQ ID NO: 8 instead of the naturally encoded amino acid tryptophan the amino acid leucine.

### Use

The present disclosure further relates to the use of one or more ALS inhibitor herbicide(s) in mutant plants according to the invention comprising mutations of all endogenous acetolactate synthase (ALS) genes, wherein all ALS genes encode an ALS polypeptide containing leucine instead of tryptophan at a position corresponding to 574 of SEQ ID NO: 10, and wherein at least one of the ALS genes encodes an ALS polypeptide which further comprises at a position corresponding to position 197 of SEQ ID NO: 10 instead of the naturally encoded amino acid proline the amino acid serine, such as B. *napus* mutants wherein an ALS I gene encodes an ALS I polypeptide containing serine instead of proline at a position 182 and leucine instead of tryptophan at a position 559 of said ALS I polypeptide and wherein an ALS III gene encodes an ALS III polypeptide containing leucine instead of tryptophan at a position 556 of said ALS III polypeptide, such as *B. napus* mutants wherein an ALS I gene encodes an ALS I polypeptide containing leucine instead of tryptophan at a position 559 of said ALS I polypeptide and wherein an ALS III gene encodes an ALS III polypeptide containing serine instead of proline at a position 179 and leucine instead of tryptophan at a position 556 of said ALS III polypeptide, or such as *B. napus* mutants wherein an ALS I gene encodes an ALS I polypeptide containing serine instead of proline at a position 182 and leucine instead of tryptophan at a position 559 of said ALS I polypeptide and wherein an ALS III gene encodes an ALS III polypeptide polypeptide containing serine instead of proline at a position 179 and leucine instead of tryptophan at a position 556 of said ALS III polypeptide, or such as *B. juncea* mutants wherein an ALS-A gene encodes an ALS-A polypeptide containing serine instead of proline at a position 179 and leucine instead of tryptophan at a position 556 of said ALS-A polypeptide and wherein an ALS-B gene encodes an ALS-B polypeptide containing leucine instead of tryptophan at a position 559 of said ALS-B polypeptide, or such as *B. juncea* mutants wherein an ALS-A gene encodes an ALS-A polypeptide containing leucine instead of tryptophan at a position 556 of said ALS-A polypeptide and wherein an ALS-B gene encodes an ALS-B polypeptide containing serine instead of proline at a position 182 and leucine instead of tryptophan at a position 559 of said ALS-B polypeptide, or such as *B. juncea* mutants wherein an ALS-A gene encodes an ALS-A polypeptide containing serine instead of proline at a position 179 and leucine instead of tryptophan at a position 556 of said ALS-A polypeptide and wherein an ALS-B gene encodes an ALS-B polypeptide containing serine instead of proline at a position 182 and leucine instead of tryptophan at a position 559 of said ALS-B polypeptide, and wherein the ALS inhibitor herbicide(s) belong to:
the group of the (sulfon)amides (group (A)) consisting of:
the subgroup (A1) of the sulfonylureas, consisting of:
   amidosulfuron [CAS RN 120923-37-7] (= A1-1);
   azimsulfuron [CAS RN 120162-55-2] (= A1-2);
   bensulfuron-methyl [CAS RN 83055-99-6] (= A1-3);
   chlorimuron-ethyl [CAS RN 90982-32-4] (= A1-4);
   chlorsulfuron [CAS RN 64902-72-3] (= A1-5);
   cinosulfuron [CAS RN 94593-91-6] (= A1-6);
   cyclosulfamuron [CAS RN 136849-15-5] (= A1-7);
   ethametsulfuron-methyl [CAS RN 97780-06-8] (= A1-8);
   ethoxysulfuron [CAS RN 126801-58-9] (= A1-9);
   flazasulfuron [CAS RN 104040-78-0] (= A1-10);
   flucetosulfuron [CAS RN 412928-75-7] (= A1-11);
   flupyrsulfuron-methyl-sodium [CAS RN 144740-54-5] (= A1-12);
   foramsulfuron [CAS RN 173159-57-4] (= A1-13);
   halosulfuron-methyl [CAS RN 100784-20-1] (= A1-14);
   imazosulfuron [CAS RN 122548-33-8] (= A1-15);
   iodosulfuron-methyl-sodium [CAS RN 144550-36-7] (= A1-16);
   mesosulfuron-methyl [CAS RN 208465-21-8] (= A1-17);
   metsulfuron-methyl [CAS RN 74223-64-6] (= A1-18);
   monosulfuron [CAS RN 155860-63-2] (= A1-19);
   nicosulfuron [CAS RN 111991-09-4] (= A1-20);
   orthosulfamuron [CAS RN 213464-77-8] (= A1-21);
   oxasulfuron [CAS RN 144651-06-9] (= A1-22);
   primisulfuron-methyl [CAS RN 86209-51-0] (= A1-23);
   prosulfuron [CAS RN 94125-34-5] (= A1-24);
   pyrazosulfuron-ethyl [CAS RN 93697-74-6] (= A1-25);
   rimsulfuron [CAS RN 122931-48-0] (= A1-26);
   sulfometuron-methyl [CAS RN 74222-97-2] (= A1-27);
   sulfosulfuron [CAS RN 141776-32-1] (= A1-28);
   thifensulfuron-methyl [CAS RN 79277-27-3] (= A1-29);
   triasulfuron [CAS RN 82097-50-5] (= A1-30);
   tribenuron-methyl [CAS RN 101200-48-0] (= A1-31);
   trifloxysulfuron [CAS RN 145099-21-4] (sodium) (= A1-32);
   triflusulfuron-methyl [CAS RN 126535-15-7] (= A1-33);
   tritosulfuron [CAS RN 142469-14-5] (= A1-34);
   NC-330 [CAS RN 104770-29-8] (= A1-35);
   NC-620 [CAS RN 868680-84-6] (= A1-36);
   TH-547 [CAS RN 570415-88-2] (= A1-37);
   monosulfuron-methyl [CAS RN 175076-90-1] (= A1-38);
   metazosulfuron [CAS RN 868680-84-6] (=A1-39) ;
   methiopyrsulfuron [CAS RN 441050-97-1] (=A1-40);
   iofensulfuron-sodium [CAS RN 1144097-30-2] (= A1-41) ;
   propyrisulfuron [CAS RN 570415-88-2] (=A1-42).
the subgroup of the sulfonylaminocarbonyltriazolinones (subgroup ((A2)), consisting of:
   flucarbazone-sodium [CAS RN 181274-17-9] (= A2-1);
   propoxycarbazone-sodium [CAS RN 181274-15-7] (= A2-2);
   thiencarbazone-methyl [CAS RN 317815-83-1] (= A2-3).
the subgroup of the triazolopyrimidines (subgroup (A3)), consisting of:
   cloransulam-methyl [147150-35-4] (= A3-1);
   diclosulam [CAS RN 145701-21-9] (= A3-2);
   florasulam [CAS RN 145701-23-1] (= A3-3);
   flumetsulam [CAS RN 98967-40-9] (= A3-4);
   metosulam [CAS RN 139528-85-1] (= A3-5);
   penoxsulam [CAS RN 219714-96-2] (= A3-6);
   pyroxsulam [CAS RN 422556-08-9] (= A3-7).
the subgroup of the sulfonanilides (subgroup (A4)), consisting of:
   compounds or salts thereof, and racemates and enantiomers thereof, from the group described by the general formula (I): 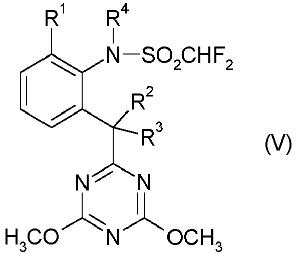 in which
   R¹ is halogen, preferably fluorine or chlorine,
   R² is hydrogen and R³ is hydroxyl or
   R² and R³ together with the carbon atom to which they are attached are a carbonyl group C=O and
   R⁴ is hydrogen or methyl;
   and more especially compounds of the below given chemical structure (A4-1) to (A4-8) 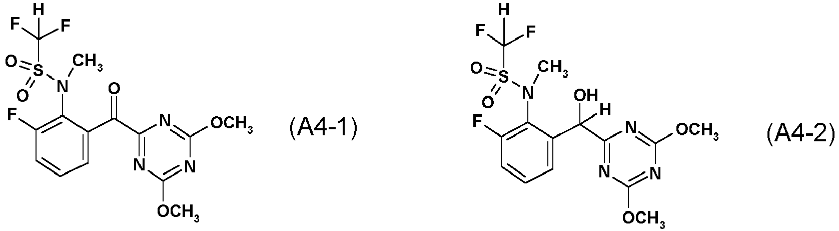 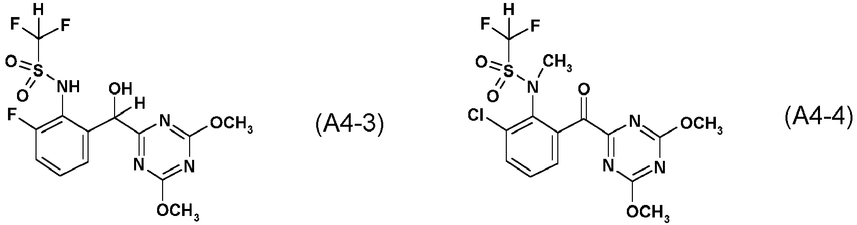 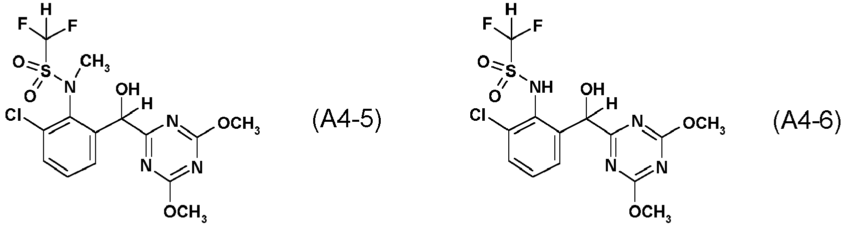 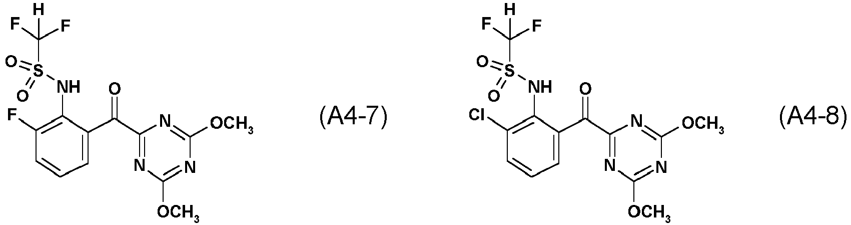
the group of the imidazolinones (group (B1)), consisting of:
   imazamethabenzmethyl [CAS RN 81405-85-8] (= B1-1);
   imazamox [CAS RN 114311-32-9] (= B1-2);
   imazapic [CAS RN 104098-48-8] (= B1-3);
   imazapyr [CAS RN 81334-34-1] (= B1-4);
   imazaquin [CAS RN 81335-37-7] (= B1-5);
   imazethapyr [CAS RN 81335-77-5] (= B1-6);
   SYP-298 [CAS RN 557064-77-4] (= B1-7);
   SYP-300 [CAS RN 374718-10-2] (= B1-8).
the group of the pyrimidinyl(thio)benzoates (group (C)), consisting of:
   the subgroup of the pyrimidinyloxybenzoeacids (subgroup (C1)) consisting of:
      bispyribac-sodium [CAS RN 125401-92-5] (= C1-1);
      pyribenzoxim [CAS RN 168088-61-7] (= C1-2);
      pyriminobac-methyl [CAS RN 136191-64-5] (= C1-3);
      pyribambenz-isopropyl [CAS RN 420138-41-6] (= CI-4);
      pyribambenz-propyl [CAS RN 420138-40-5] (= C1-5).
   the subgroup of the pyrimidinylthiobenzoeacids (subgroup (C2)), consisting of:
      pyriftalid [CAS RN 135186-78-6] (= C2-1);
      pyrithiobac-sodium [CAS RN 123343-16-8] (= C2-2).

In this context, "tolerance" or "tolerant" means that the application of one or more ALS inhibitor herbicide(s) belonging to any of the above defined groups (A), (B), (C) have reduced apparent effect(s), as compared to effect(s) on wild type plants, concerning the physiological functions/phytotoxicity when applied to the respective plant, such as *B. napus* plants or *B. juncea* plants according to the invention, having mutations of its endogenous acetolactate synthase (ALS) genes, wherein a first ALS gene, such as ALS I *B. napus,* or ALS-A *B. juncea,* gene encodes a first ALS, such as *B. napus or B. juncea,* polypeptide containing serine instead of proline at a position corresponding to position 197 of SEQ ID NO: 10 and leucine instead of tryptophan at a position corresponding to position 574 of SEQ ID NO: 10 and wherein a second ALS gene, such as an ALS III *B. napus,* or ALS-B *B. juncea,* gene encodes a second ALS, such as *B. napus* or *B. juncea,* polypeptide containing leucine instead of tryptophan at a position corresponding to position 574 of SEQ ID NO: 10, or such as *B. napus* plants or *B. juncea* plants according to the invention, having mutations of its endogenous acetolactate synthase (ALS) genes, wherein a first ALS gene, such as ALS I *B. napus,* or ALS-A *B. juncea,* gene encodes a first ALS, such as *B. napus or B. juncea,* polypeptide containing leucine instead of tryptophan at a position corresponding to position 574 of SEQ ID NO: 10 and wherein a second ALS gene, such as an ALS III *B. napus,* or ALS-B *B. juncea,* gene encodes a second ALS, such as *B. napus* or *B. juncea,* polypeptide containing serine instead of proline at a position corresponding to position 197 of SEQ ID NO: 10 and leucine instead of tryptophan at a position corresponding to position 574 of SEQ ID NO: 10, or such as *B. napus* plants or *B. juncea* plants according to the invention, having mutations of its endogenous acetolactate synthase (ALS) genes, wherein a first ALS gene, such as ALS I *B. napus,* or ALS-A *B. juncea,* gene encodes a first ALS, such as *B. napus or B. juncea,* polypeptide containing serine instead of proline at a position corresponding to position 197 of SEQ ID NO: 10 and leucine instead of tryptophan at a position corresponding to position 574 of SEQ ID NO: 10 and wherein a second ALS gene, such as an ALS III *B. napus,* or ALS-B *B. juncea,* gene encodes a second ALS, such as *B. napus* or *B. juncea,* polypeptide containing serine instead of proline at a position corresponding to position 197 of SEQ ID NO: 10 and leucine instead of tryptophan at a position corresponding to position 574 of SEQ ID NO: 10, and whereas the application of the same amount of the respective ALS inhibitor herbicide(s) on non-tolerant plants, such as *B. napus* or *B. juncea,* wild type plants leads to significant negative effects concerning plant growth, its physiological functions or shows phytotoxic sypmtoms. Qualtity and quantity of the observed effects may depend on the chemical composition of the respective ALS inhibitor heribicide(s) applied, dose rate and timing of the application as well growth conditions/stage of the treated plants.

The "CAS RN" stated in square brackets after the names (common names) mentioned under groups A to C corresponds to the "chemical abstract service registry number", a customary reference number which allows the substances named to be classified unambiguously, since the "CAS RN" distinguishes, inter alia, between isomers including stereoisomers.

ALS inhibitor herbicides which are preferably used for control of unwanted vegetation in plant growing areas, such as *B. napus* or *B. juncea* growing areas whichplants, such as *B. napus* plants or *B. juncea* plants comprise mutations of its endogenous acetolactate synthase (ALS) genes, wherein at least two ALS genes encode an ALS polypeptide containing leucine instead of tryptophan at a position corresponding to position 574 of SEQ ID NO: 10, and wherein at least one of the ALS genes encodes an ALS polypeptide which further comprises at a position corresponding to position 197 of SEQ ID NO: 10 instead of the naturally encoded amino acid proline the amino acid serine, such as *B. napus* wherein the ALS I gene encodes an ALS I polypeptide containing serine instead of proline at a position corresponding to position 182 and leucine instead of tryptophan at a position corresponding to position 559 of said first ALS I polypeptide and wherein the ALS III gene encodes an ALS III polypeptide containing leucine instead of tryptophan at a position 556 of said ALS III polypeptide, or such as *B*. *napus* wherein the ALS I gene encodes an ALS I polypeptide containing leucine instead of tryptophan at a position corresponding to position 559 of said first ALS I polypeptide and wherein the ALS III gene encodes an ALS III polypeptide containing serine instead of proline at a position corresponding to position 179 and leucine instead of tryptophan at a position 556 of said ALS III polypeptide, or such as *B. napus* wherein the ALS I gene encodes an ALS I polypeptide containing serine instead of proline at a position corresponding to position 182 and leucine instead of tryptophan at a position corresponding to position 559 of said first ALS I polypeptide and wherein the ALS III gene encodes an ALS III polypeptide containing serine instead of proline at a position corresponding to position 179 and leucine instead of tryptophan at a position 556 of said ALS III polypeptide, or such as *B. juncea* wherein the ALS-A gene encodes an ALS-A polypeptide containing serine instead of proline at a position corresponding to position 179 and leucine instead of tryptophan at a position corresponding to position 556 of said first ALS-A polypeptide and wherein the ALS-B gene encodes an ALS-B polypeptide containing leucine instead of tryptophan at a position 559 of said ALS-B polypeptide, or such as *B*. *juncea* wherein the ALS-A gene encodes an ALS-A polypeptide containing leucine instead of tryptophan at a position corresponding to position 556 of said first ALS-A polypeptide and wherein the ALS-B gene encodes an ALS-B polypeptide containing serine instead of proline at a position corresponding to position 182 and leucine instead of tryptophan at a position 559 of said ALS-B polypeptide, or such as *B. juncea* wherein the ALS-A gene encodes an ALS-A polypeptide containing serine instead of proline at a position corresponding to position 179 and leucine instead of tryptophan at a position corresponding to position 556 of said first ALS-A polypeptide and wherein the ALS-B gene encodes an ALS-B polypeptide containing serine instead of proline at a position corresponding to position 182 and leucine instead of tryptophan at a position 559 of said ALS-B polypeptide, and thereby providing tolerance against the ALS inhibitor herbicide(s) belonging to group (A) are:
amidosulfuron [CAS RN 120923-37-7] (= A1-1);
chlorimuron-ethyl [CAS RN 90982-32-4] (= A1-4);
chlorsulfuron [CAS RN 64902-72-3] (=A1-5);
ethametsulfuron-methyl [CAS RN 97780-06-8] (= A1-8);
ethoxysulfuron [CAS RN 126801-58-9] (= A1-9);
flupyrsulfuron-methyl-sodium [CAS RN 144740-54-5] (= A1-12);
foramsulfuron [CAS RN 173159-57-4] (= A1-13);
iodosulfuron-methyl-sodium [CAS RN 144550-36-7] (= A1-16);
mesosulfuron-methyl [CAS RN 208465-21-8] (= A1-17);
metsulfuron-methyl [CAS RN 74223-64-6] (= A1-18);
monosulfuron [CAS RN 155860-63-2] (= A1-19);
nicosulfuron [CAS RN 111991-09-4] (= A1-20);
rimsulfuron [CAS RN 122931-48-0] (= A1-26);
sulfosulfuron [CAS RN 141776-32-1] (= A1-28);
thifensulfuron-methyl [CAS RN 79277-27-3] (= A1-29);
tribenuron-methyl [CAS RN 101200-48-0] (= A1-31);
triflusulfuron-methyl [CAS RN 126535-15-7] (= A1-33);
iofensulfuron-sodium [CAS RN 1144097-30-2] (= A1-41);
flucarbazone-sodium [CAS RN 181274-17-9] (= A2-1);
propoxycarbazone-sodium [CAS RN 181274-15-7] (= A2-2);
thiencarbazone-methyl [CAS RN 317815-83-1] (= A2-3);
florasulam [CAS RN 145701-23-1] (= A3-3);
metosulam [CAS RN 139528-85-1] (= A3-5);
pyroxsulam [CAS RN 422556-08-9] (= A3-7);
   (A4-1); (A4-2) and (A4-3).

ALS inhibitor herbicides which are more preferably used for control of unwanted in plant growing areas, such as *B. napus* or *B. juncea* growing areas which plants, such as *B. napus* plants or *B. juncea* plants comprise mutations of its endogenous acetolactate synthase (ALS) genes, wherein at least two ALS genes encode an ALS polypeptide containing leucine instead of tryptophan at a position corresponding to position 574 of SEQ ID NO: 10, and wherein at least one of the ALS genes encodes an ALS polypeptide which further comprises at a position corresponding to position 197 of SEQ ID NO: 10 instead of the naturally encoded amino acid proline the amino acid serine, such as *B. napus* wherein the ALS I gene encodes an ALS I polypeptide containing serine instead of proline at a position corresponding to position 182 and leucine instead of tryptophan at a position corresponding to position 559 of said first ALS I polypeptide and wherein the ALS III gene encodes an ALS III polypeptide containing leucine instead of tryptophan at a position 556 of said ALS III polypeptide, or such as *B*. *napus* wherein the ALS I gene encodes an ALS I polypeptide containing leucine instead of tryptophan at a position corresponding to position 559 of said first ALS I polypeptide and wherein the ALS III gene encodes an ALS III polypeptide containing serine instead of proline at a position corresponding to position 179 and leucine instead of tryptophan at a position 556 of said ALS III polypeptide, or such as *B. napus* wherein the ALS I gene encodes an ALS I polypeptide containing serine instead of proline at a position corresponding to position 182 and leucine instead of tryptophan at a position corresponding to position 559 of said first ALS I polypeptide and wherein the ALS III gene encodes an ALS III polypeptide containing serine instead of proline at a position corresponding to position 179 and leucine instead of tryptophan at a position 556 of said ALS III polypeptide, or such as *B. juncea* wherein the ALS-A gene encodes an ALS-A polypeptide containing serine instead of proline at a position corresponding to position 179 and leucine instead of tryptophan at a position corresponding to position 556 of said first ALS-A polypeptide and wherein the ALS-B gene encodes an ALS-B polypeptide containing leucine instead of tryptophan at a position 559 of said ALS-B polypeptide, or such as *B*. *juncea* wherein the ALS-A gene encodes an ALS-A polypeptide containing leucine instead of tryptophan at a position corresponding to position 556 of said first ALS-A polypeptide and wherein the ALS-B gene encodes an ALS-B polypeptide containing serine instead of proline at a position corresponding to position 182 and leucine instead of tryptophan at a position 559 of said ALS-B polypeptide, or such as *B. juncea* wherein the ALS-A gene encodes an ALS-A polypeptide containing serine instead of proline at a position corresponding to position 179 and leucine instead of tryptophan at a position corresponding to position 556 of said first ALS-A polypeptide and wherein the ALS-B gene encodes an ALS-B polypeptide containing serine instead of proline at a position corresponding to position 182 and leucine instead of tryptophan at a position 559 of said ALS-B polypeptide, and thereby providing tolerance against the ALS inhibitor herbicide(s) belonging to group (A) are:
amidosulfuron [CAS RN 120923-37-7] (= A1-1);
ethoxysulfuron [CAS RN 126801-58-9] (= A1-9);
flupyrsulfuron-methyl-sodium [CAS RN 144740-54-5] (= A1-12);
foramsulfuron [CAS RN 173159-57-4] (= A1-13);
iodosulfuron-methyl-sodium [CAS RN 144550-36-7] (= A1-16);
mesosulfuron-methyl [CAS RN 208465-21-8] (= A1-17);
metsulfuron-methyl [CAS RN 74223-64-6] (= A1-18);
nicosulfuron [CAS RN 111991-09-4] (= A1-20);
rimsulfuron [CAS RN 122931-48-0] (= A1-26);
sulfosulfuron [CAS RN 141776-32-1] (= A1-28);
thifensulfuron-methyl [CAS RN 79277-27-3] (= A1-29);
tribenuron-methyl [CAS RN 101200-48-0] (= A1-31);
iofensulfuron-sodium [CAS RN 1144097-30-2] (= A1-41)
propoxycarbazone-sodium [CAS RN 181274-15-7] (= A2-2);
thiencarbazone-methyl [CAS RN 317815-83-1] (= A2-3);
florasulam [CAS RN 145701-23-1] (= A3-3);
metosulam [CAS RN 139528-85-1] (= A3-5); and
pyroxsulam [CAS RN 422556-08-9] (= A3-7).

ALS inhibitor herbicides which are especially preferably used for control of unwanted vegetation in plant growing areas, such as *B. napus* or *B. juncea* growing areas whichplants, such as *B. napus* plants or *B. juncea* plants comprise mutations of its endogenous acetolactate synthase (ALS) genes, wherein at least two ALS genes encode an ALS polypeptide containing leucine instead of tryptophan at a position corresponding to position 574 of SEQ ID NO: 10, and wherein at least one of the ALS genes encodes an ALS polypeptide which further comprises at a position corresponding to position 197 of SEQ ID NO: 10 instead of the naturally encoded amino acid proline the amino acid serine, such as *B. napus* wherein the ALS I gene encodes an ALS I polypeptide containing serine instead of proline at a position corresponding to position 182 and leucine instead of tryptophan at a position corresponding to position 559 of said first ALS I polypeptide and wherein the ALS III gene encodes an ALS III polypeptide containing leucine instead of tryptophan at a position 556 of said ALS III polypeptide, or such as *B. napus* wherein the ALS I gene encodes an ALS I polypeptide containing leucine instead of tryptophan at a position corresponding to position 559 of said first ALS I polypeptide and wherein the ALS III gene encodes an ALS III polypeptide containing serine instead of proline at a position corresponding to position 179 and leucine instead of tryptophan at a position 556 of said ALS III polypeptide, or such as *B. napus* wherein the ALS I gene encodes an ALS I polypeptide containing serine instead of proline at a position corresponding to position 182 and leucine instead of tryptophan at a position corresponding to position 559 of said first ALS I polypeptide and wherein the ALS III gene encodes an ALS III polypeptide containing serine instead of proline at a position corresponding to position 179 and leucine instead of tryptophan at a position 556 of said ALS III polypeptide, or such as *B. juncea* wherein the ALS-A gene encodes an ALS-A polypeptide containing serine instead of proline at a position corresponding to position 179 and leucine instead of tryptophan at a position corresponding to position 556 of said first ALS-A polypeptide and wherein the ALS-B gene encodes an ALS-B polypeptide containing leucine instead of tryptophan at a position 559 of said ALS-B polypeptide, or such as *B. juncea* wherein the ALS-A gene encodes an ALS-A polypeptide containing leucine instead of tryptophan at a position corresponding to position 556 of said first ALS-A polypeptide and wherein the ALS-B gene encodes an ALS-B polypeptide containing serine instead of proline at a position corresponding to position 182 and leucine instead of tryptophan at a position 559 of said ALS-B polypeptide, or such as *B. juncea* wherein the ALS-A gene encodes an ALS-A polypeptide containing serine instead of proline at a position corresponding to position 179 and leucine instead of tryptophan at a position corresponding to position 556 of said first ALS-A polypeptide and wherein the ALS-B gene encodes an ALS-B polypeptide containing serine instead of proline at a position corresponding to position 182 and leucine instead of tryptophan at a position 559 of said ALS-B polypeptide, and thereby providing tolerance against the ALS inhibitor herbicide(s) belonging to group (A) are:
amidosulfuron [CAS RN 120923-37-7] (= A1-1);
foramsulfuron [CAS RN 173159-57-4] (= A1-13);
iofensulfuron-sodium [CAS RN 1144097-30-2] (= A1-41); and
thiencarbazone-methyl [CAS RN 317815-83-1] (= A2-3).

Another ALS inhibitor herbicide which is preferably used for control of unwanted vegetation in plant growing areas, such as *B. napus* or *B. juncea* growing areas which plants, such as *B. napus* plants or *B. juncea* plants comprise mutations of its endogenous acetolactate synthase (ALS) genes, wherein at least two ALS genes encode an ALS polypeptide containing leucine instead of tryptophan at a position corresponding to position 574 of SEQ ID NO: 10, and wherein at least one of the ALS genes encodes an ALS polypeptide which further comprises at a position corresponding to position 197 of SEQ ID NO: 10 instead of the naturally encoded amino acid proline the amino acid serine, such as *B. napus* wherein the ALS I gene encodes an ALS I polypeptide containing serine instead of proline at a position corresponding to position 182 and leucine instead of tryptophan at a position corresponding to position 559 of said first ALS I polypeptide and wherein the ALS III gene encodes an ALS III polypeptide containing leucine instead of tryptophan at a position 556 of said ALS III polypeptide, or such as *B*. *napus* wherein the ALS I gene encodes an ALS I polypeptide containing leucine instead of tryptophan at a position corresponding to position 559 of said first ALS I polypeptide and wherein the ALS III gene encodes an ALS III polypeptide containing serine instead of proline at a position corresponding to position 179 and leucine instead of tryptophan at a position 556 of said ALS III polypeptide, or such as *B. napus* wherein the ALS I gene encodes an ALS I polypeptide containing serine instead of proline at a position corresponding to position 182 and leucine instead of tryptophan at a position corresponding to position 559 of said first ALS I polypeptide and wherein the ALS III gene encodes an ALS III polypeptide containing serine instead of proline at a position corresponding to position 179 and leucine instead of tryptophan at a position 556 of said ALS III polypeptide, or such as *B. juncea* wherein the ALS-A gene encodes an ALS-A polypeptide containing serine instead of proline at a position corresponding to position 179 and leucine instead of tryptophan at a position corresponding to position 556 of said first ALS-A polypeptide and wherein the ALS-B gene encodes an ALS-B polypeptide containing leucine instead of tryptophan at a position 559 of said ALS-B polypeptide, or such as *B*. *juncea* wherein the ALS-A gene encodes an ALS-A polypeptide containing leucine instead of tryptophan at a position corresponding to position 556 of said first ALS-A polypeptide and wherein the ALS-B gene encodes an ALS-B polypeptide containing serine instead of proline at a position corresponding to position 182 and leucine instead of tryptophan at a position 559 of said ALS-B polypeptide, or such as *B. juncea* wherein the ALS-A gene encodes an ALS-A polypeptide containing serine instead of proline at a position corresponding to position 179 and leucine instead of tryptophan at a position corresponding to position 556 of said first ALS-A polypeptide and wherein the ALS-B gene encodes an ALS-B polypeptide containing serine instead of proline at a position corresponding to position 182 and leucine instead of tryptophan at a position 559 of said ALS-B polypeptide, and thereby providing tolerance against the ALS inhibitor herbicide(s) belonging to group (B) is imazamox [CAS RN 114311-32-9] (= B1-2).

Another ALS inhibitor herbicide which is preferably used for control of unwanted vegetation in plant growing areas, such as *B. napus* or *B. juncea* growing areas whichplants, such as *B. napus* plants or *B. juncea* plants comprise mutations of its endogenous acetolactate synthase (ALS) genes, wherein at least two ALS genes encode an ALS polypeptide containing leucine instead of tryptophan at a position corresponding to position 574 of SEQ ID NO: 10,, and wherein at least one of the ALS genes encodes an ALS polypeptide which further comprises at a position corresponding to position 197 of SEQ ID NO: 10 instead of the naturally encoded amino acid proline the amino acid serine, such as *B. napus* wherein the ALS I gene encodes an ALS I polypeptide containing serine instead of proline at a position corresponding to position 182 and leucine instead of tryptophan at a position corresponding to position 559 of said first ALS I polypeptide and wherein the ALS III gene encodes an ALS III polypeptide containing leucine instead of tryptophan at a position 556 of said ALS III polypeptide, or such as *B*. *napus* wherein the ALS I gene encodes an ALS I polypeptide containing leucine instead of tryptophan at a position corresponding to position 559 of said first ALS I polypeptide and wherein the ALS III gene encodes an ALS III polypeptide containing serine instead of proline at a position corresponding to position 179 and leucine instead of tryptophan at a position 556 of said ALS III polypeptide, or such as *B. napus* wherein the ALS I gene encodes an ALS I polypeptide containing serine instead of proline at a position corresponding to position 182 and leucine instead of tryptophan at a position corresponding to position 559 of said first ALS I polypeptide and wherein the ALS III gene encodes an ALS III polypeptide containing serine instead of proline at a position corresponding to position 179 and leucine instead of tryptophan at a position 556 of said ALS III polypeptide, or such as *B. juncea* wherein the ALS-A gene encodes an ALS-A polypeptide containing serine instead of proline at a position corresponding to position 179 and leucine instead of tryptophan at a position corresponding to position 556 of said first ALS-A polypeptide and wherein the ALS-B gene encodes an ALS-B polypeptide containing leucine instead of tryptophan at a position 559 of said ALS-B polypeptide, or such as *B*. *juncea* wherein the ALS-A gene encodes an ALS-A polypeptide containing leucine instead of tryptophan at a position corresponding to position 556 of said first ALS-A polypeptide and wherein the ALS-B gene encodes an ALS-B polypeptide containing serine instead of proline at a position corresponding to position 182 and leucine instead of tryptophan at a position 559 of said ALS-B polypeptide, or such as *B. juncea* wherein the ALS-A gene encodes an ALS-A polypeptide containing serine instead of proline at a position corresponding to position 179 and leucine instead of tryptophan at a position corresponding to position 556 of said first ALS-A polypeptide and wherein the ALS-B gene encodes an ALS-B polypeptide containing serine instead of proline at a position corresponding to position 182 and leucine instead of tryptophan at a position 559 of said ALS-B polypeptide, and thereby providing tolerance against the ALS inhibitor herbicide(s) belonging to group (C) is bispyribac-sodium [CAS RN 125401-92-5] (= C1-1).

It is to be further understood that concerning all above defined ALS inhibitor herbicides and where not already specified by the respective CAS RN, all use forms, such as acids, and salts can be applied as described herein.

Additionally, the ALS inhibitor herbicide(s) that can be used may comprise further components, for example agrochemically active compounds of a different type of mode of action and/or the formulation auxiliaries and/or additives customary in crop protection, or may be used together with these.

Suitable herbicide combinations that can be used comprise effective amounts of the ALS inhibitor herbicide(s) belonging to groups (A), (B) and/or (C) and/or have synergistic actions. The synergistic actions can be observed, for example, when applying one or more ALS inhibitor herbicide(s) belonging to groups (A), (B), and/or (C) together, for example as a coformulation or as a tank mix; however, they can also be observed when the active compounds are applied at different times (splitting). It is also possible to apply the herbicides or the herbicide combinations in a plurality of portions (sequential application), for example pre-emergence applications followed by post-emergence applications or early post-emergence applications followed by medium or late post-emergence applications. Preference is given here to the joint or almost simultaneous application of the ALS-inhibitor herbicides belonging to groups (A), (B) and/or (C) of the combination in question.

The synergistic effects permit a reduction of the application rates of the individual ALS inhibitor herbicides, a higher efficacy at the same application rate, the control of species which were as yet uncontrolled (gaps), control of species which are tolerant or resistant to individual ALS inhibitor herbicides or to a number of ALS inhibitor herbicides, an extension of the period of application and/or a reduction in the number of individual applications required and - as a result for the user - weed control systems which are more advantageous economically and ecologically.

The herbicides that can be used are all acetolactate synthase (ALS) inhibitor herbicides and thus inhibit protein biosynthesis in plants.

The application rate of the ALS inhibitor herbicides belonging to groups (A), (B) or (C) (as defined above) can vary within a wide range, for example between 0.001 g and 1500 g of ai/ha (ai/ha means here and below "active substance per hectare" = based on 100% pure active compound). Applied at application rates of from 0.001 g to 1500 g of ai/ha, the herbicides belonging to classes A, B and C, preferably the compounds A1-1; A1-4; A1-9; A1-12; A1-13; A1-16; A1-17; A1-18; A1-20; A1-26; A1-28; A1-29; A1-31; A1-41; A2-2; A3-3; A3-5; A3-7, control, when used by the pre- and post-emergence method, a relatively wide spectrum of harmful plants, for example of annual and perennial mono- or dicotyledonous weeds, and also of unwanted crop plants (together also defined as "unwanted vegetation).

In many applications, the application rates are generally lower, for example in the range of from 0.001 g to 1000 g of ai/ha, preferably from 0.1 g to 500 g of ai/ha, particularly preferably from 0.5 g to 250 g of ai/ha, and even more preferably 1.0 g to 200 g of ai/ha. In cases where the application of several ALS inhibitor herbicides is conducted, the quantity represents the total quantity of all of the applied ALS inhibitor herbicides.

For example, the combinations of ALS inhibitor herbicides (belonging to groups (A), (B) and/or (C)) allow the activity to be enhanced synergistically in a manner which, by far and in an unexpected manner, exceeds the activities which can be achieved using the individual ALS inhibitor herbicides (belonging to groups (A), (B) and/or (C)).

For combinations of ALS inhibitor herbicides, the preferred conditions are illustrated below.

Of particular interest is the use of herbicidal compositions for control of unwanted vegetation in polyploid plants, such as *B. napus* or *B. juncea* plants, preferably in mutated plants as described herein having a content of the following ALS inhibitor herbicides:
(A1-1) + (A1-9); (A1-1) + (A1-12); (A1-1) + (A1-13); (A1-1) + (A1-16); (A1-1) + (A1-17); (A1-1) + (A1-18); (A1-1) + (A1-20); (A1-1) + (A1-26); (A1-1) + (A1-28); (A1-1) +(A1-29); (A1-1) + (A1-31); (A1-1) + (A1-41); (A1-1) + (A2-2); (A1-1) +(A2-3); (A1-1) + (A3-3); (A1-1) + (A3-5); (A1-1) + (A3-7); (A1-1) + (B1-2); (A1-1) + (C1-1);
(A1-9) + (A1-12); (A1-9) + (A1-13); (A1-9) + (A1-16); (A1-9) + (A1-17); (A1-9) + (A1-18); (A1-9) + (A1-20); (A1-9) + (A1-26); (A1-9) + (A1-28); (A1-9) +(A1-29); (A1-9) + (A1-31); (A1-9) + (A1-41); (A1-9) + (A2-2); (A1-9) +(A2-3); (A1-9) + (A3-3); (A1-9) + (A3-5); (A1-9) + (A3-7); (A1-9) + (B1-2); (A1-9) + (C1-1);
(A1-12) + (A1-13); (A1-12) + (A1-16); (A1-12) + (A1-17); (A1-12) + (A1-18); (A1-12) + (A1-20); (A1-12) + (A1-26); (A1-12) + (A1-28); (A1-12) +(A1-29); (A1-12) + (A1-31); (A1-12) + (A1-41); (A1-12) + (A2-2); (A1-12) +(A2-3); (A1-12) + (A3-3); (A1-12) + (A3-5); (A1-12) + (A3-7); (A1-12) + (B1-2); (A1-12) + (C1-1);
(A1-13) + (A1-16); (A1-13) + (A1-17); (A1-13) + (A1-18); (A1-13) + (A1-20); (A1-13) + (A1-26); (A1-13) + (A1-28); (A1-13) +(A1-29); (A1-13) + (A1-31); (A1-13) + (A1-41); (A1-13) + (A2-2); (A1-13) +(A2-3); (A1-13) + (A3-3); (A1-13) + (A3-5); (A1-13) + (A3-7); (A1-13) + (B1-2); (A1-13) + (C1-1);
(A1-16) + (A1-17); (A1-16) + (A1-18); (A1-16) + (A1-20); (A1-16) + (A1-26); (A1-16) + (A1-28); (A1-16) +(A1-29); (A1-16) + (A1-31); (A1-16) + (A1-41); (A1-16) + (A2-2); (A1-16) +(A2-3); (A1-16) + (A3-3); (A1-16) + (A3-5); (A1-16) + (A3-7); (A1-16) + (B1-2); (A1-16) + (C1-1);
(A1-17) + (A1-18); (A1-17) + (A1-20); (A1-17) + (A1-26); (A1-17) + (A1-28); (A1-17) +(A1-29); (A1-17) + (A1-31); (A1-17) + (A1-41); (A1-17) + (A2-2); (A1-17) +(A2-3); (A1-17) + (A3-3); (A1-17) + (A3-5); (A1-17) + (A3-7); (A1-17) + (B1-2); (A1-17) + (C1-1);
(A1-18) + (A1-20); (A1-18) + (A1-26); (A1-18) + (A1-28); (A1-18) +(A1-29); (A1-18) + (A1-31); (A1-18) + (A1-41); (A1-18) + (A2-2); (A1-18) +(A2-3); (A1-18) + (A3-3); (A1-18) + (A3-5); (A1-18) + (A3-7); (A1-18) + (B1-2); (A1-18) + (C1-1);
(A1-20) + (A1-26); (A1-20) + (A1-28); (A1-20) +(A1-29); (A1-20) + (A1-31); (A1-20) + (A1-41); (A1-20) + (A2-2); (A1-20) +(A2-3); (A1-20) + (A3-3); (A1-20) + (A3-5); (A1-20) + (A3-7); (A1-20) + (B1-2); (A1-20) + (C1-1);
(A1-26) + (A1-28); (A1-26) +(A1-29); (A1-26) + (A1-31); (A1-26) + (A1-41); (A1-26) + (A2-2); (A1-26) +(A2-3); (A1-26) + (A3-3); (A1-26) + (A3-5); (A1-26) + (A3-7); (A1-26) + (B1-2); (A1-26) + (C1-1);
(A1-28) +(A1-29); (A1-28) + (A1-31); (A1-28) + (A1-41); (A1-28) + (A2-2); (A1-28) +(A2-3); (A1-28) + (A3-3); (A1-28) + (A3-5); (A1-28) + (A3-7); (A1-28) + (B1-2); (A1-28) + (C1-1);
(A1-29) + (A1-31); (A1-29) + (A1-41); (A1-29) + (A2-2); (A1-29) +(A2-3); (A1-29) + (A3-3); (A1-29) + (A3-5); (A1-29) + (A3-7); (A1-29) + (B1-2); (A1-29) + (C1-1);
(A1-31) + (A1-41); (A1-31) + (A2-2); (A1-31) +(A2-3); (A1-31) + (A3-3); (A1-31) + (A3-5); (A1-31) + (A3-7); (A1-31) + (B1-2); (A1-31) + (C1-1);
(A1-41) + (A2-2); (A1-41) +(A2-3); (A1-41) + (A3-3); (A1-41) + (A3-5); (A1-41) + (A3-7); (A1-41) + (B1-2); (A1-41) + (C1-1);
(A2-2) +(A2-3); (A2-2) + (A3-3); (A2-2) + (A3-5); (A2-2) + (A3-7); (A2-2) + (B1-2); (A2-2) + (C1-1);
(A2-3) + (A3-3); (A2-3) + (A3-5); (A2-3) + (A3-7); (A2-3) + (B1-2); (A2-3) + (C1-1);
(A3-3) + (A3-5); (A3-3) + (A3-7); (A3-3) + (B1-2); (A3-3) + (C1-1);
(A3-5) + (A3-7); (A3-5) + (B1-2); (A3-5) + (C1-1);
(A3-7) + (B1-2); (A3-7) + (C1-1);
(B1-2) + (C1-1).

Additionally, the ALS inhibitor herbicides that can be used may comprise further components, for example agrochemically active compounds of a different type of mode of action and/or the formulation auxiliaries and/or additives customary in crop protection, or may be used together with these.

The ALS inhibitor herbicide(s) that can be used or combinations of various such ALS inhibitor herbicides may furthermore comprise various agrochemically active compounds, for example from the group of the safeners, fungicides, insecticides, or from the group of the formulation auxiliaries and additives customary in crop protection.

Also disclosed is the use of effective amounts of ALS inhibitor herbicide(s) (i.e. members of the groups (A), (B) and/or (C)) and non-ALS inhibitor herbicides (i.e. herbicides showing a mode of action that is different to the inhibition of the ALS enzyme [acetohydroxyacid synthase; EC 2.2.1.6] (group B herbicides) in order obtain synergistic effect for the control of unwanted vegetation. Such synergistic actions can be observed, for example, when applying one or more ALS inhibitor herbicides (i.e. members of the groups (A), (B), and/or (C)) and one or more non ALS inhibitor herbicides (group B herbicides) together, for example as a coformulation or as a tank mix; however, they can also be observed when the active compounds are applied at different times (splitting). It is also possible to apply the ALS inhibitor herbicides and non ALS inhibitor herbicides in a plurality of portions (sequential application), for example pre-emergence applications followed by post-emergence applications or early post-emergence applications followed by medium or late post-emergence applications. Preference is given here to the joint or almost simultaneous application of the herbicides ((A), (B) and/or (C)) and (D) of the combination in question.

Suitable partner herbicides to be applied together with ALS inhibitor herbicideds are, for example, the following herbicides which differ structurally from the herbicides belonging to the groups (A), (B), and (C) as defined above, preferably herbicidally active compounds whose action is based on inhibition of, for example, acetyl coenzyme A carboxylase, PS I, PS II, HPPDO, phytoene desaturase, protoporphyrinogen oxidase, glutamine synthetase, cellulose biosynthesis, 5-enolpyruvylshikimate 3-phosphate synthetase, as described, for example, in Weed Research 26, 441-445 (1986), or "The Pesticide Manual", 14th edition, The British Crop Protection Council, 2007, or 15th edition 2010, or in the corresponding "e-Pesticide Manual", Version 5 (2010), in each case published by the British Crop Protection Council, (hereinbelow in short also "PM"), and in the literature cited therein. Lists of common names are also available in "The Compendium of Pesticide Common Names" on the internet. Herbicides known from the literature (in brackets behind the common name hereinafter also classified by the indicators D1 to D426), which can be combined with ALS-inhibitor herbicides of groups (A), (B) and/or (C) and to be used are, for example, the active compounds listed below: (note: the herbicides are referred to either by the "common name" in accordance with the International Organization for Standardization (ISO) or by the chemical name, together where appropriate with a customary code number, and in each case include all use forms, such as acids, salts, esters and isomers, such as stereoisomers and optical isomers, in particular the commercial form or the commercial forms, unless the context indicates otherwise. The citation given is of one use form and in some cases of two or more use forms):
acetochlor (= D1), acibenzolar (= D2), acibenzolar-S-methyl (= D3), acifluorfen (= D4), acifluorfen-sodium (= D5), aclonifen (= D6), alachlor (= D7), allidochlor (= D8), alloxydim (= D9), alloxydim-sodium (= D10), ametryn (= D11), amicarbazone (= D12), amidochlor (= D13), aminocyclopyrachlor (= D14), aminopyralid (= D15), amitrole (= D16), ammonium sulfamate (= D17), ancymidol (= D18), anilofos (= D19), asulam (= D20), atrazine (= D21), azafenidin (= D22), aziprotryn (= D23), beflubutamid (= D24), benazolin (= D25), benazolin-ethyl (= D26), bencarbazone (= D27), benfluralin (= D28), benfuresate (= D29), bensulide (= D30), bentazone (= D31), benzfendizone (= D32), benzobicyclon (= D33), benzofenap (= D34), benzofluor (= D35), benzoylprop (= D36), bicyclopyrone (= D37), bifenox (= D38), bilanafos (= D39), bilanafos-sodium (= D40), bromacil (= D41), bromobutide (= D42), bromofenoxim (= D43), bromoxynil (= D44), bromuron (= D45), buminafos (= D46), busoxinone (= D47), butachlor (= D48), butafenacil (= D49), butamifos (= D50), butenachlor (= D51), butralin (= D52), butroxydim (= D53), butylate (= D54), cafenstrole (= D55), carbetamide (= D56), carfentrazone (= D57), carfentrazone-ethyl (= D58), chlomethoxyfen (= D59), chloramben (= D60), chlorazifop (= D61), chlorazifop-butyl (= D62), chlorbromuron (= D63), chlorbufam (= D64), chlorfenac (= D65), chlorfenac-sodium (= D66), chlorfenprop (= D67), chlorflurenol (= D68), chlorflurenol-methyl (= D69), chloridazon (= D70), chlormequat-chloride (= D71), chlornitrofen (= D72), chlorophthalim (= D73), chlorthal-dimethyl (= D74), chlorotoluron (= D75), cinidon (= D76), cinidon-ethyl (= D77), cinmethylin (= D78), clethodim (= D79), clodinafop (= D80), clodinafop-propargyl (= D81), clofencet (= D82), clomazone (= D83), clomeprop (= D84), cloprop (= D85), clopyralid (= D86), cloransulam (= D87), cloransulam-methyl (= D88), cumyluron (= D89), cyanamide (= D90), cyanazine (= D91), cyclanilide (= D92), cycloate (= D93), cycloxydim (= D94), cycluron (= D95), cyhalofop (= D96), cyhalofop-butyl (= D97), cyperquat (= D98), cyprazine (= D99), cyprazole (= D100), 2,4-D (= D101), 2,4-DB (= D102), daimuron/dymron (= D103), dalapon (= D104), daminozide (= D105), dazomet (= D106), n-decanol (= D-107), desmedipham (= D108), desmetryn (= D109), detosyl-pyrazolate (= D110), diallate (= D111), dicamba (= D112), dichlobenil (= D113), dichlorprop (= D114), dichlorprop-P (= D115), diclofop (= D116), diclofop-methyl (= D117), diclofop-P-methyl (= D118), diethatyl (= D119), diethatyl-ethyl (= D120), difenoxuron (= D121), difenzoquat (= D122), diflufenican (= D123), diflufenzopyr (= D124), diflufenzopyr-sodium (= D125), dimefuron (= D126), dikegulac-sodium (= D127), dimefuron (= D128), dimepiperate (= D129), dimethachlor (= D130), dimethametryn (= D131), dimethenamid (= D132), dimethenamid-P (= D133), dimethipin (= D134), dimetrasulfuron (= D135), dinitramine (= D136), dinoseb (= D137), dinoterb (= D138), diphenamid (= D139), dipropetryn (= D140), diquat (= D141), diquat-dibromide (= D142), dithiopyr (= D143), diuron (= D144), DNOC (= D145), eglinazine-ethyl (= D146), endothal (= D147), EPTC (= D148), esprocarb (= D149), ethalfluralin (= D150), ethephon (= D151), ethidimuron (= D152), ethiozin (= D153), ethofumesate (= D154), ethoxyfen (= D155), ethoxyfen-ethyl (= D156), etobenzanid (= D157), F-5331 (= 2-Chlor-4-fluor-5-[4-(3-fluorpropyl)-4,5-dihydro-5-oxo-1H-tetrazol-1-yl]-phenyl]-ethansulfonamid) (= D158), F-7967 (=3-[7-Chlor-5-fluor-2-(trifluormethyl)-1H-benzimidazol-4-yl]-1-methyl-6-(trifluonnethyl)pyrimidin-2,4(1H,3H)-dion) (= D159), fenoprop (= D160), fenoxaprop (= D161), fenoxaprop-P (= D162), fenoxaprop-ethyl (= D163), fenoxaprop-P-ethyl (= D164), fenoxasulfone (= D165), fentrazamide (= D166), fenuron (= D167), flamprop (= D168), flamprop-M-isopropyl (= D169), flamprop-M-methyl (= D170), fluazifop (= D171), fluazifop-P (= D172), fluazifop-butyl (= D173), fluazifop-P-butyl (= D174), fluazolate (= D175), fluchloralin (= D176), flufenacet (thiafluamide) (= D177), flufenpyr (= D178), flufenpyr-ethyl (= D179), flumetralin (= D180), flumiclorac (= D 181), flumiclorac-pentyl (= D182), flumioxazin (= D183), flumipropyn (= D184), fluometuron (= D185), fluorodifen (= D186), fluoroglycofen (= D187), fluoroglycofen-ethyl (= D188), flupoxam (= D189), flupropacil (= D190), flupropanate (= D191), flurenol (= D192), flurenol-butyl (= D193), fluridone (= D194), flurochloridone (= D195), fluroxypyr (= D196), fluroxypyr-meptyl (= D197), flurprimidol (= D198), flurtamone (= D199), fluthiacet (= D200), fluthiacet-methyl (= D201), fluthiamide (= D202), fomesafen (= 203), forchlorfenuron (= D204), fosamine (= D205), furyloxyfen (= D206), gibberellic acid (= D207), glufosinate (= D208), glufosinate-ammonium (= D209), glufosinate-P (= D210), glufosinate-P-ammonium (= D211), glufosinate-P-sodium (= D212), glyphosate (= D213), glyphosate-isopropylammonium (= D214), H-9201 (=O-(2,4-Dimethyl-6-nitrophenyl)-O-ethyl-isopropylphosphoramidothioat) (= D215), halosafen (= D216), haloxyfop (= D217), haloxyfop-P (= D218), haloxyfop-ethoxyethyl (= D219), haloxyfop-P-ethoxyethyl (= D220), haloxyfop-methyl (= D221), haloxyfop-P-methyl (= D222), hexazinone (= D223), HW-02 (= 1-(Dimethoxyphosphoryl)-ethyl(2,4-dichlorphenoxy)acetate) (= D224), inabenfide (= D225), indanofan (= D226), indaziflam (= D227), indol-3-acetic acid (IAA) (= D228), 4-indol-3-ylbutyric acid (IBA) (= D229), ioxynil (= D230), ipfencarbazone (= D231), isocarbamid (= D232), isopropalin (= D233), isoproturon (= D234), isouron (= D235), isoxaben (= D236), isoxachlortole (= D237), isoxaflutole (= D238), isoxapyrifop (= D239), KUH-043 (= 3-({[5-(Difluormethyl)-1-methyl-3-(trifluormethyl)-1H-pyrazol-4-yl]methyl}sulfonyl)-5,5-dimethyl-4,5-dihydro-1,2-oxazol) (= D240), karbutilate (= D241), ketospiradox (= D242), lactofen (= D243), lenacil (= D244), linuron (= D245), male ic hydrazide (= D246), MCPA (= D247), MCPB (= D248), MCPB-methyl, -ethyl and -sodium (= D249), mecoprop (= D250), mecoprop-sodium (= D251), mecoprop-butotyl (= D252), mecoprop-P-butotyl (= D253), mecoprop-P-dimethylammonium (= D254), mecoprop-P-2-ethylhexyl (= D255), mecoprop-P-potassium (= D256), mefenacet (= D257), mefluidide (= D258), mepiquat-chloride (= D259), mesotrione (= D260), methabenzthiazuron (= D261), metam (= D262), metamifop (= D263), metamitron (= D264), metazachlor (= D265), metazole (= D266), methiopyrsulfuron (= D267), methiozolin (= D268), methoxyphenone (= D269), methyldymron (= D270), 1-methylcyclopropen (= D271), methylisothiocyanat (= D272), metobenzuron (= D273), metobromuron (= D274), metolachlor (= D275), S-metolachlor (= D-276), metoxuron (= D277), metribuzin (= D278), molinate (= D279), monalide (= D280), monocarbamide (= D281), monocarbamide-dihydrogensulfate (= D282), monolinuron (= D283), monosulfuron-ester (= D284), monuron (= D285), MT-128 (= 6-Chlor-N-[(2E)-3-chlorprop-2-en-1-yl]-5-methyl-N-phenylpyridazin-3-amine) (= D286), MT-5950 (= N-[3-Chlor-4-(1-methylethyl)-phenyl]-2-methylpentanamide) (= D287), NGGC-011 (= D288), naproanilide (= D289), napropamide (= D290), naptalam (= D291), NC-310 (= 4-(2,4-Dichlorobenzoyl)-1-methyl-5-benzyloxypyrazole) (= D292), neburon (= D293), nipyraclofen (= D294), nitralin (= D295), nitrofen (= D296), nitrophenolat-sodium (isomer mixture) (= D297), nitrofluorfen (= D298), nonanoic acid (= D299), norflurazon (= D300), orbencarb (= D301), oryzalin (= D302), oxadiargyl (= D303), oxadiazon (= D304), oxaziclomefone (= D305), oxyfluorfen (= D306), paclobutrazol (= D307), paraquat (= D308), paraquat-dichloride (= D309), pelargonic acid (nonanoic acid) (= D310), pendimethalin (= D311), pendralin (= D312), pentanochlor (= D313), pentoxazone (= D314), perfluidone (= D315), pethoxamid (= D317), phenisopham (= D318), phenmedipham (= D319), phenmedipham-ethyl (= D320), picloram (= D321), picolinafen (= D322), pinoxaden (= D323), piperophos (= D324), pirifenop (= D325), pirifenop-butyl (= D326), pretilachlor (= D327), probenazole (= D328), profluazol (= D329), procyazine (= D330), prodiamine (= D331), prifluraline (= D332), profoxydim (= D333), prohexadione (= D334), prohexadione-calcium (= D335), prohydrojasmone (= D336), prometon (= D337), prometryn (= D338), propachlor (= D339), propanil (= D340), propaquizafop (= D341), propazine (= D342), propham (= D343), propisochlor (= D344), propyzamide (= D345), prosulfalin (= D346), prosulfocarb (= D347), prynachlor (= D348), pyraclonil (= D349), pyraflufen (= D350), pyraflufen-ethyl (= D351), pyrasulfotole (= D352), pyrazolynate (pyrazolate) (= D353), pyrazoxyfen (= D354), pyribambenz (= D355), pyributicarb (= D356), pyridafol (= D357), pyridate (= D358), pyriminobac (= D359), pyrimisulfan (= D360), pyroxasulfone (= D361), quinclorac (= D362), quinmerac (= D363), quinoclamine (= D364), quizalofop (= D365), quizalofop-ethyl (= D366), quizalofop-P (= D367), quizalofop-P-ethyl (= D368), quizalofop-P-tefuryl (= D369), saflufenacil (= D370), secbumeton (= D371), sethoxydim (= D372), siduron (= D373), simazine (= D374), simetryn (= D375), SN-106279 (= Methyl-(2R)-2-({7-[2-chlor-4-(trifluormethyl)phenoxy]-2-naphthyl}oxy)-propanoate) (= D376), sulcotrione (= D377), sulfallate (CDEC) (= D378), sulfentrazone (= D379), sulfosate (glyphosate-trimesium) (= D380), SYN-523 (= D381), SYP-249 (= 1-Ethoxy-3-methyl-1-oxobut-3-en-2-yl-5-[2-chlor-4-(trifluormethyl)phenoxy]-2-nitrobenzoate) (= D382), tebutam (= D383), tebuthiuron (= D384), tecnazene (= D385), tefuryltrione (= D386), tembotrione (= D387), tepraloxydim (= D388), terbacil (= D389), terbucarb (= D390), terbuchlor (= D391), terbumeton (= D392), terbuthylazine (= D393), terbutryn (= D394), thenylchlor (= D395), thiafluamide (= D396), thiazafluron (= D397), thiazopyr (= D398), thidiazimin (= D399), thidiazuron (= D400), thiobencarb (= D401), tiocarbazil (= D402), topramezone (= D403), tralkoxydim (= D404), triallate (= D405), triaziflam (= D406), triazofenamide (= D407), trichloracetic acid (TCA) (= D408), triclopyr (= D409), tridiphane (= D410), trietazine (= D411), trifluralin (=D412), trimeturon (= D413), trinexapac (= D414), trinexapac-ethyl (= D415), tsitodef (= D416), uniconazole (= D417), uniconazole-P (= D418), vernolate (= D419), ZJ-0862 (= 3,4-Dichlor-N-{2-[(4,6-dimethoxypyrimidin-2-yl)oxy]benzyl}anilne) (= D420), the below compounds defined by their chemical structure, respectively: 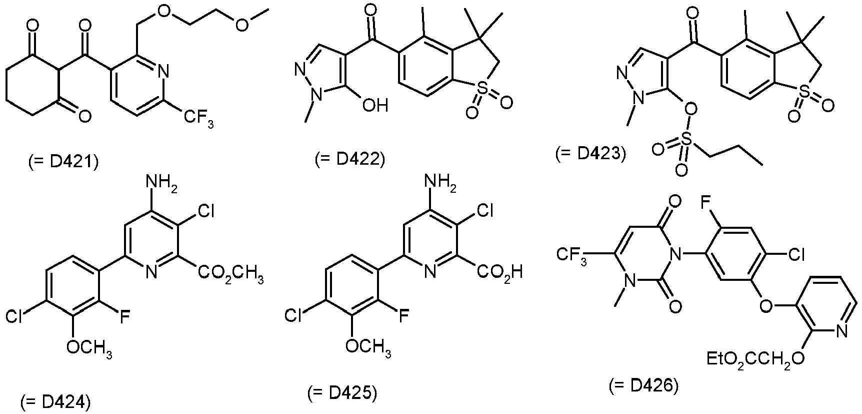 and propachlor (D 427).

Preferably, further herbicides which differ structurally and via their mode of action from the ALS inhibitor herbicides belonging to the groups (A), (B), and (C) as defined above which can be applied for control of unwanted vegetation in ALS inhibitor herbicide tolerant *B. napus* plants, preferably in mutated *B. napus* plants as described herein. In connection with ALS inhibitor herbicides belonging to the groups (A), (B), and (C) are those selected from the group consisting of acetochlor (= D1), carbetamide (= D56), fenoxaprop-P-ethyl (= D164), fluazifop-P-butyl (= D174), haloxyfop-P-methyl (= D222), metolachlor (= D275), dimethenamid (= D132), napropamide (= D290), pethoxamid (= D317), propaquizafop (= D341), propisochlor (= D344), propyzamide (= D345), quinmerac (= D363), propachlor (D 427), clomazone (= D83), clopyralid (= D86), dimethachlor (= D130), metazachlor (= D265), picloram (= D321), and quizalofop-P-ethyl (= D368).

Even more preferably, further herbicides which differ from the ALS inhibitor herbicides belonging to the groups (A), (B), and (C) as defined above and which can be applied in connection with ALS inhibitor herbicides belonging to the groups (A), (B), and (C) are those selected from the group consisting of clomazone (= D83), clopyralid (= D86), dimethachlor (= D130), metazachlor (= D265), picloram (= D321), and quizalofop-P-ethyl (= D368).

Mixtures containing ALS inhibitor herbicides and non ALS inhibitor herbicides, compositions comprising mixtures of one or more ALS inhibitor herbicide(s) (compounds belonging to one or more of groups (A), (B) and (C)) and non ALS inhibitor heribicide(s) (group (D) members; as defined above) that are of very particular interest in order to be used for control of unwanted vegetation are:
(A1-1) + (D83); (A1-1) + (D86); (A1-1) + (D130); (A1-1) + (D265); (A1-1) + (D321); (A1-1) + (D368);
(A1-9) + (D83); (A1-9) + (D86); (A1-9) + (D130); (A1-9) + (D265); (A1-9) + (D321); (A1-9) + (D368);
(A1-12) + (D83); (A1-12) + (D86); (A1-12) + (D130); (A1-12) + (D265); (A1-12) + (D321); (A1-12) + (D368);
(A1-13) + (D83); (A1-13) + (D86); (A1-13) + (D130); (A1-13) + (D265); (A1-13) + (D321); (A1-13) + (D368);
(A1-16) + (D83); (A1-16) + (D86); (A1-16) + (D130); (A1-16) + (D265); (A1-16) + (D321); (A1-16) + (D368);
(A1-17) + (D83); (A1-17) + (D86); (A1-17) + (D130); (A1-17) + (D265); (A1-17) + (D321); (A1-17) + (D368);
(A1-18) + (D83); (A1-18) + (D86); (A1-18) + (D130); (A1-18) + (D265); (A1-18) + (D321); (A1-18) + (D368);
(A1-20) + (D83); (A1-20) + (D86); (A1-20) + (D130); (A1-20) + (D265); (A1-20) + (D321); (A1-20) + (D368);
(A1-26) + (D83); (A1-26) + (D86); (A1-26) + (D130); (A1-26) + (D265); (A1-26) + (D321); (A1-26) + (D368);
(A1-28) + (D83); (A1-28) + (D86); (A1-28) + (D130); (A1-28) + (D265); (A1-28) + (D321); (A1-28) + (D368);
(A1-29) + (D83); (A1-29) + (D86); (A1-29) + (D130); (A1-29) + (D265); (A1-29) + (D321); (A1-29) + (D368);
(A1-31) + (D83); (A1-31) + (D86); (A1-31) + (D130); (A1-31) + (D265); (A1-31) + (D321); (A1-31) + (D368);
(A1-41) + (D83); (A1-41) + (D86); (A1-41) + (D130); (A1-41) + (D265); (A1-41) + (D321); (A1-41) + (D368);
(A2-2) + (D83); (A2-2) + (D86); (A2-2) + (D130); (A2-2) + (D265); (A2-2) + (D321); (A2-2) + (D368);
(A2-3) + (D83); (A2-3) + (D86); (A2-3) + (D130); (A2-3) + (D265); (A2-3) + (D321); (A2-3) + (D368);
(A3-3) + (D83); (A3-3) + (D86); (A3-3) + (D130); (A3-3) + (D265); (A3-3) + (D321); (A3-3) + (D368);
(A3-5) + (D83); (A3-5) + (D86); (A3-5) + (D130); (A3-5) + (D265); (A3-5) + (D321); (A3-5) + (D368);
(A3-7) + (D83); (A3-7) + (D86); (A3-7) + (D130); (A3-7) + (D265); (A3-7) + (D321); (A3-7) + (D368);
(A4-1) + (D83); (A4-1) + (D86); (A4-1) + (D130); (A4-1) + (D265); (A4-1) + (D321); (A4-1) + (D368);
(A4-2) + (D83); (A4-2) + (D86); (A4-2) + (D130); (A4-2) + (D265); (A4-2) + (D321); (A4-2) + (D368);
(A4-3) + (D83); (A4-3) + (D86); (A4-3) + (D130); (A4-3) + (D265); (A4-3) + (D321); (A4-3) + (D368);
(A4-2) + (D83); (A4-2) + (D86); (A4-2) + (D130); (A4-2) + (D265); (A4-2) + (D321); (A4-2) + (D368);
(B1-2) + (D83); (B1-2) + (D86); (B1-2) + (D130); (B1-2) + (D265); (B1-2) + (D321); (B1-2) + (D368);
(C1-1) + (D83); (C1-1) + (D86); (C1-1) + (D130); (C1-1) + (D265); (C1-1) + (D321); (C1-1) + (D368).

The application of ALS inhibitor herbicides also act efficiently on perennial weeds which produce shoots from rhizomes, root stocks and other perennial organs and which are difficult to control. Here, the substances can be applied, for example, by the pre-sowing method, the pre-emergence method or the post-emergence method, for example jointly or separately. Preference is given, for example, to application by the post-emergence method, in particular to the emerged harmful plants.

Specific examples may be mentioned of some representatives of the monocotyledonous and dicotyledonous weed flora which can be controlled by the ALS inhibitor herbicides, without the enumeration being restricted to certain species.

Examples of weed species on which the application as described herein acts efficiently are, from amongst the monocotyledonous weed species, *Avena spp., Alopecurus spp., Apera spp., Brachiaria spp., Bromus spp., Digitaria spp., Lolium spp., Echinochloa spp., Panicum spp., Phalaris spp., Poa spp., Setaria spp.,* volunteer cereals (*Triticum sp., Hordeum sp.*) and also Cyperus species from the annual group, and, among the perennial species, Agropyron, Cynodon, Imperata and Sorghum and also perennial Cyperus species.

In the case of the dicotyledonous weed species, the spectrum of action extends to genera such as, for example, *Aethusa spp., Amaranthus spp., Capsella spp, Centaurea spp., Chenopodium spp., Chrysanthemum spp., Galium spp., Geranium spp., Lamium spp., Matricaria spp., Myosotis spp., Papaver spp., Polygonum spp., Sinapis spp., Solanum spp., Stellaria spp., Thlaspi spp., Urtica spp., Veronica spp.* and *Viola spp., Xanthium spp.,* among the annuals, and Convolvulus, Cirsium, Rumex and Artemisia in the case of the perennial weeds.

Also described is a polyploid *Brassica* plant, such as *B. napus* or *B. juncea,* plant as described herein to which one or more ALS inhibitor herbicide(s) alone or in combination with one or more herbicide(s) that do(es) not belong to the class of ALS inhibitor herbicides are applied for control of unwanted vegetation in polyploid plant, such as *Brassica* plant, such as *B. napus* or *B. juncea,* plant comprising two ALS polypeptides containing leucine instead of tryptophan at a position of said ALS polypeptide corresponding to position 574 of SEQ ID NO: 10, and wherein at least one of the ALS genes encodes an ALS polypeptide which further comprises at a position corresponding to position 197 of SEQ ID NO: 10 instead of the naturally encoded amino acid proline the amino acid serine, such as an ALS I polypeptide containing serine instead of proline at a position corresponding to position 197 and leucine instead of tryptophan at a position corresponding to position 574 of SEQ ID NO: 10 of said ALS I *Brassica,* such as *B. napus,* polypeptide, and an ALS III polypeptide containing leucine instead of tryptophan at a position corresponding to position 574 of SEQ ID NO: 10 of said ALS III *Brassica,* such as *B. napus* polypeptide, or such as an ALS I polypeptide containing leucine instead of tryptophan at a position corresponding to position 574 of SEQ ID NO: 10 of said ALS I *Brassica,* such as *B. napus,* polypeptide, and an ALS III polypeptide containing serine instead of proline at a position corresponding to position 197 and leucine instead of tryptophan at a position corresponding to position 574 of SEQ ID NO: 10 of said ALS III *Brassica,* such as *B. napus* polypeptide, or such as an ALS I polypeptide containing serine instead of proline at a position corresponding to position 197 and leucine instead of tryptophan at a position corresponding to position 574 of SEQ ID NO: 10 of said ALS I *Brassica,* such as *B. napus,* polypeptide, and an ALS III polypeptide containing serine instead of proline at a position corresponding to position 197 and leucine instead of tryptophan at a position corresponding to position 574 of SEQ ID NO: 10 of said ALS III *Brassica,* such as *B. napus* polypeptide, or such as an ALS-A polypeptide containing serine instead of proline at a position corresponding to position 197 and leucine instead of tryptophan at a position corresponding to position 574 of SEQ ID NO: 10 of said ALS-A *Brassica,* such as *B. juncea,* polypeptide, and an ALS-B polypeptide containing leucine instead of tryptophan at a position corresponding to position 574 of SEQ ID NO: 10 of said ALS-B *Brassica,* such as *B. juncea* polypeptide, or such as an ALS-A polypeptide containing leucine instead of tryptophan at a position corresponding to position 574 of SEQ ID NO: 10 of said ALS-A *Brassica,* such as *B. juncea,* polypeptide, and an ALS-B polypeptide containing serine instead of proline at a position corresponding to position 197 and leucine instead of tryptophan at a position corresponding to position 574 of SEQ ID NO: 10 of said ALS-B *Brassica,* such as *B. juncea* polypeptide, or such as an ALS-A polypeptide containing serine instead of proline at a position corresponding to position 197 and leucine instead of tryptophan at a position corresponding to position 574 of SEQ ID NO: 10 of said ALS-A *Brassica,* such as *B. juncea,* polypeptide, and an ALS-B polypeptide containing serine instead of proline at a position corresponding to position 197 and leucine instead of tryptophan at a position corresponding to position 574 of SEQ ID NO: 10 of said ALS-B *Brassica,* such as *B. juncea* polypeptide.

Also described is an allotetraploid *Brassica* plant, such as *B. napus* or *B. juncea* plant as described herein to which one or more ALS inhibitor herbicide(s) alone or in combination with one or more herbicide(s) that do(es) not belong to the class of ALS inhibitor herbicides are applied for control of unwanted vegetation in polyploid plant, such as *Brassica,* such as *B. napus* or *B. juncea,* plant comprising mutations of at least two endogenous acetolactate synthase (ALS) genes, wherein said gene encodes an ALS polypeptide containing leucine instead of tryptophan at a position corresponding to position 574 of SEQ ID NO: 10, and wherein at least one of the ALS genes encodes an ALS polypeptide which further comprises at a position corresponding to position 197 of SEQ ID NO: 10 instead of the naturally encoded amino acid proline the amino acid serine, such as *Brassica,* such as B. *napus,* genes, wherein the ALS I *Brassica,* such as *B. napus,* gene encodes an ALS I *Brassica,* such as *B. napus,* polypeptide containing serine instead of proline at a position corresponding to position 197 of SEQ ID NO: 10 and leucine instead of tryptophan at a position corresponding to position 574 of SEQ ID NO: 10 and wherein the ALS III *Brassica,* such as *B. napus,* gene encodes an ALS III *Brassica,* such as *B. napus,* polypeptide containing leucine instead of tryptophan at a position corresponing to poistion 574 of SEQ ID NO: 10, or such as *B. napus,* gene encodes an ALS I *Brassica,* such as *B. napus,* polypeptide containing leucine instead of tryptophan at a position corresponding to position 574 of SEQ ID NO: 10 and wherein the ALS III *Brassica,* such as *B. napus,* gene encodes an ALS III *Brassica,* such as *B. napus,* polypeptide containing serine instead of proline at a position corresponding to position 197 of SEQ ID NO: 10 and leucine instead of tryptophan at a position corresponing to poistion 574 of SEQ ID NO: 10, or such as *Brassica,* such as B. *napus,* genes, wherein the ALS I *Brassica,* such as *B. napus,* gene encodes an ALS I *Brassica,* such as *B. napus,* polypeptide containing serine instead of proline at a position corresponding to position 197 of SEQ ID NO: 10 and leucine instead of tryptophan at a position corresponding to position 574 of SEQ ID NO: 10 and wherein the ALS III *Brassica,* such as *B. napus,* gene encodes an ALS III *Brassica,* such as *B. napus,* polypeptide containing serine instead of proline at a position corresponding to position 197 of SEQ ID NO: 10 and leucine instead of tryptophan at a position corresponing to poistion 574 of SEQ ID NO: 10, or such as *B. juncea,* genes, wherein the ALS-A Brassica, such as *B. juncea,* gene encodes an ALS-A *Brassica,* such as *B. juncea,* polypeptide containing serine instead of proline at a position corresponding to position 197 of SEQ ID NO: 10 and leucine instead of tryptophan at a position corresponding to position 574 of SEQ ID NO: 10 and wherein the ALS-B *Brassica,* such as *B. juncea,* gene encodes an ALS-B *Brassica,* such as *B. juncea,* polypeptide containing leucine instead of tryptophan at a position corresponing to poistion 574 of SEQ ID NO: 10, or such as *B. juncea,* genes, wherein the ALS-A Brassica, such as *B. juncea,* gene encodes an ALS-A *Brassica,* such as *B. juncea,* polypeptide containing leucine instead of tryptophan at a position corresponding to position 574 of SEQ ID NO: 10 and wherein the ALS-B *Brassica,* such as *B. juncea,* gene encodes an ALS-B *Brassica,* such as *B. juncea,* polypeptide containing serine instead of proline at a position corresponding to position 197 of SEQ ID NO: 10 and leucine instead of tryptophan at a position corresponing to poistion 574 of SEQ ID NO: 10, or such as *B. juncea,* genes, wherein the ALS-A Brassica, such as *B. juncea,* gene encodes an ALS-A *Brassica,* such as *B. juncea,* polypeptide containing serine instead of proline at a position corresponding to position 197 of SEQ ID NO: 10 and leucine instead of tryptophan at a position corresponding to position 574 of SEQ ID NO: 10 and wherein the ALS-B *Brassica,* such as *B. juncea,* gene encodes an ALS-B *Brassica,* such as *B. juncea,* polypeptide containing serine instead of proline at a position corresponding to position 197 of SEQ ID NO: 10 and leucine instead of tryptophan at a position corresponing to poistion 574 of SEQ ID NO: 10.

In yet another embodiment, a plant, such as *Brassica,* such as *B. napus* or *B. juncea,* plant as described herein is homozygous regarding the mutation of the ALS genes as described herein.

Also described is the use of one or more ALS inhibitor herbicide(s) alone or in combination with one or more non ALS inhibitor herbicide(s) for weed control in plant growing areas, such as in *B. napus* or in *B. juncea* growing areas which plants comprise at least two endogenous ALS genes, wherein said ALS genes comprise a codon encoding Leu instead of Trp at a position corresponding to position 1720-1722 of the nucleotide sequence of SEQ ID NO: 9, and wherein at least one of said ALS genes further comprises a codon encoding Ser instead of Pro at a position corresponding to position 589-591 of the nucleotide sequence of SEQ ID NO: 9, which plants are heterozygous or homozygous, preferably homozygous concerning the mutations in codon of the endogenous ALS I gene corresponding to the codon at position 589-591 of SEQ ID NO: 9 and in codon of the endogenous ALS I gene corresponding to the codon at position 1720-1722 of SEQ ID NO: 9, such as *B. napus* plants which comprise an endogenous ALS I gene, wherein the ALS I gene comprises a codon encoding Ser instead of Pro at a position corresponding to position 544-546 and a codon encoding Leu instead of Trp at a position corresponding to position 1675-1677 of the nucleotide sequence of the *B. napus* ALS I gene shown in SEQ ID NO: 1, and an endogenous ALS III gene, wherein the ALS III gene comprises Leu instead of Trp at a position corresponding to position 1666-1668 of the nucleotide sequence of the *B. napus* ALS III gene shown in SEQ ID NO: 3, which plants are heterozygous or homozygous, preferably homozygous concerning the mutation in codon 544-546 and 1675-1677 of the endogenous ALS I gene and the mutation in codon 1666-1668 of the endogenous ALS III gene, or such as *B. napus* plants which comprise an endogenous ALS I gene, wherein the ALS I gene comprises a codon encoding Leu instead of Trp at a position corresponding to position 1675-1677 of the nucleotide sequence of the *B. napus* ALS I gene shown in SEQ ID NO: 1, and an endogenous ALS III gene, wherein the ALS III gene comprises Ser instead of Pro at a position corresponding to position 535-537, and Leu instead of Trp at a position corresponding to position 1666-1668 of the nucleotide sequence of the *B. napus* ALS III gene shown in SEQ ID NO: 3, which plants are heterozygous or homozygous, preferably homozygous concerning the mutation in codon 1675-1677 of the endogenous ALS I gene and the mutation in codon 535-537 and 1666-1668 of the endogenous ALS III gene, or such as *B. napus* plants which comprise an endogenous ALS I gene, wherein the ALS I gene comprises a codon encoding Ser instead of Pro at a position corresponding to position 544-546 and a codon encoding Leu instead of Trp at a position corresponding to position 1675-1677 of the nucleotide sequence of the *B. napus* ALS I gene shown in SEQ ID NO: 1, and an endogenous ALS III gene, wherein the ALS III gene comprises Ser instead of Pro at a position corresponding to position 535-537, and Leu instead of Trp at a position corresponding to position 1666-1668 of the nucleotide sequence of the *B. napus* ALS III gene shown in SEQ ID NO: 3, which plants are heterozygous or homozygous, preferably homozygous concerning the mutation in codon 544-546 and 1675-1677 of the endogenous ALS I gene and the mutation in codon 535-537 and 1666-1668 of the endogenous ALS III gene, or such as *B. juncea* plants which comprise an endogenous ALS-A gene, wherein the ALS-A gene comprises a codon encoding Ser instead of Pro at a position corresponding to position 535-537 and a codon encoding Leu instead of Trp at a position corresponding to position 1666-1668 of the nucleotide sequence of the *B. juncea* ALS-A gene shown in SEQ ID NO: 5, and an endogenous ALS-B gene, wherein the ALS-B gene comprises Leu instead of Trp at a position corresponding to position 1675-1677 of the nucleotide sequence of the *B. juncea* ALS-B gene shown in SEQ ID NO: 7, which plants are heterozygous or homozygous, preferably homozygous concerning the mutation in codon 535-537 and 1666-1668 of the endogenous ALS-A gene and the mutation in codon 1675-1677 of the endogenous ALS-B gene, or such as *B. juncea* plants which comprise an endogenous ALS-A gene, wherein the ALS-A gene comprises a codon encoding Leu instead of Trp at a position corresponding to position 1666-1668 of the nucleotide sequence of the *B. juncea* ALS-A gene shown in SEQ ID NO: 5, and an endogenous ALS-B gene, wherein the ALS-B gene comprises Ser instead of Pro at a position corresponding to position 544-546 and Leu instead of Trp at a position corresponding to position 1675-1677 of the nucleotide sequence of the *B. juncea* ALS-B gene shown in SEQ ID NO: 7, which plants are heterozygous or homozygous, preferably homozygous concerning the mutation in codon 1666-1668 of the endogenous ALS-A gene and the mutation in codon 544-546 and 1675-1677 of the endogenous ALS-B gene, or such as *B. juncea* plants which comprise an endogenous ALS-A gene, wherein the ALS-A gene comprises a codon encoding Ser instead of Pro at a position corresponding to position 535-537 and a codon encoding Leu instead of Trp at a position corresponding to position 1666-1668 of the nucleotide sequence of the *B. juncea* ALS-A gene shown in SEQ ID NO: 5, and an endogenous ALS-B gene, wherein the ALS-B gene comprises Ser instead of Pro at a position corresponding to position 544-546 and Leu instead of Trp at a position corresponding to position 1675-1677 of the nucleotide sequence of the *B. juncea* ALS-B gene shown in SEQ ID NO: 7, which plants are heterozygous or homozygous, preferably homozygous concerning the mutation in codon 535-537 and 1666-1668 of the endogenous ALS-A gene and the mutation in codon 544-546 and 1675-1677 of the endogenous ALS-B gene,.

Owing to their herbicidal and plant growth-regulatory properties, ALS inhibitor herbicides belonging to one or more of the groups (A), (B), and (C) either alone or in combination with non ALS inhibitor heribicides can be employed for controlling harmful plants in known plant, such as *Brassica,* such as *B. napus* or *B. juncea,* plants but also in tolerant or genetically modified crop plants that do already exists or need still to be developed. In general, the transgenic plants are distinguished by specific advantageous properties, in addition to tolerances to the ALS inhibitor herbicides according to the invention, for example, by tolerances to non ALS inhibitor herbicides, resistances to plant diseases or the causative organisms of plant diseases such as certain insects or microorganisms, such as fungi, bacteria or viruses. Other specific chracteristics relate, for example, to the harvested material with regard to quantity, quality, storability, composition and specific constituents. Thus, transgenic plants are known whose oil content is increased, or whose oil quality is altered, or those where the harvested material has a different fatty acid composition.

Conventional methods of generating novel plants which have modified properties in comparison to plants occurring to date consist, for example, in traditional breeding methods and the generation of mutants. Alternatively, novel plants with altered properties can be generated with the aid of recombinant methods (see, for example, EP-A-0221044, EP-A-0131624). For example, the following have been described in several cases:
- the modification, by recombinant technology, of crop plants with the aim of modifying the starch synthesized in the plants (for example WO 92/11376, WO 92/14827, WO 91/19806),
- transgenic crop plants which exhibit tolerance to non ALS inhibitor herbicides,
- transgenic crop plants with the capability of producing *Bacillus thuringiensis* toxins (Bt toxins), which make the plants resistant to certain pests (EP-A-0142924, EP-A-0193259),
- transgenic crop plants with a modified fatty acid composition (WO 91/13972).

The plants according to the invention may additionally contain an endogenous or a transgene, which confers herbicide resistance, such as the bar or pat gene, which confer resistance to glufosinate ammonium (Liberty or Basta) [EP 0 242 236 and EP 0 242 246]; or any modified EPSPS gene, such as the 2mEPSPS gene from maize [EP0 508 909 and EP 0 507 698], or glyphosate acetyltransferase, or glyphosate oxidoreductase, which confer resistance to glyphosate (RoundupReady), or bromoxynitril nitrilase to confer bromoxynitril tolerance,. Further, the plants according to the invention may additionally contain an endogenous or a transgene which confers increased oil content or improved oil composition, such as a 12:0 ACP thioesteraseincrease to obtain high laureate; which confers increased digestibility, such as 3-phytase; which confers pollination control, such as such as barnase under control of an anther-specific promoter to obtain male sterility, or barstar under control of an anther-specific promoter to confer restoration of male sterility, or such as the Ogura cytoplasmic male sterility and nuclear restorer of fertility.

A large number of techniques in molecular biology are known in principle with the aid of which novel transgenic plants with modified properties can be generated; see, for example, Sambrook et al., 1989, Molecular Cloning, A Laboratory Manual, 2nd Edition, Cold Spring Harbor Laboratory Press, Cold Spring Harbor, NY; or Winnacker "Gene und Klone", VCH Weinheim 2nd Edition 1996 or Christou, "Trends in Plant Science" 1 (1996) 423-431).

To carry out such recombinant manipulations, nucleic acid molecules which allow mutagenesis or sequence changes by recombination of DNA sequences can be introduced into plasmids. For example, the abovementioned standard methods allow base exchanges to be carried out, subsequences to be removed, or natural or synthetic sequences to be added. To connect the DNA fragments to each other, adapters or linkers may be added to the fragments.

For example, the generation of plant cells with a reduced activity of a gene product can be achieved by expressing at least one corresponding antisense RNA, a sense RNA for achieving a cosuppression effect or by expressing at least one suitably constructed ribozyme which specifically cleaves transcripts of the abovementioned gene product.

To this end, it is possible to use DNA molecules which encompass the entire coding sequence of a gene product inclusive of any flanking sequences which may be present, and also DNA molecules which only encompass portions of the coding sequence, it being necessary for these portions to be long enough to have an antisense effect in the cells. The use of DNA sequences which have a high degree of homology to the coding sequences of a gene product, but are not completely identical to them, is also possible.

When expressing nucleic acid molecules in plants, the protein synthesized can be localized in any desired compartment of the plant cell. However, to achieve localization in a particular compartment, it is possible, for example, to link the coding region with DNA sequences which ensure localization in a particular compartment. Such sequences are known to those skilled in the art (see, for example, Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

The transgenic plant cells can be regenerated by known techniques to give rise to entire plants. Thus, transgenic plants, such as *Brassica,* such as *B. napus* or *B. juncea,* plants can be obtained whose properties are altered by overexpression, suppression or inhibition of homologous (= natural) genes or gene sequences or the expression of heterologous (= foreign) genes or gene sequences.

The present disclosure furthermore provides a method for controlling unwanted plants in plant, such as *B. napus* or *B. juncea* growing areas of plants, such as *B. napus* or *B. juncea* plants according to the invention as described herein which comprises applying one or more ALS inhibitor herbicides belonging to groups (A), (B) and/or (C) to the plants (for example harmful plants, such as monocotyledonous or dicotyledonous weeds or unwanted crop plants), the seed (seeds or vegetative propagation organs, such as tubers or shoot parts) or to the area in which the plants grow (for example the area under cultivation), for example together or separately.

The present disclosure furthermore provides a method for controlling unwanted plants in growing areas of plants, such as *B. napus* or *B. juncea* plants according to the invention as described herein which comprises applying one or more ALS inhibitor herbicide(s) belonging to groups (A), (B) and/or (C) alone or in combination with non ALS inhibitor herbicides belonging to class (D) compound according to the invention to the plants (for example harmful plants, such as monocotyledonous or dicotyledonous weeds or unwanted crop plants), the seed (seeds or vegetative propagation organs, such as tubers or shoot parts) or to the area in which the plants grow (for example the area under cultivation), for example together or separately. One or more non ALS inhibitor herbicides may be applied in combination with one or more ALS inhibitor herbicide(s) before, after or simultaneously with the ALS inhibitor herbicide(s) to the plants, the seed or the area in which the plants grow (for example the area under cultivation).

"Unwanted plants" or "unwanted vegetation" are to be understood as meaning all plants which grow in locations where they are unwanted. This can, for example, be harmful plants (for example monocotyledonous or dicotyledonous species or other unwanted crop plants (volunteers)) such as *Geranium dissectum, Centaurea cyanus, Sinapis arvensis* and/or *Alopecurus myosuroides.*

An unwanted plant can be at least one dicotyledonous plant selected from the group consisting of Aethusa cynapium, Agrostemma githago, Amaranthus sp., Ambrosia artemisifolia, Ammi majus, Anagallis arvensis, Anchusa officinalis, Anthemis sp., Aphanes arvensis, Arabidopsis thaliana, Artemisia vulgaris, Atriplex sp., Bidens sp., Bifora radians, Brassica nigra, Calendula arvensis, Capsella bursa pastoris, Cardamine hirsute, Cardaria draba, Centaurea cyanus, Cerastium arvense, Chaenorhinum minus, Chenopodium sp., Chrysanthemum segetum, Cirsium arvense, Convolvulus sp., Coronopus sp., Datura stramonium, Daucus carota, Descurainia sophia, Diplotaxis muralis, Echium vulgare, Erigeron Canadensis, Erodium circutarium, Erysium cheiranthoides, Euphorbia sp., Filaginella uliginosa, Fumaria officinalis, Galeopsis sp., Galeopsis tetraclit, Galinsoga parviflora, Galium aparine, Geranium sp., Juncus bufonius, Kickxia spuria, Lactuca sericola, Lamium sp, Lapsana communis, Lathyrus tuberosus, Legousia speculum-veneris, Linaria vulgaris, Lithospermum arvense, Lycopsis arvensis, Malva sp., Matricaria sp., Menta arvensis,Mercurialis annua, Myagrum perfoliatum, Myosotis arvensis, Papaver sp., Picris echioides, Polygonum sp., Portulaca oleracea, Ranunculus sp., Raphanus raphanistrum, Rumex sp., Scandix pecten-veneris, Senecio vulgaris, Silene sp., Sinapis arvensis, Sisymbrium officinale, Solanum nigrum, Sonchus sp., Spergula arvensis, Stachys arvensis, Stellaria media, Thlaspi arvense, Tussilago farfara, Urtica urens, Verbena officinalis, Veronica sp., Vicia sp., Viola arvensis and Xanthium sp. An unwanted plant can be at least one plant selected from the group consisting of Aethusa cynapium, Galium aparine, Geranium sp., Lamium sp, Matricaria sp., Myosotis arvensis, Papaver sp., Polygonum sp., Sisymbrium officinale, Stellaria media, Thlaspi arvense, Urtica urens and Viola arvensis.

An unwanted plant can be at least one monocotyledonous plant selected from the group consisting of *Agropyron repens, Alopecurus myosuroides, Apera spica-venti, Avena sp.,* Bromus *sp., Cyperus sp., Digitaria sp., Echinochloa sp., Hordeum murinum, Lolium multiflorum, Panicum dichotomiflorum, Phalaris canariensis, Poa sp., Setaria sp., Sorghum halepense, Leptochloa filiformis.* An unwanted plant can be at least one plant selected from the group consisting of *Agropyron repens, Alopecurus myosuroides, Apera spica-venti, Avena sp.* and *Poa sp.*

An unwanted plant can be at least one monocotyledonous plant selected from the group consisting *of Beta vulgaris, Helianthus* annuus, *Solanum tuberosum, Triticum vulgare, Hordeum vulgare, Secale cereale, Avena sativa.* An unwanted plant can be *Triticum vulgare* and *Hordeum vulgare.*

The herbicide combinations to be used as described herein can be prepared by known processes, for example as mixed formulations of the individual components, if appropriate with further active compounds, additives and/or customary formulation auxiliaries, which combinations are then applied in a customary manner diluted with water, or as tank mixes by joint dilution of the components, formulated separately or formulated partially separately, with water. Also possible is the split application of the separately formulated or partially separately formulated individual components.

It is also possible to apply ALS inhibitor herbicides or the combination comprising ALS inhibitor herbicide(s) and non ALS inhibitor herbicide(s) in a plurality of portions (sequential application) using, for example, pre-emergence applications followed by post-emergence applications or using early post-emergence applications followed by medium or late post-emergence applications. Preference is given here to the joint or almost simultaneous application of the active compounds of the combination in question.

The herbicides belonging to any of the above defined groups (A), (B), (C) and (D) and to be applied as described herein can be converted jointly or separately into customary formulations, such as solutions, emulsions suspensions, powders, foams, pastes, granules, aerosols, natural and synthetic materials impregnated with active compound and microencapsulations in polymeric materials. The formulations may comprise the customary auxiliaries and additives.

These formulations are produced in a known manner, for example by mixing the active compounds with extenders, that is liquid solvents, pressurized liquefied gases and/or solid carriers, if appropriate with the use of surfactants, that is emulsifiers and/or dispersants, and/or foam formers.

If the extender used is water, it is also possible to use, for example, organic solvents as auxiliary solvents. Suitable liquid solvents are essentially: aromatics, such as xylene, toluene, alkylnaphthalenes, chlorinated aromatics or chlorinated aliphatic hydrocarbons, such as chlorobenzenes, chloroethylenes, or methylene chloride, aliphatic hydrocarbons, such as cyclohexane or paraffins, for example mineral oil fractions, mineral and vegetable oils, alcohols, such as butanol or glycol, and ethers and esters thereof, ketones, such as acetone, methyl ethyl ketone, methyl isobutyl ketone or cyclohexanone, strongly polar solvents, such as dimethylformamide or dimethyl sulfoxide, and also water.

Suitable solid carriers are: for example ammonium salts and ground natural minerals, such as kaolins, clays, talc, chalk, quartz, attapulgite, montmorillonite or diatomaceous earth, and ground synthetic minerals, such as finely divided silica, alumina and silicates; suitable solid carriers for granules are: for example crushed and fractionated natural rocks, such as calcite, marble, pumice, sepiolite and dolomite, and also synthetic granules of inorganic and organic meals, and granules of organic material, such as sawdust, coconut shells, corn cobs and tobacco stalks; suitable emulsifiers and/or foam formers are: for example nonionic and anionic emulsifiers, such as polyoxyethylene fatty acid esters, polyoxyethylene fatty alcohol ethers, for example alkylaryl polyglycol ethers, alkylsulfonates, alkyl sulfates, arylsulfonates and also protein hydrolysates; suitable dispersants are: for example lignosulfite waste liquors and methylcellulose.

Tackifiers such as carboxymethylcellulose and natural and synthetic polymers in the form of powders, granules or latices, such as gum arabic, polyvinyl alcohol and polyvinyl acetate, and also natural phospholipids, such as cephalins and lecithins and synthetic phospholipids, can be used in the formulations. Other possible additives are mineral and vegetable oils.

The herbicidal action of the herbicide combinations to be used as described herein can be improved, for example, by surfactants, preferably by wetting agents from the group of the fatty alcohol polyglycol ethers. The fatty alcohol polyglycol ethers preferably comprise 10 - 18 carbon atoms in the fatty alcohol radical and 2-20 ethylene oxide units in the polyglycol ether moiety. The fatty alcohol polyglycol ethers may be present in nonionic form, or ionic form, for example in the form of fatty alcohol polyglycol ether sulfates, which may be used, for example, as alkali metal salts (for example sodium salts and potassium salts) or ammonium salts, or even as alkaline earth metal salts, such as magnesium salts, such as C₁₂/C₁₄-fatty alcohol diglycol ether sulfate sodium (Genapol® LRO, Clariant GmbH); see, for example, EP-A-0476555, EP-A-0048436, EP-A-0336151 or US-A-4,400,196 and also Proc. EWRS Symp. "Factors Affecting Herbicidal Activity and Selectivity", 227 - 232 (1988). Nonionic fatty alcohol polyglycol ethers are, for example, (C₁₀-C₁₈)-, preferably (C₁₀-C₁₄)-fatty alcohol polyglycol ethers (for example isotridecyl alcohol polyglycol ethers) which comprise, for example, 2 - 20, preferably 3 - 15, ethylene oxide units, for example those from the Genapol® X-series, such as Genapol® X-030, Genapol® X-060, Genapol® X-080 or Genapol® X-150 (all from Clariant GmbH).

Also disclosed herein is the combination of ALS inhibitor herbicides belonging to any of the groups (A), (B), and (C) as described herein with the wetting agents mentioned above from the group of the fatty alcohol polyglycol ethers which preferably contain 10 - 18 carbon atoms in the fatty alcohol radical and 2 - 20 ethylene oxide units in the polyglycol ether moiety and which may be present in nonionic or ionic form (for example as fatty alcohol polyglycol ether sulfates). Preference is given to C₁₂/C₁₄-fatty alcohol diglycol ether sulfate sodium (Genapol® LRO, Clariant GmbH) and isotridecyl alcohol polyglycol ether having 3 - 15 ethylene oxide units, for example from the Genapol® X-series, such as Genapol® X-030, Genapol® X-060, Genapol® X-080 and Genapol® X-150 (all from Clariant GmbH). Furthermore, it is known that fatty alcohol polyglycol ethers, such as nonionic or ionic fatty alcohol polyglycol ethers (for example fatty alcohol polyglycol ether sulfates) are also suitable for use as penetrants and activity enhancers for a number of other herbicides (see, for example, EP-A-0502014).

Furthermore, it is known that fatty alcohol polyglycol ethers, such as nonionic or ionic fatty alcohol polyglycol ethers (for example fatty alcohol polyglycol ether sulfates) are also suitable for use as penetrants and activity enhancers for a number of other herbicides (see, for example, EP-A-0502014).

The herbicidal action of the herbicide combinations as described herein can also be enhanced by using vegetable oils. The term vegetable oils is to be understood as meaning oils of oleaginous plant species, such as soybean oil, rapeseed oil, corn oil, sunflower oil, cottonseed oil, linseed oil, coconut oil, palm oil, thistle oil or castor oil, in particular rapeseed oil, and also their transesterification products, for example alkyl esters, such as rapeseed oil methyl ester or rapeseed oil ethyl ester.

The vegetable oils are preferably esters of C₁₀-C₂₂-, preferably C₁₂-C₂₀-, fatty acids. The C₁₀-C₂₂-fatty acid esters are, for example, esters of unsaturated or saturated C₁₀-C₂₂-fatty acids, in particular those having an even number of carbon atoms, for example erucic acid, lauric acid, palmitic acid and in particular C₁₈-fatty acids, such as stearic acid, oleic acid, linoleic acid or linolenic acid.

Examples of C₁₀-C₂₂-fatty acid esters are esters obtained by reacting glycerol or glycol with the C₁₀-C₂₂-fatty acids contained, for example, in oils of oleaginous plant species, or C₁-C₂₀-alkyl-C₁₀-C₂₂-fatty acid esters which can be obtained, for example, by transesterification of the aforementioned glycerol- or glycol-C₁₀-C₂₂-fatty acid esters with C₁-C₂₀-alcohols (for example methanol, ethanol, propanol or butanol). The transesterification can be carried out by known methods as described, for example, in Römpp Chemie Lexikon, 9th edition, Volume 2, page 1343, Thieme Verlag Stuttgart.

Preferred C₁-C₂₀-alkyl-C₁₀-C₂₂-fatty acid esters are methyl esters, ethyl esters, propyl esters, butyl esters, 2-ethylhexyl esters and dodecyl esters. Preferred glycol- and glycerol-C₁₀-C₂₂-fatty acid esters are the uniform or mixed glycol esters and glycerol esters of C₁₀-C₂₂-fatty acids, in particular fatty acids having an even number of carbon atoms, for example erucic acid, lauric acid, palmitic acid and, in particular, C₁₈-fatty acids, such as stearic acid, oleic acid, linoleic acid or linolenic acid.

In the herbicidal compositions to be used as described herein, the vegetable oils can be present, for example, in the form of commercially available oil-containing formulation additives, in particular those based on rapeseed oil, such as Hasten® (Victorian Chemical Company, Australia, hereinbelow referred to as Hasten, main ingredient: rapeseed oil ethyl ester), Actirob®B (Novance, France, hereinbelow referred to as ActirobB, main ingredient: rapeseed oil methyl ester), Rako-Binol® (Bayer AG, Germany, hereinbelow referred to as Rako-Binol, main ingredient: rapeseed oil), Renol® (Stefes, Germany, hereinbelow referred to as Renol, vegetable oil ingredient: rapeseed oil methyl ester) or Stefes Mero® (Stefes, Germany, hereinbelow referred to as Mero, main ingredient: rapeseed oil methyl ester).

Herbicidal combinations that can be used can be formulated with the vegetable oils mentioned above, such as rapeseed oil, preferably in the form of commercially available oil-containing formulation additives, in particular those based on rapeseed oil, such as Hasten® (Victorian Chemical Company, Australia, hereinbelow referred to as Hasten, main ingredient: rapeseed oil ethyl ester), Actirob®B (Novance, France, hereinbelow referred to as ActirobB, main ingredient: rapeseed oil methyl ester), Rako-Binol® (Bayer AG, Germany, hereinbelow referred to as Rako-Binol, main ingredient: rapeseed oil), Renol® (Stefes, Germany, hereinbelow referred to as Renol, vegetable oil ingredient: rapeseed oil methyl ester) or Stefes Mero® (Stefes, Germany, hereinbelow referred to as Mero, main ingredient: rapeseed oil methyl ester).

It is possible to use colorants, such as inorganic pigments, for example iron oxide, titanium oxide, Prussian Blue, and organic dyes, such as alizarin dyes, azo dyes and metal phthalocyanine dyes, and trace nutrients such as salts of iron, manganese, boron, copper, cobalt, molybdenum and zinc.

The formulations that can be used generally comprise from 0.1 to 95% by weight of active compounds, preferably from 0.5 to 90% by weight.

As such or in their formulations, the ALS inhibitor herbicides belonging to any of the above defined groups (A), (B), and (C) can also be used as a mixture with other agrochemically active compounds, such as known non ALS inibitor herbicides, for controlling unwanted vegetation, for example for controlling weeds or for controlling unwanted crop plants, finished formulations or tank mixes, for example, being possible.

The use of a mixture of ALS inhibitor herbicides belonging to any of the above defined groups (A), (B), and (C) with other known active compounds, such as fungicides, insecticides, acaricides, nematicides, safeners, bird repellants, plant nutrients and soil structure improvers is likewise possible.

The ALS inhibitor herbicides belonging to any of the above defined groups (A), (B), (C) can be used as such, in the form of their formulations or in the use forms prepared therefrom by further dilution, such as ready-to-use solutions, suspensions, emulsions, powders, pastes and granules. Application is carried out in a customary manner, for example by watering, spraying, atomizing, broadcasting.

One or more of the ALS inhibitor herbicides belonging to any of the above defined groups (A), (B), and (C) can be applied either alone or in combination with one or more non ALS inhibitor herbicides belonging to group (DO) to the plants (for example harmful plants, such as monocotyledonous or dicotyledonous weeds or unwanted crop plants), the seed (for example grains, seeds or vegetative propagation organs, such as tubers or shoot parts with buds) or the area under cultivation (for example the soil), preferably to the green plants and parts of plants and, if appropriate, additionally the soil. One possible use is the joint application of the active compounds in the form of tank mixes, where the optimally formulated concentrated formulations of the individual active compounds are, together, mixed in a tank with water, and the spray liquor obtained is applied.

Also described is a method to increase the tolerance to ALS inhibitor herbicide(s) of polyploid plants, such as *Brassica napus* or *Brassica juncea* plants, said method comprising introducing a first ALS allele encoding an ALS polypeptide comprising at a position corresponding to position 197 of SEQ ID NO: 10 instead of the naturally encoded amino acid proline the amino acid serine, and at a position corresponding to position 574 of SEQ ID NO: 10 instead of the naturally encoded amino acid tryptophan the amino acid leucine, such as an ALS I allele encoding an ALS I polypeptide comprising at a position corresponding to position 182 of SEQ ID NO: 2 instead of the naturally encoded amino acid proline the amino acid serine and at a position corresponding to position 559 of SEQ ID NO: 2 instead of the naturally encoded amino acid tryptophan the amino acid leucine, or such as an ALS-A allele encoding an ALS-A polypeptide comprising at a position corresponding to position 179 of SEQ ID NO: 6 instead of the naturally encoded amino acid proline the amino acid serine and at a position corresponding to position 556 of SEQ ID NO: 6 instead of the naturally encoded amino acid tryptophan the amino acid leucine, and a second ALS allele encoding an ALS polypeptide comprising at a position corresponding to position 574 of SEQ ID NO: 10 instead of the naturally encoded amino acid tryptophan the amino acid leucine, such as an ALS III allele encoding an ALS III polypeptide comprising at a position corresponding to position 556 of SEQ ID NO: 4 instead of the naturally encoded amino acid tryptophan the amino acid leucine, or such as an ALS-B allele encoding an ALS-B polypeptide comprising at a position corresponding to position 559 of SEQ ID NO: 8 instead of the naturally encoded amino acid tryptophan the amino acid leucine, in the genome of said plant, or said method comprising introducing a first ALS allele encoding an ALS polypeptide comprising at a position corresponding to position 574 of SEQ ID NO: 10 instead of the naturally encoded amino acid tryptophan the amino acid leucine, such as an ALS I allele encoding an ALS I polypeptide comprising at a position corresponding to position 559 of SEQ ID NO: 2 instead of the naturally encoded amino acid tryptophan the amino acid leucine, or such as an ALS-A allele encoding an ALS-A polypeptide comprising at a position corresponding to position 556 of SEQ ID NO: 6 instead of the naturally encoded amino acid tryptophan the amino acid leucine, and a second ALS allele encoding an ALS polypeptide comprising at a position corresponding to position 197 of SEQ ID NO: 10 instead of the naturally encoded amino acid proline the amino acid serine, and at a position corresponding to position 574 of SEQ ID NO: 10 instead of the naturally encoded amino acid tryptophan the amino acid leucine, such as an ALS III allele encoding an ALS III polypeptide comprising at a position corresponding to position 179 of SEQ ID NO: 4 instead of the naturally encoded amino acid proline the amino acid serine and at a position corresponding to position 556 of SEQ ID NO: 4 instead of the naturally encoded amino acid tryptophan the amino acid leucine, or such as an ALS-B allele encoding an ALS-B polypeptide comprising at a position corresponding to position 182 of SEQ ID NO: 8 instead of the naturally encoded amino acid proline the amino acid serine and at a position corresponding to position 559 of SEQ ID NO: 8 instead of the naturally encoded amino acid tryptophan the amino acid leucine, in the genome of said plant, or said method comprising introducing a first and a second ALS allele encoding an ALS polypeptide comprising at a position corresponding to position 197 of SEQ ID NO: 10 instead of the naturally encoded amino acid proline the amino acid serine, and at a position corresponding to position 574 of SEQ ID NO: 10 instead of the naturally encoded amino acid tryptophan the amino acid leucine, such as a first ALS allele which is an ALS I allele encoding an ALS I polypeptide comprising at a position corresponding to position 182 of SEQ ID NO: 2 instead of the naturally encoded amino acid proline the amino acid serine and at a position corresponding to position 559 of SEQ ID NO: 2 instead of the naturally encoded amino acid tryptophan the amino acid leucine and a second ALS allele which is an ALS III allele encoding an ALS III polypeptide comprising at a position corresponding to position 179 of SEQ ID NO: 4 instead of the naturally encoded amino acid proline the amino acid serine and at a position corresponding to position 556 of SEQ ID NO: 4 instead of the naturally encoded amino acid tryptophan the amino acid leucine, or such as a first ALS allele which is ALS-A allele encoding an ALS-A polypeptide comprising at a position corresponding to position 179 of SEQ ID NO: 6 instead of the naturally encoded amino acid proline the amino acid serine and at a position corresponding to position 556 of SEQ ID NO: 6 instead of the naturally encoded amino acid tryptophan the amino acid leucine, and a second ALS allele which is an ALS-B allele encoding an ALS-B polypeptide comprising at a position corresponding to position 182 of SEQ ID NO: 8 instead of the naturally encoded amino acid proline the amino acid serine and at a position corresponding to position 559 of SEQ ID NO: 8 instead of the naturally encoded amino acid tryptophan the amino acid leucine, in the genome of said plant.

An increase in tolerance to ALS inhibitor herbicide(s) can be an increase in tolerance to one or to more of the ALS inhibitor herbicides as described elsewhere in this application.

Introducing an ALS allele, such as an ALS I allele and an ALS III allele or such as an ALS-A allele and an ALS-B allele according to the invention can be, for example, generation of the ALS I mutation as described in the below examples. Introducing an ALS allele, such as an ALS I or an ALS III allele or such as an ALS-A allele and an ALS-B allele according to the invention can also be by crossing with a plant comprising an ALS allele according to the invention and selection of progeny plants comprising the ALS alleles according to the invention.

Progeny plants can be selected by their tolerance to ALS inhibitor herbicide(s). Progeny plants can also be selected using molecular methods well known in the art, such as, for example, direct sequencing or using molecular markers (e.g. AFLP, PCR, Invader™, TaqMan®, KASP, and the like).

### Agronomically exploitable

The skilled person will understand that it is generally preferred that the polyploid plants, such as *B. napus* or *B. juncea* plants of the present invention and parts thereof are agronomically exploitable.

"Agronomically exploitable" means that the plants, such as *B. napus* or *B. juncea* plants and parts thereof are useful for agronomical purposes. For example, the *B. napus* plants should serve for the purpose of being useful for rape seed oil production for, e.g., bio fuel or bar oil for chainsaws, animal feed or honey production, for oil, meal, grain, starch, flour, protein, fiber, industrial chemical, pharmaceutical or neutraceutical production. The term "agronomically exploitable" when used herein also includes that the plants, such as *B. napus* or *B. juncea* plants of the present invention are less sensitive against an ALS-inhibitor herbicide, such as 5 times, or 10 times, or 50 times, or 100 times, or 500 times, or 1000 times, or 2000 times less sensitive as compared to wild type plants. The ALS inhibitor herbicide is one or more described herein, preferably it is foramsulfuron either alone or in combination with one or more further ALS-inhibitor herbicide(s) either from the sub-class of the sulfonyurea herbicides or any other sub-class of the ALS-inhbitor herbicides, most preferably it is foramsulfuron in combination with a further sulfonylurea herbicide and/or an ALS-inhibitor of the sulfonylaminocarbonyltriazolinone herbicide sub-class.

Also described is the use of the plants, such as *B. napus* or *B. juncea* plant described herein and/or the harvestable parts or propagation material described herein for the manufacture/breeding of said plants. Methods for the manufacture/breeding of plants, such as *B. napus* or *B. juncea* plants are described herein elsewhere. Such manufacture/breeding methods may be used to generate plants of the present invention further comprising novel plant traits such as stress-resistance, like but not limited to drought, heat, cold, or salt stress and the like.

The present disclosure envisages the use of the herbicide tolerant plant described herein and/or harvestable parts or propagation material derived thereof in a screening method for the selection of ALS inhibitor herbicides.

A better understanding of the present invention and of its many advantages will be had from the following examples, offered for illustrative purposes only, and are not intended to limit the scope of the present invention in any way.

The sequence listing contained in the file named "BCS13-2013_ST25.txt", which is 257 kilobytes (size as measured in Microsoft Windows®), contains 38 sequences SEQ ID NO: 1 through SEQ ID NO: 38 is filed herewith by electronic submission.

### SEQUENCES

*A. thaliana* sequences SEQ ID NOs: 9 (nucleotide AY042819) and 10 (protein AAK68759), and wild type *B. napus* sequences SEQ ID NOs: 1 (ALS1 nucleotide Z11524) and 3 (ALS3 nucleotide Z11526) were taken from the ncbi-genebank (see world wide web: http://www.ncbi.nlm.nih.gov/genbank/). SEQ ID NOs: 2 and 4 are the protein sequences encoded by SEQ ID NOs: 1 and 3, respectively.
SEQ ID No.1: Nucleic acid sequence encoding *B. napus* wild type ALS I gb Z11524.
SEQ ID No.2: *B. napus* ALS I amino acid sequence derived from SEQ ID No.1.
SEQ ID No.3: Nucleic acid sequence encoding *B. napus* wild type ALS III gb Z11526.
SEQ ID No.4: *B. napus* ALS III amino acid sequence derived from SEQ ID No.3.
SEQ ID No.5: Nucleic acid sequence encoding *B. juncea* wild type ALS-A.
SEQ ID No.6: *B. juncea* ALS-A amino acid sequence derived from SEQ ID No.5.
SEQ ID No.7: Nucleic acid sequence encoding *B. juncea* wild type ALS-B.
SEQ ID No.8: *B. juncea* ALS-B amino acid sequence derived from SEQ ID No.7.
SEQ ID No.9: Nucleic acid sequence encoding *A. thaliana* ALS gene.
SEQ ID No.10: *A. thaliana* amino acid sequence derived from SEQ ID No.9.
SEQ ID No.11: Nucleic acid sequence encoding *Gossypium hirsutum* ALS gene.
SEQ ID No.12: *Gossypium hirsutum* amino acid sequence derived from SEQ ID No.19.
SEQ ID No.13: Nucleic acid sequence encoding *Gossypium hirsutum* ALS gene.
SEQ ID No.14: *Gossypium hirsutum* amino acid sequence derived from SEQ ID No.21.
SEQ ID No.15: Nucleic acid sequence encoding *Glycine max* ALS gene.
SEQ ID No.16: *Glycine max* amino acid sequence derived from SEQ ID No.23.
SEQ ID No.17: Nucleic acid sequence encoding *Glycine max* ALS gene.
SEQ ID No.18: *Glycine max* amino acid sequence derived from SEQ ID No.25.
SEQ ID No.19: Nucleic acid sequence encoding *Glycine max* ALS gene.
SEQ ID No.20: *Glycine max* amino acid sequence derived from SEQ ID No.27.
SEQ ID No.21: Nucleic acid sequence encoding *Glycine max* ALS gene.
SEQ ID No.22: *Glycine max* amino acid sequence derived from SEQ ID No.29.
SEQ ID No.23: Nucleic acid sequence encoding *Nicotiana tabacum* ALS gene.
SEQ ID No.24: *Nicotiana tabacum* amino acid sequence derived from SEQ ID No.31.
SEQ ID No.25: Nucleic acid sequence encoding *Nicotiana tabacum* ALS gene.
SEQ ID No.26: *Nicotiana tabacum* amino acid sequence derived from SEQ ID No.33.
SEQ ID No.27: Nucleic acid sequence encoding *Solanum tuberosum* ALS gene.
SEQ ID No.28: *Solanum tuberosum* amino acid sequence derived from SEQ ID No.35.
SEQ ID No.29: Nucleic acid sequence encoding *Solanum tuberosum* ALS gene.
SEQ ID No.30: *Solanum tuberosum* amino acid sequence derived from SEQ ID No.37.
SEQ ID No.31: Nucleic acid sequence encoding *Solanum tuberosum* ALS gene.
SEQ ID No.32: *Solanum tuberosum* amino acid sequence derived from SEQ ID No.39.
SEQ ID No.33: Nucleic acid sequence encoding *Triticum aestivum* ALS gene.
SEQ ID No.34: *Triticum aestivum* amino acid sequence derived from SEQ ID No.41.
SEQ ID No.35: Nucleic acid sequence encoding *Triticum aestivum* ALS gene.
SEQ ID No.36: *Triticum aestivum* amino acid sequence derived from SEQ ID No.43.
SEQ ID No.37: Nucleic acid sequence encoding *Saccharum officinarum* ALS gene.
SEQ ID No.38: *Saccharum officinarum* amino acid sequence derived from SEQ ID No.45.

### EXAMPLES

### Example 1 - Generation and isolation of mutant Brassica AHAS alleles

### Mutant HETO134 and HETO 133

*Brassica napus* lines with the HETO134 mutation, i.e. comprising a G to T substitution at position 1676 of ALS I, resulting in a Tryptophan to Leucine amino acid substitution at position 559 of the encoded protein, and with the HETO133 mutation, i.e. comprising a G to T substitution at position 1667 of ALS III, resulting in a Tryptophan to Leucine amino acid substitution at position 556 of the encoded protein, were generated as follows.

### Seedling germination and callus induction

Aliquots of seeds were sterilized by rinsing for 1 min in 70% ethanol followed by 15 minutes agitation in bleach (6% active chlorine dilution). After 3 washes in sterile water the seeds were sown on 5 cm Petri plates containing 5 ml of M-205 germination medium (25 seeds per plate). M-205 medium is half strength MS macro and micro salts (Murashige and Skoog, 1962), half strength B5 vitamins (Gamborg et al. 1968) containing 10 g/l sucrose and solidified with 8 g/l plant agar (pH 5.6). Plates of seeds were transferred to 21 sterile glass containers and incubated for 5 days in the light at 24°C.

Five day old seedlings were used for the preparation of hypocotyl segments 7-10 mm in length. Hypocotyl segments were transferred to 14 cm Petri plates containing 75 ml of M-338 [H76] callus induction medium (25 explants/plate). M-338 [H76] medium is MS salts and vitamins (Murashige and Skoog, 1962), 20 g/l sucrose, 0.5 g/l MES, 0.5 g/l adenine sulphate, 5 mg/l silver nitrate, 0.5 mg/1 2,4-D, 0.2 mg/l kinetin and solidified with 5.5 g/l agarose (pH 5.7). Dishes were sealed and cultured at 24°C with standard light conditions (16h/day). After 3 weeks, calli developing at the ends of the explants were transferred to fresh M-338 [H76] plates (25 calli/plate). Calli were subcultured to fresh medium every 3 weeks (larger calli cut into two pieces).

### Selection of mutant HETO134 and of mutant HETO133

Hypocotyl derived callus (9 weeks old) was used for the selection of herbicide resistant mutants. Small pieces of callus were plated on M-338 [H76] medium containing 25 nM of the ALS inhibitor Foramsulfuron (CAS RN 173159-57-4). The starting concentration of 25 nM Foramsulfuron was chosen to be partially inhibitory to callus growth but not lethal. Dishes were cultured at 24°C with standard light conditions (16h/day). After 3 weeks, the calli were divided into 2 pieces each and replated on M-338 [H76] medium containing 50 nM Foramsulfuron. Further increases in Foramsulfuron concentration (e.g. 75 nM, 100 nM, 150 nM, or higher) were made each subculture until rapidly growing green (resistant) calli could clearly be identified from the brown non-resistant tissues. Individual mutant clones were transferred to 9 cm dishes containing M-338 [H76] medium and 250 nM Foramsulfuron (a level clearly inhibitory to WT tissues).

The presence in HETO134 of a single point mutation in the tryptophan 559 codon (corresponding to the tryptophan 574 codon in *A. thaliana),* i.e. a G to T substitution at position 1676 of the coding sequence of ALS I gene, resulting in a Tryptophan to Leucine amino acid substitution at position 559 of the encoded protein, was confirmed by sequence analysis.

The presence in HETO133 of a single point mutation in the tryptophan 556 codon (corresponding to the tryptophan 574 codon in *A. thaliana),* i.e. a G to T substitution at position 1667 of the coding sequence of ALS III gene, resulting in a Tryptophan to Leucine amino acid substitution at position 556 of the encoded protein, was confirmed by sequence analysis.

### Mutant HETO132

*Brassica napus* lines with the HETO132 mutation, i.e. comprising a C to T substitution at position 535 and a G to T substitution at position 1667 in ALS III, resulting in a Proline to Serine amino acid substitution at position 179 and a tryptophan to leucine substitution at position 556 of the ALS III protein were generated as follows.

First, *Brassica napus* lines with the HETO111 mutation, i.e. comprising a C to T substitution at position 535 in ALS III, resulting in a Proline to Serine amino acid substitution at position 179 of the ALS III protein, were generated and identified as described in WO 2011/076345. Based on lines with the HETO111 mutation, lines with the HETO132 mutation were obtained using methods essentially similar to those for obtaining HETO134 (see above), with the modification that the starting material for selection was seed from lines with the HETO111 mutation, and that selection of the mutants took place using higher levels of Foramsulfuron. Following callus induction on M-338 [H76] medium without selection, calli were plated on M-338 [H76] medium containing 0.5 µM Foramsulfuron. After 3 weeks the calli were divided into smaller pieces and replated to M-338 [H76] medium containing 1.0 µM Foramsulfuron. Further increases in Foramsulfuron concentration (e.g. 1.5 µM, 2.0 µM, 2.5 µM 3.0 µM or higher) were made at each subculture until rapidly growing green (resistant) calli could clearly be identified from the brown non-resistant tissues.

The presence in HETO132 of a point mutation in the proline 179 codon and the tryptophan 556 codon (corresponding to the proline 197 and the tryptophan 574 codon in *A. thaliana,* respectively), i.e. a C to T substitution at position 535 and a G to T substitution at position 1667 of the coding sequence of ALS III gene, resulting in a Proline to Serine amino acid substitution at position 179, and of a Tryptophan to Leucine amino acid substitution at position 556 of the encoded protein, respectively was confirmed by sequence analysis.

### Mutant HETO136

*Brassica napus* lines with the HETO136 mutation, i.e. comprising a C to T substitution at position 544 and a G to T substitution at position 1676 in ALS I, resulting in a Proline to Serine amino acid substitution at position 182 and a tryptophan to leucine substitution at position 559 of the ALS I protein were generated as follows.

First, *Brassica napus* lines with the HETO108 mutation, i.e. comprising a C to T substitution at position 544 in ALS I, resulting in a Proline to Serine amino acid substitution at position 182 of the ALS I protein, were generated and identified as described in WO 2011/076345. Based on lines with the HETO108 mutation, lines with the HETO136 mutation were obtained using methods essentially similar to those for obtaining HETO132 (see above), with the modification that the starting material for selection was seed from lines with the HETO108 mutation.

The presence in HETO136 of a point mutation in the proline 182 codon and the tryptophan 559 codon (corresponding to the proline 197 and the tryptophan 574 codon in *A. thaliana,* respectively), i.e. a C to T substitution at position 544 and a G to T substitution at position 1676 of the coding sequence of ALS I gene, resulting in a Proline to Serine amino acid substitution at position 182, and of a Tryptophan to Leucine amino acid substitution at position 559 of the encoded protein, respectively was confirmed by sequence analysis.

Shoots were recovered from mutant HETO132, HETO133, HETO134 and HETO136 calli following transfer to M338 [H67] regeneration medium without herbicide selection. M-338 [H67] medium is identical to M-338 [H76] except it contains 3 mg/l zeatin, 0.1 mg/l NAA instead in place of 2,4-D and kinetin. Small shoots were excised from the calli and transferred to Magenta boxes containing 50 ml of M-338 [H13] medium without Foramsulfuron selection for further development. M-338 [H13] medium is identical to M-338 [H67] except it contains 2.5 µg/l zeatin and no NAA. Shoots with normal looking leaves were transferred to 21 sterile glass containers with 100 ml of M-400 rooting medium. M-400 medium is half strength MS salts and vitamins (Murashige and Skoog, 1962) containing 15 g/l sucrose and solidified with 6 g/l plant agar (pH 6.0). After 4 weeks of culture rooted plants were transferred to the glasshouse.

Seeds comprising HETO132 have been deposited at the NCIMB Limited (Ferguson Building, Craibstone Estate, Bucksburn, Aberdeen, Scotland, AB21 9YA, UK) on July 5, 2013, under accession number NCIMB 42153. Of the deposited seeds, 25% is homozygous for the *HETO132* mutation and 50% is heterozygous for the *HETO132* mutation, which can be identified using methods as described elsewhere in this application. Seeds homozygous for HETO132 have been deposited at the NCIMB Limited (Ferguson Building, Craibstone Estate, Bucksburn, Aberdeen, Scotland, AB21 9YA, UK) on July 1, 2014, under accession number NCIMB 42259.

Seeds comprising HETO136 have been deposited at the NCIMB Limited (Ferguson Building, Craibstone Estate, Bucksburn, Aberdeen, Scotland, AB21 9YA, UK) on July 1, 2014, under accession number NCIMB 42260. Of the deposited seeds, 25% is homozygous for the *HETO136* mutation and 50% is heterozygous for the *HETO136* mutation, which can be identified using methods as described elsewhere in this application.

Seeds comprising HETO134 and HETO133 were obtained by crossing plants comprising HETO134 with plants comprising HETO133. Seeds comprising and HETO134 and HETO133 have been deposited at the NCIMB Limited (Ferguson Building, Craibstone Estate, Bucksburn, Aberdeen, Scotland, AB21 9YA, UK) on May 20, 2013, under accession number NCIMB 42145. Of the deposited seeds, 25% is homozygous for the *HETO134* mutation and 50% is heterozygous for the *HETO134,* and of the deposited seeds 25% is homozygous for the *HETO133* mutation and 50% is heterozygous for the *HETO133* mutation, which can be identified using methods as described elsewhere in this application. Seeds homozygous for HETO133 and HETO134 have been deposited at the NCIMB Limited (Ferguson Building, Craibstone Estate, Bucksburn, Aberdeen, Scotland, AB21 9YA, UK) on May 8, 2014, under accession number NCIMB 42235.

### Example 2 - Combination of HETO132 and HETO134 alleles

Heterozygous *Brassica* plants comprising HETO132 are crossed with heterozygous *Brassica* plants comprising HETO134. The F1 plants have been selfed to obtain the following genotypes:

| ALS I | ALS III |
|---|---|
| -/- | -/- |
| -/- | HETO132/- |
| -/- | HETO132/HETO132 |
| HETO134/- | -/- |
| HETO134/- | HETO132/- |
| HETO134/- | HETO132/HETO132 |
| HETO134/HETO134 | -/- |
| HETO134/HETO134 | HETO132/- |
| HETO134/HETO134 | HETO132/HETO132 |

wherein - indicates the wild-type allele for ALS I and ALS III.

### Example 3 - Measurement of herbicide tolerance of Brassica plants comprising mutant AHAS alleles

The correlation between the presence of mutant *AHAS* alleles in a *Brassica* plant grown in the greenhouse and tolerance to thiencarbazone-methyl and foramsulfuron was determined as follows. Treatment post-emergence at the 1-2 leaf stage was carried out in a spray cabinet with a dose of 5 g a.i./ha of thiencarbazone-methyl and 8.75 g a.i./ha of foramsulfuron. The plants were evaluated for phenotype (height, side branching and leave morphology) on scale of 5 to 1, where; type 5 = normal (corresponding to wildtype unsprayed phenotype); type 4 = normal height, some side branching, normal leaves; type 3 = intermediate height, intermediate side branching, normal leaves; type 2 = short, severe side branching ("bushy"), some leave malformations; type 1 = short, severe side branching ("bushy"), severe leave malformations. Phytotoxicity (PPTOX) was determined and evaluated on a scale of 1 to 9, where 1 = completely yellowing, 5 = 50% of plant is yellow and 9 = no yellowing. For assessment of vigor scores, plants were evaluated on a scale of 1 to 9, where 1=very poor (+/-dead), 5=average, 9=vigorous.

Plants comprising HETO132 (P197S-W574L on ALS III) and HETO134 (W574L on ALS I) were compared to plants comprising W574L on ALS III and P197S on ALS I, to plants comprising W574L on both ALS III and ALS I but lacking the P197S mutation. The HETO132 allele (P197S-W574L on ALS III) was also combined with ALS I having the P197S mutation, and with ALS I comprising on one allele P197S and on the other allele W574L (P197S/W574L).

It can be seen from Table 1 that the plants comprising HETO132 and HETO 134 in homozygous form have the highest herbicide tolerance of all genotypes tested. Moreover, it can be seen that, for plants comprising W574L on all alleles of all ALS genes, the addition of P197S on ALS III further increases herbicide tolerance (compare P197S-W574L/P197S-W574L, W574L/W574L with W574L/W574L, W574L/W574L).

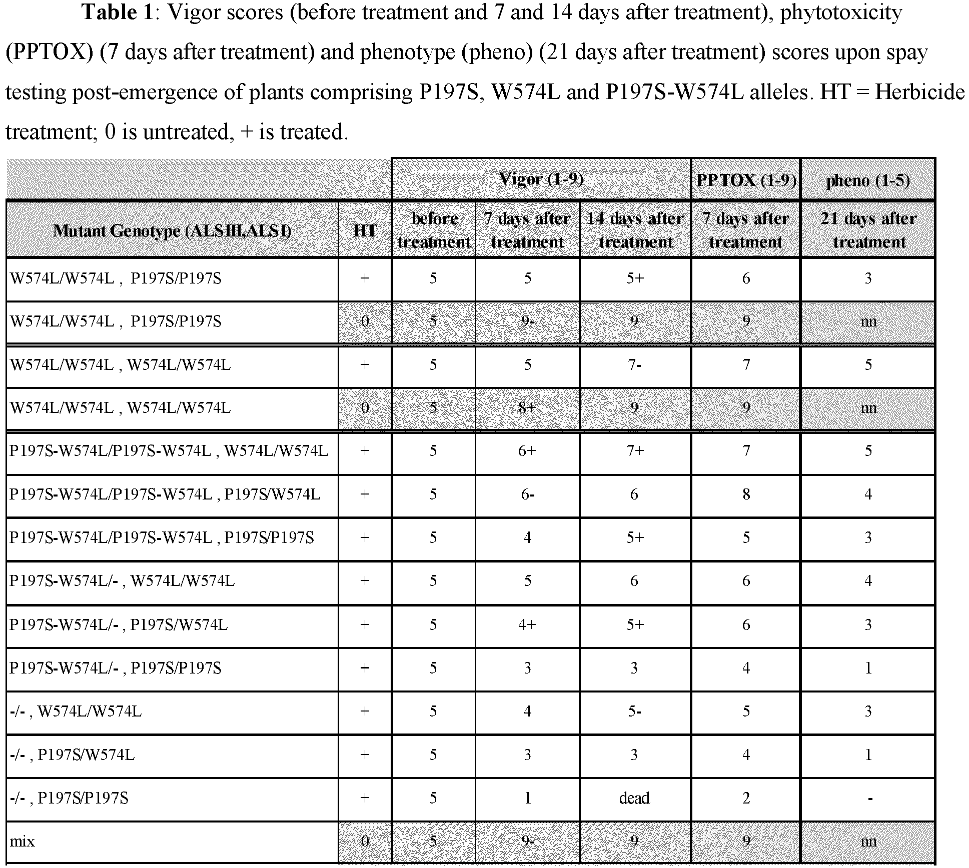

Further combinations of HETO132, HETO133, HETO134 and HETO136 were made by different crossing plants comprising said mutations. The ALS alleles having the P197S mutation only were derived from HETO111 and HETO108. Plants were sprayed at the 1-2 leaf stage with a dose of 60 g a.i./ha of thiencarbazone-methyl and 100 g a.i./ha of foramsulfuron. Vigor was determined before treatment, and 12 days after treatment; PPTOX was determined 12 days after treatment, and Phenotype was determined 14 days after treatment. The results of this analysis are shown in Table 2.

**Table 2: Vigor scores (before treatment and 12 days after treatment), phytotoxicity (PPTOX) (12 days after treatment) and phenotype (pheno) (14 days after treatment) scores upon spay testing post-emergence of plants comprising P197S, W574L and P197S-W574L alleles. HT = Herbicide treatment; 0 is untreated, + is treated.**

| | | **Vigor (1-9)** | | | | **PPTOX (1-9)** | **Pheno (1-5)** |
|---|---|---|---|---|---|---|---|
| **Mutant Genotype (ALSIII, ALSI)** | **HT** | **before treatment** | | | **12 days after treatment** | **12 days after treatment** | **14 days after treatment** |
| | | Vigor | Leaf Blistering | Leaf Chlorosis | | | |
| P197S-W574L/P197S-W574L, P197S/P197S | + | 5 | 8 | 9 | 4 | 5 | 3 |
| P197S-W574L/P197S-W574L, P197S/P197S | 0 | | | | 9 | 9 | 5 |
| W574L/W574L, W574L/W574L | + | 4+ | 8 | 9 | 5 | 7 | 4 |
| W574L/W574L, W574L/W574L | 0 | | | | 9- | 9 | 5 |
| P197S-W574L /P197S-W574L, W574L/W574L | + | 5 | 7 | 8 | 8 | 8 | 5 |
| P197S-W574L /P197S-W574L, W574L/W574L | 0 | | | | 9- | 9 | 5 |
| P197S-W574L /P197S-W574L, W574L/W574L | + | 4 | 7 | 7- | 8 | 8- | 5 |
| P197S-W574L /P197S-W574L, W574L/W574L | 0 | | | | 9 | 9 | 5 |
| P197S-W574L/P197S-W574L, P197S-W574L/P197S-W574L | + | 3-5 | 8 | 7 | 8 | 8 | 5 |
| P197S-W574L/P197S-W574L, P197S-W574L/P197S | + | | | | 7 | 7 | 5 |
| P197S-W574L/P197S-W574L, P197S/P197S | + | | | | 5 | 5 | 3 |
| P197S-W574L/P197S, P197S-W574L/P197S-W574L | + | | | | 6 | 6 | 4 |
| P197S/P197S, P197S-W574L/P197S-W574L | + | | | | 4 | 5 | 3 |
| P197S-W574L/P197S, P197S-W574L/P197S | + | | | | 4+ | 5 | 3 |
| P197S-W574L/P197S, P197S/P197S | + | | | | 2+ | 3 | 1 |
| P197S/P197S, P197S-W574L/P197S | + | | | | 2+ | 3 | 1 |
| P197S/P197S, P197S/P197S | + | | | | 1 | 1 | - |
| Mix P197S-W574L, P197S-W574L | 0 | | | | 9- | 9 | 5 |

Table 2 shows that, for plants comprising W574L on all alleles of all ALS genes, the addition of P197S on ALS III, or on both ALS I and ALS III, further increases herbicide tolerance (compare P197S-W574L/P197S-W574L, W574L/W574L with W574L/W574L, W574L/W574L). It can further be seen that addition of W574L to an allele already comprising P197S further increases herbicide tolerance.

### Example 4 - Measurement of herbicide tolerance of Brassica plants comprising mutant AHAS alleles in the field

Seeds of spring oilseed rape homozygous for HETO132 and HETO134, and seeds of spring oilseed rape homozygous for HETO133 and HETO 134 were sown in a field according to typical practical agricultural methods. Several ALS inhibitor herbicides were applied to the oilseed rape plants by using specific spray equipment for small plot applications. At 9, 16 and 24 days after application, the visible phytotoxicity on the oilseed rape plant was assessed according to a scale from 0% to 100%: 0% = no phytotoxic effects, comparable to untreated 100% = complete control, all plants killed. The results are shown in Table 3. The use of all herbicides in the test lead to a clearly better tolerance of the spring oilseed rape lines homozygous for HETO132 and HETO134 as compared to a standard variety not comprising ALS alleles according to the invention. Moreover, in particular 24 days after application, plants comprising HETO132 and HETO 134 showed a better tolerance than plants comprising HETO133 and HETO134, showing that the addition of P197S on ALSIII in plants comprising W574L on all ALS genes, further increases herbicide tolerance to the majority of tested herbicides, including higher concentrations foramsulfuron and thiencarbazone-methyl.

### Example 5 - In vitro ALS inhibitor sensitivity and kinetic parameters of proteins encoded by different AHAS mutants according to the invention

The ALS inhibitor sensitivity and kinetic parameters of AHAS proteins comprising the P197S mutation, the W574L mutation, and both the P197S and W574L mutation in one ALS protein, were determined essentially as described in WO 2013/127766. ALS inhibitor sensitivity of a mixture of two ALS proteins, one of which comprising the P197S and the other comprising the W574L mutations were calculated as described in WO 2013/127766. The results of this analysis are shown in Table 4. It is shown that AHAS protein comprising both the P197S and W574 mutation on the same protein have increased tolerance to the herbicides that were tested as compared to proteins comprising the P197S mutation, as compared to proteins comprising the W574L mutation, and as compared to the mixture of W574L proteins and P197S proteins.

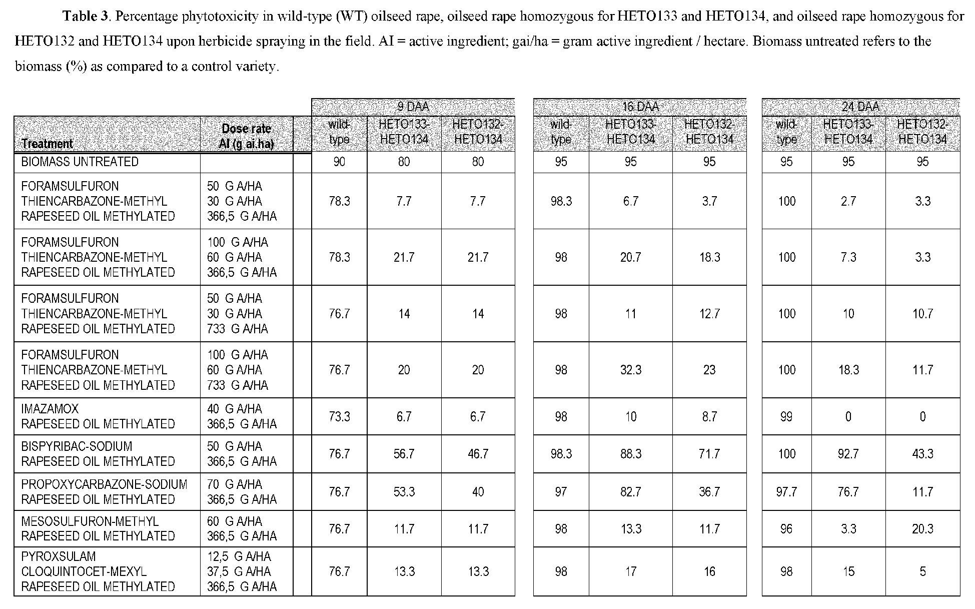

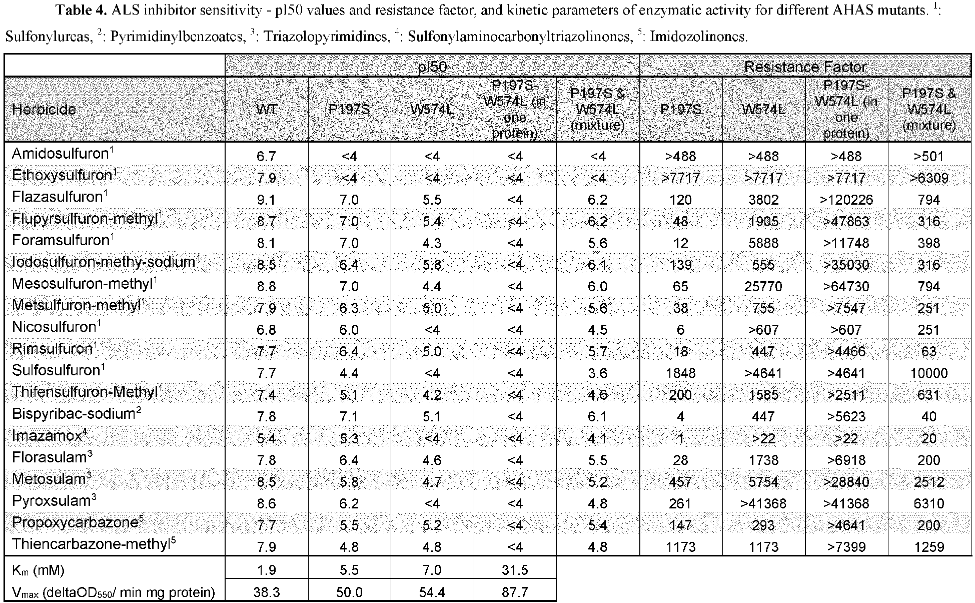

### Example 6 - Detection and/or transfer of mutant AHAS alleles into (elite) Brassica lines

The mutant AHAS genes are transferred into (elite) *Brassica* breeding lines by the following method: A plant containing a mutant AHAS gene (donor plant), is crossed with an (elite) *Brassica* line (elite parent / recurrent parent) or variety lacking the mutant *AHAS* gene. The following introgression scheme is used (the mutant *AHAS* allele is abbreviated to *ahas* while the wild type is depicted as *AHAS*):
Initial cross: *ahas* / *ahas* (donor plant) X *AHAS*/*AHAS* (elite parent)
F1 plant: *AHAS*/*ahas*
BC1 cross: *AHAS*/*ahas* X *AHAS*/*AHAS* (recurrent parent)
BC1 plants: *50% AHAS*/*ahas* and *50% AHAS* / *HAS*
The 50% *ahas* / *AHAS* are selected by direct sequencing or using molecular markers (e.g. AFLP, PCR, Invader™, TaqMan® and the like) for the mutant *AHAS* allele *(ahas).*
BC2 cross: *AHAS* /*AHAS* (BC1 plant) X *AHAS*/*AHAS* (recurrent parent)
BC2 plants: 50% *AHAS*/*ahas* and 50% *AHAS*/*AHAS*
The 50% *AHAS* / *AHAS* are selected by direct sequencing or using molecular markers for the mutant *AHAS* allele (*ahas*).
Backcrossing is repeated until BC3 to BC6
BC3-6 plants: 50% AHAS / ahas and 50% AHAS / ahas
The 50% AHAS / ahas are selected using molecular markers for the mutant AHAS allele (ahas). To reduce the number of backcrossings (e.g. until BC3 in stead of BC6), molecular markers can be used specific for the genetic background of the elite parent.
BC3-6 S1 cross: AHAS / ahas X AHAS / ahas
BC3-6 S1 plants: 25% AHAS / AHAS and 50% AHAS / ahas and 25% ahas / ahas
Plants containing ahas are selected using molecular markers for the mutant AHAS allele (AHAS). Individual BC3-6 S1 or BC3-6 S2 plants that are homozygous for the mutant AHAS allele (ahas / ahas) are selected using molecular markers for the mutant and the wild-type AHAS alleles. These plants are then used for seed production.

To select for plants comprising a point mutation in an AHAS allele, direct sequencing by standard sequencing techniques known in the art, such as those described in Example 1, can be used.

## Claims

1. An allotetraploid ALS inhibitor herbicide tolerant *Brassica* plant or parts thereof comprising at least two ALS genes, wherein all endogenous ALS genes encode an ALS polypeptide comprising at a position corresponding to position 574 of SEQ ID NO: 10 instead of the naturally encoded amino acid tryptophan the amino acid leucine, and wherein at least one of the endogenous ALS genes encodes an ALS polypeptide which further comprises at a position corresponding to position 197 of SEQ ID NO: 10 instead of the naturally encoded amino acid proline the amino acid serine, **characterized in that** said plant or parts thereof is homozygous for said ALS genes encoding an ALS polypeptide comprising at a position corresponding to position 574 of SEQ ID NO: 10 instead of the naturally encoded amino acid tryptophan the amino acid leucine, and is homozygous for said ALS genes encoding an ALS polypeptide comprising at a position corresponding to position 197 of SEQ ID NO: 10 instead of the naturally encoded amino acid proline the amino acid serine and comprising at a position corresponding to position 574 of SEQ ID NO: 10 instead of the naturally encoded amino acid tryptophan the amino acid leucine.

2. The plant or parts thereof according to claim 1, wherein all ALS genes encode an ALS polypeptide comprising at a position corresponding to position 574 of SEQ ID NO: 10 instead of the naturally encoded amino acid tryptophan the amino acid leucine, and comprising at a position corresponding to position 197 of SEQ ID NO: 10 instead of the naturally encoded amino acid proline the amino acid serine.

3. The plant or parts thereof according to claim 1 or 2, which is selected from the group consisting of:
a. *Brassica napus* comprising an ALS I gene encoding an ALS I polypeptide comprising at a position corresponding to position 182 of SEQ ID NO: 2 instead of the naturally encoded amino acid proline the amino acid serine and comprising at a position corresponding to position 559 of SEQ ID NO: 2 instead of the naturally encoded amino acid tryptophan the amino acid leucine, and an ALS III gene encoding an ALS III polypeptide comprising at a position corresponding to position 556 of SEQ ID NO: 4 instead of the naturally encoded amino acid tryptophan the amino acid leucine;
b. *Brassica napus* comprising an ALS I gene encoding an ALS I polypeptide comprising at a position corresponding to position 559 of SEQ ID NO: 2 instead of the naturally encoded amino acid tryptophan the amino acid leucine, and an ALS III gene encoding an ALS III polypeptide comprising at a position corresponding to position 179 of SEQ ID NO: 4 instead of the naturally encoded amino acid proline the amino acid serine and comprising at a position corresponding to position 556 of SEQ ID NO: 4 instead of the naturally encoded amino acid tryptophan the amino acid leucine;
c. *Brassica napus* comprising an ALS I gene encoding an ALS I polypeptide comprising at a position corresponding to position 182 of SEQ ID NO: 2 instead of the naturally encoded amino acid proline the amino acid serine and comprising at a position corresponding to position 559 of SEQ ID NO: 2 instead of the naturally encoded amino acid tryptophan the amino acid leucine, and an ALS III gene encoding an ALS III polypeptide comprising at a position corresponding to position 179 of SEQ ID NO: 4 instead of the naturally encoded amino acid proline the amino acid serine and comprising at a position corresponding to position 556 of SEQ ID NO: 4 instead of the naturally encoded amino acid tryptophan the amino acid leucine;
d. *Brassica juncea* comprising an ALS-A gene encoding an ALS-A polypeptide comprising at a position corresponding to position 179 of SEQ ID NO: 6 instead of the naturally encoded amino acid proline the amino acid serine and comprising at a position corresponding to position 556 of SEQ ID NO: 6 instead of the naturally encoded amino acid tryptophan the amino acid leucine, and an ALS-B gene encoding an ALS-B polypeptide comprising at a position corresponding to position 559 of SEQ ID NO: 8 instead of the naturally encoded amino acid tryptophan the amino acid leucine;
e. *Brassica juncea* comprising an ALS-A gene encoding an ALS-A polypeptide comprising at a position corresponding to position 556 of SEQ ID NO: 6 instead of the naturally encoded amino acid tryptophan the amino acid leucine, and an ALS-B gene encoding an ALS-B polypeptide comprising at a position corresponding to position 182 of SEQ ID NO: 8 instead of the naturally encoded amino acid proline the amino acid serine and comprising at a position corresponding to position 559 of SEQ ID NO: 8 instead of the naturally encoded amino acid tryptophan the amino acid leucine; and
f. *Brassica juncea* comprising an ALS-A gene encoding an ALS-A polypeptide comprising at a position corresponding to position 179 of SEQ ID NO: 6 instead of the naturally encoded amino acid proline the amino acid serine and comprising at a position corresponding to position 556 of SEQ ID NO: 6 instead of the naturally encoded amino acid tryptophan the amino acid leucine, and an ALS-B gene encoding an ALS-B polypeptide comprising at a position corresponding to position 182 of SEQ ID NO: 8 instead of the naturally encoded amino acid proline the amino acid serine and comprising at a position corresponding to position 559 of SEQ ID NO: 8 instead of the naturally encoded amino acid tryptophan the amino acid leucine.

4. The *Brassica* plant or parts thereof according to claim 3, which is selected from the group consisting of:
a. *B. napus* comprising an ALS I gene encoding an ALS I polypeptide which is at least 90% identical to SEQ ID NO: 2 of which the proline at position 182 is substituted with a serine, and of which the tryptophan at position 559 is substituted with a leucine, and an ALS III gene encoding an ALS III polypeptide which is at least 90% identical to SEQ ID NO: 4 of which the tryptophan at position 556 is substituted with a leucine;
b. *B. napus* comprising an ALS I gene encoding an ALS I polypeptide which is at least 90% identical to SEQ ID NO: 2 of which the tryptophan at position 559 is substituted with a leucine, and an ALS III gene encoding an ALS III polypeptide which is at least 90% identical to SEQ ID NO: 4 of which the proline at position 179 is substituted with a serine and of which the tryptophan at position 556 is substituted with a leucine;
c. *B. napus* comprising an ALS I gene encoding an ALS I polypeptide which is at least 90% identical to SEQ ID NO: 2 of which the proline at position 182 is substituted with a serine, and of which the tryptophan at position 559 is substituted with a leucine, and an ALS III gene encoding an ALS III polypeptide which is at least 90% identical to SEQ ID NO: 4 of which the proline at position 179 is substituted with a serine and of which the tryptophan at position 556 is substituted with a leucine;
d. *B. juncea* comprising an ALS-A gene encoding an ALS-A polypeptide which is at least 90% identical to SEQ ID NO: 6 of which the proline at position 179 is substituted with a serine and of which the tryptophan at position 556 is substituted with a leucine, and an ALS-B gene encoding an ALS-B polypeptide which is at least 90% identical to SEQ ID NO: 8 of which the tryptophan at position 559 is substituted with a leucine;
e. *B. juncea* comprising an ALS-A gene encoding an ALS-A polypeptide which is at least 90% identical to SEQ ID NO: 6 of which the tryptophan at position 556 is substituted with a leucine, and an ALS-B gene encoding an ALS-B polypeptide which is at least 90% identical to SEQ ID NO: 8 of which the proline at position 182 is substituted with a serine, and of which the tryptophan at position 559 is substituted with a leucine; and
f. *B. juncea* comprising an ALS-A gene encoding an ALS-A polypeptide which is at least 90% identical to SEQ ID NO: 6 of which the proline at position 179 is substituted with a serine and of which the tryptophan at position 556 is substituted with a leucine, and an ALS-B gene encoding an ALS-B polypeptide which is at least 90% identical to SEQ ID NO: 8 of which the proline at position 182 is substituted with a serine, and of which the tryptophan at position 559 is substituted with a leucine.

5. The *Brassica* plant or parts thereof according to claim 4, which is *B. napus,* which is selected from the group consisting of:
a. a *B. napus* plant or parts thereof wherein said ALS I gene comprises the nucleotide sequence corresponding to SEQ ID NO: 1 of which the C at position 544 is substituted with T and of which the G at position 1676 is substituted with T, and said ALS III gene comprises the nucleotide sequence corresponding to SEQ ID NO: 3 of which the G at position 1667 is substituted with T, and which is obtainable from seeds deposited at NCIMB under accession numbers NCIMB 42145, NCIMB 42235, and/or NCIMB 42260;
b. a *B. napus* plant or parts thereof wherein said ALS I gene comprises the nucleotide sequence corresponding to SEQ ID NO: 1 of which the G at position 1676 is substituted with T, and said ALS III gene comprises the nucleotide sequence corresponding to SEQ ID NO: 3 of which the C at position 535 is substituted with T and of which the G at position 1667 is substituted with T, and which is obtainable from seeds deposited at NCIMB under accession numbers NCIMB 42145, NCIMB 42235, NCIMB 42153, and/or NCIMB 42259; and
c. a *B. napus* plant or parts thereof wherein said ALS I gene comprises the nucleotide sequence corresponding to SEQ ID NO: 1 of which the C at position 544 is substituted with T and of which the G at position 1676 is substituted with T, and said ALS III gene comprises the nucleotide sequence corresponding to SEQ ID NO: 3 of which the C at position 535 is substituted with T and of which the G at position 1667 is substituted with T, and which is obtainable from seeds deposited at NCIMB under accession numbers NCIMB 42153, NCIMB 42259, and/or NCIMB 42260.

6. The *Brassica* plant or parts thereof according to claim 4, which is *B. napus,* which is selected from the group consisting of:
a. a *B. napus* plant or parts thereof wherein said ALS I gene comprises the nucleotide sequence corresponding to SEQ ID NO: 1 of which the C at position 544 is substituted with T and of which the G at position 1676 is substituted with T, and said ALS III gene comprises the nucleotide sequence corresponding to SEQ ID NO: 3 of which the G at position 1667 is substituted with T, reference seed of said plant having been deposited at NCIMB under accession numbers NCIMB 42145, 42235 and NCIMB 42260,
b. a *B. napus* plant or parts thereof wherein said ALS I gene comprises the nucleotide sequence corresponding to SEQ ID NO: 1 of which the G at position 1676 is substituted with T, and said ALS III gene comprises the nucleotide sequence corresponding to SEQ ID NO: 3 of which the C at position 535 is substituted with T and of which the G at position 1667 is substituted with T, reference seed of said plant having been deposited at NCIMB under accession numbers NCIMB 42145, NCIMB 42235, NCIMB 42153, and NCIMB 42259, and
c. a *B. napus* plant or parts thereof wherein said ALS I gene comprises the nucleotide sequence corresponding to SEQ ID NO: 1 of which the C at position 544 is substituted with T and of which the G at position 1676 is substituted with T, and said ALS III gene comprises the nucleotide sequence corresponding to SEQ ID NO: 3 of which the C at position 535 is substituted with T and of which the G at position 1667 is substituted with T, reference seed of said plant having been deposited at NCIMB under accession numbers NCIMB 42153, NCIMB 42259, and NCIMB 42260.

7. The plant or parts thereof according to any one of claims 1 to 6 which are tolerant to one or more ALS-inhibitor herbicides belonging to the group consisting of sulfonylurea herbicides, sulfonylaminocarbonyltriazolinone herbicides, imidazolinone herbicides, triazolopyrimidine herbicides, and pyrimidinyl(thio)benzoate herbicides.

8. Progeny of a plant according to any one of claims 1 to 7 obtained by further breeding with said plant, wherein all ALS genes of said progeny encode an ALS polypeptide comprising at a position corresponding to position 574 of SEQ ID NO: 10 instead of the naturally encoded amino acid tryptophan the amino acid leucine and wherein at least one of the ALS genes encodes an ALS polypeptide which further comprises at a position corresponding to position 197 of SEQ ID NO: 10 instead of the naturally encoded amino acid proline the amino acid serine.

9. A hybrid plant produced from crossing a parent plant according to any one of claims 1 to 7 with a second parent plant and harvesting a resultant hybrid seed and growing said seed, wherein all ALS genes of said hybrid plant encode an ALS polypeptide comprising at a position corresponding to position 574 of SEQ ID NO: 10 instead of the naturally encoded amino acid tryptophan the amino acid leucine, and wherein at least one of the ALS genes encodes an ALS polypeptide which further comprises at a position corresponding to position 197 of SEQ ID NO: 10 instead of the naturally encoded amino acid proline the amino acid serine.

## Patentansprüche

1. Allotetraploide gegenüber ALS-Inhibitor-Herbiziden tolerante *Brassica-*Pflanze oder Teile davon, umfassend wenigstens zwei ALS-Gene, wobei alle endogenen ALS-Gene ein ALS-Polypeptid codieren, das an einer Position 574 von SEQ ID NO: 10 entsprechenden Position statt der natürlich codierten Aminosäure Tryptophan die Aminosäure Leucin umfasst, und wobei wenigstens eines der endogenen ALS-Gene ein ALS-Polypeptid codiert, das weiter an einer Position 197 von SEQ ID NO: 10 entsprechenden Position statt der natürlich codierten Aminosäure Prolin die Aminosäure Serin umfasst, **dadurch gekennzeichnet, dass** die Pflanze oder Teile davon homozygot für die ALS-Gene, die ein ALS-Polypeptid codieren, das an einer Position 574 von SEQ ID NO: 10 entsprechenden Position statt der natürlich codierten Aminosäure Tryptophan die Aminosäure Leucin umfasst, und homozygot für die ALS-Gene, die ein ALS-Polypeptid codieren, das an einer Position 197 von SEQ ID NO: 10 entsprechenden Position statt der natürlich codierten Aminosäure Prolin die Aminosäure Serin und an einer Position 574 von SEQ ID NO: 10 entsprechenden Position statt der natürlich codierten Aminosäure Tryptophan die Aminosäure Leucin umfasst, ist.

2. Pflanze oder Teile davon nach Anspruch 1, wobei alle ALS-Gene ein ALS-Polypeptid codieren, das an einer Position 574 von SEQ ID NO: 10 entsprechenden Position statt der natürlich codierten Aminosäure Tryptophan die Aminosäure Leucin und an einer Position 197 von SEQ ID NO: 10 entsprechenden Position statt der natürlich codierten Aminosäure Prolin die Aminosäure Serin umfasst.

3. Pflanze oder Teile davon nach Anspruch 1 oder 2, die ausgewählt ist aus der Gruppe bestehend aus:
a. *Brassica napus,* umfassend ein ALS-I-Gen, das ein ALS-I-Polypeptid codiert, das an einer Position 182 von SEQ ID NO: 2 entsprechenden Position statt der natürlich codierten Aminosäure Prolin die Aminosäure Serin und an einer Position 559 von SEQ ID NO: 2 entsprechenden Position statt der natürlich codierten Aminosäure Tryptophan die Aminosäure Leucin umfasst, und ein ALS-III-Gen, das ein ALS-III-Polypeptid codiert, das an einer Position 556 von SEQ ID NO: 4 entsprechenden Position statt der natürlich codierten Aminosäure Tryptophan die Aminosäure Leucin umfasst;
b. *Brassica napus,* umfassend ein ALS-I-Gen, das ein ALS-I-Polypeptid codiert, das an einer Position 559 von SEQ ID NO: 2 entsprechenden Position statt der natürlich codierten Aminosäure Tryptophan die Aminosäure Leucin umfasst, und ein ALS-III-Gen, das ein ALS-III-Polypeptid codiert, das an einer Position 179 von SEQ ID NO: 4 entsprechenden Position statt der natürlich codierten Aminosäure Prolin die Aminosäure Serin und an einer Position 556 von SEQ ID NO: 4 entsprechenden Position statt der natürlich codierten Aminosäure Tryptophan die Aminosäure Leucin umfasst;
c. *Brassica napus,* umfassend ein ALS-I-Gen, das ein ALS-I-Polypeptid codiert, das an einer Position 182 von SEQ ID NO: 2 entsprechenden Position statt der natürlich codierten Aminosäure Prolin die Aminosäure Serin und an einer Position 559 von SEQ ID NO: 2 entsprechenden Position statt der natürlich codierten Aminosäure Tryptophan die Aminosäure Leucin umfasst, und ein ALS-III-Gen, das ein ALS-III-Polypeptid codiert, das an einer Position 179 von SEQ ID NO: 4 entsprechenden Position statt der natürlich codierten Aminosäure Prolin die Aminosäure Serin und an einer Position 556 von SEQ ID NO: 4 entsprechenden Position statt der natürlich codierten Aminosäure Tryptophan die Aminosäure Leucin umfasst;
d. *Brassica juncea,* umfassend ein ALS-A-Gen, das ein ALS-A-Polypeptid codiert, das an einer Position 179 von SEQ ID NO: 6 entsprechenden Position statt der natürlich codierten Aminosäure Prolin die Aminosäure Serin und an einer Position 556 von SEQ ID NO: 6 entsprechenden Position statt der natürlich codierten Aminosäure Tryptophan die Aminosäure Leucin umfasst, und ein ALS-B-Gen, das ein ALS-B-Polypeptid codiert, das an einer Position 559 von SEQ ID NO: 8 entsprechenden Position statt der natürlich codierten Aminosäure Tryptophan die Aminosäure Leucin umfasst;
e. *Brassica juncea,* umfassend ein ALS-A-Gen, das ein ALS-A-Polypeptid codiert, das an einer Position 556 von SEQ ID NO: 6 entsprechenden Position statt der natürlich codierten Aminosäure Tryptophan die Aminosäure Leucin umfasst, und ein ALS-B-Gen, das ein ALS-B-Polypeptid codiert, das an einer Position 182 von SEQ ID NO: 8 entsprechenden Position statt der natürlich codierten Aminosäure Prolin die Aminosäure Serin und an einer Position 559 von SEQ ID NO: 8 entsprechenden Position statt der natürlich codierten Aminosäure Tryptophan die Aminosäure Leucin umfasst; und
f. *Brassica juncea,* umfassend ein ALS-A-Gen, das ein ALS-A-Polypeptid codiert, das an einer Position 179 von SEQ ID NO: 6 entsprechenden Position statt der natürlich codierten Aminosäure Prolin die Aminosäure Serin und an einer Position 556 von SEQ ID NO: 6 entsprechenden Position statt der natürlich codierten Aminosäure Tryptophan die Aminosäure Leucin umfasst, und ein ALS-B-Gen, das ein ALS-B-Polypeptid codiert, das an einer Position 182 von SEQ ID NO: 8 entsprechenden Position statt der natürlich codierten Aminosäure Prolin die Aminosäure Serin und an einer Position 559 von SEQ ID NO: 8 entsprechenden Position statt der natürlich codierten Aminosäure Tryptophan die Aminosäure Leucin umfasst.

4. Brassica-Pflanze oder Teile davon nach Anspruch 3, die ausgewählt ist aus der Gruppe bestehend aus:
a. *B. napus,* umfassend ein ALS-I-Gen, das ein ALS-I-Polypeptid codiert, das zu wenigstens 90% mit SEQ ID NO: 2 identisch ist, von der das Prolin an Position 182 durch ein Serin substituiert ist und von der das Tryptophan an Position 559 durch ein Leucin substituiert ist, und ein ALS-III-Gen, das ein ALS-III-Polypeptid codiert, das zu wenigstens 90% mit SEQ ID NO: 4 identisch ist, von der das Tryptophan an Position 556 durch ein Leucin substituiert ist;
b. *B. napus,* umfassend ein ALS-I-Gen, das ein ALS-I-Polypeptid codiert, das zu wenigstens 90% mit SEQ ID NO: 2 identisch ist, von der das Tryptophan an Position 559 durch ein Leucin substituiert ist, und ein ALS-III-Gen, das ein ALS-III-Polypeptid codiert, das zu wenigstens 90% mit SEQ ID NO: 4 identisch ist, von der das Prolin an Position 179 durch ein Serin substituiert ist und von der das Tryptophan an Position 556 durch ein Leucin substituiert ist;
c. *B. napus,* umfassend ein ALS-I-Gen, das ein ALS-I-Polypeptid codiert, das zu wenigstens 90% mit SEQ ID NO: 2 identisch ist, von der das Prolin an Position 182 durch ein Serin substituiert ist und von der das Tryptophan an Position 559 durch ein Leucin substituiert ist, und ein ALS-III-Gen, das ein ALS-III-Polypeptid codiert, das zu wenigstens 90% mit SEQ ID NO: 4 identisch ist, von der das Prolin an Position 179 durch ein Serin substituiert ist und von der das Tryptophan an Position 556 durch ein Leucin substituiert ist;
d. *B. juncea,* umfassend ein ALS-A-Gen, das ein ALS-A-Polypeptid codiert, das zu wenigstens 90% mit SEQ ID NO: 6 identisch ist, von der das Prolin an Position 179 durch ein Serin substituiert ist und von der das Tryptophan an Position 556 durch ein Leucin substituiert ist, und ein ALS-B-Gen, das ein ALS-B-Polypeptid codiert, das zu wenigstens 90% mit SEQ ID NO: 8 identisch ist, von der das Tryptophan an Position 559 durch ein Leucin substituiert ist;
e. *B. juncea,* umfassend ein ALS-A-Gen, das ein ALS-A-Polypeptid codiert, das zu wenigstens 90% mit SEQ ID NO: 6 identisch ist, von der das Tryptophan an Position 556 durch ein Leucin substituiert ist, und ein ALS-B-Gen, das ein ALS-B-Polypeptid codiert, das zu wenigstens 90% mit SEQ ID NO: 8 identisch ist, von der das Prolin an Position 182 durch ein Serin substituiert ist und von der das Tryptophan an Position 559 durch ein Leucin substituiert ist; und
f. *B. juncea,* umfassend ein ALS-A-Gen, das ein ALS-A-Polypeptid codiert, das zu wenigstens 90% mit SEQ ID NO: 6 identisch ist, von der das Prolin an Position 179 durch ein Serin substituiert ist und von der das Tryptophan an Position 556 durch ein Leucin substituiert ist, und ein ALS-B-Gen, das ein ALS-B-Polypeptid codiert, das zu wenigstens 90% mit SEQ ID NO: 8 identisch ist, von der das Prolin an Position 182 durch ein Serin substituiert ist und von der das Tryptophan an Position 559 durch ein Leucin substituiert ist.

5. Brassica-Pflanze oder Teile davon nach Anspruch 4, bei der es sich um *B. napus* handelt, die ausgewählt ist aus der Gruppe bestehend aus:
a. einer *B*. *napus*-Pflanze oder Teilen davon, wobei das ALS-I-Gen die SEQ ID NO: 1 entsprechende Nukleotidsequenz, von der das C an Position 544 durch T und das G an Position 1676 durch T substituiert ist, umfasst und das ALS-III-Gen die SEQ ID NO: 3 entsprechende Nukleotidsequenz, von der das G an Position 1667 durch T substituiert ist, umfasst, und die aus Samen erhältlich ist, die bei NCIMB unter Zugangsnummer NCIMB 42145, NCIMB 42235 und/oder NCIMB 42260 hinterlegt sind;
b. einer *B*. *napus*-Pflanze oder Teilen davon, wobei das ALS-I-Gen die SEQ ID NO: 1 entsprechende Nukleotidsequenz, von der das G an Position 1676 durch T substituiert ist, umfasst und das ALS-III-Gen die SEQ ID NO: 3 entsprechende Nukleotidsequenz, von der das C an Position 535 durch T und das G an Position 1667 durch T substituiert ist, umfasst, und die aus Samen erhältlich ist, die bei NCIMB unter Zugangsnummer NCIMB 42145, NCIMB 42235, NCIMB 42153 und/oder NCIMB 42259 hinterlegt sind; und
c. einer *B. napus*-Pflanze oder Teilen davon, wobei das ALS-I-Gen die SEQ ID NO: 1 entsprechende Nukleotidsequenz, von der das C an Position 544 durch T und das G an Position 1676 durch T substituiert ist, umfasst und das ALS-III-Gen die SEQ ID NO: 3 entsprechende Nukleotidsequenz, von der das C an Position 535 durch T und das G an Position 1667 durch T substituiert ist, umfasst, und die aus Samen erhältlich ist, die bei NCIMB unter Zugangsnummer NCIMB 42153, NCIMB 42259 und/oder NCIMB 42260 hinterlegt sind.

6. *Brassica*-Pflanze oder Teile davon nach Anspruch 4, bei der es sich um *B. napus* handelt, die ausgewählt ist aus der Gruppe bestehend aus:
a. einer *B. napus*-Pflanze oder Teilen davon, wobei das ALS-I-Gen die SEQ ID NO: 1 entsprechende Nukleotidsequenz, von der das C an Position 544 durch T und das G an Position 1676 durch T substituiert ist, umfasst und das ALS-III-Gen die SEQ ID NO: 3 entsprechende Nukleotidsequenz, von der das G an Position 1667 durch T substituiert ist, umfasst, wobei Referenzsamen der Pflanze bei NCIMB unter Zugangsnummer NCIMB 42145, 42235 und NCIMB 42260 hinterlegt wurde;
b. einer *B. napus*-Pflanze oder Teilen davon, wobei das ALS-I-Gen die SEQ ID NO: 1 entsprechende Nukleotidsequenz, von der das G an Position 1676 durch T substituiert ist, umfasst und das ALS-III-Gen die SEQ ID NO: 3 entsprechende Nukleotidsequenz, von der das C an Position 535 durch T und das G an Position 1667 durch T substituiert ist, umfasst, wobei Referenzsamen der Pflanze bei NCIMB unter Zugangsnummer NCIMB 42145, NCIMB 42235, NCIMB 42153 und NCIMB 42259 hinterlegt wurde; und
c. einer *B. napus*-Pflanze oder Teilen davon, wobei das ALS-I-Gen die SEQ ID NO: 1 entsprechende Nukleotidsequenz, von der das C an Position 544 durch T und das G an Position 1676 durch T substituiert ist, umfasst und das ALS-III-Gen die SEQ ID NO: 3 entsprechende Nukleotidsequenz, von der das C an Position 535 durch T und das G an Position 1667 durch T substituiert ist, umfasst, wobei Referenzsamen der Pflanze bei NCIMB unter Zugangsnummer NCIMB 42153, NCIMB 42259 und NCIMB 42260 hinterlegt wurde.

7. Pflanze oder Teile davon nach einem der Ansprüche 1 bis 6, die gegenüber einem oder mehreren ALS-Inhibitor-Herbiziden, die zur Gruppe bestehend aus Sulfonylharnstoff-Herbiziden, Sulfonylaminocarbonyltriazolinon-Herbiziden, Imidazolinon-Herbiziden, Triazolopyrimidin-Herbiziden und Pyrimidinyl(thio)benzoat-Herbiziden gehören, tolerant sind.

8. Nachkommen einer Pflanze nach einem der Ansprüche 1 bis 7, erhalten durch weitere Züchtung mit der Pflanze, wobei alle ALS-Gene der Nachkommen ein ALS-Polypeptid codieren, das an einer Position 574 von SEQ ID NO: 10 entsprechenden Position statt der natürlich codierten Aminosäure Tryptophan die Aminosäure Leucin umfasst, und wobei wenigstens eines der ALS-Gene ein ALS-Polypeptid codiert, das weiter an einer Position 197 von SEQ ID NO: 10 entsprechenden Position statt der natürlich codierten Aminosäure Prolin die Aminosäure Serin umfasst.

9. Hybridpflanze, erzeugt aus Kreuzen einer Elternpflanze nach einem der Ansprüche 1 bis 7 mit einer zweiten Elternpflanze und Ernten eines resultierenden Hybridsamens und Kultivieren des Samens, wobei alle ALS-Gene der Hybridpflanze ein ALS-Polypeptid codieren, das an einer Position 574 von SEQ ID NO: 10 entsprechenden Position statt der natürlich codierten Aminosäure Tryptophan die Aminosäure Leucin umfasst, und wobei wenigstens eines der ALS-Gene ein ALS-Polypeptid codiert, das weiter an einer Position 197 von SEQ ID NO: 10 entsprechenden Position statt der natürlich codierten Aminosäure Prolin die Aminosäure Serin umfasst.

## Revendications

1. Plante *Brassica* allotétraploïde tolérante aux herbicides inhibiteurs d'ALS ou parties de celle-ci comprenant au moins deux gènes ALS, dans lesquelles tous les gènes ALS endogènes codent pour un polypeptide ALS comprenant, à une position correspondant à la position 574 de SEQ ID NO : 10, à la place de l'acide aminé tryptophane codé naturellement, l'acide aminé leucine, et dans lesquelles au moins un des gènes ALS endogènes code pour un polypeptide ALS qui comprend en outre, à une position correspondant à la position 197 de SEQ ID NO : 10, à la place de l'acide aminé proline codé naturellement, l'acide aminé sérine, **caractérisées en ce que** ladite plante ou des parties de celle-ci sont homozygotes pour lesdits gènes ALS codant pour un polypeptide ALS comprenant, à une position correspondant à la position 574 de SEQ ID NO : 10, à la place de l'acide aminé tryptophane codé naturellement, l'acide aminé leucine, et sont homozygotes pour lesdits gènes ALS codant pour un polypeptide ALS comprenant, à une position correspondant à la position 197 de SEQ ID NO : 10, à la place de l'acide aminé proline codé naturellement, l'acide aminé sérine et comprenant, à une position correspondant à la position 574 de SEQ ID NO : 10, à la place de l'acide aminé tryptophane codé naturellement, l'acide aminé leucine.

2. Plante ou parties de celle-ci selon la revendication 1, dans lesquelles tous les gènes ALS codent pour un polypeptide ALS comprenant, à une position correspondant à la position 574 de SEQ ID NO : 10, à la place de l'acide aminé tryptophane codé naturellement, l'acide aminé leucine, et comprenant, à une position correspondant à la position 197 de SEQ ID NO : 10, à la place de l'acide aminé proline codé naturellement, l'acide aminé sérine.

3. Plante ou parties de celle-ci selon la revendication 1 ou 2, qui sont choisies dans le groupe constitué de :
a. *Brassica napus* comprenant un gène ALS I codant pour un polypeptide ALS I comprenant, à une position correspondant à la position 182 de SEQ ID NO : 2, à la place de l'acide aminé proline codé naturellement, l'acide aminé sérine et comprenant, à une position correspondant à la position 559 de SEQ ID NO : 2, à la place de l'acide aminé tryptophane codé naturellement, l'acide aminé leucine, et un gène ALS III codant pour un polypeptide ALS III comprenant, à une position correspondant à la position 556 de SEQ ID NO : 4, à la place de l'acide aminé tryptophane codé naturellement, l'acide aminé leucine ;
b. *Brassica napus* comprenant un gène ALS I codant pour un polypeptide ALS I comprenant, à une position correspondant à la position 559 de SEQ ID NO : 2, à la place de l'acide aminé tryptophane codé naturellement, l'acide aminé leucine, et un gène ALS III codant pour un polypeptide ALS III comprenant, à une position correspondant à la position 179 de SEQ ID NO : 4, à la place de l'acide aminé proline codé naturellement, l'acide aminé sérine et comprenant, à une position correspondant à la position 556 de SEQ ID NO : 4, à la place de l'acide aminé tryptophane codé naturellement, l'acide aminé leucine ;
c. *Brassica napus* comprenant un gène ALS I codant pour un polypeptide ALS I comprenant, à une position correspondant à la position 182 de SEQ ID NO : 2, à la place de l'acide aminé proline codé naturellement, l'acide aminé sérine et comprenant, à une position correspondant à la position 559 de SEQ ID NO : 2, à la place de l'acide aminé tryptophane codé naturellement, l'acide aminé leucine, et un gène ALS III codant pour un polypeptide ALS III comprenant, à une position correspondant à la position 179 de SEQ ID NO : 4, à la place de l'acide aminé proline codé naturellement, l'acide aminé sérine et comprenant, à une position correspondant à la position 556 de SEQ ID NO : 4, à la place de l'acide aminé tryptophane codé naturellement, l'acide aminé leucine ;
d. *Brassica juncea* comprenant un gène ALS-A codant pour un polypeptide ALS-A comprenant, à une position correspondant à la position 179 de SEQ ID NO : 6, à la place de l'acide aminé proline codé naturellement, l'acide aminé sérine et comprenant, à une position correspondant à la position 556 de SEQ ID NO : 6, à la place de l'acide aminé tryptophane codé naturellement, l'acide aminé leucine, et un gène ALS-B codant pour un polypeptide ALS-B comprenant, à une position correspondant à la position 559 de SEQ ID NO : 8, à la place de l'acide aminé tryptophane codé naturellement, l'acide aminé leucine ;
e. *Brassica juncea* comprenant un gène ALS-A codant pour un polypeptide ALS-A comprenant, à une position correspondant à la position 556 de SEQ ID NO : 6, à la place de l'acide aminé tryptophane codé naturellement, l'acide aminé leucine, et un gène ALS-B codant pour un polypeptide ALS-B comprenant, à une position correspondant à la position 182 de SEQ ID NO : 8, à la place de l'acide aminé proline codé naturellement, l'acide aminé sérine et comprenant, à une position correspondant à la position 559 de SEQ ID NO : 8, à la place de l'acide aminé tryptophane codé naturellement, l'acide aminé leucine ; et
f. *Brassica juncea* comprenant un gène ALS-A codant pour un polypeptide ALS-A comprenant, à une position correspondant à la position 179 de SEQ ID NO : 6, à la place de l'acide aminé proline codé naturellement, l'acide aminé sérine et comprenant, à une position correspondant à la position 556 de SEQ ID NO : 6, à la place de l'acide aminé tryptophane codé naturellement, l'acide aminé leucine, et un gène ALS-B codant pour un polypeptide ALS-B comprenant, à une position correspondant à la position 182 de SEQ ID NO : 8, à la place de l'acide aminé proline codé naturellement, l'acide aminé sérine et comprenant, à une position correspondant à la position 559 de SEQ ID NO : 8, à la place de l'acide aminé tryptophane codé naturellement, l'acide aminé leucine.

4. Plante *Brassica* ou parties de celle-ci selon la revendication 3, qui sont choisies dans le groupe constitué de :
a. *B. napus* comprenant un gène ALS I codant pour un polypeptide ALS I qui est au moins 90 % identique à SEQ ID NO : 2 dont la proline à la position 182 est substituée par une sérine, et dont le tryptophane à la position 559 est substitué par une leucine, et un gène ALS III codant pour un polypeptide ALS III qui est au moins 90 % identique à SEQ ID NO : 4 dont le tryptophane à la position 556 est substitué par une leucine ;
b. *B. napus* comprenant un gène ALS I codant pour un polypeptide ALS I qui est au moins 90 % identique à SEQ ID NO : 2 dont le tryptophane à la position 559 est substitué par une leucine, et un gène ALS III codant pour un polypeptide ALS III qui est au moins 90 % identique à SEQ ID NO : 4 dont la proline à la position 179 est substituée par une sérine et dont le tryptophane à la position 556 est substitué par une leucine ;
c. *B. napus* comprenant un gène ALS I codant pour un polypeptide ALS I qui est au moins 90 % identique à SEQ ID NO : 2 dont la proline à la position 182 est substituée par une sérine, et dont le tryptophane à la position 559 est substitué par une leucine, et un gène ALS III codant pour un polypeptide ALS III qui est au moins 90 % identique à SEQ ID NO : 4 dont la proline à la position 179 est substituée par une sérine et dont le tryptophane à la position 556 est substitué par une leucine ;
d. *B. juncea* comprenant un gène ALS-A codant pour un polypeptide ALS-A qui est au moins 90 % identique à SEQ ID NO : 6 dont la proline à la position 179 est substituée par une sérine et dont le tryptophane à la position 556 est substitué par une leucine, et un gène ALS-B codant pour un polypeptide ALS-B qui est au moins 90 % identique à SEQ ID NO : 8 dont le tryptophane à la position 559 est substitué par une leucine ;
e. *B. juncea* comprenant un gène ALS-A codant pour un polypeptide ALS-A qui est au moins 90 % identique à SEQ ID NO : 6 dont le tryptophane à la position 556 est substitué par une leucine, et un gène ALS-B codant pour un polypeptide ALS-B qui est au moins 90 % identique à SEQ ID NO : 8 dont la proline à la position 182 est substituée par une sérine, et dont le tryptophane à la position 559 est substitué par une leucine ; et
f. *B. juncea* comprenant un gène ALS-A codant pour un polypeptide ALS-A qui est au moins 90 % identique à SEQ ID NO : 6 dont la proline à la position 179 est substituée par une sérine et dont le tryptophane à la position 556 est substitué par une leucine, et un gène ALS-B codant pour un polypeptide ALS-B qui est au moins 90 % identique à SEQ ID NO : 8 dont la proline à la position 182 est substituée par une sérine, et dont le tryptophane à la position 559 est substitué par une leucine.

5. Plante *Brassica* ou parties de celle-ci selon la revendication 4, qui sont *B. napus,* qui sont choisies dans le groupe constitué de :
a. une plante de *B. napus* ou des parties de celle-ci dans lesquelles ledit gène ALS I comprend la séquence nucléotidique correspondant à SEQ ID NO : 1 dont le C à la position 544 est substitué par T et dont le G à la position 1676 est substitué par T, et ledit gène ALS III comprend la séquence nucléotidique correspondant à SEQ ID NO : 3 dont le G à la position 1667 est substitué par T, et qui peuvent être obtenues à partir de semences déposées au NCIMB sous les numéros d'accession NCIMB 42145, NCIMB 42235 et/ou NCIMB 42260 ;
b. une plante de *B. napus* ou des parties de celle-ci dans lesquelles ledit gène ALS I comprend la séquence nucléotidique correspondant à SEQ ID NO : 1 dont le G à la position 1676 est substitué par T, et ledit gène ALS III comprend la séquence nucléotidique correspondant à SEQ ID NO : 3 dont le C à la position 535 est substitué par T et dont le G à la position 1667 est substitué par T, et qui peuvent être obtenues à partir de semences déposées au NCIMB sous les numéros d'accession NCIMB 42145, NCIMB 42235, NCIMB 42153 et/ou NCIMB 42259 ; et
c. une plante de *B. napus* ou des parties de celle-ci dans lesquelles ledit gène ALS I comprend la séquence nucléotidique correspondant à SEQ ID NO : 1 dont le C à la position 544 est substitué par T et dont le G à la position 1676 est substitué par T, et ledit gène ALS III comprend la séquence nucléotidique correspondant à SEQ ID NO : 3 dont le C à la position 535 est substitué par T et dont le G à la position 1667 est substitué par T, et qui peuvent être obtenues à partir de semences déposées au NCIMB sous les numéros d'accession NCIMB 42153, NCIMB 42259 et/ou NCIMB 42260.

6. Plante *Brassica* ou parties de celle-ci selon la revendication 4, qui sont *B. napus,* qui sont choisies dans le groupe constitué de :
a. une plante de *B. napus* ou des parties de celle-ci dans lesquelles ledit gène ALS I comprend la séquence nucléotidique correspondant à SEQ ID NO : 1 dont le C à la position 544 est substitué par T et dont le G à la position 1676 est substitué par T, et ledit gène ALS III comprend la séquence nucléotidique correspondant à SEQ ID NO : 3 dont le G à la position 1667 est substitué par T, une semence de référence de ladite plante ayant été déposée au NCIMB sous les numéros d'accession NCIMB 42145, 42235 et NCIMB 42260,
b. une plante de *B. napus* ou des parties de celle-ci dans lesquelles ledit gène ALS I comprend la séquence nucléotidique correspondant à SEQ ID NO : 1 dont le G à la position 1676 est substitué par T, et ledit gène ALS III comprend la séquence nucléotidique correspondant à SEQ ID NO : 3 dont le C à la position 535 est substitué par T et dont le G à la position 1667 est substitué par T, une semence de référence de ladite plante ayant été déposée au NCIMB sous les numéros d'accession NCIMB 42145, NCIMB 42235, NCIMB 42153 et NCIMB 42259, et
c. une plante de *B. napus* ou des parties de celle-ci dans lesquelles ledit gène ALS I comprend la séquence nucléotidique correspondant à SEQ ID NO : 1 dont le C à la position 544 est substitué par T et dont le G à la position 1676 est substitué par T, et ledit gène ALS III comprend la séquence nucléotidique correspondant à SEQ ID NO : 3 dont le C à la position 535 est substitué par T et dont le G à la position 1667 est substitué par T, une semence de référence de ladite plante ayant été déposée au NCIMB sous les numéros d'accession NCIMB 42153, NCIMB 42259, et NCIMB 42260.

7. Plante ou parties de celle-ci selon l'une quelconque des revendications 1 à 6 qui sont tolérantes à un ou plusieurs herbicides inhibiteurs d'ALS appartenant au groupe constitué des herbicides sulfonylurées, herbicides sulfonylaminocarbonyltriazolinones, herbicides imidazolinones, herbicides triazolopyrimidines et herbicides pyrimidinyl(thio)benzoates.

8. Descendance d'une plante selon l'une quelconque des revendications 1 à 7 obtenue par reproduction consécutive avec ladite plante, dans laquelle tous les gènes ALS de ladite descendance codent pour un polypeptide ALS comprenant, à une position correspondant à la position 574 de SEQ ID NO : 10, à la place de l'acide aminé tryptophane codé naturellement, l'acide aminé leucine et dans laquelle au moins un des gènes ALS code pour un polypeptide ALS qui comprend en outre, à une position correspondant à la position 197 de SEQ ID NO : 10, à la place de l'acide aminé proline codé naturellement, l'acide aminé sérine.

9. Plante hybride produite par croisement d'une plante parente selon l'une quelconque des revendications 1 à 7 avec une deuxième plante parente et récolte d'une semence hybride résultante et culture de ladite semence, dans laquelle tous les gènes ALS de ladite plante hybride codent pour un polypeptide ALS comprenant, à une position correspondant à la position 574 de SEQ ID NO : 10, à la place de l'acide aminé tryptophane codé naturellement, l'acide aminé leucine, et dans laquelle au moins un des gènes ALS code pour un polypeptide ALS qui comprend en outre, à une position correspondant à la position 197 de SEQ ID NO : 10, à la place de l'acide aminé proline codé naturellement, l'acide aminé sérine.
